# EUROPEAN PATENT APPLICATION

(11) **EP 3 778 644 A2**
(43) Date of publication of application: **17.02.2021**
(21) Application number: 20197862.4
(22) Date of filing: 23.12.2015
(51) Int. Cl.: C07K 16/28, G01N 33/574, C12Q 1/6886, C07K 14/71

(54) **FGFR-TACC FUSION PROTEINS AND METHODS THEREOF**

(30) Priority: 23.12.2014 US 201462096311 P
(62) Divisional of application: 15873797.3
(71) Applicant: The Trustees of Columbia University in the City of New York, New York, NY 10027 (US)
(72) Inventor: LASORELLA, Anna, New York, 10021 (US); IAVARONE, Antonio, New York, 10021 (US)
(74) Representative: Bassil, Nicholas Charles

(57) **Abstract**

The invention discloses FGFR-TACC oncogenic fusion proteins. The invention provides methods for treating and diagnosing gene-fusion based cancers.

SEQUENCE LISTING

<110> THE TRUSTEES OF COLUMBIA UNIVERSITY IN THE CITY OF NEW YORK

<120> FUSION PROTEINS AND METHODS THEREOF

<130> B051-0219EP3

<140>
<141>

<150>
<151> 2014-12-23

<160> 560

<170> PatentIn version 3.5

<210> 1
<211> 178
<212> DNA
<213> Artificial Sequence

<220>
<223> Description of Artificial Sequence: Synthetic
      polynucleotide

<220>
<221> CDS
<222> (2)..(178)

<400> 1
g tgc tgg cat gcc gcg ccc tcc cag agg ccc acc ttc aag cag ctg gtg      49
  Cys Trp His Ala Ala Pro Ser Gln Arg Pro Thr Phe Lys Gln Leu Val
  1               5                   10                  15

gag gac ctg gac cgt gtc ctt acc gtg acg tcc acc gac ttt aag gag      97
Glu Asp Leu Asp Arg Val Leu Thr Val Thr Ser Thr Asp Phe Lys Glu
            20                  25                  30

tcg gcc ttg agg aag cag tcc tta tac ctc aag ttc gac ccc ctc ctg      145
Ser Ala Leu Arg Lys Gln Ser Leu Tyr Leu Lys Phe Asp Pro Leu Leu
        35                  40                  45

agg gac agt cct ggt aga cca gtg ccc gtg gcc                          178
Arg Asp Ser Pro Gly Arg Pro Val Pro Val Ala
    50                  55

<210> 2
<211> 91
<212> DNA
<213> Artificial Sequence

<220>
<223> Description of Artificial Sequence: Synthetic
      oligonucleotide

<400> 2
ctttaaggag tcggccttga ggaagcagtc cttatacctc aagttcgacc ccctcctgag      60

ggacagtcct ggtagaccag tgcccgtggc c                                     91

<210> 3
<211> 91
<212> DNA
<213> Artificial Sequence

<220>
<223> Description of Artificial Sequence: Synthetic
      oligonucleotide

<400> 3
actttaagga gtcggccttg aggaagcagt ccttatacct caagttcgac cccctcctga      60

gggacagtcc tggtagacca gtgcccgtgg g                                     91


<210> 4
<211> 91
<212> DNA
<213> Artificial Sequence

<220>
<223> Description of Artificial Sequence: Synthetic
      oligonucleotide

<400> 4
gactttaagg agtcggcctt gaggaagcag tccttatacc tcaagttcga ccccctcctg      60

agggacagtc ctggtagacc agtgcccgtg g                                     91


<210> 5
<211> 91
<212> DNA
<213> Artificial Sequence

<220>
<223> Description of Artificial Sequence: Synthetic
      oligonucleotide

<400> 5
cgactttaag gagtcggcct tgaggaagca gtccttatac ctcaagttcg accccctcct      60

gagggacagt cctggtagac cagtgcccgt t                                     91


<210> 6
<211> 91
<212> DNA
<213> Artificial Sequence

<220>
<223> Description of Artificial Sequence: Synthetic
      oligonucleotide

<400> 6
ccgactttaa ggagtcggcc ttgaggaagc agtccttata cctcaagttc gaccccctcc      60

tgagggacag tcctggtaga ccagtgcccg g                                     91


<210> 7

<211> 91
<212> DNA
<213> Artificial Sequence

<220>
<223> Description of Artificial Sequence: Synthetic
      oligonucleotide

<400> 7
accgacttta aggagtcggc cttgaggaag cagtccttat acctcaagtt cgaccccctc        60

ctgagggaca gtcctggtag accagtgccc c                                      91


<210> 8
<211> 91
<212> DNA
<213> Artificial Sequence

<220>
<223> Description of Artificial Sequence: Synthetic
      oligonucleotide

<400> 8
   caccgacttt aaggagtcgg ccttgaggaa gcagtcctta tacctcaagt tcgaccccct        60
   cctgagggac agtcctggta gaccagtgcc c                                      91


<210> 9
<211> 91
<212> DNA
<213> Artificial Sequence

<220>
<223> Description of Artificial Sequence: Synthetic
      oligonucleotide

<400> 9
ccaccgactt taaggagtcg gccttgagga agcagtcctt atacctcaag ttcgaccccc        60

tcctgaggga cagtcctggt agaccagtgc c                                      91


<210> 10
<211> 91
<212> DNA
<213> Artificial Sequence

<220>
<223> Description of Artificial Sequence: Synthetic
      oligonucleotide

<400> 10
tccaccgact ttaaggagtc ggccttgagg aagcagtcct tatacctcaa gttcgacccc        60

ctcctgaggg acagtcctgg tagaccagtg g                                      91


<210> 11
<211> 91
<212> DNA
<213> Artificial Sequence

<220>
<223> Description of Artificial Sequence: Synthetic
      oligonucleotide

<400> 11
gtccaccgac tttaaggagt cggccttgag gaagcagtcc ttatacctca agttcgaccc      60

cctcctgagg gacagtcctg gtagaccagt t                                     91


<210> 12
<211> 91
<212> DNA
<213> Artificial Sequence

<220>
<223> Description of Artificial Sequence: Synthetic
      oligonucleotide

<400> 12
cgtccaccga ctttaaggag tcggccttga ggaagcagtc cttatgcctc aagttcggcc      60

ccctcctgag ggacagtcct ggtagaccag g                                     91


<210> 13
<211> 90
<212> DNA
<213> Artificial Sequence

<220>
<223> Description of Artificial Sequence: Synthetic
      oligonucleotide

<400> 13
gacgtccacc gactttaagg agtcggcctt gaggaagcag tccttatacc tcaagttcga      60

ccccctcctg agggacagtc ctggtagacg                                      90


<210> 14
<211> 91
<212> DNA
<213> Artificial Sequence

<220>
<223> Description of Artificial Sequence: Synthetic
      oligonucleotide

<400> 14
tgacgtccac cgactttaag gagtcggcct tgaggaagca gtccttatac ctcaagttcg      60

accccctcct gagggacagt cctggtagac c                                     91


<210> 15
<211> 91
<212> DNA
<213> Artificial Sequence

<220>
<223> Description of Artificial Sequence: Synthetic

oligonucleotide

<400> 15
gtgacgtcca ccgactttaa ggagtcggcc ttgaggaagc agtccttata cctcaagttc 60

gaccccctcc tgagggacag tcctggtaga a 91


<210> 16
<211> 91
<212> DNA
<213> Artificial Sequence

<220>
<223> Description of Artificial Sequence: Synthetic
      oligonucleotide

<400> 16
cgtgacgtcc accgacttta aggagtcggc cttgaggaag cagtccttat acctcaagtt 60

cgaccccctc ctgagggaca gtcctggtag g 91


<210> 17
<211> 91
<212> DNA
<213> Artificial Sequence

<220>
<223> Description of Artificial Sequence: Synthetic
      oligonucleotide

<400> 17
ccgtgacgtc caccgacttt aaggagtcgg ccttgaggaa gcagtcctta tacctcaagt 60

tcgaccccct cctgagggac agtcctggta a 91


<210> 18
<211> 91
<212> DNA
<213> Artificial Sequence

<220>
<223> Description of Artificial Sequence: Synthetic
      oligonucleotide

<400> 18
cttaccgtga cgtccaccga ctttaaggag tcggccttga ggaagcagtc cttatacctc 60

aagttcgacc ccctcctgag ggacagtcct t 91


<210> 19
<211> 91
<212> DNA
<213> Artificial Sequence

<220>
<223> Description of Artificial Sequence: Synthetic
      oligonucleotide

<400> 19

gtccttaccg tgacgtccac cgactttaag gagtcggcct tgaggaagca gtccttatac 60

ctcaagttcg accccctcct gagggacagt t 91

<210> 20
<211> 91
<212> DNA
<213> Artificial Sequence

<220>
<223> Description of Artificial Sequence: Synthetic
      oligonucleotide

<400> 20
tgtccttacc gtgacgtcca ccgactttaa ggagtcggcc ttgaggaagc agtccttata 60

cctcaagttc gaccccctcc tgagggacag g 91

<210> 21
<211> 91
<212> DNA
<213> Artificial Sequence

<220>
<223> Description of Artificial Sequence: Synthetic
      oligonucleotide

<400> 21
gtgtccttac cgtgacgtcc accgacttta aggagtcggc cttgaggaag cagtccttat 60

acctcaagtt cgaccccctc ctgagggaca a 91

<210> 22
<211> 91
<212> DNA
<213> Artificial Sequence

<220>
<223> Description of Artificial Sequence: Synthetic
      oligonucleotide

<400> 22
cgtgtcctta ccgtgacgtc caccgacttt aaggagtcgg ccttgaggaa gcagtcctta 60

tacctcaagt tcgaccccct cctgagggac c 91

<210> 23
<211> 91
<212> DNA
<213> Artificial Sequence

<220>
<223> Description of Artificial Sequence: Synthetic
      oligonucleotide

<400> 23
accgtgtcct taccgtgacg tccaccgact ttaaggagtc ggccttgagg aagcagtcct 60

tatacctcaa gttcgacccc ctcctgaggg g 91

<210> 24
<211> 91
<212> DNA
<213> Artificial Sequence

<220>
<223> Description of Artificial Sequence: Synthetic
      oligonucleotide

<400> 24
gaccgtgtcc ttaccgtgac gtccaccgac tttaaggagt cggccttgag gaagcagtcc      60

ttatacctca agttcgaccc cctcctgagg g                                     91


<210> 25
<211> 91
<212> DNA
<213> Artificial Sequence

<220>
<223> Description of Artificial Sequence: Synthetic
      oligonucleotide

<400> 25
ggaccgtgtc cttaccgtga cgtccaccga ctttaaggag tcggccttga ggaagcagtc      60

cttatacctc aagttcgacc ccctcctgag g                                     91


<210> 26
<211> 91
<212> DNA
<213> Artificial Sequence

<220>
<223> Description of Artificial Sequence: Synthetic
      oligonucleotide

<400> 26
ctggaccgtg tccttaccgt gacgtccacc gactttaagg agtcggcctt gaggaagcag      60

tccttatacc tcaagttcga ccccctcctg g                                     91


<210> 27
<211> 91
<212> DNA
<213> Artificial Sequence

<220>
<223> Description of Artificial Sequence: Synthetic
      oligonucleotide

<400> 27
cctggaccgt gtccttaccg tgacgtccac cgactttaag gagtcggcct tgaggaagca      60

gtccttatac ctcaagttcg accccctcct t                                     91


<210> 28

<211> 91
<212> DNA
<213> Artificial Sequence

<220>
<223> Description of Artificial Sequence: Synthetic
      oligonucleotide

<400> 28
gacctggacc gtgtccttac cgtgacgtcc accgacttta aggagtcggc cttgaggaag      60

cagtccttat acctcaagtt cgaccccctc c                                      91


<210> 29
<211> 91
<212> DNA
<213> Artificial Sequence

<220>
<223> Description of Artificial Sequence: Synthetic
      oligonucleotide

<400> 29
ggacctggac cgtgtcctta ccgtgacgtc caccgacttt aaggagtcgg ccttgaggaa      60

gcagtcctta tacctcaagt tcgaccccct t                                      91


<210> 30
<211> 91
<212> DNA
<213> Artificial Sequence

<220>
<223> Description of Artificial Sequence: Synthetic
      oligonucleotide

<400> 30
aggacctgga ccgtgtcctt accgtgacgt ccaccgactt taaggagtcg gccttgagga      60

agcagtcctt atacctcaag ttcgaccccc c                                     91


<210> 31
<211> 90
<212> DNA
<213> Artificial Sequence

<220>
<223> Description of Artificial Sequence: Synthetic
      oligonucleotide

<400> 31
gaggacctgg accgtgtcct taccgtgacg tccaccgact ttaaggagtc ggccttgagg      60

aagcagtcct tatacctcaa gttcgacccc                                       90


<210> 32
<211> 90
<212> DNA
<213> Artificial Sequence

<220>
<223> Description of Artificial Sequence: Synthetic
      oligonucleotide

<400> 32
ggaggacctg gaccgtgtcc ttaccgtgac gtccaccgac tttaaggagt cggccttgag      60

gaagcagtcc ttatacctca agttcgaccc                                        90


<210> 33
<211> 90
<212> DNA
<213> Artificial Sequence

<220>
<223> Description of Artificial Sequence: Synthetic
      oligonucleotide

<400> 33
tggaggacct ggaccgtgtc cttaccgtga cgtccaccga ctttaaggag tcggccttga      60

ggaagcagtc cttatacctc aagttcgacc                                        90


<210> 34
<211> 90
<212> DNA
<213> Artificial Sequence

<220>
<223> Description of Artificial Sequence: Synthetic
      oligonucleotide

<400> 34
gtggaggacc tggaccgtgt ccttaccgtg acgtccaccg actttaagga gtcggccttg      60

aggaagcagt ccttatacct caagttcgac                                        90


<210> 35
<211> 90
<212> DNA
<213> Artificial Sequence

<220>
<223> Description of Artificial Sequence: Synthetic
      oligonucleotide

<400> 35
ggtggaggac ctggaccgtg tccttaccgt gacgtccacc gactttaagg agtcggcctt      60

gaggaagcag tccttatacc tcaagttcga                                        90


<210> 36
<211> 90
<212> DNA
<213> Artificial Sequence

<220>
<223> Description of Artificial Sequence: Synthetic

oligonucleotide

<400> 36
tggtggagga cctggaccgt gtccttaccg tgacgtccac cgactttaag gagtcggcct 60

tgaggaagca gtccttatac ctcaagttcg 90


<210> 37
<211> 90
<212> DNA
<213> Artificial Sequence

<220>
<223> Description of Artificial Sequence: Synthetic
      oligonucleotide

<400> 37
ctggtggagg acctggaccg tgtccttacc gtgacgtcca ccgactttaa ggagtcggcc 60

ttgaggaagc agtccttata cctctaatca 90


<210> 38
<211> 90
<212> DNA
<213> Artificial Sequence

<220>
<223> Description of Artificial Sequence: Synthetic
      oligonucleotide

<400> 38
gctggtggag gacctggacc gtgtccttac cgtgacgtcc accggcttta aggagtcggc 60

ctcgaggaag cagccctttt acctcaagtt 90


<210> 39
<211> 90
<212> DNA
<213> Artificial Sequence

<220>
<223> Description of Artificial Sequence: Synthetic
      oligonucleotide

<400> 39
agctggtgga ggacctggac cgtgtcctta ccgtgacgtc caccgacttt aaggagtcgg 60

ccttgaggaa gcagtcctta tacctcaagt 90


<210> 40
<211> 90
<212> DNA
<213> Artificial Sequence

<220>
<223> Description of Artificial Sequence: Synthetic
      oligonucleotide

<400> 40

cagctggtgg aggacctgga ccgtgtcctt accgtgacgt ccaccgactt taaggagtcg 60

gccttgagga agcagtcctt atacctcaag 90

<210> 41
<211> 90
<212> DNA
<213> Artificial Sequence

<220>
<223> Description of Artificial Sequence: Synthetic
      oligonucleotide

<400> 41
gcagctggtg gaggacctgg accgtgtcct taccgtgacg tccaccgact ttaaggagtc 60

ggccttgagg aagcagtcct tatacctcaa 90

<210> 42
<211> 90
<212> DNA
<213> Artificial Sequence

<220>
<223> Description of Artificial Sequence: Synthetic
      oligonucleotide

<400> 42
agcagctggt ggaggacctg gaccgtgtcc ttaccgtgac gtccaccgac tttaaggagt 60

cggccttgag gaagcagtcc ttatacctca 90

<210> 43
<211> 90
<212> DNA
<213> Artificial Sequence

<220>
<223> Description of Artificial Sequence: Synthetic
      oligonucleotide

<400> 43
aagcagctgg tggaggacct ggaccgtgtc cttaccgtga cgtccaccga ctttaaggag 60

tcggccttga ggaagcagtc cctatacccc 90

<210> 44
<211> 90
<212> DNA
<213> Artificial Sequence

<220>
<223> Description of Artificial Sequence: Synthetic
      oligonucleotide

<400> 44
caagcagctg gtggaggacc tggaccgtgt ccttaccgtg acgtccaccg actttaagga 60

gtcggccttg aggaagcagt ccttatacct 90

<210> 45
<211> 90
<212> DNA
<213> Artificial Sequence

<220>
<223> Description of Artificial Sequence: Synthetic
      oligonucleotide

<400> 45
tcaagcagct ggtggaggac ctggaccgtg tccttaccgt gacgtccacc gactttaagg      60

agtcggcctt gaggaagcag tccttatacc                                      90


<210> 46
<211> 90
<212> DNA
<213> Artificial Sequence

<220>
<223> Description of Artificial Sequence: Synthetic
      oligonucleotide

<400> 46
ttcaagcagc tggtggagga cctggaccgt gtccttaccg tgacgtccac cgactttaag      60

gagtcggcct tgaggaagca gtccttatac                                      90


<210> 47
<211> 90
<212> DNA
<213> Artificial Sequence

<220>
<223> Description of Artificial Sequence: Synthetic
      oligonucleotide

<400> 47
cttcaagcag ctggtggagg acctggaccg tgtccttacc gtgacgtcca ccgactttaa      60

ggagtcggcc ttgaggaagc agtccttata                                      90


<210> 48
<211> 90
<212> DNA
<213> Artificial Sequence

<220>
<223> Description of Artificial Sequence: Synthetic
      oligonucleotide

<400> 48
ccttcaagca gctggtggag gacctggacc gtgtccttac cgtgacgtcc accgacttta      60

aggagtcggc cttgaggaag cagtccttat                                      90


<210> 49

<211> 90
<212> DNA
<213> Artificial Sequence

<220>
<223> Description of Artificial Sequence: Synthetic
      oligonucleotide

<400> 49
accttcaagc agctggtgga ggacctggac cgtgtcctta ccgtgacgtc caccgacttt      60

aaggagtcgg ccttgaggaa gcagtcctta                                        90


<210> 50
<211> 90
<212> DNA
<213> Artificial Sequence

<220>
<223> Description of Artificial Sequence: Synthetic
      oligonucleotide

<400> 50
caccttcaag cagctggtgg aggacctgga ccgtgtcctt accgtgacgt ccaccgactt      60

taaggagtcg gccttgagga agcagtcctt                                        90


<210> 51
<211> 90
<212> DNA
<213> Artificial Sequence

<220>
<223> Description of Artificial Sequence: Synthetic
      oligonucleotide

<400> 51
ccaccttcaa gcagctggtg gaggacctgg accgtgtcct taccgtgacg tccaccgact      60

ttaaggagtc ggccttgagg aagcagtcct                                        90


<210> 52
<211> 90
<212> DNA
<213> Artificial Sequence

<220>
<223> Description of Artificial Sequence: Synthetic
      oligonucleotide

<400> 52
cccaccttca agcagctggt ggaggacctg gaccgtgtcc ttaccgtgac gtccaccgac      60

tttaaggagt cggccttgag gaagcagtcc                                        90


<210> 53
<211> 90
<212> DNA
<213> Artificial Sequence

<220>
<223> Description of Artificial Sequence: Synthetic
      oligonucleotide

<400> 53
ggcccacctt caagcagctg gtggaggacc tggaccgtgt ccttaccgtg acgtccaccg      60

actttaagga gtcggccttg aggaagcagt                                        90


<210> 54
<211> 90
<212> DNA
<213> Artificial Sequence

<220>
<223> Description of Artificial Sequence: Synthetic
      oligonucleotide

<400> 54
aggcccacct tcaagcagct ggtggaggac ctggaccgtg tccttaccgt gacgtccacc      60

gactttaagg agtcggcctt gaggaagcag                                        90


<210> 55
<211> 90
<212> DNA
<213> Artificial Sequence

<220>
<223> Description of Artificial Sequence: Synthetic
      oligonucleotide

<400> 55
gaggcccacc ttcaagcagc tggtggagga cctggaccgt gtccttaccg tgacgtccac      60

cgactttaag gagtcggcct tgaggaagca                                        90


<210> 56
<211> 90
<212> DNA
<213> Artificial Sequence

<220>
<223> Description of Artificial Sequence: Synthetic
      oligonucleotide

<400> 56
agaggcccac cttcaagcag ctggtggagg acctggaccg tgtccttacc gtgacgtcca      60

ccgactttaa ggagtcggcc ttgaggaagc                                        90


<210> 57
<211> 90
<212> DNA
<213> Artificial Sequence

<220>
<223> Description of Artificial Sequence: Synthetic

oligonucleotide

<400> 57
cagaggccca ccttcaagca gctggtggag gacctggacc gtgtccttac cgtgacgtcc 60

accgacttta aggagtcggc cttgaggaag 90


<210> 58
<211> 90
<212> DNA
<213> Artificial Sequence

<220>
<223> Description of Artificial Sequence: Synthetic
      oligonucleotide

<400> 58
ccagaggccc accttcaagc agctggtgga ggacctggac cgtgtcctta ccgtgacgtc 60

caccgacttt aaggagtcgg ccttgaggaa 90


<210> 59
<211> 90
<212> DNA
<213> Artificial Sequence

<220>
<223> Description of Artificial Sequence: Synthetic
      oligonucleotide

<400> 59
cccagaggcc caccttcaag cagctggtgg aggacctgga ccgtgtcctt accgtgacgt 60

ccaccgactt taaggagtcg gccttgagga 90


<210> 60
<211> 90
<212> DNA
<213> Artificial Sequence

<220>
<223> Description of Artificial Sequence: Synthetic
      oligonucleotide

<400> 60
tcccagaggc ccaccttcaa gcagctggtg gaggacctgg accgtgtcct taccgtgacg 60

tccaccgact ttaaggagtc ggccttgagg 90


<210> 61
<211> 90
<212> DNA
<213> Artificial Sequence

<220>
<223> Description of Artificial Sequence: Synthetic
      oligonucleotide

<400> 61

ctcccagagg cccaccttca agcagctggt ggaggacctg gaccgtgtcc ttaccgtgac 60

gtccaccgac tttaaggagt cggccttgag 90

<210> 62
<211> 90
<212> DNA
<213> Artificial Sequence

<220>
<223> Description of Artificial Sequence: Synthetic
      oligonucleotide

<400> 62
cctcccagag gcccaccttc aagcagctgg tggaggacct ggaccgtgtc cttaccgtga 60

cgtccaccga ctttaaggag tcggccttga 90

<210> 63
<211> 90
<212> DNA
<213> Artificial Sequence

<220>
<223> Description of Artificial Sequence: Synthetic
      oligonucleotide

<400> 63
ccctcccaga ggcccacctt caagcagctg gtggaggacc tggaccgtgt ccttaccgtg 60

acgtccaccg actttaagga gtcggccttg 90

<210> 64
<211> 90
<212> DNA
<213> Artificial Sequence

<220>
<223> Description of Artificial Sequence: Synthetic
      oligonucleotide

<400> 64
gcgccctccc agaggcccac cttcaagcag ctggtggagg acctggaccg tgtccttacc 60

gtgacgtcca ccgactttaa ggagtcggcc 90

<210> 65
<211> 90
<212> DNA
<213> Artificial Sequence

<220>
<223> Description of Artificial Sequence: Synthetic
      oligonucleotide

<400> 65
cgcgccctcc cagaggccca ccttcaagca gctggtggag gacctggacc gtgtccttac 60

cgtgacgtcc accgacttta aggagtcggc 90

<210> 66
<211> 90
<212> DNA
<213> Artificial Sequence

<220>
<223> Description of Artificial Sequence: Synthetic
      oligonucleotide

<400> 66
ccgcgccctc ccagaggccc accttcaagc agctggtgga ggacctggac cgtgtcctta      60

ccgtgacgtc caccgacttt aaggagtcgg                                        90


<210> 67
<211> 90
<212> DNA
<213> Artificial Sequence

<220>
<223> Description of Artificial Sequence: Synthetic
      oligonucleotide

<400> 67
gccgcgccct cccagaggcc caccttcaag cagctggtgg aggacctgga ccgtgtcctt      60

accgtgacgt ccaccgactt taaggagtcg                                        90


<210> 68
<211> 90
<212> DNA
<213> Artificial Sequence

<220>
<223> Description of Artificial Sequence: Synthetic
      oligonucleotide

<400> 68
tgccgcgccc tcccagaggc ccaccttcaa gcagctggtg gaggacctgg accgtgtcct      60

taccgtgacg tccaccgact ttaaggagtc                                        90


<210> 69
<211> 90
<212> DNA
<213> Artificial Sequence

<220>
<223> Description of Artificial Sequence: Synthetic
      oligonucleotide

<400> 69
atgccgcgcc ctcccagagg cccaccttca agcagctggt ggaggacctg gaccgtgtcc      60

ttaccgtgac gtccaccgac tttaaggagt                                        90


<210> 70

<211> 90
<212> DNA
<213> Artificial Sequence

<220>
<223> Description of Artificial Sequence: Synthetic
      oligonucleotide

<400> 70
catgccgcgc cctcccagag gcccaccttc aagcagctgg tggaggacct ggaccgtgtc      60

cttaccgtga cgtccaccga ctttaaggag                                        90


<210> 71
<211> 90
<212> DNA
<213> Artificial Sequence

<220>
<223> Description of Artificial Sequence: Synthetic
      oligonucleotide

<400> 71
gcatgccgcg ccctcccaga ggcccacctt caagcagctg gtggaggacc tggaccgtgt      60

ccttaccgtg acgtccaccg actttaagga                                        90


<210> 72
<211> 90
<212> DNA
<213> Artificial Sequence

<220>
<223> Description of Artificial Sequence: Synthetic
      oligonucleotide

<400> 72
ggcatgccgc gccctcccag aggcccacct tcaagcagct ggtggaggac ctggaccgtg      60

tccttaccgt gacgtccacc gactttaagg                                        90


<210> 73
<211> 90
<212> DNA
<213> Artificial Sequence

<220>
<223> Description of Artificial Sequence: Synthetic
      oligonucleotide

<400> 73
tggcatgccg cgccctccca gaggcccacc ttcaagcagc tggtggagga cctggaccgt      60

gtccttaccg tgacgtccac cgactttaag                                        90


<210> 74
<211> 90
<212> DNA
<213> Artificial Sequence

<220>
<223> Description of Artificial Sequence: Synthetic
      oligonucleotide

<400> 74
ctggcatgcc gcgccctccc agaggcccac cttcaagcag ctggtggagg acctggaccg      60

tgtccttacc gtgacgtcca ccgactttaa                                      90


<210> 75
<211> 90
<212> DNA
<213> Artificial Sequence

<220>
<223> Description of Artificial Sequence: Synthetic
      oligonucleotide

<400> 75
gctggcatgc cgcgccctcc cagaggccca ccttcaagca gctggtggag gacctggacc      60

gtgtccttac cgtgacgtcc accgacttta                                      90


<210> 76
<211> 90
<212> DNA
<213> Artificial Sequence

<220>
<223> Description of Artificial Sequence: Synthetic
      oligonucleotide

<400> 76
tgctggcatg ccgcgccctc ccagaggccc accttcaagc agctggtgga ggacctggac      60

cgtgtcctta ccgtgacgtc caccgacttt                                      90


<210> 77
<211> 90
<212> DNA
<213> Artificial Sequence

<220>
<223> Description of Artificial Sequence: Synthetic
      oligonucleotide

<400> 77
gtgctggcat gccgcgccct cccagaggcc caccttcaag cagctggtgg aggacctgga      60

ccgtgtcctt accgtgacgt ccaccgactt                                      90


<210> 78
<211> 59
<212> PRT
<213> Artificial Sequence

<220>
<223> Description of Artificial Sequence: Synthetic

polypeptide

<400> 78
Cys Trp His Ala Ala Pro Ser Gln Arg Pro Thr Phe Lys Gln Leu Val
1               5                   10                  15

Glu Asp Leu Asp Arg Val Leu Thr Val Thr Ser Thr Asp Phe Lys Glu
            20                  25                  30

Ser Ala Leu Arg Lys Gln Ser Leu Tyr Leu Lys Phe Asp Pro Leu Leu
        35                  40                  45

Arg Asp Ser Pro Gly Arg Pro Val Pro Val Ala
    50                  55

<210> 79
<211> 1048
<212> PRT
<213> Artificial Sequence

<220>
<223> Description of Artificial Sequence: Synthetic
      polypeptide

<400> 79
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser

385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Phe Lys Glu Ser Ala Leu Arg Lys Gln Ser
        755                 760                 765

Leu Tyr Leu Lys Phe Asp Pro Leu Leu Arg Asp Ser Pro Gly Arg Pro
    770                 775                 780

Val Pro Val Ala Thr Glu Thr Ser Ser Met His Gly Ala Asn Glu Thr
785                 790                 795                 800

Pro Ser Gly Arg Pro Arg Glu Ala Lys Leu Val Glu Phe Asp Phe Leu
                805                 810                 815

Gly Ala Leu Asp Ile Pro Val Pro Gly Pro Pro Pro Gly Val Pro Ala
            820                 825                 830

Pro Gly Gly Pro Pro Leu Ser Thr Gly Pro Ile Val Asp Leu Leu Gln
        835                 840                 845

Tyr Ser Gln Lys Asp Leu Asp Ala Val Val Lys Ala Thr Gln Glu Glu
    850                 855                 860

Asn Arg Glu Leu Arg Ser Arg Cys Glu Glu Leu His Gly Lys Asn Leu
865                 870                 875                 880

Glu Leu Gly Lys Ile Met Asp Arg Phe Glu Glu Val Val Tyr Gln Ala
                885                 890                 895

Met Glu Glu Val Gln Lys Gln Lys Glu Leu Ser Lys Ala Glu Ile Gln
            900                 905                 910

Lys Val Leu Lys Glu Lys Asp Gln Leu Thr Thr Asp Leu Asn Ser Met
        915                 920                 925

Glu Lys Ser Phe Ser Asp Leu Phe Lys Arg Phe Glu Lys Gln Lys Glu
    930                 935                 940

Val Ile Glu Gly Tyr Arg Lys Asn Glu Glu Ser Leu Lys Lys Cys Val
945                 950                 955                 960

Glu Asp Tyr Leu Ala Arg Ile Thr Gln Glu Gly Gln Arg Tyr Gln Ala
                965                 970                 975

Leu Lys Ala His Ala Glu Glu Lys Leu Gln Leu Ala Asn Glu Glu Ile
            980                 985                 990

Ala Gln Val Arg Ser Lys Ala Gln  Ala Glu Ala Leu Ala  Leu Gln Ala
        995                 1000                 1005

Ser Leu  Arg Lys Glu Gln Met  Arg Ile Gln Ser Leu  Glu Lys Thr
    1010                 1015                 1020

Val Glu  Gln Lys Thr Lys Glu  Asn Glu Glu Leu Thr  Arg Ile Cys
    1025                 1030                 1035

Asp Asp  Leu Ile Ser Lys Met  Glu Lys Ile
    1040                 1045

<210> 80
<211> 26
<212> DNA
<213> Artificial Sequence

<220>
<223> Description of Artificial Sequence: Synthetic
      oligonucleotide

<220>
<221> CDS
<222> (2)..(25)

<400> 80
g acg tcc acc gac ttt aag gag tcg g                                26
  Thr Ser Thr Asp Phe Lys Glu Ser
  1               5

<210> 81
<211> 27

<212> DNA
<213> Artificial Sequence

<220>
<223> Description of Artificial Sequence: Synthetic
      oligonucleotide

<220>
<221> CDS
<222> (2)..(25)

<400> 81
g acg tcc acc gac gta aag gcg aca ca                                27
  Thr Ser Thr Asp Val Lys Ala Thr
  1               5

<210> 82
<211> 27
<212> DNA
<213> Artificial Sequence

<220>
<223> Description of Artificial Sequence: Synthetic
      oligonucleotide

<220>
<221> CDS
<222> (1)..(27)

<400> 82
gcc gtc ccc ggc cat ccc tca gga cgt                                 27
Ala Val Pro Gly His Pro Ser Gly Arg
1               5

<210> 83
<211> 26
<212> DNA
<213> Artificial Sequence

<220>
<223> Description of Artificial Sequence: Synthetic
      oligonucleotide

<220>
<221> CDS
<222> (2)..(25)

<400> 83
g acc tcc aac cag ggg ctg ctg gag t                                 26
  Thr Ser Asn Gln Gly Leu Leu Glu
  1               5

<210> 84
<211> 27
<212> DNA
<213> Artificial Sequence

<220>
<223> Description of Artificial Sequence: Synthetic
      oligonucleotide

<220>
<221> CDS
<222> (2)..(25)

<400> 84
g acg tcc acc gac gtg cca ggc cca cc                                    27
  Thr Ser Thr Asp Val Pro Gly Pro
  1               5

<210> 85
<211> 8
<212> PRT
<213> Artificial Sequence

<220>
<223> Description of Artificial Sequence: Synthetic
      peptide

<400> 85
Thr Ser Thr Asp Phe Lys Glu Ser
1               5

<210> 86
<211> 8
<212> PRT
<213> Artificial Sequence

<220>
<223> Description of Artificial Sequence: Synthetic
      peptide

<400> 86
Thr Ser Thr Asp Val Lys Ala Thr
1               5

<210> 87
<211> 9
<212> PRT
<213> Artificial Sequence

<220>
<223> Description of Artificial Sequence: Synthetic
      peptide

<400> 87
Ala Val Pro Gly His Pro Ser Gly Arg
1               5

<210> 88
<211> 8
<212> PRT
<213> Artificial Sequence

<220>

<223> Description of Artificial Sequence: Synthetic
      peptide

<400> 88
Thr Ser Asn Gln Gly Leu Leu Glu
1               5


<210> 89
<211> 8
<212> PRT
<213> Artificial Sequence

<220>
<223> Description of Artificial Sequence: Synthetic
      peptide

<400> 89
Thr Ser Thr Asp Val Pro Gly Pro
1               5


<210> 90
<211> 806
<212> PRT
<213> Homo sapiens

<400> 90
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu

645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly
    770                 775                 780

Asp Asp Ser Val Phe Ala His Asp Leu Leu Pro Pro Ala Pro Pro Ser
785                 790                 795                 800

Ser Gly Gly Ser Arg Thr
                805

<210> 91
<211> 4304
<212> DNA
<213> Homo sapiens

<400> 91
gtcgcgggca gctggcgccg cgcggtcctg ctctgccggt cgcacggacg caccggcggg      60

ccgccggccg gagggacggg gcgggagctg ggcccgcgga cagcgagccg gagcgggagc     120

cgcgcgtagc gagccgggct ccggcgctcg ccagtctccc gagcggcgcc cgcctcccgc     180

cggtgcccgc gccgggccgt ggggggcagc atgcccgcgc gcgctgcctg aggacgccgc     240

ggcccccgcc cccgccatgg gcgcccctgc ctgcgccctc gcgctctgcg tggccgtggc     300

catcgtggcc ggcgcctcct cggagtcctt ggggacggag cagcgcgtcg tggggcgagc     360

ggcagaagtc ccgggcccag agcccggcca gcaggagcag ttggtcttcg gcagcgggga     420

tgctgtggag ctgagctgtc ccccgcccgg gggtggtccc atggggccca ctgtctgggt 480

caaggatggc acagggctgg tgccctcgga gcgtgtcctg gtggggcccc agcggctgca 540

ggtgctgaat gcctcccacg aggactccgg ggcctacagc tgccggcagc ggctcacgca 600

gcgcgtactg tgccacttca gtgtgcgggt gacagacgct ccatcctcgg gagatgacga 660

agacggggag gacgaggctg aggacacagg tgtggacaca ggggcccctt actggacacg 720

gcccgagcgg atggacaaga agctgctggc cgtgccggcc gccaacaccg tccgcttccg 780

ctgcccagcc gctggcaacc ccactccctc catctcctgg ctgaagaacg gcagggagtt 840

ccgcggcgag caccgcattg gaggcatcaa gctgcggcat cagcagtgga gcctggtcat 900

ggaaagcgtg gtgccctcgg accgcggcaa ctacacctgc gtcgtggaga acaagtttgg 960

cagcatccgg cagacgtaca cgctggacgt gctggagcgc tccccgcacc ggcccatcct 1020

gcaggcgggg ctgccggcca accagacggc ggtgctgggc agcgacgtgg agttccactg 1080

caaggtgtac agtgacgcac agccccacat ccagtggctc aagcacgtgg aggtgaatgg 1140

cagcaaggtg ggcccggacg gcacacccta cgttaccgtg ctcaagacgg cgggcgctaa 1200

caccaccgac aaggagctag aggttctctc cttgcacaac gtcacctttg aggacgccgg 1260

ggagtacacc tgcctggcgg gcaattctat tgggttttct catcactctg cgtggctggt 1320

ggtgctgcca gccgaggagg agctggtgga ggctgacgag gcgggcagtg tgtatgcagg 1380

catcctcagc tacggggtgg gcttcttcct gttcatcctg gtggtggcgg ctgtgacgct 1440

ctgccgcctg cgcagccccc ccaagaaagg cctgggctcc cccaccgtgc acaagatctc 1500

ccgcttcccg ctcaagcgac aggtgtccct ggagtccaac gcgtccatga gctccaacac 1560

accactggtg cgcatcgcaa ggctgtcctc aggggagggc cccacgctgg ccaatgtctc 1620

cgagctcgag ctgcctgccg accccaaatg ggagctgtct cgggcccggc tgaccctggg 1680

caagcccctt ggggagggct gcttcggcca ggtggtcatg gcggaggcca tcggcattga 1740

caaggaccgg gccgccaagc ctgtcaccgt agccgtgaag atgctgaaag acgatgccac 1800

tgacaaggac ctgtcggacc tggtgtctga gatggagatg atgaagatga tcgggaaaca 1860

caaaaacatc atcaacctgc tgggcgcctg cacgcagggc gggcccctgt acgtgctggt 1920

ggagtacgcg gccaagggta acctgcggga gtttctgcgg gcgcggcggc ccccgggcct 1980

ggactactcc ttcgacacct gcaagccgcc cgaggagcag ctcaccttca aggacctggt 2040

gtcctgtgcc taccaggtgg cccggggcat ggagtacttg gcctcccaga agtgcatcca 2100

cagggacctg gctgcccgca atgtgctggt gaccgaggac aacgtgatga agatcgcaga 2160

cttcgggctg gcccgggacg tgcacaacct cgactactac aagaagacaa ccaacggccg 2220

gctgcccgtg aagtggatgg cgcctgaggc cttgtttgac cgagtctaca ctcaccagag 2280

tgacgtctgg tcctttgggg tcctgctctg ggagatcttc acgctggggg gctccccgta 2340

ccccggcatc cctgtggagg agctcttcaa gctgctgaag gagggccacc gcatggacaa 2400

gcccgccaac tgcacacacg acctgtacat gatcatgcgg gagtgctggc atgccgcgcc 2460

ctcccagagg cccaccttca agcagctggt ggaggacctg gaccgtgtcc ttaccgtgac 2520

gtccaccgac gagtacctgg acctgtcggc gcctttcgag cagtactccc cgggtggcca 2580

ggacaccccc agctccagct cctcagggga cgactccgtg tttgcccacg acctgctgcc 2640

cccggcccca cccagcagtg ggggctcgcg gacgtgaagg gccactggtc cccaacaatg 2700

tgaggggtcc ctagcagccc accctgctgc tggtgcacag ccactccccg gcatgagact 2760

cagtgcagat ggagagacag ctacacagag ctttggtctg tgtgtgtgtg tgtgcgtgtg 2820

tgtgtgtgtg tgtgcacatc cgcgtgtgcc tgtgtgcgtg cgcatcttgc ctccaggtgc 2880

agaggtaccc tgggtgtccc cgctgctgtg caacggtctc ctgactggtg ctgcagcacc 2940

gaggggcctt tgttctgggg ggacccagtg cagaatgtaa gtgggcccac ccggtgggac 3000

ccccgtgggg cagggagctg ggcccgacat ggctccggcc tctgcctttg caccacggga 3060

catcacaggg tgggcctcgg cccctcccac acccaaagct gagcctgcag ggaagcccca 3120

catgtccagc accttgtgcc tggggtgtta gtggcaccgc ctccccacct ccaggctttc 3180

ccacttccca ccctgcccct cagagactga aattacgggt acctgaagat gggagccttt 3240

accttttatg caaaaggttt attccggaaa ctagtgtaca tttctataaa tagatgctgt 3300

gtatatggta tatatacata tatatatata acatatatgg aagaggaaaa ggctggtaca 3360

acggaggcct gcgaccctgg gggcacagga ggcaggcatg gccctgggcg gggcgtgggg 3420

gggcgtggag ggaggcccca gggggtctca cccatgcaag cagaggacca gggccttttc 3480

tggcaccgca gttttgtttt aaaactggac ctgtatattt gtaaagctat ttatgggccc 3540

ctggcactct tgttcccaca ccccaacact tccagcattt agctggccac atggcggaga 3600

gttttaattt ttaacttatt gacaaccgag aaggtttatc ccgccgatag agggacggcc 3660

aagaatgtac gtccagcctg ccccggagct ggaggatccc ctccaagcct aaaaggttgt 3720

taatagttgg aggtgattcc agtgaagata ttttatttcc tttgtccttt ttcaggagaa 3780

ttagatttct ataggatttt tctttaggag atttattttt tggacttcaa agcaagctgg 3840

tattttcata caaattcttc taattgctgt gtgtcccagg cagggagacg gtttccaggg 3900

aggggccggc cctgtgtgca ggttccgatg ttattagatg ttacaagttt atatatatct 3960

atatatataa tttattgagt ttttacaaga tgtatttgtt gtagacttaa cacttcttac 4020

gcaatgcttc tagagtttta tagcctggac tgctaccttt caaagcttgg agggaagccg 4080

tgaattcagt tggttcgttc tgtactgtta ctgggccctg agtctgggca gctgtccctt 4140

gcttgcctgc agggccatgg ctcagggtgg tctcttcttg gggcccagtg catggtggcc 4200

agaggtgtca cccaaaccgg caggtgcgat tttgttaacc cagcgacgaa ctttccgaaa 4260

aataaagaca cctggttgct aacctggaaa aaaaaaaaaa aaaa 4304

<210> 92
<211> 838
<212> PRT
<213> Homo sapiens

<400> 92
Met Ser Leu Gln Val Leu Asn Asp Lys Asn Val Ser Asn Glu Lys Asn
1               5                   10                  15

Thr Glu Asn Cys Asp Phe Leu Phe Ser Pro Pro Glu Val Thr Gly Arg
            20                  25                  30

Ser Ser Val Leu Arg Val Ser Gln Lys Glu Asn Val Pro Pro Lys Asn
        35                  40                  45

Leu Ala Lys Ala Met Lys Val Thr Phe Gln Thr Pro Leu Arg Asp Pro
    50                  55                  60

Gln Thr His Arg Ile Leu Ser Pro Ser Met Ala Ser Lys Leu Glu Ala
65                  70                  75                  80

Pro Phe Thr Gln Asp Asp Thr Leu Gly Leu Glu Asn Ser His Pro Val
                85                  90                  95

Trp Thr Gln Lys Glu Asn Gln Gln Leu Ile Lys Glu Val Asp Ala Lys
            100                 105                 110

Thr Thr His Gly Ile Leu Gln Lys Pro Val Glu Ala Asp Thr Asp Leu
        115                 120                 125

Leu Gly Asp Ala Ser Pro Ala Phe Gly Ser Gly Ser Ser Ser Glu Ser
    130                 135                 140

Gly Pro Gly Ala Leu Ala Asp Leu Asp Cys Ser Ser Ser Ser Gln Ser
145                 150                 155                 160

Pro Gly Ser Ser Glu Asn Gln Met Val Ser Pro Gly Lys Val Ser Gly
                165                 170                 175

Ser Pro Glu Gln Ala Val Glu Glu Asn Leu Ser Ser Tyr Ser Leu Asp
            180                 185                 190

Arg Arg Val Thr Pro Ala Ser Glu Thr Leu Glu Asp Pro Cys Arg Thr
        195                 200                 205

Glu Ser Gln His Lys Ala Glu Thr Pro His Gly Ala Glu Glu Glu Cys
    210                 215                 220

Lys Ala Glu Thr Pro His Gly Ala Glu Glu Glu Cys Arg His Gly Gly
225                 230                 235                 240

Val Cys Ala Pro Ala Ala Val Ala Thr Ser Pro Pro Gly Ala Ile Pro
                245                 250                 255

Lys Glu Ala Cys Gly Gly Ala Pro Leu Gln Gly Leu Pro Gly Glu Ala
            260                 265                 270

Leu Gly Cys Pro Ala Gly Val Gly Thr Pro Val Pro Ala Asp Gly Thr
        275                 280                 285

Gln Thr Leu Thr Cys Ala His Thr Ser Ala Pro Glu Ser Thr Ala Pro
    290                 295                 300

Thr Asn His Leu Val Ala Gly Arg Ala Met Thr Leu Ser Pro Gln Glu
305                 310                 315                 320

Glu Val Ala Ala Gly Gln Met Ala Ser Ser Ser Arg Ser Gly Pro Val
                325                 330                 335

Lys Leu Glu Phe Asp Val Ser Asp Gly Ala Thr Ser Lys Arg Ala Pro
            340                 345                 350

Pro Pro Arg Arg Leu Gly Glu Arg Ser Gly Leu Lys Pro Pro Leu Arg
        355                 360                 365

Lys Ala Ala Val Arg Gln Gln Lys Ala Pro Gln Glu Val Glu Glu Asp
    370                 375                 380

Asp Gly Arg Ser Gly Ala Gly Glu Asp Pro Pro Met Pro Ala Ser Arg
385                 390                 395                 400

Gly Ser Tyr His Leu Asp Trp Asp Lys Met Asp Asp Pro Asn Phe Ile
                405                 410                 415

Pro Phe Gly Gly Asp Thr Lys Ser Gly Cys Ser Glu Ala Gln Pro Pro
            420                 425                 430

Glu Ser Pro Glu Thr Arg Leu Gly Gln Pro Ala Ala Glu Gln Leu His
        435                 440                 445

Ala Gly Pro Ala Thr Glu Glu Pro Gly Pro Cys Leu Ser Gln Gln Leu
    450                 455                 460

His Ser Ala Ser Ala Glu Asp Thr Pro Val Val Gln Leu Ala Ala Glu
465                 470                 475                 480

Thr Pro Thr Ala Glu Ser Lys Glu Arg Ala Leu Asn Ser Ala Ser Thr
                485                 490                 495

Ser Leu Pro Thr Ser Cys Pro Gly Ser Glu Pro Val Pro Thr His Gln
            500                 505                 510

Gln Gly Gln Pro Ala Leu Glu Leu Lys Glu Glu Ser Phe Arg Asp Pro
        515                 520                 525

Ala Glu Val Leu Gly Thr Gly Ala Glu Val Asp Tyr Leu Glu Gln Phe
    530                 535                 540

Gly Thr Ser Ser Phe Lys Glu Ser Ala Leu Arg Lys Gln Ser Leu Tyr
545                 550                 555                 560

Leu Lys Phe Asp Pro Leu Leu Arg Asp Ser Pro Gly Arg Pro Val Pro
                565                 570                 575

Val Ala Thr Glu Thr Ser Ser Met His Gly Ala Asn Glu Thr Pro Ser
            580                 585                 590

Gly Arg Pro Arg Glu Ala Lys Leu Val Glu Phe Asp Phe Leu Gly Ala
        595                 600                 605

Leu Asp Ile Pro Val Pro Gly Pro Pro Pro Gly Val Pro Ala Pro Gly
    610                 615                 620

Gly Pro Pro Leu Ser Thr Gly Pro Ile Val Asp Leu Leu Gln Tyr Ser
625                 630                 635                 640

Gln Lys Asp Leu Asp Ala Val Val Lys Ala Thr Gln Glu Glu Asn Arg
                645                 650                 655

Glu Leu Arg Ser Arg Cys Glu Glu Leu His Gly Lys Asn Leu Glu Leu
            660                 665                 670

Gly Lys Ile Met Asp Arg Phe Glu Glu Val Val Tyr Gln Ala Met Glu
        675                 680                 685

Glu Val Gln Lys Gln Lys Glu Leu Ser Lys Ala Glu Ile Gln Lys Val
    690                 695                 700

Leu Lys Glu Lys Asp Gln Leu Thr Thr Asp Leu Asn Ser Met Glu Lys
705                 710                 715                 720

Ser Phe Ser Asp Leu Phe Lys Arg Phe Glu Lys Gln Lys Glu Val Ile
                725                 730                 735

Glu Gly Tyr Arg Lys Asn Glu Glu Ser Leu Lys Lys Cys Val Glu Asp
            740                 745                 750

Tyr Leu Ala Arg Ile Thr Gln Glu Gly Gln Arg Tyr Gln Ala Leu Lys
        755                 760                 765

Ala His Ala Glu Glu Lys Leu Gln Leu Ala Asn Glu Glu Ile Ala Gln
    770                 775                 780

Val Arg Ser Lys Ala Gln Ala Glu Ala Leu Ala Leu Gln Ala Ser Leu
785                 790                 795                 800

Arg Lys Glu Gln Met Arg Ile Gln Ser Leu Glu Lys Thr Val Glu Gln
                805                 810                 815

Lys Thr Lys Glu Asn Glu Glu Leu Thr Arg Ile Cys Asp Asp Leu Ile
            820                 825                 830

Ser Lys Met Glu Lys Ile
        835

<210> 93
<211> 2847
<212> DNA
<213> Homo sapiens

<400> 93
gcgtttgaaa ctccggcgcg ccggcggcca tcaagggcta gaagcgcgac ggcggtagca      60

gctaggcttg gcccccggcg tggagcagac gcggacccct ccttcctggc ggcggcggcg     120

cgggctcaga gcccggcaac gggcgggcgg gcagaatgag tctgcaggtc ttaaacgaca     180

aaaatgtcag caatgaaaaa aatacagaaa attgcgactt cctgttttcg ccaccagaag     240

ttaccggaag atcgtctgtt cttcgtgtgt cacagaaaga aaatgtgcca cccaagaacc     300

tggccaaagc tatgaaggtg acttttcaga cacctctgcg ggatccacag acgcacagga     360

ttctaagtcc tagcatggcc agcaaacttg aggctccttt cactcaggat gacacccttg     420

gactggaaaa ctcacacccg gtctggacac agaaagagaa ccaacagctc atcaaggaag     480

tggatgccaa aactactcat ggaattctac agaaaccagt ggaggctgac accgacctcc     540

tgggggatgc aagcccagcc tttgggagtg gcagctccag cgagtctggc ccaggtgccc     600

tggctgacct ggactgctca agctcttccc agagcccagg aagttctgag aaccaaatgg     660

tgtctccagg aaaagtgtct ggcagccctg agcaagccgt ggaggaaaac cttagttcct     720

attccttaga cagaagagtg acacccgcct ctgagaccct agaagaccct tgcaggacag 780

agtcccagca caaagcggag actccgcacg gagccgagga agaatgcaaa gcggagactc 840

cgcacggagc cgaggaggaa tgccggcacg gtggggtctg tgctcccgca gcagtggcca 900

cttcgcctcc tggtgcaatc cctaaggaag cctgcggagg agcacccctg cagggtctgc 960

ctggcgaagc cctgggctgc cctgcgggtg tgggcacccc cgtgccagca gatggcactc 1020

agacccttac ctgtgcacac acctctgctc ctgagagcac agccccaacc aaccacctgg 1080

tggctggcag ggccatgacc ctgagtcctc aggaagaagt ggctgcaggc caaatggcca 1140

gctcctcgag gagcggacct gtaaaactag aatttgatgt atctgatggc gccaccagca 1200

aaagggcacc cccaccaagg agactgggag agaggtccgg cctcaagcct cccttgagga 1260

aagcagcagt gaggcagcaa aaggccccgc aggaggtgga ggaggacgac ggtaggagcg 1320

gagcaggaga ggaccccccc atgccagctt ctcggggctc ttaccacctc gactgggaca 1380

aaatggatga cccaaacttc atcccgttcg gaggtgacac caagtctggt tgcagtgagg 1440

cccagccccc agaaagccct gagaccaggc tgggccagcc agcggctgaa cagttgcatg 1500

ctgggcctgc cacggaggag ccaggtccct gtctgagcca gcagctgcat tcagcctcag 1560

cggaggacac gcctgtggtg cagttggcag ccgagacccc aacagcagag agcaaggaga 1620

gagccttgaa ctctgccagc acctcgcttc ccacaagctg tccaggcagt gagccagtgc 1680

ccacccatca gcaggggcag cctgccttgg agctgaaaga ggagagcttc agagaccccg 1740

ctgaggttct aggcacgggc gcggaggtgg attacctgga gcagtttgga acttcctcgt 1800

ttaaggagtc ggccttgagg aagcagtcct tatacctcaa gttcgacccc ctcctgaggg 1860

acagtcctgg tagaccagtg cccgtggcca ccgagaccag cagcatgcac ggtgcaaatg 1920

agactccctc aggacgtccg cgggaagcca agcttgtgga gttcgatttc ttgggagcac 1980

tggacattcc tgtgccaggc ccacccccag gtgttcccgc gcctgggggc ccacccctgt 2040

ccaccggacc tatagtggac ctgctccagt acagccagaa ggacctggat gcagtggtaa 2100

aggcgacaca ggaggagaac cgggagctga ggagcaggtg tgaggagctc cacgggaaga 2160

acctggaact ggggaagatc atggacaggt tcgaagaggt tgtgtaccag gccatggagg 2220

aagttcagaa gcagaaggaa ctttccaaag ctgaaatcca gaaagttcta aaagaaaaag 2280

accaacttac cacagatctg aactccatgg agaagtcctt ctccgacctc ttcaagcgtt 2340

ttgagaaaca gaaagaggtg atcgagggct accgcaagaa cgaagagtca ctgaagaagt 2400

gcgtggagga ttacctggca aggatcaccc aggagggcca gaggtaccaa gccctgaagg 2460

cccacgcgga ggagaagctg cagctggcaa acgaggagat cgcccaggtc cggagcaagg 2520

cccaggcgga agcgttggcc ctccaggcca gcctgaggaa ggagcagatg cgcatccagt 2580

cgctggagaa gacagtggag cagaagacta aagagaacga ggagctgacc aggatctgcg 2640

acgacctcat ctccaagatg gagaagatct gacctccacg gagccgctgt ccccgccccc 2700

ctgctcccgt ctgtctgtcc tgtctgattc tcttaggtgt catgttcttt tttctgtctt 2760

gtcttcaact tttttaaaaa ctagattgct ttgaaaacat gactcaataa aagtttcctt 2820

tcaatttaaa cactgaaaaa aaaaaaa 2847

<210> 94
<211> 3578
<212> DNA
<213> Homo sapiens

<400> 94
gtcgcgggca gctggcgccg cgcggtcctg ctctgccggt cgcacggacg caccggcggg 60

ccgccggccg gagggacggg gcgggagctg ggcccgcgga cagcgagccg gagcgggagc 120

cgcgcgtagc gagccgggct ccggcgctcg ccagtctccc gagcggcgcc cgcctcccgc 180

cggtgcccgc gccgggccgt ggggggcagc atgcccgcgc gcgctgcctg aggacgccgc 240

ggcccccgcc cccgccatgg gcgcccctgc ctgcgccctc gcgctctgcg tggccgtggc 300

catcgtggcc ggcgcctcct cggagtcctt ggggacggag cagcgcgtcg tggggcgagc 360

ggcagaagtc ccgggcccag agcccggcca gcaggagcag ttggtcttcg gcagcgggga 420

tgctgtggag ctgagctgtc ccccgcccgg gggtggtccc atggggccca ctgtctgggt 480

caaggatggc acagggctgg tgccctcgga gcgtgtcctg gtggggcccc agcggctgca 540

ggtgctgaat gcctcccacg aggactccgg ggcctacagc tgccggcagc ggctcacgca 600

gcgcgtactg tgccacttca gtgtgcgggt gacagacgct ccatcctcgg gagatgacga 660

agacggggag gacgaggctg aggacacagg tgtggacaca ggggcccctt actggacacg 720

gcccgagcgg atggacaaga agctgctggc cgtgccggcc gccaacaccg tccgcttccg 780

ctgcccagcc gctggcaacc ccactccctc catctcctgg ctgaagaacg gcagggagtt 840

ccgcggcgag caccgcattg gaggcatcaa gctgcggcat cagcagtgga gcctggtcat 900

ggaaagcgtg gtgccctcgg accgcggcaa ctacacctgc gtcgtggaga acaagtttgg 960

cagcatccgg cagacgtaca cgctggacgt gctggagcgc tccccgcacc ggcccatcct 1020

gcaggcgggg ctgccggcca accagacggc ggtgctgggc agcgacgtgg agttccactg 1080

caaggtgtac agtgacgcac agccccacat ccagtggctc aagcacgtgg aggtgaatgg 1140

cagcaaggtg ggcccggacg gcacacccta cgttaccgtg ctcaagacgg cgggcgctaa 1200

caccaccgac aaggagctag aggttctctc cttgcacaac gtcacctttg aggacgccgg 1260

ggagtacacc tgcctggcgg gcaattctat tgggttttct catcactctg cgtggctggt 1320

ggtgctgcca gccgaggagg agctggtgga ggctgacgag gcgggcagtg tgtatgcagg 1380

catcctcagc tacggggtgg gcttcttcct gttcatcctg gtggtggcgg ctgtgacgct 1440

ctgccgcctg cgcagccccc ccaagaaagg cctgggctcc cccaccgtgc acaagatctc 1500

ccgcttcccg ctcaagcgac aggtgtccct ggagtccaac gcgtccatga gctccaacac 1560

accactggtg cgcatcgcaa ggctgtcctc aggggagggc cccacgctgg ccaatgtctc 1620

cgagctcgag ctgcctgccg accccaaatg ggagctgtct cgggcccggc tgaccctggg 1680

caagcccctt ggggagggct gcttcggcca ggtggtcatg gcggaggcca tcggcattga 1740

caaggaccgg gccgccaagc ctgtcaccgt agccgtgaag atgctgaaag acgatgccac 1800

tgacaaggac ctgtcggacc tggtgtctga gatggagatg atgaagatga tcgggaaaca 1860

caaaaacatc atcaacctgc tgggcgcctg cacgcagggc gggcccctgt acgtgctggt 1920

ggagtacgcg gccaagggta acctgcggga gtttctgcgg gcgcggcggc ccccgggcct 1980

ggactactcc ttcgacacct gcaagccgcc cgaggagcag ctcaccttca aggacctggt 2040

gtcctgtgcc taccaggtgg cccggggcat ggagtacttg gcctcccaga agtgcatcca 2100

cagggacctg gctgcccgca atgtgctggt gaccgaggac aacgtgatga agatcgcaga 2160

cttcgggctg gcccgggacg tgcacaacct cgactactac aagaagacaa ccaacggccg 2220

gctgcccgtg aagtggatgg cgcctgaggc cttgtttgac cgagtctaca ctcaccagag 2280

tgacgtctgg tcctttgggg tcctgctctg ggagatcttc acgctggggg gctccccgta 2340

ccccggcatc cctgtggagg agctcttcaa gctgctgaag gagggccacc gcatggacaa 2400

gcccgccaac tgcacacacg acctgtacat gatcatgcgg gagtgctggc atgccgcgcc 2460

ctcccagagg cccaccttca agcagctggt ggaggacctg gaccgtgtcc ttaccgtgac 2520

gtccaccgac tttaaggagt cggccttgag gaagcagtcc ttatacctca agttcgaccc 2580

cctcctgagg gacagtcctg gtagaccagt gcccgtggcc accgagacca gcagcatgca 2640

cggtgcaaat gagactccct caggacgtcc gcgggaagcc aagcttgtgg agttcgattt 2700

cttgggagca ctggacattc ctgtgccagg cccaccccca ggtgttcccg cgcctggggg 2760

cccacccctg tccaccggac ctatagtgga cctgctccag tacagccaga aggacctgga 2820

tgcagtggta aaggcgacac aggaggagaa ccgggagctg aggagcaggt gtgaggagct 2880

ccacgggaag aacctggaac tggggaagat catggacagg ttcgaagagg ttgtgtacca 2940

ggccatggag gaagttcaga agcagaagga actttccaaa gctgaaatcc agaaagttct 3000

aaaagaaaaa gaccaactta ccacagatct gaactccatg gagaagtcct tctccgacct 3060

cttcaagcgt tttgagaaac agaaagaggt gatcgagggc taccgcaaga acgaagagtc 3120

actgaagaag tgcgtggagg attacctggc aaggatcacc caggagggcc agaggtacca 3180

agccctgaag gcccacgcgg aggagaagct gcagctggca aacgaggaga tcgcccaggt 3240

ccggagcaag gcccaggcgg aagcgttggc cctccaggcc agcctgagga aggagcagat 3300

gcgcatccag tcgctggaga agacagtgga gcagaagact aaagagaacg aggagctgac     3360

caggatctgc gacgacctca tctccaagat ggagaagatc tgacctccac ggagccgctg     3420

tccccgcccc cctgctcccg tctgtctgtc ctgtctgatt ctcttaggtg tcatgttctt     3480

ttttctgtct tgtcttcaac ttttttaaaa actagattgc tttgaaaaca tgactcaata     3540

aaagtttcct ttcaatttaa acactgaaaa aaaaaaaa                             3578


<210> 95
<211> 152
<212> DNA
<213> Artificial Sequence

<220>
<223> Description of Artificial Sequence: Synthetic
      polynucleotide

<400> 95
catcctgccc cccagagtgc tgaggtgtgg ggcgggcctt ctggggcaca gcctgggcac       60

agaggtggct gtgcgaaggt cgctgagggt ccaggcttcc acccagtgtc cccgcagtca      120

gctgcccacc agcagcctcc ccgggactct cc                                    152


<210> 96
<211> 77
<212> DNA
<213> Artificial Sequence

<220>
<223> Description of Artificial Sequence: Synthetic
      oligonucleotide

<400> 96
ggctgggcga agttcgctga gggtccaggc ttcctcccag tgtccccgca gtcagctgcc       60

caccagcagc ctccccc                                                      77


<210> 97
<211> 77
<212> DNA
<213> Artificial Sequence

<220>
<223> Description of Artificial Sequence: Synthetic
      oligonucleotide

<400> 97
acagcctggg cacagaggtg gctgtgcgaa ggtcgctgag ggtccaggct tccacccagt       60

gtccccgcag tcagctt                                                      77


<210> 98
<211> 77
<212> DNA
<213> Artificial Sequence

<220>
<223> Description of Artificial Sequence: Synthetic
      oligonucleotide

<400> 98
ggcacagcct gggcacagag gtggctgtgc gaaggtcgct gagggtccag gcttccaccc      60

agtgtccccg cagtcaa                                                     77


<210> 99
<211> 77
<212> DNA
<213> Artificial Sequence

<220>
<223> Description of Artificial Sequence: Synthetic
      oligonucleotide

<400> 99
ttctggggca cagcctgggc acagaggtgg ctgtgcgaag gtcgctgagg gtccaggctt      60

ccacccagtg tccccgg                                                     77


<210> 100
<211> 77
<212> DNA
<213> Artificial Sequence

<220>
<223> Description of Artificial Sequence: Synthetic
      oligonucleotide

<400> 100
cttctgtggc acagcctggc cacagaggtg gctgtgcgaa ggtcgctgag ggtccaggct      60

tccacccagt gtccccc                                                     77


<210> 101
<211> 77
<212> DNA
<213> Artificial Sequence

<220>
<223> Description of Artificial Sequence: Synthetic
      oligonucleotide

<400> 101
gccttctggg gcacagcctg ggcacagagg tggctgtgcg aaggtcgctg agggtccagg      60

cttccaccca gtgtccc                                                     77


<210> 102
<211> 77
<212> DNA
<213> Artificial Sequence

<220>
<223> Description of Artificial Sequence: Synthetic

oligonucleotide

<400> 102
ggtgtggggc gggccggctg gggcacagcc ggggcacaga ggtggctgtg cgaaggtcgc 60

tgagggtcca ggcttcc 77


<210> 103
<211> 77
<212> DNA
<213> Artificial Sequence

<220>
<223> Description of Artificial Sequence: Synthetic
      oligonucleotide

<400> 103
ggtgtggggc gggccttctg gggcacagcc tgggcacaga ggtggctgtg cgaaggtcgc 60

tgagggtcca ggcttcc 77


<210> 104
<211> 77
<212> DNA
<213> Artificial Sequence

<220>
<223> Description of Artificial Sequence: Synthetic
      oligonucleotide

<400> 104
tgctgaggtg tggggcgggc cttctggggc acagcctggg cacagaggtg gctgtgcgaa 60

ggtcgctgag ggtccaa 77


<210> 105
<211> 77
<212> DNA
<213> Artificial Sequence

<220>
<223> Description of Artificial Sequence: Synthetic
      oligonucleotide

<400> 105
agtgctgagg tgtggggcgg gccttctggg gcacagcctg ggcacagagg tggctgtgcg 60

aaggtcgctg agggtcc 77


<210> 106
<211> 77
<212> DNA
<213> Artificial Sequence

<220>
<223> Description of Artificial Sequence: Synthetic
      oligonucleotide

<400> 106

cccagagtgc tgaggtgtgg ggcgggcctt ctggggcaca gcctgggcac agaggtggct 60

gtgcgaaggt cgctgaa 77

<210> 107
<211> 76
<212> DNA
<213> Artificial Sequence

<220>
<223> Description of Artificial Sequence: Synthetic
      oligonucleotide

<400> 107
cctgcccccc agagtgctga ggtgtggggc gggccttctg gggcacagcc tgggcacaga 60

ggtggctgtg cgaagg 76

<210> 108
<211> 153
<212> DNA
<213> Artificial Sequence

<220>
<223> Description of Artificial Sequence: Synthetic
      polynucleotide

<400> 108
gtgctggctc tggcctggtg ccacccgcct atgcccctcc ccctgccgtc cccggccatc 60

ctgcccccca gagtgccggg ggctaagggc cagggaggtc acctgcacac tcccaccccc 120

ggtcacccgc acactcccac ccccggtcac cca 153

<210> 109
<211> 76
<212> DNA
<213> Artificial Sequence

<220>
<223> Description of Artificial Sequence: Synthetic
      oligonucleotide

<400> 109
agagtgcagg gggctaaggg ccagggaggt cacctgcaca ctcccacccc cggtcacccg 60

cacactccca cccccg 76

<210> 110
<211> 76
<212> DNA
<213> Artificial Sequence

<220>
<223> Description of Artificial Sequence: Synthetic
      oligonucleotide

<400> 110
ggccatcctg ccccccagag tgcagggggc taagggccag ggaggtcccc tgcacactcc 60

ctcccccggt cacccg                                                    76


<210> 111
<211> 76
<212> DNA
<213> Artificial Sequence

<220>
<223> Description of Artificial Sequence: Synthetic
      oligonucleotide

<400> 111
tccccctgcc gtccccggcc atcctgcccc ccagagtgca gggggctaag ggccagggag      60

gtcacctgca cactcc                                                    76


<210> 112
<211> 152
<212> DNA
<213> Artificial Sequence

<220>
<223> Description of Artificial Sequence: Synthetic
      polynucleotide

<400> 112
tgacgtccac cgacgtgagt gctggctctg gcctggtgcc acccgcctat gcccctcccc      60

ctgccgtccc cggccatccc tcaggacgtc cgcgggaagc caagcttgtg gagttcgatt     120

tcttgggagc actggacatt cctgtaagtc ct                                  152


<210> 113
<211> 76
<212> DNA
<213> Artificial Sequence

<220>
<223> Description of Artificial Sequence: Synthetic
      oligonucleotide

<400> 113
atccctcagg acgtccgcgg gaagccaagc ttgtggagtt cgatttcttg ggagcactgg      60

acattcctgt aagtcc                                                    76


<210> 114
<211> 76
<212> DNA
<213> Artificial Sequence

<220>
<223> Description of Artificial Sequence: Synthetic
      oligonucleotide

<400> 114
catccctcag gacgtccgcg ggaagccaag cttgtggagt tcgatttctt gggagcactg      60

gacattcctg taagtc                                                    76

<210> 115
<211> 76
<212> DNA
<213> Artificial Sequence

<220>
<223> Description of Artificial Sequence: Synthetic
      oligonucleotide

<400> 115
catccctcag gacgtccgcg ggaagccaag cttgtggagt tcgatttctt gggagcactg      60

gacattcctg taagtc                                                    76

<210> 116
<211> 76
<212> DNA
<213> Artificial Sequence

<220>
<223> Description of Artificial Sequence: Synthetic
      oligonucleotide

<400> 116
gccatccctc aggacgtccg cgggaagcca agcttgtgga gttcgatttc ttgggagcac      60

tggacattcc tgtaag                                                    76

<210> 117
<211> 76
<212> DNA
<213> Artificial Sequence

<220>
<223> Description of Artificial Sequence: Synthetic
      oligonucleotide

<400> 117
ccggccatcc ctcaggacgt ccgcgggaag ccaagcttgt ggagttcgat ttcttgggag      60

cactggacat tcctgt                                                    76

<210> 118
<211> 76
<212> DNA
<213> Artificial Sequence

<220>
<223> Description of Artificial Sequence: Synthetic
      oligonucleotide

<400> 118
cacggccatc ccggaggacg tccgcgggaa cccaagcttg tggagttcga tttcttggta      60

gcactggaca ttcctg                                                    76

<210> 119
<211> 76
<212> DNA
<213> Artificial Sequence

<220>
<223> Description of Artificial Sequence: Synthetic
      oligonucleotide

<400> 119
cccggccatc cctcaggacg tccgcgggaa gccaagcttg tggagttcga tttcttggga      60

gcactggaca ttcctg                                                      76


<210> 120
<211> 76
<212> DNA
<213> Artificial Sequence

<220>
<223> Description of Artificial Sequence: Synthetic
      oligonucleotide

<400> 120
tccccggcca tccctcagga cgtccgcggg aagccaagct tgtggagttc gatttcttgg      60

gagcactgga cattcc                                                      76


<210> 121
<211> 76
<212> DNA
<213> Artificial Sequence

<220>
<223> Description of Artificial Sequence: Synthetic
      oligonucleotide


<220>
<221> modified_base
<222> (21)..(21)
<223> a, c, t, g, unknown or other

<400> 121
tccccggcca tccctcagga ngtccgcggg aagccaagct tgtggagttc gatttcttgg      60

gagcactgga cattcc                                                      76


<210> 122
<211> 76
<212> DNA
<213> Artificial Sequence

<220>
<223> Description of Artificial Sequence: Synthetic
      oligonucleotide

<400> 122
gtccccggcc atccctcagg acgtccgcgg gaagccaagc ttgtggagtt cgatttcttg      60

ggagcactgg acattc                                                    76

<210> 123
<211> 76
<212> DNA
<213> Artificial Sequence

<220>
<223> Description of Artificial Sequence: Synthetic
      oligonucleotide

<400> 123
ccgtccccgg ccatccctca ggacgtccgc gggaagccaa gcttgtggag ttcgatttct      60

tgggagcact ggacat                                                    76

<210> 124
<211> 76
<212> DNA
<213> Artificial Sequence

<220>
<223> Description of Artificial Sequence: Synthetic
      oligonucleotide

<400> 124
tccccctgcc gtccccggcc atccctcagg acgtccgcgg gaagccaagc ttgtggagtt      60

cgatttcttg ggagca                                                    76

<210> 125
<211> 76
<212> DNA
<213> Artificial Sequence

<220>
<223> Description of Artificial Sequence: Synthetic
      oligonucleotide

<400> 125
atgcccctcc ccctgccgtc cccggccatc cctcaggacg tccgcgggaa gccaagcttg      60

tggagttcga tttctt                                                    76

<210> 126
<211> 76
<212> DNA
<213> Artificial Sequence

<220>
<223> Description of Artificial Sequence: Synthetic
      oligonucleotide

<400> 126
cctatgcccc tccccctgcc gcccccggcc atccctcagg acgtccgcgg gaagccaagc      60

ttgtggagtt cgattt                                                    76

<210> 127
<211> 76
<212> DNA
<213> Artificial Sequence

<220>
<223> Description of Artificial Sequence: Synthetic
      oligonucleotide

<400> 127
ctggcctggt gccacccgcc tatgcccctc cccctgccgt ccccggccat ccctcaggac      60

gtccgcggga agccaa                                                      76


<210> 128
<211> 76
<212> DNA
<213> Artificial Sequence

<220>
<223> Description of Artificial Sequence: Synthetic
      oligonucleotide

<400> 128
gagctggcct ggtgccacac gcctatgccc ctccccctgc cgtccccggc gatccatcag      60

gaagtccgcg ggacga                                                      76


<210> 129
<211> 76
<212> DNA
<213> Artificial Sequence

<220>
<223> Description of Artificial Sequence: Synthetic
      oligonucleotide


<220>
<221> modified_base
<222> (40)..(40)
<223> a, c, t, g, unknown or other

<400> 129
ggctctggcc tggtgccacc cgcctatgcc cctccccctn ccgtccccgg ccatccctca      60

ggacgtccgc gggaag                                                      76


<210> 130
<211> 76
<212> DNA
<213> Artificial Sequence

<220>
<223> Description of Artificial Sequence: Synthetic
      oligonucleotide

<400> 130
gtgctggctc tggcctggtg ccacccgcct atgcccctcc ccctgccgtc cccggccatc      60

cctcaggacg tccgcg                                                    76

<210> 131
<211> 76
<212> DNA
<213> Artificial Sequence

<220>
<223> Description of Artificial Sequence: Synthetic
      oligonucleotide

<400> 131
ccaccgacgt gagtgctggc tctggcctgg tgccacccgc ctatgcccct ccccctgccg      60

tccccggcca tccctc                                                    76

<210> 132
<211> 151
<212> DNA
<213> Artificial Sequence

<220>
<223> Description of Artificial Sequence: Synthetic
      polynucleotide

<400> 132
tggaccgtgt ccttaccgtg acgtccaccg acgtgagtgc tggctctggc ctggtgccac      60

ccgcctatgc ccctcccctg gcccttagcc cccgtgtgtg ttaggggatg gcagtcagac     120

ctgatcactt gccctcttgt ccccagttta a                                   151

<210> 133
<211> 80
<212> DNA
<213> Artificial Sequence

<220>
<223> Description of Artificial Sequence: Synthetic
      oligonucleotide

<400> 133
tgcccctccc ctgcccttag cccccctgtg tgtgttaggg gatggcagtc agacctgatc      60

acttgccctc ttgtccgtcc                                                80

<210> 134
<211> 80
<212> DNA
<213> Artificial Sequence

<220>
<223> Description of Artificial Sequence: Synthetic
      oligonucleotide

<400> 134
atgcccctcc cctgccctta gcccccctgt gtgtgttagg ggatggcagt cagacctgat      60

cacttgccct cttgtctgtc                                                80

<210> 135
<211> 80
<212> DNA
<213> Artificial Sequence

<220>
<223> Description of Artificial Sequence: Synthetic
      oligonucleotide

<400> 135
gcctatgccc ctcccctgcc cttagccccc ctgtgtgtgt taggggatgg cagtcagacc      60

tgatcacttg ccctctctct                                                  80


<210> 136
<211> 80
<212> DNA
<213> Artificial Sequence

<220>
<223> Description of Artificial Sequence: Synthetic
      oligonucleotide

<400> 136
gtgccacccg cctatgcccc tcccctgccc ttagcccccc tgtgtgtgtt aggggatggc      60

agtcagacct gatcactcac                                                  80


<210> 137
<211> 80
<212> DNA
<213> Artificial Sequence

<220>
<223> Description of Artificial Sequence: Synthetic
      oligonucleotide

<400> 137
gctctggcct ggtgcccccc gcctatgccc ctcccctgcc cttagccccc ctgtgtgtgt      60

taggggatgg cagtcagtca                                                  80


<210> 138
<211> 80
<212> DNA
<213> Artificial Sequence

<220>
<223> Description of Artificial Sequence: Synthetic
      oligonucleotide

<400> 138
gctctggcct ggtgcccccc gcctatgccc ctcccctgcc cttagccccc ctgtgtgtgt      60

taggggatgg cagtcagtca                                                  80


<210> 139

<211> 81
<212> DNA
<213> Artificial Sequence

<220>
<223> Description of Artificial Sequence: Synthetic
      oligonucleotide

<400> 139
gctgcctctg tcctggtgcc ccccgcctat gcccctcccc tgcccttagc ccccctgtgt      60

gtgttagggg atggcatggc a                                                81


<210> 140
<211> 81
<212> DNA
<213> Artificial Sequence

<220>
<223> Description of Artificial Sequence: Synthetic
      oligonucleotide

<400> 140
   cacccgccgg gggtgcgggc tctggcctgg tgccccccgc ctatgcccct cccctgccct      60
   tagcccccct gtgtgttgtg t                                               81


<210> 141
<211> 80
<212> DNA
<213> Artificial Sequence

<220>
<223> Description of Artificial Sequence: Synthetic
      oligonucleotide

<400> 141
ccccccgacg tgagtgctgg ctcgggcctg gtcccccccg cctatgcccc tcccctgccc      60

ttagcccccc tgtgtgtgtg                                                  80


<210> 142
<211> 82
<212> DNA
<213> Artificial Sequence

<220>
<223> Description of Artificial Sequence: Synthetic
      oligonucleotide

<400> 142
gacgtccacc gacgtgagtg ctggctctgg cctggtgcca cccgcctatg cccctcccct      60

gcccttagac cccctgcccc tg                                               82


<210> 143
<211> 84
<212> DNA
<213> Artificial Sequence

<220>
<223> Description of Artificial Sequence: Synthetic
      oligonucleotide

<400> 143
cttaccgtga cgtccaccga cgtgagtgct ggctctggcc tggtgccacc cgcctatgcc        60

cctcccctgc ccttaggccc ttag                                               84


<210> 144
<211> 82
<212> DNA
<213> Artificial Sequence

<220>
<223> Description of Artificial Sequence: Synthetic
      oligonucleotide

<400> 144
cccttcaggt cccccccccc gacgtgagtg ctggctctgg cctggtgcca cccgcctatg        60

cccctcccct gcccttgccc tt                                                 82


<210> 145
<211> 82
<212> DNA
<213> Artificial Sequence

<220>
<223> Description of Artificial Sequence: Synthetic
      oligonucleotide

<400> 145
tgtccttacc gtgacgtcca ccgacgtgag tgctggctct ggcctggtgc cacccgccta        60

tgcccctccc ctgcccctgc cc                                                 82


<210> 146
<211> 820
<212> PRT
<213> Homo sapiens

<400> 146
Met Trp Ser Trp Lys Cys Leu Leu Phe Trp Ala Val Leu Val Thr Ala
1               5                   10                  15


Thr Leu Cys Thr Ala Arg Pro Ser Pro Thr Leu Pro Glu Gln Ala Gln
            20                  25                  30


Pro Trp Gly Ala Pro Val Glu Val Glu Ser Phe Leu Val His Pro Gly
        35                  40                  45


Asp Leu Leu Gln Leu Arg Cys Arg Leu Arg Asp Asp Val Gln Ser Ile
    50                  55                  60

Asn Trp Leu Arg Asp Gly Val Gln Leu Ala Glu Ser Asn Arg Thr Arg
65                  70                  75                  80

Ile Thr Gly Glu Glu Val Glu Val Gln Asp Ser Val Pro Ala Asp Ser
                85                  90                  95

Gly Leu Tyr Ala Cys Val Thr Ser Ser Pro Ser Gly Ser Asp Thr Thr
            100                 105                 110

Tyr Phe Ser Val Asn Val Ser Asp Ala Leu Pro Ser Ser Glu Asp Asp
        115                 120                 125

Asp Asp Asp Asp Asp Ser Ser Ser Glu Glu Lys Glu Thr Asp Asn Thr
    130                 135                 140

Lys Pro Asn Arg Met Pro Val Ala Pro Tyr Trp Thr Ser Pro Glu Lys
145                 150                 155                 160

Met Glu Lys Lys Leu His Ala Val Pro Ala Ala Lys Thr Val Lys Phe
                165                 170                 175

Lys Cys Pro Ser Ser Gly Thr Pro Asn Pro Thr Leu Arg Trp Leu Lys
            180                 185                 190

Asn Gly Lys Glu Phe Lys Pro Asp His Arg Ile Gly Gly Tyr Lys Val
        195                 200                 205

Arg Tyr Ala Thr Trp Ser Ile Ile Met Asp Ser Val Val Pro Ser Asp
    210                 215                 220

Lys Gly Asn Tyr Thr Cys Ile Val Glu Asn Glu Tyr Gly Ser Ile Asn
225                 230                 235                 240

His Thr Tyr Gln Leu Asp Val Val Glu Arg Ser Pro His Arg Pro Ile
                245                 250                 255

Leu Gln Ala Gly Leu Pro Ala Asn Lys Thr Val Ala Leu Gly Ser Asn
            260                 265                 270

Val Glu Phe Met Cys Lys Val Tyr Ser Asp Pro Gln Pro His Ile Gln
        275                 280                 285

Trp Leu Lys His Ile Glu Val Asn Gly Ser Lys Ile Gly Pro Asp Asn
    290                 295                 300

Leu Pro Tyr Val Gln Ile Leu Lys Thr Ala Gly Val Asn Thr Thr Asp
305                 310                 315                 320

Lys Glu Met Glu Val Leu His Leu Arg Asn Val Ser Phe Glu Asp Ala
                325                 330                 335

Gly Glu Tyr Thr Cys Leu Ala Gly Asn Ser Ile Gly Leu Ser His His
            340                 345                 350

Ser Ala Trp Leu Thr Val Leu Glu Ala Leu Glu Glu Arg Pro Ala Val
        355                 360                 365

Met Thr Ser Pro Leu Tyr Leu Glu Ile Ile Ile Tyr Cys Thr Gly Ala
    370                 375                 380

Phe Leu Ile Ser Cys Met Val Gly Ser Val Ile Val Tyr Lys Met Lys
385                 390                 395                 400

Ser Gly Thr Lys Lys Ser Asp Phe His Ser Gln Met Ala Val His Lys
                405                 410                 415

Leu Ala Lys Ser Ile Pro Leu Arg Arg Gln Val Ser Ala Asp Ser Ser
            420                 425                 430

Ala Ser Met Asn Ser Gly Val Leu Leu Val Arg Pro Ser Arg Leu Ser
        435                 440                 445

Ser Ser Gly Thr Pro Met Leu Ala Gly Val Ser Glu Tyr Glu Leu Pro
    450                 455                 460

Glu Asp Pro Arg Trp Glu Leu Pro Arg Asp Arg Leu Val Leu Gly Lys
465                 470                 475                 480

Pro Leu Gly Glu Gly Cys Phe Gly Gln Val Val Leu Ala Glu Ala Ile
                485                 490                 495

Gly Leu Asp Lys Asp Lys Pro Asn Arg Val Thr Lys Val Ala Val Lys
            500                 505                 510

Met Leu Lys Ser Asp Ala Thr Glu Lys Asp Leu Ser Asp Leu Ile Ser
        515                 520                 525

Glu Met Glu Met Met Lys Met Ile Gly Lys His Lys Asn Ile Ile Asn
    530                 535                 540

Leu Leu Gly Ala Cys Thr Gln Asp Gly Pro Leu Tyr Val Ile Val Glu
545                 550                 555                 560

Tyr Ala Ser Lys Gly Asn Leu Arg Glu Tyr Leu Gln Ala Arg Arg Pro
                565                 570                 575

Pro Gly Leu Glu Tyr Cys Tyr Asn Pro Ser His Asn Pro Glu Glu Gln
        580                 585                 590

Leu Ser Ser Lys Asp Leu Val Ser Cys Ala Tyr Gln Val Ala Arg Gly
        595                 600                 605

Met Glu Tyr Leu Ala Ser Lys Lys Cys Ile His Arg Asp Leu Ala Ala
    610                 615                 620

Arg Asn Val Leu Val Thr Glu Asp Asn Val Met Lys Ile Ala Asp Phe
625                 630                 635                 640

Gly Leu Ala Arg Asp Ile His His Ile Asp Tyr Tyr Lys Lys Thr Thr
                645                 650                 655

Asn Gly Arg Leu Pro Val Lys Trp Met Ala Pro Glu Ala Leu Phe Asp
            660                 665                 670

Arg Ile Tyr Thr His Gln Ser Asp Val Trp Ser Phe Gly Val Leu Leu
        675                 680                 685

Trp Glu Ile Phe Thr Leu Gly Gly Ser Pro Tyr Pro Gly Val Pro Val
    690                 695                 700

Glu Glu Leu Phe Lys Leu Leu Lys Glu Gly His Arg Met Asp Lys Pro
705                 710                 715                 720

Ser Asn Cys Thr Asn Glu Leu Tyr Met Met Met Arg Asp Cys Trp His
                725                 730                 735

Ala Val Pro Ser Gln Arg Pro Thr Phe Lys Gln Leu Val Glu Asp Leu
            740                 745                 750

Asp Arg Ile Val Ala Leu Thr Ser Asn Gln Glu Tyr Leu Asp Leu Ser
        755                 760                 765

Met Pro Leu Asp Gln Tyr Ser Pro Ser Phe Pro Asp Thr Arg Ser Ser
    770                 775                 780

Thr Cys Ser Ser Gly Glu Asp Ser Val Phe Ser His Glu Pro Leu Pro
785                 790                 795                 800

Glu Glu Pro Cys Leu Pro Arg His Pro Ala Gln Leu Ala Asn Gly Gly
                805                 810                 815

Leu Lys Arg Arg

            820

<210> 147
<211> 5895
<212> DNA
<213> Homo sapiens

<400> 147
agatgcaggg gcgcaaacgc caaaggagac caggctgtag gaagagaagg gcagagcgcc 60

ggacagctcg gcccgctccc cgtcctttgg ggccgcggct ggggaactac aaggcccagc 120

aggcagctgc agggggcgga ggcggaggag ggaccagcgc gggtgggagt gagagagcga 180

gccctcgcgc cccgccggcg catagcgctc ggagcgctct tgcggccaca ggcgcggcgt 240

cctcggcggc gggcggcagc tagcgggagc cgggacgccg gtgcagccgc agcgcgcgga 300

ggaacccggg tgtgccggga gctgggcggc cacgtccgga cgggaccgag acccctcgta 360

gcgcattgcg gcgacctcgc cttccccggc cgcgagcgcg ccgctgcttg aaaagccgcg 420

gaacccaagg acttttctcc ggtccgagct cggggcgccc cgcagggcgc acggtacccg 480

tgctgcagtc gggcacgccg cggcgccggg gcctccgcag ggcgatggag cccggtctgc 540

aaggaaagtg aggcgccgcc gctgcgttct ggaggagggg ggcacaaggt ctggagaccc 600

cgggtggcgg acgggagccc tccccccgcc ccgcctccgg ggcaccagct ccggctccat 660

tgttcccgcc cgggctggag gcgccgagca ccgagcgccg ccgggagtcg agcgccggcc 720

gcggagctct tgcgaccccg ccaggacccg aacagagccc gggggcggcg ggccggagcc 780

ggggacgcgg gcacacgccc gctcgcacaa gccacggcgg actctcccga ggcggaacct 840

ccacgccgag cgagggtcag tttgaaaagg aggatcgagc tcactgtgga gtatccatgg 900

agatgtggag ccttgtcacc aacctctaac tgcagaactg ggatgtggag ctggaagtgc 960

ctcctcttct gggctgtgct ggtcacagcc acactctgca ccgctaggcc gtccccgacc 1020

ttgcctgaac aagcccagcc ctggggagcc cctgtggaag tggagtcctt cctggtccac 1080

cccggtgacc tgctgcagct tcgctgtcgg ctgcgggacg atgtgcagag catcaactgg 1140

ctgcgggacg gggtgcagct ggcggaaagc aaccgcaccc gcatcacagg ggaggaggtg 1200

gaggtgcagg actccgtgcc cgcagactcc ggcctctatg cttgcgtaac cagcagcccc 1260

tcgggcagtg acaccaccta cttctccgtc aatgtttcag atgctctccc ctcctcggag 1320

gatgatgatg atgatgatga ctcctcttca gaggagaaag aaacagataa caccaaacca 1380

aaccgtatgc ccgtagctcc atattggaca tccccagaaa agatggaaaa gaaattgcat 1440

gcagtgccgg ctgccaagac agtgaagttc aaatgccctt ccagtgggac cccaaacccc 1500

acactgcgct ggttgaaaaa tggcaaagaa ttcaaacctg accacagaat tggaggctac 1560

aaggtccgtt atgccacctg gagcatcata atggactctg tggtgccctc tgacaagggc 1620

aactacacct gcattgtgga gaatgagtac ggcagcatca accacacata ccagctggat 1680

gtcgtggagc ggtcccctca ccggcccatc ctgcaagcag ggttgcccgc caacaaaaca 1740

gtggccctgg gtagcaacgt ggagttcatg tgtaaggtgt acagtgaccc gcagccgcac 1800

atccagtggc taaagcacat cgaggtgaat gggagcaaga ttggcccaga caacctgcct 1860

tatgtccaga tcttgaagac tgctggagtt aataccaccg acaaagagat ggaggtgctt 1920

cacttaagaa atgtctcctt tgaggacgca ggggagtata cgtgcttggc gggtaactct 1980

atcggactct cccatcactc tgcatggttg accgttctgg aagccctgga agagaggccg 2040

gcagtgatga cctcgcccct gtacctggag atcatcatct attgcacagg ggccttcctc 2100

atctcctgca tggtggggtc ggtcatcgtc tacaagatga agagtggtac caagaagagt 2160

gacttccaca gccagatggc tgtgcacaag ctggccaaga gcatccctct gcgcagacag 2220

gtgtctgctg actccagtgc atccatgaac tctggggttc ttctggttcg gccatcacgg 2280

ctctcctcca gtgggactcc catgctagca ggggtctctg agtatgagct tcccgaagac 2340

cctcgctggg agctgcctcg ggacagactg gtcttaggca aacccctggg agagggctgc 2400

tttgggcagg tggtgttggc agaggctatc gggctggaca aggacaaacc caaccgtgtg 2460

accaaagtgg ctgtgaagat gttgaagtcg gacgcaacag agaaagactt gtcagacctg 2520

atctcagaaa tggagatgat gaagatgatc gggaagcata agaatatcat caacctgctg 2580

ggggcctgca cgcaggatgg tcccttgtat gtcatcgtgg agtatgcctc caagggcaac 2640

ctgcgggagt acctgcaggc ccggaggccc ccagggctgg aatactgcta caaccccagc 2700

cacaacccag aggagcagct ctcctccaag gacctggtgt cctgcgccta ccaggtggcc 2760

cgaggcatgg agtatctggc ctccaagaag tgcatacacc gagacctggc agccaggaat 2820

gtcctggtga cagaggacaa tgtgatgaag atagcagact ttggcctcgc acgggacatt 2880

caccacatcg actactataa aaagacaacc aacggccgac tgcctgtgaa gtggatggca 2940

cccgaggcat tatttgaccg gatctacacc caccagagtg atgtgtggtc tttcggggtg 3000

ctcctgtggg agatcttcac tctgggcggc tccccatacc ccggtgtgcc tgtggaggaa 3060

cttttcaagc tgctgaagga gggtcaccgc atggacaagc ccagtaactg caccaacgag 3120

ctgtacatga tgatgcggga ctgctggcat gcagtgccct cacagagacc caccttcaag 3180

cagctggtgg aagacctgga ccgcatcgtg gccttgacct ccaaccagga gtacctggac 3240

ctgtccatgc ccctggacca gtactccccc agctttcccg acacccggag ctctacgtgc 3300

tcctcagggg aggattccgt cttctctcat gagccgctgc ccgaggagcc ctgcctgccc 3360

cgacacccag cccagcttgc caatggcgga ctcaaacgcc gctgactgcc acccacacgc 3420

cctccccaga ctccaccgtc agctgtaacc ctcacccaca gcccctgctg ggcccaccac 3480

ctgtccgtcc ctgtcccctt tcctgctggc aggagccggc tgcctaccag gggccttcct 3540

gtgtggcctg ccttcacccc actcagctca cctctccctc cacctcctct ccacctgctg 3600

gtgagaggtg caaagaggca gatctttgct gccagccact tcatcccctc ccagatgttg 3660

gaccaacacc cctccctgcc accaggcact gcctggaggg cagggagtgg gagccaatga 3720

acaggcatgc aagtgagagc ttcctgagct ttctcctgtc ggtttggtct gttttgcctt 3780

cacccataag cccctcgcac tctggtggca ggtgccttgt cctcagggct acagcagtag 3840

ggaggtcagt gcttcgtgcc tcgattgaag gtgacctctg ccccagatag gtggtgccag 3900

tggcttatta attccgatac tagtttgctt tgctgaccaa atgcctggta ccagaggatg 3960

gtgaggcgaa ggccaggttg ggggcagtgt tgtggccctg gggcccagcc ccaaactggg 4020

ggctctgtat atagctatga agaaaacaca aagtgtataa atctgagtat atatttacat 4080

gtctttttaa aagggtcgtt accagagatt tacccatcgg gtaagatgct cctggtggct 4140

gggaggcatc agttgctata tattaaaaac aaaaaagaaa aaaaaggaaa atgtttttaa 4200

aaaggtcata tattttttgc tacttttgct gttttatttt tttaaattat gttctaaacc 4260

tattttcagt ttaggtccct caataaaaat tgctgctgct tcatttatct atgggctgta 4320

tgaaaagggt gggaatgtcc actggaaaga agggacaccc acgggccctg gggctaggtc 4380

tgtcccgagg gcaccgcatg ctcccggcgc aggttccttg taacctcttc ttcctaggtc 4440

ctgcacccag acctcacgac gcacctcctg cctctccgct gcttttggaa agtcagaaaa 4500

agaagatgtc tgcttcgagg gcaggaaccc catccatgca gtagaggcgc tgggcagaga 4560

gtcaaggccc agcagccatc gaccatggat ggtttcctcc aaggaaaccg gtggggttgg 4620

gctggggagg gggcacctac ctaggaatag ccacggggta gagctacagt gattaagagg 4680

aaagcaaggg cgcggttgct cacgcctgta atcccagcac tttgggacac cgaggtgggc 4740

agatcacttc aggtcaggag tttgagacca gcctggccaa cttagtgaaa ccccatctct 4800

actaaaaatg caaaaattat ccaggcatgg tggcacacgc ctgtaatccc agctccacag 4860

gaggctgagg cagaatccct tgaagctggg aggcggaggt tgcagtgagc cgagattgcg 4920

ccattgcact ccagcctggg caacagagaa aacaaaaagg aaaacaaatg atgaaggtct 4980

gcagaaactg aaacccagac atgtgtctgc cccctctatg tgggcatggt tttgccagtg 5040

cttctaagtg caggagaaca tgtcacctga ggctagtttt gcattcaggt ccctggcttc 5100

gtttcttgtt ggtatgcctc cccagatcgt ccttcctgta tccatgtgac cagactgtat 5160

ttgttgggac tgtcgcagat cttggcttct tacagttctt cctgtccaaa ctccatcctg 5220

tccctcagga acggggggaa aattctccga atgtttttgg ttttttggct gcttggaatt 5280

tacttctgcc acctgctggt catcactgtc ctcactaagt ggattctggc tcccccgtac 5340

ctcatggctc aaactaccac tcctcagtcg ctatattaaa gcttatattt tgctggatta 5400

ctgctaaata caaaagaaag ttcaatatgt tttcatttct gtagggaaaa tgggattgct 5460

gctttaaatt tctgagctag ggattttttg gcagctgcag tgttggcgac tattgtaaaa 5520

ttctctttgt ttctctctgt aaatagcacc tgctaacatt acaatttgta tttatgttta 5580

aagaaggcat catttggtga acagaactag gaaatgaatt tttagctctt aaaagcattt 5640

gctttgagac cgcacaggag tgtctttcct tgtaaaacag tgatgataat ttctgccttg 5700

gccctacctt gaagcaatgt tgtgtgaagg gatgaagaat ctaaaagtct tcataagtcc 5760

ttgggagagg tgctagaaaa atataaggca ctatcataat tacagtgatg tccttgctgt 5820

tactactcaa atcacccaca aatttcccca aagactgcgc tagctgtcaa ataaaagaca 5880

gtgaaattga cctga 5895

<210> 148
<211> 805
<212> PRT
<213> Homo sapiens

<400> 148
Met Ala Phe Ser Pro Trp Gln Ile Leu Ser Pro Val Gln Trp Ala Lys
1               5                   10                  15

Trp Thr Trp Ser Ala Val Arg Gly Gly Ala Ala Gly Glu Asp Glu Ala
            20                  25                  30

Gly Gly Pro Glu Gly Asp Pro Glu Glu Glu Asp Ser Gln Ala Glu Thr
        35                  40                  45

Lys Ser Leu Ser Phe Ser Ser Asp Ser Glu Gly Asn Phe Glu Thr Pro
    50                  55                  60

Glu Ala Glu Thr Pro Ile Arg Ser Pro Phe Lys Glu Ser Cys Asp Pro
65                  70                  75                  80

Ser Leu Gly Leu Ala Gly Pro Gly Ala Lys Ser Gln Glu Ser Gln Glu
                85                  90                  95

Ala Asp Glu Gln Leu Val Ala Glu Val Val Glu Lys Cys Ser Ser Lys
            100                 105                 110

Thr Cys Ser Lys Pro Ser Glu Asn Glu Val Pro Gln Gln Ala Ile Asp
        115                 120                 125

Ser His Ser Val Lys Asn Phe Arg Glu Glu Pro Glu His Asp Phe Ser
    130                 135                 140

Lys Ile Ser Ile Val Arg Pro Phe Ser Ile Glu Thr Lys Asp Ser Thr
145                 150                 155                 160

Asp Ile Ser Ala Val Leu Gly Thr Lys Ala Ala His Gly Cys Val Thr
                165                 170                 175

Ala Val Ser Gly Lys Ala Leu Pro Ser Ser Pro Pro Asp Ala Leu Gln
            180                 185                 190

Asp Glu Ala Met Thr Glu Gly Ser Met Gly Val Thr Leu Glu Ala Ser
        195                 200                 205

Ala Glu Ala Asp Leu Lys Ala Gly Asn Ser Cys Pro Glu Leu Val Pro
    210                 215                 220

Ser Arg Arg Ser Lys Leu Arg Lys Pro Lys Pro Val Pro Leu Arg Lys
225                 230                 235                 240

Lys Ala Ile Gly Gly Glu Phe Ser Asp Thr Asn Ala Ala Val Glu Gly
                245                 250                 255

Thr Pro Leu Pro Lys Ala Ser Tyr His Phe Ser Pro Glu Glu Leu Asp
            260                 265                 270

Glu Asn Thr Ser Pro Leu Leu Gly Asp Ala Arg Phe Gln Lys Ser Pro
        275                 280                 285

Pro Asp Leu Lys Glu Thr Pro Gly Thr Leu Ser Ser Asp Thr Asn Asp
    290                 295                 300

Ser Gly Val Glu Leu Gly Glu Glu Ser Arg Ser Ser Pro Leu Lys Leu
305                 310                 315                 320

Glu Phe Asp Phe Thr Glu Asp Thr Gly Asn Ile Glu Ala Arg Lys Ala
                325                 330                 335

Leu Pro Arg Lys Leu Gly Arg Lys Leu Gly Ser Thr Leu Thr Pro Lys
            340                 345                 350

Ile Gln Lys Asp Gly Ile Ser Lys Ser Ala Gly Leu Glu Gln Pro Thr
        355                 360                 365

Asp Pro Val Ala Arg Asp Gly Pro Leu Ser Gln Thr Ser Ser Lys Pro
    370                 375                 380

Asp Pro Ser Gln Trp Glu Ser Pro Ser Phe Asn Pro Phe Gly Ser His
385                 390                 395                 400

Ser Val Leu Gln Asn Ser Pro Pro Leu Ser Ser Glu Gly Ser Tyr His

                405                 410                 415

Phe Asp Pro Asp Asn Phe Asp Glu Ser Met Asp Pro Phe Lys Pro Thr
            420                 425                 430

Thr Thr Leu Thr Ser Ser Asp Phe Cys Ser Pro Thr Gly Asn His Val
        435                 440                 445

Asn Glu Ile Leu Glu Ser Pro Lys Lys Ala Lys Ser Arg Leu Ile Thr
    450                 455                 460

Ser Gly Cys Lys Val Lys Lys His Glu Thr Gln Ser Leu Ala Leu Asp
465                 470                 475                 480

Ala Cys Ser Arg Asp Glu Gly Ala Val Ile Ser Gln Ile Ser Asp Ile
                485                 490                 495

Ser Asn Arg Asp Gly His Ala Thr Asp Glu Glu Lys Leu Ala Ser Thr
            500                 505                 510

Ser Cys Gly Gln Lys Ser Ala Gly Ala Glu Val Lys Gly Glu Pro Glu
        515                 520                 525

Glu Asp Leu Glu Tyr Phe Glu Cys Ser Asn Val Pro Val Ser Thr Ile
    530                 535                 540

Asn His Ala Phe Ser Ser Ser Glu Ala Gly Ile Glu Lys Glu Thr Cys
545                 550                 555                 560

Gln Lys Met Glu Glu Asp Gly Ser Thr Val Leu Gly Leu Leu Glu Ser
                565                 570                 575

Ser Ala Glu Lys Ala Pro Val Ser Val Ser Cys Gly Gly Glu Ser Pro
            580                 585                 590

Leu Asp Gly Ile Cys Leu Ser Glu Ser Asp Lys Thr Ala Val Leu Thr
        595                 600                 605

Leu Ile Arg Glu Glu Ile Ile Thr Lys Glu Ile Glu Ala Asn Glu Trp
    610                 615                 620

Lys Lys Lys Tyr Glu Glu Thr Arg Gln Glu Val Leu Glu Met Arg Lys
625                 630                 635                 640

Ile Val Ala Glu Tyr Glu Lys Thr Ile Ala Gln Met Ile Glu Asp Glu
                645                 650                 655

Gln Arg Thr Ser Met Thr Ser Gln Lys Ser Phe Gln Gln Leu Thr Met
            660                 665                 670

Glu Lys Glu Gln Ala Leu Ala Asp Leu Asn Ser Val Glu Arg Ser Leu
        675                 680                 685

Ser Asp Leu Phe Arg Arg Tyr Glu Asn Leu Lys Gly Val Leu Glu Gly
    690                 695                 700

Phe Lys Lys Asn Glu Glu Ala Leu Lys Lys Cys Ala Gln Asp Tyr Leu
705                 710                 715                 720

Ala Arg Val Lys Gln Glu Glu Gln Arg Tyr Gln Ala Leu Lys Ile His
                725                 730                 735

Ala Glu Glu Lys Leu Asp Lys Ala Asn Glu Glu Ile Ala Gln Val Arg
            740                 745                 750

Thr Lys Ala Lys Ala Glu Ser Ala Ala Leu His Ala Gly Leu Arg Lys
        755                 760                 765

Glu Gln Met Lys Val Glu Ser Leu Glu Arg Ala Leu Gln Gln Lys Asn
    770                 775                 780

Gln Glu Ile Glu Glu Leu Thr Lys Ile Cys Asp Glu Leu Ile Ala Lys
785                 790                 795                 800

Leu Gly Lys Thr Asp
                805

<210> 149
<211> 7802
<212> DNA
<213> Homo sapiens

<400> 149
agctgatgcg cgccccgccg gccgggaggc gggagtccgc gagccgggag cgggagcagc 60

agaggtctag cagccgggcg ccgcgggccg ggggcctgag gaggccacag gacgggcgtc 120

ttcccggcta gtggagcccg gcgcggggcc cgctgcggcc gcaccgtgag gggaggaggc 180

cgaggaggac gcagcgccgg ctgccggcgg gaggaagcgc tccaccaggg cccccgacgg 240

cactcgttta accacatccg cgcctctgct ggaaacgctt gctggcgcct gtcaccggtt 300

ccctccattt tgaaagggaa aaaggctctc cccacccatt cccctgcccc taggagctgg 360

agccggagga gccgcgctca tggcgttcag cccgtggcag atcctgtccc ccgtgcagtg 420

ggcgaaatgg acgtggtctg cggtacgcgg cggggccgcc ggcgaggacg aggctggcgg 480

gcccgagggc gaccccgagg aggaggattc gcaagccgag accaaatcct tgagtttcag 540

ctcggattct gaaggtaatt ttgagactcc tgaagctgaa accccgatcc gatcaccttt 600

caaggagtcc tgtgatccat cactcggatt ggcaggacct ggggccaaaa gccaagaatc 660

acaagaagct gatgaacagc ttgtagcaga agtggttgaa aaatgttcat ctaagacttg 720

ttctaaacct tcagaaaatg aagtgccaca gcaggccatt gactctcact cagtcaagaa 780

tttcagagaa gaacctgaac atgattttag caaaatttcc atcgtgaggc cattttcaat 840

agaaacgaag gattccacgg atatctcggc agtcctcgga acaaaagcag ctcatggctg 900

tgtaactgca gtctcaggca aggctctgcc ttccagcccg ccagacgccc tccaggacga 960

ggcgatgaca gaaggcagca tgggggtcac cctcgaggcc tccgcagaag ctgatctaaa 1020

agctggcaac tcctgtccag agcttgtgcc cagcagaaga agcaagctga gaaagcccaa 1080

gcctgtcccc ctgaggaaga aagcaattgg aggagagttc tcagacacca acgctgctgt 1140

ggagggcaca cctctcccca aggcatccta tcacttcagt cctgaagagt tggatgagaa 1200

cacaagtcct ttgctaggag atgccaggtt ccagaagtct ccccctgacc ttaaagaaac 1260

tcccggcact ctcagtagtg acaccaacga ctcaggggtt gagctggggg aggagtcgag 1320

gagctcacct ctcaagcttg agtttgattt cacagaagat acaggaaaca tagaggccag 1380

gaaagccctt ccaaggaagc ttggcaggaa actgggtagc acactgactc ccaagataca 1440

aaaagatggc atcagtaagt cagcaggttt agaacagcct acagacccag tggcacgaga 1500

cgggcctctc tcccaaacat cttccaagcc agatcctagt cagtgggaaa gccccagctt 1560

caaccccttt gggagccact ctgttctgca gaactcccca cccctctctt ctgagggctc 1620

ctaccacttt gacccagata actttgacga atccatggat ccctttaaac caactacgac 1680

cttaacaagc agtgactttt gttctcccac tggtaatcac gttaatgaaa tcttagaatc 1740

acccaagaag gcaaagtcgc gtttaataac gagtggctgt aaggtgaaga agcatgaaac 1800

tcagtctctc gccctggatg catgttctcg ggatgaaggg gcagtgatct cccagatttc 1860

agacatttct aatagggatg gccatgctac tgatgaggag aaactggcat ccacgtcatg 1920

tggtcagaaa tcagctggtg ccgaggtgaa aggtgagcca gaggaagacc tggagtactt 1980

tgaatgttcc aatgttcctg tgtctaccat aaatcatgcg ttttcatcct cagaagcagg 2040

catagagaag gagacgtgcc agaagatgga agaagacggg tccactgtgc ttgggctgct 2100

ggagtcctct gcagagaagg cccctgtgtc ggtgtcctgt ggaggtgaga gccccctgga 2160

tgggatctgc ctcagcgaat cagacaagac agccgtgctc accttaataa gagaagagat 2220

aattactaaa gagattgaag caaatgaatg gaagaagaaa tacgaagaga cccggcaaga 2280

agttttggag atgaggaaaa ttgtagctga atatgaaaag actattgctc aaatgattga 2340

agatgaacaa aggacaagta tgacctctca gaagagcttc cagcaactga ccatggagaa 2400

ggaacaggcc ctggctgacc ttaactctgt ggaaaggtcc ctttctgatc tcttcaggag 2460

atatgagaac ctgaaaggtg ttctggaagg gttcaagaag aatgaagaag ccttgaagaa 2520

atgtgctcag gattacttag ccagagttaa acaagaggag cagcgatacc aggccctgaa 2580

aatccacgca gaagagaaac tggacaaagc caatgaagag attgctcagg ttcgaacaaa 2640

agcaaaggct gagagtgcag ctctccatgc tggactccgc aaagagcaga tgaaggtgga 2700

gtccctggaa agggccctgc agcagaagaa ccaagaaatt gaagaactga caaaaatctg 2760

tgatgagctg attgcaaagc tgggaaagac tgactgagac actccccctg ttagctcaac 2820

agatctgcat ttggctgctt ctcttgtgac cacaattatc ttgccttatc caggaataat 2880

tgcccctttg cagagaaaaa aaaaaactta aaaaaagcac atgcctactg ctgcctgtcc 2940

cgctttgctg ccaatgcaac agccctggaa gaaaccctag agggttgcat agtctagaaa 3000

ggagtgtgac ctgacagtgc tggagcctcc tagtttcccc ctatgaaggt tcccttaggc 3060

tgctgagttt gggtttgtga tttatcttta gtttgtttta aagtcatctt tactttccca 3120

aatgtgttaa atttgtaact cctctttggg gtcttctcca ccacctgtct gatttttttg 3180

tgatctgttt aatcttttaa ttttttagta tcagtggttt tatttaagga gacagtttgg 3240

cctattgtta cttccaattt ataatcaaga aggggctctg gatccccttt taaattacac 3300

acactctcac acacatacat gtatgtttat agatgctgct gctcttttcc ctgaagcata 3360

gtcaagtaag aactgctcta cagaaggaca tatttccttg gatgtgagac cctattttga 3420

aatagagtcc tgactcagaa caccaactta agaatttggg ggattaaaga tgtgaagacc 3480

acagtcttgg gttttcatat ctggagaaga ctatttgcca tgacgttttg ttgccctggt 3540

atttggacac tcctcagctt taatgggtgt ggccccctta gggttagtcc tcagactaat 3600

gatagtgtct gctttctgca tgaacggcaa tatgggactc cctccaagct agggtttggc 3660

aagtctgccc tagagtcatt tactctcctc tgcctccatt tgttaataca gaatcaacat 3720

ttagtcttca ttatcttttt tttttttttt gagacagagt ttcgatctat tttaagtatg 3780

tgaagaaaat ctacttgtaa aaggctcaga tcttaattaa aaggtaattg tagcacatta 3840

ccaattataa ggtgaagaaa tgtttttttc ccaagtgtga tgcattgttc ttcagatgtt 3900

gaaaagaaag caaaaaatac cttctaactt aagacagaat ttttaacaaa atgagcagta 3960

aaagtcacat gaaccactcc aaaaatcagt gcattttgca tatttttaaa caaagacagc 4020

ttgttgaata ctgagaagag gagtgcaagg agaaggtctg tactaacaaa gccaaattcc 4080

tcaagctctt actggactca gttcagagtg gtgggccatt aaccccaaca tggaattttt 4140

ccatataaat ctcaatgaat tccctttcat ttgaataggc aaacccaaat ccatgcaagt 4200

gttttaaagc actgtcctgt cttaatctta catgctgaaa gtcttcatgg tgatatgcac 4260

tatattcagt atacgtatgt tttcctactt ctcttgtaaa actgttgcat gatccaactt 4320

cagcaatgaa ttgtgcctag tggagaacct ctatagatct taaaaaatga attattcttt 4380

agcagtgtat tactcacatg ggtgcaatct ttagccccag ggaggtcaat aatgtctttt 4440

aaagccagaa gtcacatttt accaatatgc atttatcata attggtgctt aggctgtata 4500

ttcaagcctg ttgtcttaac attttgtata aaaaagaaca acagaaatta tctgtcattt 4560

gagaagtggc ttgacaatca tttgagcttt gaaagcagtc actgtggtgt aatatgaatg 4620

ctgtcctagt ggtcatagta ccaagggcac gtgtctcccc ttggtataac tgatttcctt 4680

tttagtcctc tactgctaaa taagttaatt ttgcattttg cagaaagaaa cattgattgc 4740

taaatctttt tgctgctgtg ttttggtgtt ttcatgttta cttgttttat attgatctgt 4800

tttaagtatg agaggcttat agtgccctcc attgtaaatc catagtcatc tttttaagct 4860

tattgtgttt aagaaagtag ctatgtgtta aacagaggtg atggcagccc ttccctagca 4920

cactggtgga agagacccct taagaacctg accccagtga atgaagctga tgcacaggga 4980

gcaccaaagg accttcgtta agtgataatt gtcctggcct ctcagccatg accgttatga 5040

ggaaatatcc cccattcgaa cttaacagat gcctcctctc caaagagaat taaaatcgta 5100

gcttgtacag atcaagagaa tatactgggc agaatgaagt atgtttgttt atttttcttt 5160

aaaaataaag gattttggaa ctctggagag taagaatata gtatagagtt tgcctcaaca 5220

catgtgaggg ccaaataacc tgctagctag gcagtaataa actctgttac agaagagaaa 5280

aagggccggg cacagtggct tattcctgta atcccaacac tgtggaaggc cgaggcagga 5340

ggatcacttg agtccaggag tttgaaacct acctaggcaa catggtgaaa ccttgtctct 5400

accaaaataa aaattagctg ggcatggtgg cacgtgcctg tggtcccagc tacttgggag 5460

gctgaggtgg gagcctggga ggtcaaggct gcagtgagcc atgatcatgc cactgcactc 5520

catcctgggt gacagcaaga tcttgtctca aaaaaaaaaa aaaaaaaaaa aaaaccagga 5580

gtgaaaaagg aaagtagaag gcagctgctg gcctagatgt tggtttggga atattaggtg 5640

atcctgttga gattctggat ccagagcaat ttctttagct tttgactttg ccaaagtgta 5700

gatagccttt atccagcagt attttaagtg gggaatgcaa cgtgaggcca actgaacaat 5760

tccccccgtg gctgcccaga tagtcacagt caaggttgga gagtctcctt ccagccagtg 5820

acctacccaa accttttgtt ctgtaaaact gctctggaaa taccgggaag cccagttttc 5880

tcacgtggtt tctagcttct tcagactcag cccaaattag gaagtgcaga agcacatgat 5940

ggtgaaaaac ctaggatttg gcagccttcc agaatggtat ggaatctgag ggaagattta 6000

tgtttcgttt tggaggatag ctcaagttga attttctttc cagccagtta ccctttcaac 6060

ctacccatac tttgtacaac tcttacacaa atacttagat atttattaga tagccctgaa 6120

ttcactctaa ttataaacag ggagtgtaaa ctgcccccag atgttcctgg gctgggtaaa 6180

agcagctgga gtgaagcact cattttccat aaaggtaaca aagggcagct cagtggttac 6240

tcaagctcaa aagggttttt ttaagagcaa gcattggtta agtctgtgta tactgagttg 6300

gaagtgattt cagcacattc ttttttagtg gagtgaaagt tctgaagccc ccttttaact 6360

tcctcttggt ttttcattat aattggtagc catctcatga actgtctctg actgttgtct 6420

ctttgtggtc atgtgattgt gagcttgctt tctgacttgc atttctgact ttatcctgtt 6480

gttaggaaga tagaaactag gttttgaaag attacatgat tcaagcgagg gattttaaag 6540

taaagatgta tttattctga agaatctaaa agataacaga ttatttgctt atgaaagaac 6600

aatatagtct gggaatccca gaatgtcaag ccaaaggtct aagaagtcat ctccttcaaa 6660

tactttaata aagaagtatt tcgaggagat atctgtccaa aaaggtttga ctggcctcca 6720

gattccagtt atttttaaaa agcaacttac cactaaatcc ttgagtctcc atagagtaac 6780

agtaaagaaa ctgatgtaac agactctcct ctcaaaggat ctcctctgga agagactatc 6840

agcggcagca ttctccaggg aagacccatc ccctagtgcc agagcttgca tcctggagac 6900

taaagattgc acttttttgt agttttttgt ccaaatgcaa tcccatttct gtgcctctta 6960

gcatgcagtt agatttggac aaacaagatt cctaaggaat gactttatta actataatat 7020

ggttacagct attatataaa tatatattct ggttatagtt ctaatatgga gatgttgtgt 7080

gcaatgctgg cctgtggtgg tctgtgtaat gctttaactt gtatggagga ggccaggctc 7140

agagctgaga tgtggcctga accttccctg tatcgatcct ttaatttaga actgtcaaga 7200

tgtcactttc tccccctctg ccttttagtg gtatctgaca tatactcaaa acagtaattt 7260

cctggtcaca tcattaactg ctaattctgt atttataaag aattttcaga tggacatgta 7320

caaatttgaa ctcaaaccat ccccagtcca gatacagggc agcgtgtagg tgaccacacc 7380

agagcctcag cctcggtcct tctcagccgt cgggatagga tccaggcatt tcttttaaat 7440

ctcagaggta gcagtaaact tttcagtatt gctgttagca agtgtgtgtt tgccaataga 7500

tacccattat actaatgtgc caagtaaatg ttcattgcac atctgcttcc actgtgttcc 7560

cacgggtgcc atgaagtgtg tgaggagccc ctcatctgga gggatgagtg ctgcgttgac 7620

tactgctatc aggattgtgt tgtgtggaat attcatctac ataaatttta tatgcacagt 7680

aatttccctt tttatatgtc aagtaactat ttgtaaaagt tatactcaca aattattata 7740

atgattacta atatattttt tccatgtttc attgcctgaa taaaaactgt ttaccactgt 7800

ta 7802

<210> 150
<211> 907
<212> PRT
<213> Artificial Sequence

<220>

<223> Description of Artificial Sequence: Synthetic
      polypeptide

<400> 150
Met Trp Ser Trp Lys Cys Leu Leu Phe Trp Ala Val Leu Val Thr Ala
1               5                   10                  15

Thr Leu Cys Thr Ala Arg Pro Ser Pro Thr Leu Pro Glu Gln Asp Ala
            20                  25                  30

Leu Pro Ser Ser Glu Asp Asp Asp Asp Asp Asp Asp Ser Ser Ser Glu
        35                  40                  45

Glu Lys Glu Thr Asp Asn Thr Lys Pro Asn Pro Val Ala Pro Tyr Trp
    50                  55                  60

Thr Ser Pro Glu Lys Met Glu Lys Lys Leu His Ala Val Pro Ala Ala
65                  70                  75                  80

Lys Thr Val Lys Phe Lys Cys Pro Ser Ser Gly Thr Pro Asn Pro Thr
                85                  90                  95

Leu Arg Trp Leu Lys Asn Gly Lys Glu Phe Lys Pro Asp His Arg Ile
            100                 105                 110

Gly Gly Tyr Lys Val Arg Tyr Ala Thr Trp Ser Ile Ile Met Asp Ser
        115                 120                 125

Val Val Pro Ser Asp Lys Gly Asn Tyr Thr Cys Ile Val Glu Asn Glu
    130                 135                 140

Tyr Gly Ser Ile Asn His Thr Tyr Gln Leu Asp Val Val Glu Arg Ser
145                 150                 155                 160

Pro His Arg Pro Ile Leu Gln Ala Gly Leu Pro Ala Asn Lys Thr Val
                165                 170                 175

Ala Leu Gly Ser Asn Val Glu Phe Met Cys Lys Val Tyr Ser Asp Pro
            180                 185                 190

Gln Pro His Ile Gln Trp Leu Lys His Ile Glu Val Asn Gly Ser Lys
        195                 200                 205

Ile Gly Pro Asp Asn Leu Pro Tyr Val Gln Ile Leu Lys Thr Ala Gly
    210                 215                 220

Val Asn Thr Thr Asp Lys Glu Met Glu Val Leu His Leu Arg Asn Val
225                 230                 235                 240

Ser Phe Glu Asp Ala Gly Glu Tyr Thr Cys Leu Ala Gly Asn Ser Ile
                245                 250                 255

Gly Leu Ser His His Ser Ala Trp Leu Thr Val Leu Glu Ala Leu Glu
            260                 265                 270

Glu Arg Pro Ala Val Met Thr Ser Pro Leu Tyr Leu Glu Ile Ile Ile
        275                 280                 285

Tyr Cys Thr Gly Ala Phe Leu Ile Ser Cys Met Val Gly Ser Val Ile
    290                 295                 300

Val Tyr Lys Met Lys Ser Gly Thr Lys Lys Ser Asp Phe His Ser Gln
305                 310                 315                 320

Met Ala Val His Lys Leu Ala Lys Ser Ile Pro Leu Arg Arg Gln Val
                325                 330                 335

Thr Val Ser Ala Asp Ser Ser Ala Ser Met Asn Ser Gly Val Leu Leu
            340                 345                 350

Val Arg Pro Ser Arg Leu Ser Ser Ser Gly Thr Pro Met Leu Ala Gly
        355                 360                 365

Val Ser Glu Tyr Glu Leu Pro Glu Asp Pro Arg Trp Glu Leu Pro Arg
    370                 375                 380

Asp Arg Leu Val Leu Gly Lys Pro Leu Gly Glu Gly Cys Phe Gly Gln
385                 390                 395                 400

Val Val Leu Ala Glu Ala Ile Gly Leu Asp Lys Asp Lys Pro Asn Arg
                405                 410                 415

Val Thr Lys Val Ala Val Lys Met Leu Lys Ser Asp Ala Thr Glu Lys
            420                 425                 430

Asp Leu Ser Asp Leu Ile Ser Glu Met Glu Met Met Lys Met Ile Gly
        435                 440                 445

Lys His Lys Asn Ile Ile Asn Leu Leu Gly Ala Cys Thr Gln Asp Gly
    450                 455                 460

Pro Leu Tyr Val Ile Val Glu Tyr Ala Ser Lys Gly Asn Leu Arg Glu
465                 470                 475                 480

Tyr Leu Gln Ala Arg Arg Pro Pro Gly Leu Glu Tyr Cys Tyr Asn Pro
                485                 490                 495

Ser His Asn Pro Glu Glu Gln Leu Ser Ser Lys Asp Leu Val Ser Cys
                500                 505                 510

Ala Tyr Gln Val Ala Arg Gly Met Glu Tyr Leu Ala Ser Lys Lys Cys
        515                 520                 525

Ile His Arg Asp Leu Ala Ala Arg Asn Val Leu Val Thr Glu Asp Asn
    530                 535                 540

Val Met Lys Ile Ala Asp Phe Gly Leu Ala Arg Asp Ile His His Ile
545                 550                 555                 560

Asp Tyr Tyr Lys Lys Thr Thr Asn Gly Arg Leu Pro Val Lys Trp Met
                565                 570                 575

Ala Pro Glu Ala Leu Phe Asp Arg Ile Tyr Thr His Gln Ser Asp Val
                580                 585                 590

Trp Ser Phe Gly Val Leu Leu Trp Glu Ile Phe Thr Leu Gly Gly Ser
        595                 600                 605

Pro Tyr Pro Gly Val Pro Val Glu Glu Leu Phe Lys Leu Leu Lys Glu
    610                 615                 620

Gly His Arg Met Asp Lys Pro Ser Asn Cys Thr Asn Glu Leu Tyr Met
625                 630                 635                 640

Met Met Arg Asp Cys Trp His Ala Val Pro Ser Gln Arg Pro Thr Phe
                645                 650                 655

Lys Gln Leu Val Glu Asp Leu Asp Arg Ile Val Ala Leu Thr Ser Asn
                660                 665                 670

Gln Gly Leu Leu Glu Ser Ser Ala Glu Lys Ala Pro Val Ser Val Ser
        675                 680                 685

Cys Gly Gly Glu Ser Pro Leu Asp Gly Ile Cys Leu Ser Glu Ser Asp
    690                 695                 700

Lys Thr Ala Val Leu Thr Leu Ile Arg Glu Glu Ile Ile Thr Lys Glu
705                 710                 715                 720

Ile Glu Ala Asn Glu Trp Lys Lys Lys Tyr Glu Glu Thr Arg Gln Glu
                725                 730                 735

Val Leu Glu Met Arg Lys Ile Val Ala Glu Tyr Glu Lys Thr Ile Ala

740                 745                 750

Gln Met Ile Glu Asp Glu Gln Arg Thr Ser Met Thr Ser Gln Lys Ser
        755                 760                 765

Phe Gln Gln Leu Thr Met Glu Lys Glu Gln Ala Leu Ala Asp Leu Asn
    770                 775                 780

Ser Val Glu Arg Ser Leu Ser Asp Leu Phe Arg Arg Tyr Glu Asn Leu
785                 790                 795                 800

Lys Gly Val Leu Glu Gly Phe Lys Lys Asn Glu Glu Ala Leu Lys Lys
                805                 810                 815

Cys Ala Gln Asp Tyr Leu Ala Arg Val Lys Gln Glu Glu Gln Arg Tyr
            820                 825                 830

Gln Ala Leu Lys Ile His Ala Glu Glu Lys Leu Asp Lys Ala Asn Glu
        835                 840                 845

Glu Ile Ala Gln Val Arg Thr Lys Ala Lys Ala Glu Ser Ala Ala Leu
    850                 855                 860

His Ala Gly Leu Arg Lys Glu Gln Met Lys Val Glu Ser Leu Glu Arg
865                 870                 875                 880

Ala Leu Gln Gln Lys Asn Gln Glu Ile Glu Glu Leu Thr Lys Ile Cys
                885                 890                 895

Asp Glu Leu Ile Ala Lys Leu Gly Lys Thr Asp
            900                 905

<210> 151
<211> 2989
<212> DNA
<213> Artificial Sequence

<220>
<223> Description of Artificial Sequence: Synthetic
      polynucleotide

<400> 151
atgtggagct ggaagtgcct cctcttctgg gctgtgctgg tcacagccac actctgcacc      60

gctaggccgt ccccgacctt gcctgaacaa gcccagccct ggggagcccc tgtggaagtg     120

gagtccttcc tggtccaccc cggtgacctg ctgcagcttc gctgtcggct gcgggacgat     180

gtgcagagca tcaactggct gcgggacggg gtgcagctgg cggaaagcaa ccgcacccgc     240

atcacagggg aggaggtgga ggtgcaggac tccgtgcccg cagactccgg cctctatgct     300

tgcgtaacca gcagcccctc gggcagtgac accacctact tctccgtcaa tgtttcagat 360

gctctcccct cctcggagga tgatgatgat gatgatgact cctcttcaga ggagaaagaa 420

acagataaca ccaaaccaaa ccgtatgccc gtagctccat attggacatc cccagaaaag 480

atggaaaaga aattgcatgc agtgccggct gccaagacag tgaagttcaa atgcccttcc 540

agtgggaccc caaaccccac actgcgctgg ttgaaaaatg gcaaagaatt caaacctgac 600

cacagaattg gaggctacaa ggtccgttat gccacctgga gcatcataat ggactctgtg 660

gtgccctctg acaagggcaa ctacacctgc attgtggaga atgagtacgg cagcatcaac 720

cacacatacc agctggatgt cgtggagcgg tcccctcacc ggcccatcct gcaagcaggg 780

ttgcccgcca acaaaacagt ggccctgggt agcaacgtgg agttcatgtg taaggtgtac 840

agtgacccgc agccgcacat ccagtggcta aagcacatcg aggtgaatgg gagcaagatt 900

ggcccagaca acctgcctta tgtccagatc ttgaagactg ctggagttaa taccaccgac 960

aaagagatgg aggtgcttca cttaagaaat gtctcctttg aggacgcagg ggagtatacg 1020

tgcttggcgg gtaactctat cggactctcc catcactctg catggttgac cgttctggaa 1080

gccctggaag agaggccggc agtgatgacc tcgcccctgt acctggagat catcatctat 1140

tgcacagggg ccttcctcat ctcctgcatg gtggggtcgg tcatcgtcta caagatgaag 1200

agtggtacca agaagagtga cttccacagc cagatggctg tgcacaagct ggccaagagc 1260

atccctctgc gcagacaggt gtctgctgac tccagtgcat ccatgaactc tggggttctt 1320

ctggttcggc catcacggct ctcctccagt gggactccca tgctagcagg ggtctctgag 1380

tatgagcttc ccgaagaccc tcgctgggag ctgcctcggg acagactggt cttaggcaaa 1440

cccctgggag agggctgctt tgggcaggtg gtgttggcag aggctatcgg gctggacaag 1500

gacaaaccca accgtgtgac caaagtggct gtgaagatgt tgaagtcgga cgcaacagag 1560

aaagacttgt cagacctgat ctcagaaatg gagatgatga agatgatcgg gaagcataag 1620

aatatcatca acctgctggg ggcctgcacg caggatggtc ccttgtatgt catcgtggag 1680

tatgcctcca agggcaacct gcgggagtac ctgcaggccc ggaggccccc agggctggaa 1740

tactgctaca accccagcca caacccagag gagcagctct cctccaagga cctggtgtcc 1800

tgcgcctacc aggtggcccg aggcatggag tatctggcct ccaagaagtg catacaccga 1860

gacctggcag ccaggaatgt cctggtgaca gaggacaatg tgatgaagat agcagacttt 1920

ggcctcgcac gggacattca ccacatcgac tactataaaa agacaaccaa cggccgactg 1980

cctgtgaagt ggatggcacc cgaggcatta tttgaccgga tctacaccca ccagagtgat 2040

gtgtggtctt tcggggtgct cctgtgggag atcttcactc tgggcggctc cccatacccc 2100

ggtgtgcctg tggaggaact tttcaagctg ctgaaggagg gtcaccgcat ggacaagccc 2160

agtaactgca ccaacgagct gtacatgatg atgcgggact gctggcatgc agtgccctca 2220

cagagaccca ccttcaagca gctggtggaa gacctggacc gcatcgtggc cttgacctcc 2280

aaccagtggg ctgctggagt cctctgcaga gaaggcccct gtgtcggtgt cctgtggagg 2340

tgagagcccc ctggatggga tctgcctcag cgaatcagac aagacagccg tgctcacctt 2400

aataagagaa gagataatta ctaaagagat tgaagcaaat gaatggaaga agaaatacga 2460

agagacccgg caagaagttt tggagatgag gaaaattgta gctgaatatg aaaagactat 2520

tgctcaaatg attgaagatg aacaaaggac aagtatgacc tctcagaaga gcttccagca 2580

actgaccatg gagaaggaac aggccctggc tgaccttaac tctgtggaaa ggtccctttc 2640

tgatctcttc aggagatatg agaacctgaa aggtgttctg gaagggttca agaagaatga 2700

agaagccttg aagaaatgtg ctcaggatta cttagccaga gttaaacaag aggagcagcg 2760

ataccaggcc ctgaaaatcc acgcagaaga gaaactggac aaagccaatg aagagattgc 2820

tcaggttcga acaaaagcaa aggctgagag tgcagctctc catgctggac tccgcaaaga 2880

gcagatgaag gtggagtccc tggaaagggc cctgcagcag aagaaccaag aaattgaaga 2940

actgacaaaa atctgtgatg agctgattgc aaagctggga aagactgac 2989

<210> 152
<211> 821
<212> PRT
<213> Homo sapiens

<400> 152

<210> 153
<211> 4654
<212> DNA
<213> Homo sapiens

<400> 153
ggcggcggct ggaggagagc gcggtggaga gccgagcggg cgggcggcgg gtgcggagcg      60

ggcgagggag cgcgcgcggc cgccacaaag ctcgggcgcc gcggggctgc atgcggcgta     120

cctggcccgg cgcggcgact gctctccggg ctggcggggg ccggccgcga gccccggggg 180

ccccgaggcc gcagcttgcc tgcgcgctct gagccttcgc aactcgcgag caaagtttgg 240

tggaggcaac gccaagcctg agtcctttct tcctctcgtt ccccaaatcc gagggcagcc 300

cgcgggcgtc atgcccgcgc tcctccgcag cctggggtac gcgtgaagcc cgggaggctt 360

ggcgccggcg aagacccaag gaccactctt ctgcgtttgg agttgctccc cgcaaccccg 420

ggctcgtcgc tttctccatc ccgacccacg cggggcgcgg ggacaacaca ggtcgcggag 480

gagcgttgcc attcaagtga ctgcagcagc agcggcagcg cctcggttcc tgagcccacc 540

gcaggctgaa ggcattgcgc gtagtccatg cccgtagagg aagtgtgcag atgggattaa 600

cgtccacatg gagatatgga agaggaccgg ggattggtac cgtaaccatg gtcagctggg 660

gtcgtttcat ctgcctggtc gtggtcacca tggcaacctt gtccctggcc cggccctcct 720

tcagtttagt tgaggatacc acattagagc cagaagagcc accaaccaaa taccaaatct 780

ctcaaccaga agtgtacgtg gctgcgccag gggagtcgct agaggtgcgc tgcctgttga 840

aagatgccgc cgtgatcagt tggactaagg atggggtgca cttggggccc aacaatagga 900

cagtgcttat tggggagtac ttgcagataa agggcgccac gcctagagac tccggcctct 960

atgcttgtac tgccagtagg actgtagaca gtgaaacttg gtacttcatg gtgaatgtca 1020

cagatgccat ctcatccgga gatgatgagg atgacaccga tggtgcggaa gattttgtca 1080

gtgagaacag taacaacaag agagcaccat actggaccaa cacagaaaag atggaaaagc 1140

ggctccatgc tgtgcctgcg gccaacactg tcaagtttcg ctgcccagcc ggggggaacc 1200

caatgccaac catgcggtgg ctgaaaaacg ggaaggagtt taagcaggag catcgcattg 1260

gaggctacaa ggtacgaaac cagcactgga gcctcattat ggaaagtgtg gtcccatctg 1320

acaagggaaa ttatacctgt gtagtggaga atgaatacgg gtccatcaat cacacgtacc 1380

acctggatgt tgtggagcga tcgcctcacc ggcccatcct ccaagccgga ctgccggcaa 1440

atgcctccac agtggtcgga ggagacgtag agtttgtctg caaggtttac agtgatgccc 1500

agccccacat ccagtggatc aagcacgtgg aaaagaacgg cagtaaatac gggcccgacg 1560

ggctgcccta cctcaaggtt ctcaaggccg ccggtgttaa caccacggac aaagagattg 1620

aggttctcta tattcggaat gtaacttttg aggacgctgg ggaatatacg tgcttggcgg 1680

gtaattctat tgggatatcc tttcactctg catggttgac agttctgcca gcgcctggaa 1740

gagaaaagga gattacagct tccccagact acctggagat agccatttac tgcatagggg 1800

tcttcttaat cgcctgtatg gtggtaacag tcatcctgtg ccgaatgaag aacacgacca 1860

agaagccaga cttcagcagc cagccggctg tgcacaagct gaccaaacgt atccccctgc 1920

ggagacaggt aacagtttcg gctgagtcca gctcctccat gaactccaac accccgctgg 1980

tgaggataac aacacgcctc tcttcaacgg cagacacccc catgctggca ggggtctccg 2040

agtatgaact tccagaggac ccaaaatggg agtttccaag agataagctg acactgggca 2100

agcccctggg agaaggttgc tttgggcaag tggtcatggc ggaagcagtg ggaattgaca 2160

aagacaagcc caaggaggcg gtcaccgtgg ccgtgaagat gttgaaagat gatgccacag 2220

agaaagacct ttctgatctg gtgtcagaga tggagatgat gaagatgatt gggaaacaca 2280

agaatatcat aaatcttctt ggagcctgca cacaggatgg gcctctctat gtcatagttg 2340

agtatgcctc taaaggcaac ctccgagaat acctccgagc ccggaggcca cccgggatgg 2400

agtactccta tgacattaac cgtgttcctg aggagcagat gaccttcaag gacttggtgt 2460

catgcaccta ccagctggcc agaggcatgg agtacttggc ttcccaaaaa tgtattcatc 2520

gagatttagc agccagaaat gttttggtaa cagaaaacaa tgtgatgaaa atagcagact 2580

ttggactcgc cagagatatc aacaatatag actattacaa aaagaccacc aatgggcggc 2640

ttccagtcaa gtggatggct ccagaagccc tgtttgatag agtatacact catcagagtg 2700

atgtctggtc cttcggggtg ttaatgtggg agatcttcac tttagggggc tcgccctacc 2760

cagggattcc cgtggaggaa ctttttaagc tgctgaagga aggacacaga atggataagc 2820

cagccaactg caccaacgaa ctgtacatga tgatgaggga ctgttggcat gcagtgccct 2880

cccagagacc aacgttcaag cagttggtag aagacttgga tcgaattctc actctcacaa 2940

ccaatgagga atacttggac ctcagccaac ctctcgaaca gtattcacct agttaccctg 3000

acacaagaag ttcttgttct tcaggagatg attctgtttt ttctccagac cccatgcctt 3060

acgaaccatg ccttcctcag tatccacaca taaacggcag tgttaaaaca tgaatgactg 3120

tgtctgcctg tccccaaaca ggacagcact gggaacctag ctacactgag cagggagacc 3180

atgcctccca gagcttgttg tctccacttg tatatatgga tcagaggagt aaataattgg 3240

aaaagtaatc agcatatgtg taaagattta tacagttgaa aacttgtaat cttccccagg 3300

aggagaagaa ggtttctgga gcagtggact gccacaagcc accatgtaac ccctctcacc 3360

tgccgtgcgt actggctgtg gaccagtagg actcaaggtg gacgtgcgtt ctgccttcct 3420

tgttaatttt gtaataattg gagaagattt atgtcagcac acacttacag agcacaaatg 3480

cagtatatag gtgctggatg tatgtaaata tattcaaatt atgtataaat atatattata 3540

tatttacaag gagttatttt ttgtattgat tttaaatgga tgtcccaatg cacctagaaa 3600

attggtctct ctttttttaa tagctatttg ctaaatgctg ttcttacaca taatttctta 3660

attttcaccg agcagaggtg gaaaaatact tttgctttca gggaaaatgg tataacgtta 3720

atttattaat aaattggtaa tatacaaaac aattaatcat ttatagtttt ttttgtaatt 3780

taagtggcat ttctatgcag gcagcacagc agactagtta atctattgct tggacttaac 3840

tagttatcag atcctttgaa aagagaatat ttacaatata tgactaattt ggggaaaatg 3900

aagttttgat ttatttgtgt ttaaatgctg ctgtcagacg attgttctta gacctcctaa 3960

atgccccata ttaaaagaac tcattcatag gaaggtgttt cattttggtg tgcaaccctg 4020

tcattacgtc aacgcaacgt ctaactggac ttcccaagat aaatggtacc agcgtcctct 4080

taaaagatgc cttaatccat tccttgagga cagaccttag ttgaaatgat agcagaatgt 4140

gcttctctct ggcagctggc cttctgcttc tgagttgcac attaatcaga ttagcctgta 4200

ttctcttcag tgaattttga taatggcttc cagactcttt ggcgttggag acgcctgtta 4260

ggatcttcaa gtcccatcat agaaaattga aacacagagt tgttctgctg atagttttgg 4320

ggatacgtcc atctttttaa gggattgctt tcatctaatt ctggcaggac ctcaccaaaa 4380

gatccagcct catacctaca tcagacaaaa tatcgccgtt gttccttctg tactaaagta 4440

ttgtgttttg ctttggaaac acccactcac tttgcaatag ccgtgcaaga tgaatgcaga 4500

ttacactgat cttatgtgtt acaaaattgg agaaagtatt taataaaacc tgttaatttt 4560

tatactgaca ataaaaatgt ttctacagat attaatgtta acaagacaaa ataaatgtca 4620

cgcaacttat ttttttaata aaaaaaaaaa aaaa 4654

<210> 154
<211> 2948
<212> PRT
<213> Homo sapiens

<400> 154
Met Gly Asn Glu Asn Ser Thr Ser Asp Asn Gln Arg Thr Leu Ser Ala
1               5                   10                  15

Gln Thr Pro Arg Ser Ala Gln Pro Pro Gly Asn Ser Gln Asn Ile Lys
            20                  25                  30

Arg Lys Gln Gln Asp Thr Pro Gly Ser Pro Asp His Arg Asp Ala Ser
        35                  40                  45

Ser Ile Gly Ser Val Gly Leu Gly Gly Phe Cys Thr Ala Ser Glu Ser
    50                  55                  60

Ser Ala Ser Leu Asp Pro Cys Leu Val Ser Pro Glu Val Thr Glu Pro
65                  70                  75                  80

Arg Lys Asp Pro Gln Gly Ala Arg Gly Pro Glu Gly Ser Leu Leu Pro
                85                  90                  95

Ser Pro Pro Pro Ser Gln Glu Arg Glu His Pro Ser Ser Ser Met Pro
            100                 105                 110

Phe Ala Glu Cys Pro Pro Glu Gly Cys Leu Ala Ser Pro Ala Ala Ala
        115                 120                 125

Pro Glu Asp Gly Pro Gln Thr Gln Ser Pro Arg Arg Glu Pro Ala Pro
    130                 135                 140

Asn Ala Pro Gly Asp Ile Ala Ala Ala Phe Pro Ala Glu Arg Asp Ser
145                 150                 155                 160

Ser Thr Pro Tyr Gln Glu Ile Ala Ala Val Pro Ser Ala Gly Arg Glu
                165                 170                 175

Arg Gln Pro Lys Glu Glu Gly Gln Lys Ser Ser Phe Ser Phe Ser Ser
            180                 185                 190

Gly Ile Asp Gln Ser Pro Gly Met Ser Pro Val Pro Leu Arg Glu Pro
        195                 200                 205

Met Lys Ala Pro Leu Cys Gly Glu Gly Asp Gln Pro Gly Gly Phe Glu
    210                 215                 220

Ser Gln Glu Lys Glu Ala Ala Gly Gly Phe Pro Pro Ala Glu Ser Arg
225                 230                 235                 240

Gln Gly Val Ala Ser Val Gln Val Thr Pro Glu Ala Pro Ala Ala Ala
                245                 250                 255

Gln Gln Gly Thr Glu Ser Ser Ala Val Leu Glu Lys Ser Pro Leu Lys
            260                 265                 270

Pro Met Ala Pro Ile Pro Gln Asp Pro Ala Pro Arg Ala Ser Asp Arg
        275                 280                 285

Glu Arg Gly Gln Gly Glu Ala Pro Pro Gln Tyr Leu Thr Asp Asp Leu
    290                 295                 300

Glu Phe Leu Arg Ala Cys His Leu Pro Arg Ser Asn Ser Gly Ala Ala
305                 310                 315                 320

Pro Glu Ala Glu Val Asn Ala Ala Ser Gln Glu Ser Cys Gln Gln Pro
                325                 330                 335

Val Gly Ala Tyr Leu Pro His Ala Glu Leu Pro Trp Gly Leu Pro Ser
            340                 345                 350

Pro Ala Leu Val Pro Glu Ala Gly Gly Ser Gly Lys Glu Ala Leu Asp
        355                 360                 365

Thr Ile Asp Val Gln Gly His Pro Gln Thr Gly Met Arg Gly Thr Lys

    370                 375                 380

Pro Asn Gln Val Val Cys Val Ala Ala Gly Gly Gln Pro Glu Gly Gly
385                 390                 395                 400

Leu Pro Val Ser Pro Glu Pro Ser Leu Leu Thr Pro Thr Glu Glu Ala
                405                 410                 415

His Pro Ala Ser Ser Leu Ala Ser Phe Pro Ala Ala Gln Ile Pro Ile
            420                 425                 430

Ala Val Glu Glu Pro Gly Ser Ser Ser Arg Glu Ser Val Ser Lys Ala
        435                 440                 445

Gly Met Pro Val Ser Ala Asp Ala Ala Lys Glu Val Val Asp Ala Gly
    450                 455                 460

Leu Val Gly Leu Glu Arg Gln Val Ser Asp Leu Gly Ser Lys Gly Glu
465                 470                 475                 480

His Pro Glu Gly Asp Pro Gly Glu Val Pro Ala Pro Ser Pro Gln Glu
                485                 490                 495

Arg Gly Glu His Leu Asn Thr Glu Gln Ser His Glu Val Gln Pro Gly
            500                 505                 510

Val Pro Pro Pro Pro Leu Pro Lys Glu Gln Ser His Glu Val Gln Pro
        515                 520                 525

Gly Ala Pro Pro Pro Pro Leu Pro Lys Ala Pro Ser Glu Ser Ala Arg
    530                 535                 540

Gly Pro Pro Gly Pro Thr Asp Gly Ala Lys Val His Glu Asp Ser Thr
545                 550                 555                 560

Ser Pro Ala Val Ala Lys Glu Gly Ser Arg Ser Pro Gly Asp Ser Pro
                565                 570                 575

Gly Gly Lys Glu Glu Ala Pro Glu Pro Pro Asp Gly Gly Asp Pro Gly
            580                 585                 590

Asn Leu Gln Gly Glu Asp Ser Gln Ala Phe Ser Ser Lys Arg Asp Pro
        595                 600                 605

Glu Val Gly Lys Asp Glu Leu Ser Lys Pro Ser Ser Asp Ala Glu Ser
    610                 615                 620

Arg Asp His Pro Ser Ser His Ser Ala Gln Pro Pro Arg Lys Gly Gly
625                 630                 635                 640

Ala Gly His Thr Asp Gly Pro His Ser Gln Thr Ala Glu Ala Asp Ala
                645                 650                 655

Ser Gly Leu Pro His Lys Leu Gly Glu Glu Asp Pro Val Leu Pro Pro
            660                 665                 670

Val Pro Asp Gly Ala Gly Glu Pro Thr Val Pro Glu Gly Ala Ile Trp
        675                 680                 685

Glu Gly Ser Gly Leu Gln Pro Lys Cys Pro Asp Thr Leu Gln Ser Arg
    690                 695                 700

Glu Gly Leu Gly Arg Met Glu Ser Phe Leu Thr Leu Glu Ser Glu Lys
705                 710                 715                 720

Ser Asp Phe Pro Pro Thr Pro Val Ala Glu Val Ala Pro Lys Ala Gln
                725                 730                 735

Glu Gly Glu Ser Thr Leu Glu Ile Arg Lys Met Gly Ser Cys Asp Gly
            740                 745                 750

Glu Gly Leu Leu Thr Ser Pro Asp Gln Pro Arg Gly Pro Ala Cys Asp
        755                 760                 765

Ala Ser Arg Gln Glu Phe His Ala Gly Val Pro His Pro Pro Gln Gly
    770                 775                 780

Glu Asn Leu Ala Ala Asp Leu Gly Leu Thr Ala Leu Ile Leu Asp Gln
785                 790                 795                 800

Asp Gln Gln Gly Ile Pro Ser Cys Pro Gly Glu Gly Trp Ile Arg Gly
                805                 810                 815

Ala Ala Ser Glu Trp Pro Leu Leu Ser Ser Glu Lys His Leu Gln Pro
            820                 825                 830

Ser Gln Ala Gln Pro Glu Thr Ser Ile Phe Asp Val Leu Lys Glu Gln
        835                 840                 845

Ala Gln Pro Pro Glu Asn Gly Lys Glu Thr Ser Pro Ser His Pro Gly
    850                 855                 860

Phe Lys Asp Gln Gly Ala Asp Ser Ser Gln Ile His Val Pro Val Glu
865                 870                 875                 880

Pro Gln Glu Asp Asn Asn Leu Pro Thr His Gly Gly Gln Glu Gln Ala
                885                 890                 895

Leu Gly Ser Glu Leu Gln Ser Gln Leu Pro Lys Gly Thr Leu Ser Asp
            900                 905                 910

Thr Pro Thr Ser Ser Pro Thr Asp Met Val Trp Glu Ser Ser Leu Thr
        915                 920                 925

Glu Glu Ser Glu Leu Ser Ala Pro Thr Arg Gln Lys Leu Pro Ala Leu
    930                 935                 940

Gly Glu Lys Arg Pro Glu Gly Ala Cys Gly Asp Gly Gln Ser Ser Arg
945                 950                 955                 960

Val Ser Pro Pro Ala Ala Asp Val Leu Lys Asp Phe Ser Leu Ala Gly
                965                 970                 975

Asn Phe Ser Arg Lys Glu Thr Cys Cys Thr Gly Gln Gly Pro Asn Lys
            980                 985                 990

Ser Gln Gln Ala Leu Ala Asp Ala  Leu Glu Glu Gly Ser  Gln His Glu
        995                 1000                1005

Glu Ala  Cys Gln Arg His Pro  Gly Ala Ser Glu Ala  Ala Asp Gly
    1010                1015                1020

Cys Ser  Pro Leu Trp Gly Leu  Ser Lys Arg Glu Met  Ala Ser Gly
    1025                1030                1035

Asn Thr  Gly Glu Ala Pro Pro  Cys Gln Pro Asp Ser  Val Ala Leu
    1040                1045                1050

Leu Asp  Ala Val Pro Cys Leu  Pro Ala Leu Ala Pro  Ala Ser Pro
    1055                1060                1065

Gly Val  Thr Pro Thr Gln Asp  Ala Pro Glu Thr Glu  Ala Cys Asp
    1070                1075                1080

Glu Thr  Gln Glu Gly Arg Gln  Gln Pro Val Pro Ala  Pro Gln Gln
    1085                1090                1095

Lys Met  Glu Cys Trp Ala Thr  Ser Asp Ala Glu Ser  Pro Lys Leu
    1100                1105                1110

Leu Ala  Ser Phe Pro Ser Ala  Gly Glu Gln Gly Gly  Glu Ala Gly
    1115                1120                1125

Ala Ala Glu Thr Gly Gly Ser Ala Gly Ala Gly Asp Pro Gly Lys
    1130                1135                1140

Gln Gln Ala Pro Glu Lys Pro Gly Glu Ala Thr Leu Ser Cys Gly
    1145                1150                1155

Leu Leu Gln Thr Glu His Cys Leu Thr Ser Gly Glu Glu Ala Ser
    1160                1165                1170

Thr Ser Ala Leu Arg Glu Ser Cys Gln Ala Glu His Pro Met Ala
    1175                1180                1185

Ser Cys Gln Asp Ala Leu Leu Pro Ala Arg Glu Leu Gly Gly Ile
    1190                1195                1200

Pro Arg Ser Thr Met Asp Phe Ser Thr His Gln Ala Val Pro Asp
    1205                1210                1215

Pro Lys Glu Leu Leu Leu Ser Gly Pro Pro Glu Val Ala Ala Pro
    1220                1225                1230

Asp Thr Pro Tyr Leu His Val Asp Ser Ala Ala Gln Arg Gly Ala
    1235                1240                1245

Glu Asp Ser Gly Val Lys Ala Val Ser Ser Ala Asp Pro Arg Ala
    1250                1255                1260

Pro Gly Glu Ser Pro Cys Pro Val Gly Glu Pro Pro Leu Ala Leu
    1265                1270                1275

Glu Asn Ala Ala Ser Leu Lys Leu Phe Ala Gly Ser Leu Ala Pro
    1280                1285                1290

Leu Leu Gln Pro Gly Ala Ala Gly Gly Glu Ile Pro Ala Val Gln
    1295                1300                1305

Ala Ser Ser Gly Ser Pro Lys Ala Arg Thr Thr Glu Gly Pro Val
    1310                1315                1320

Asp Ser Met Pro Cys Leu Asp Arg Met Pro Leu Leu Ala Lys Gly
    1325                1330                1335

Lys Gln Ala Thr Gly Glu Glu Lys Ala Ala Thr Ala Pro Gly Ala
    1340                1345                1350

Gly Ala Lys Ala Ser Gly Glu Gly Met Ala Gly Asp Ala Ala Gly

1355                1360                1365

Glu Thr Glu Gly Ser Met Glu Arg Met Gly Glu Pro Ser Gln Asp
    1370                1375                1380

Pro Lys Gln Gly Thr Ser Gly Gly Val Asp Thr Ser Ser Glu Gln
    1385                1390                1395

Ile Ala Thr Leu Thr Gly Phe Pro Asp Phe Arg Glu His Ile Ala
    1400                1405                1410

Lys Ile Phe Glu Lys Pro Val Leu Gly Ala Leu Ala Thr Pro Gly
    1415                1420                1425

Glu Lys Ala Gly Ala Gly Arg Ser Ala Val Gly Lys Asp Leu Thr
    1430                1435                1440

Arg Pro Leu Gly Pro Glu Lys Leu Leu Asp Gly Pro Pro Gly Val
    1445                1450                1455

Asp Val Thr Leu Leu Pro Ala Pro Pro Ala Arg Leu Gln Val Glu
    1460                1465                1470

Lys Lys Gln Gln Leu Ala Gly Glu Ala Glu Ile Ser His Leu Ala
    1475                1480                1485

Leu Gln Asp Pro Ala Ser Asp Lys Leu Leu Gly Pro Ala Gly Leu
    1490                1495                1500

Thr Trp Glu Arg Asn Leu Pro Gly Ala Gly Val Gly Lys Glu Met
    1505                1510                1515

Ala Gly Val Pro Pro Thr Leu Arg Glu Asp Glu Arg Pro Glu Gly
    1520                1525                1530

Pro Gly Ala Ala Trp Pro Gly Leu Glu Gly Gln Ala Tyr Ser Gln
    1535                1540                1545

Leu Glu Arg Ser Arg Gln Glu Leu Ala Ser Gly Leu Pro Ser Pro
    1550                1555                1560

Ala Ala Thr Gln Glu Leu Pro Val Glu Arg Ala Ala Ala Phe Gln
    1565                1570                1575

Val Ala Pro His Ser His Gly Glu Glu Ala Val Ala Gln Asp Arg
    1580                1585                1590

Ile Pro Ser Gly Lys Gln His Gln Glu Thr Ser Ala Cys Asp Ser
    1595                1600                1605

Pro His Gly Glu Asp Gly Pro Gly Asp Phe Ala His Thr Gly Val
    1610                1615                1620

Pro Gly His Val Pro Arg Ser Thr Cys Ala Pro Ser Pro Gln Arg
    1625                1630                1635

Glu Val Leu Thr Val Pro Glu Ala Asn Ser Glu Pro Trp Thr Leu
    1640                1645                1650

Asp Thr Leu Gly Gly Glu Arg Arg Pro Gly Val Thr Ala Gly Ile
    1655                1660                1665

Leu Glu Met Arg Asn Ala Leu Gly Asn Gln Ser Thr Pro Ala Pro
    1670                1675                1680

Pro Thr Gly Glu Val Ala Asp Thr Pro Leu Glu Pro Gly Lys Val
    1685                1690                1695

Ala Gly Ala Ala Gly Glu Ala Glu Gly Asp Ile Thr Leu Ser Thr
    1700                1705                1710

Ala Glu Thr Gln Ala Cys Ala Ser Gly Asp Leu Pro Glu Ala Gly
    1715                1720                1725

Thr Thr Arg Thr Phe Ser Val Val Ala Gly Asp Leu Val Leu Pro
    1730                1735                1740

Gly Ser Cys Gln Asp Pro Ala Cys Ser Asp Lys Ala Pro Gly Met
    1745                1750                1755

Glu Gly Thr Ala Ala Leu His Gly Asp Ser Pro Ala Arg Pro Gln
    1760                1765                1770

Gln Ala Lys Glu Gln Pro Gly Pro Glu Arg Pro Ile Pro Ala Gly
    1775                1780                1785

Asp Gly Lys Val Cys Val Ser Ser Pro Pro Glu Pro Asp Glu Thr
    1790                1795                1800

His Asp Pro Lys Leu Gln His Leu Ala Pro Glu Glu Leu His Thr
    1805                1810                1815

Asp Arg Glu Ser Pro Arg Pro Gly Pro Ser Met Leu Pro Ser Val
    1820                1825                1830

Pro Lys Lys Asp Ala Pro Arg Val Met Asp Lys Val Thr Ser Asp
    1835                1840                1845

Glu Thr Arg Gly Ala Glu Gly Thr Glu Ser Ser Pro Val Ala Asp
    1850                1855                1860

Asp Ile Ile Gln Pro Ala Ala Pro Ala Asp Leu Glu Ser Pro Thr
    1865                1870                1875

Leu Ala Ala Ser Ser Tyr His Gly Asp Val Val Gly Gln Val Ser
    1880                1885                1890

Thr Asp Leu Ile Ala Gln Ser Ile Ser Pro Ala Ala Ala His Ala
    1895                1900                1905

Gly Leu Pro Pro Ser Ala Ala Glu His Ile Val Ser Pro Ser Ala
    1910                1915                1920

Pro Ala Gly Asp Arg Val Glu Ala Ser Thr Pro Ser Cys Pro Asp
    1925                1930                1935

Pro Ala Lys Asp Leu Ser Arg Ser Ser Asp Ser Glu Glu Ala Phe
    1940                1945                1950

Glu Thr Pro Glu Ser Thr Thr Pro Val Lys Ala Pro Pro Ala Pro
    1955                1960                1965

Pro Pro Pro Pro Pro Glu Val Ile Pro Glu Pro Glu Val Ser Thr
    1970                1975                1980

Gln Pro Pro Pro Glu Glu Pro Gly Cys Gly Ser Glu Thr Val Pro
    1985                1990                1995

Val Pro Asp Gly Pro Arg Ser Asp Ser Val Glu Gly Ser Pro Phe
    2000                2005                2010

Arg Pro Pro Ser His Ser Phe Ser Ala Val Phe Asp Glu Asp Lys
    2015                2020                2025

Pro Ile Ala Ser Ser Gly Thr Tyr Asn Leu Asp Phe Asp Asn Ile
    2030                2035                2040

Glu Leu Val Asp Thr Phe Gln Thr Leu Glu Pro Arg Ala Ser Asp
    2045                2050                2055

Ala Lys Asn Gln Glu Gly Lys Val Asn Thr Arg Arg Lys Ser Thr
    2060                2065                2070

Asp Ser Val Pro Ile Ser Lys Ser Thr Leu Ser Arg Ser Leu Ser
    2075                2080                2085

Leu Gln Ala Ser Asp Phe Asp Gly Ala Ser Ser Ser Gly Asn Pro
    2090                2095                2100

Glu Ala Val Ala Leu Ala Pro Asp Ala Tyr Ser Thr Gly Ser Ser
    2105                2110                2115

Ser Ala Ser Ser Thr Leu Lys Arg Thr Lys Lys Pro Arg Pro Pro
    2120                2125                2130

Ser Leu Lys Lys Lys Gln Thr Thr Lys Lys Pro Thr Glu Thr Pro
    2135                2140                2145

Pro Val Lys Glu Thr Gln Gln Glu Pro Asp Glu Glu Ser Leu Val
    2150                2155                2160

Pro Ser Gly Glu Asn Leu Ala Ser Glu Thr Lys Thr Glu Ser Ala
    2165                2170                2175

Lys Thr Glu Gly Pro Ser Pro Ala Leu Leu Glu Glu Thr Pro Leu
    2180                2185                2190

Glu Pro Ala Val Gly Pro Lys Ala Ala Cys Pro Leu Asp Ser Glu
    2195                2200                2205

Ser Ala Glu Gly Val Val Pro Pro Ala Ser Gly Gly Gly Arg Val
    2210                2215                2220

Gln Asn Ser Pro Pro Val Gly Arg Lys Thr Leu Pro Leu Thr Thr
    2225                2230                2235

Ala Pro Glu Ala Gly Glu Val Thr Pro Ser Asp Ser Gly Gly Gln
    2240                2245                2250

Glu Asp Ser Pro Ala Lys Gly Leu Ser Val Arg Leu Glu Phe Asp
    2255                2260                2265

Tyr Ser Glu Asp Lys Ser Ser Trp Asp Asn Gln Gln Glu Asn Pro
    2270                2275                2280

Pro Pro Thr Lys Lys Ile Gly Lys Lys Pro Val Ala Lys Met Pro
    2285                2290                2295

Leu Arg Arg Pro Lys Met Lys Lys Thr Pro Glu Lys Leu Asp Asn

2300                2305                2310

Thr Pro Ala Ser Pro Pro Arg Ser Pro Ala Glu Pro Asn Asp Ile
    2315                2320                2325

Pro Ile Ala Lys Gly Thr Tyr Thr Phe Asp Ile Asp Lys Trp Asp
    2330                2335                2340

Asp Pro Asn Phe Asn Pro Phe Ser Ser Thr Ser Lys Met Gln Glu
    2345                2350                2355

Ser Pro Lys Leu Pro Gln Gln Ser Tyr Asn Phe Asp Pro Asp Thr
    2360                2365                2370

Cys Asp Glu Ser Val Asp Pro Phe Lys Thr Ser Ser Lys Thr Pro
    2375                2380                2385

Ser Ser Pro Ser Lys Ser Pro Ala Ser Phe Glu Ile Pro Ala Ser
    2390                2395                2400

Ala Met Glu Ala Asn Gly Val Asp Gly Asp Gly Leu Asn Lys Pro
    2405                2410                2415

Ala Lys Lys Lys Lys Thr Pro Leu Lys Thr Asp Thr Phe Arg Val
    2420                2425                2430

Lys Lys Ser Pro Lys Arg Ser Pro Leu Ser Asp Pro Pro Ser Gln
    2435                2440                2445

Asp Pro Thr Pro Ala Ala Thr Pro Glu Thr Pro Pro Val Ile Ser
    2450                2455                2460

Ala Val Val His Ala Thr Asp Glu Glu Lys Leu Ala Val Thr Asn
    2465                2470                2475

Gln Lys Trp Thr Cys Met Thr Val Asp Leu Glu Ala Asp Lys Gln
    2480                2485                2490

Asp Tyr Pro Gln Pro Ser Asp Leu Ser Thr Phe Val Asn Glu Thr
    2495                2500                2505

Lys Phe Ser Ser Pro Thr Glu Glu Leu Asp Tyr Arg Asn Ser Tyr
    2510                2515                2520

Glu Ile Glu Tyr Met Glu Lys Ile Gly Ser Ser Leu Pro Gln Asp
    2525                2530                2535

Asp Asp Ala Pro Lys Lys Gln Ala Leu Tyr Leu Met Phe Asp Thr
    2540                2545                2550

Ser Gln Glu Ser Pro Val Lys Ser Ser Pro Val Arg Met Ser Glu
    2555                2560                2565

Ser Pro Thr Pro Cys Ser Gly Ser Ser Phe Glu Glu Thr Glu Ala
    2570                2575                2580

Leu Val Asn Thr Ala Ala Lys Asn Gln His Pro Val Pro Arg Gly
    2585                2590                2595

Leu Ala Pro Asn Gln Glu Ser His Leu Gln Val Pro Glu Lys Ser
    2600                2605                2610

Ser Gln Lys Glu Leu Glu Ala Met Gly Leu Gly Thr Pro Ser Glu
    2615                2620                2625

Ala Ile Glu Ile Thr Ala Pro Glu Gly Ser Phe Ala Ser Ala Asp
    2630                2635                2640

Ala Leu Leu Ser Arg Leu Ala His Pro Val Ser Leu Cys Gly Ala
    2645                2650                2655

Leu Asp Tyr Leu Glu Pro Asp Leu Ala Glu Lys Asn Pro Pro Leu
    2660                2665                2670

Phe Ala Gln Lys Leu Gln Glu Glu Leu Glu Phe Ala Ile Met Arg
    2675                2680                2685

Ile Glu Ala Leu Lys Leu Ala Arg Gln Ile Ala Leu Ala Ser Arg
    2690                2695                2700

Ser His Gln Asp Ala Lys Arg Glu Ala Ala His Pro Thr Asp Val
    2705                2710                2715

Ser Ile Ser Lys Thr Ala Leu Tyr Ser Arg Ile Gly Thr Ala Glu
    2720                2725                2730

Val Glu Lys Pro Ala Gly Leu Leu Phe Gln Gln Pro Asp Leu Asp
    2735                2740                2745

Ser Ala Leu Gln Ile Ala Arg Ala Glu Ile Ile Thr Lys Glu Arg
    2750                2755                2760

Glu Val Ser Glu Trp Lys Asp Lys Tyr Glu Glu Ser Arg Arg Glu
    2765                2770                2775

Val Met Glu Met Arg Lys Ile Val Ala Glu Tyr Glu Lys Thr Ile
    2780                2785                2790

Ala Gln Met Ile Glu Asp Glu Gln Arg Glu Lys Ser Val Ser His
    2795                2800                2805

Gln Thr Val Gln Gln Leu Val Leu Glu Lys Glu Gln Ala Leu Ala
    2810                2815                2820

Asp Leu Asn Ser Val Glu Lys Ser Leu Ala Asp Leu Phe Arg Arg
    2825                2830                2835

Tyr Glu Lys Met Lys Glu Val Leu Glu Gly Phe Arg Lys Asn Glu
    2840                2845                2850

Glu Val Leu Lys Arg Cys Ala Gln Glu Tyr Leu Ser Arg Val Lys
    2855                2860                2865

Lys Glu Glu Gln Arg Tyr Gln Ala Leu Lys Val His Ala Glu Glu
    2870                2875                2880

Lys Leu Asp Arg Ala Asn Ala Glu Ile Ala Gln Val Arg Gly Lys
    2885                2890                2895

Ala Gln Gln Glu Gln Ala Ala His Gln Ala Ser Leu Arg Lys Glu
    2900                2905                2910

Gln Leu Arg Val Asp Ala Leu Glu Arg Thr Leu Glu Gln Lys Asn
    2915                2920                2925

Lys Glu Ile Glu Glu Leu Thr Lys Ile Cys Asp Glu Leu Ile Ala
    2930                2935                2940

Lys Met Gly Lys Ser
    2945

<210> 155
<211> 9706
<212> DNA
<213> Homo sapiens

<400> 155
gcctgctcca agggaaggat caggagagaa gaaacgcaaa tcccagaacc gtgccaacat 60

ataaaacccc acattaaggg ttgtacagtg cactgggatt tctcaagtca cccgcttggt 120

cctcttccaa gtatacttta cttcctttca ttcctctcta aaactttttt aaaaactttc 180

actcctgctc taaaagttat cttggtttct tactctacct tatgcccctt gggcgaattt 240

tttcctctga ggagggaaga atagagttgc tgctgcagac acatcagatt ccctactggt 300

aacagctgga gtgcgtcacc tctgacaaaa ttctggggac gctgggaaca ctgaatcaac 360

atgggcaatg agaacagcac ctcggacaac cagaggactt tatcagctca gactccaagg 420

tccgcgcagc cacccgggaa cagtcagaat ataaaaagga agcagcagga cacgcccgga 480

agccctgacc acagagacgc gtccagcatt ggcagcgttg ggcttggagg cttctgcacc 540

gcttctgaga gttctgccag cctggatcca tgccttgtgt ccccagaggt gactgagcca 600

aggaaggacc cacagggagc cagggggcca gaaggttctt tgctgcccag cccaccaccg 660

tcccaggagc gagagcaccc ctcgtcctcc atgccctttg ccgagtgtcc cccggaaggt 720

tgcttggcaa gtccagcagc ggcacctgaa gatggtcctc agactcagtc tcccaggagg 780

gaacctgccc caaatgcccc aggagacatc gcggcggcat ttcccgctga gagggacagc 840

tctactccat accaagagat tgctgccgtc cccagtgctg gaagagagag acagccgaag 900

gaagaaggac agaagtcctc cttctccttc tccagtggca tcgaccagtc acctggaatg 960

tcgccagtac ccctcagaga gccaatgaag gcaccgctgt gtggagaggg ggaccagcct 1020

ggtggttttg agtcccaaga gaaagaggct gcaggtggct ttccccctgc agagtccagg 1080

cagggggtgg cttctgtgca agtgacccct gaggcccctg ctgcagccca gcagggcaca 1140

gaaagctcag cggtcttgga gaagtccccc ctaaaaccca tggccccgat cccacaagat 1200

ccagccccaa gagcctcaga cagagaaaga ggccaagggg aggcgccgcc tcagtattta 1260

acagatgact tggaattcct cagggcctgc catctcccta ggagcaattc aggggctgcc 1320

ccagaagcag aagtgaatgc cgcttcccag gagagctgcc agcagccagt gggagcatat 1380

ctgccgcacg cagagctgcc ctggggcttg ccaagtcctg ccctggtgcc agaggctggg 1440

ggctctggga aggaggctct ggacaccatt gatgttcagg gtcacccaca gacagggatg 1500

cgaggaacca agcccaatca agttgtctgt gtggcagcag gcggccagcc cgaagggggt 1560

ttgcctgtga gccctgaacc ttccctgctc actccgactg aggaagcaca tccagcttca 1620

agcctcgctt cattcccagc tgctcagatt cctattgctg tagaagaacc tggatcatca 1680

tccagggaat cagtttccaa ggctgggatg ccagtttctg cagatgcagc caaagaggtg 1740

gtggatgcag ggttggtggg actggagagg caggtgtcag atcttggaag caagggagag 1800

catccagaag gggaccctgg agaggttcct gccccatcac cccaggagag gggagagcac 1860

ttgaacacgg agcaaagcca tgaggtccaa ccaggagtac caccccctcc tcttcccaag 1920

gagcaaagcc atgaggtcca accaggagca ccaccccctc ctcttcccaa ggcaccaagt 1980

gaaagtgcca gagggccacc ggggccaacg gatggagcca aggtccatga agattccaca 2040

agcccagccg tggctaaaga aggaagcaga tcacctggtg acagccctgg aggaaaggag 2100

gaagccccag agccacctga tggtggagac ccagggaacc tgcaaggaga ggactctcag 2160

gctttcagca gcaagcgtga tccagaagta ggcaaagatg agctttcaaa gccaagcagt 2220

gatgcagaga gcagagacca tcccagctca cactcagcac agccacccag aaaggggggt 2280

gctgggcaca cggacgggcc ccactctcag acagcagagg ctgatgcatc tggcctacca 2340

cacaagctgg gtgaggagga ccccgtcctg ccccctgtgc cagatggagc tggtgagccc 2400

actgttcccg aaggagccat ctgggagggg tcaggattgc agcccaaatg tcctgacacc 2460

cttcagagca gggaaggatt gggaagaatg gagtctttcc tgactttaga atcagagaaa 2520

tcagattttc caccaactcc tgttgcagag gttgcaccca aagcccagga aggtgagagc 2580

acattggaaa taaggaagat gggcagctgt gatggggagg gcttgctgac gtccccagat 2640

caaccccgcg ggccggcgtg tgatgcgtcg agacaggaat ttcatgctgg ggtgccacat 2700

cccccccagg gggagaactt ggcagcagac ctggggctca cggcactcat cctggaccaa 2760

gatcagcagg gaatcccatc ctgcccaggg gaaggctgga taagaggagc tgcatccgag 2820

tggcccctac tatcttctga gaagcatctc cagccatccc aggcacaacc agagacatcc 2880

atctttgacg tgctcaagga gcaggcccag ccacctgaaa atgggaaaga gacttctcca 2940

agccatccag gttttaagga ccagggagca gattcttccc aaatccatgt acctgtggaa 3000

cctcaggaag ataacaactt gcccactcat ggaggacagg agcaggcttt gggatcagaa 3060

cttcaaagtc agctccccaa aggcaccctg tctgatactc caacttcatc tcccactgac 3120

atggtttggg agagttctct gacagaagag tcagaattgt cagcaccaac gagacagaag 3180

ttgcctgcac taggggagaa gcggccagag ggagcatgcg gtgatggtca gtcctcgagg 3240

gtctcgcctc cagcagcaga tgtcttaaaa gacttttctc ttgcagggaa cttcagcaga 3300

aaggaaactt gctgcactgg gcaggggcca aacaagtctc aacaggcatt ggctgatgcc 3360

ttggaagaag gcagccagca tgaagaagca tgtcaaaggc atccaggagc ttctgaagca 3420

gctgatggtt gttccccact ctggggcttg agtaagaggg agatggcaag tggaaacaca 3480

ggggaggccc caccttgtca gcctgactca gtagctctcc tggatgcagt tccctgcctg 3540

ccagccctgg cgcccgccag ccccggagtc acacccaccc aggatgcccc agagacagag 3600

gcatgtgatg aaacccagga aggcaggcag caaccagtgc cggccccgca gcagaaaatg 3660

gagtgctggg ccacttcgga tgcagagtcc ccaaagcttc ttgcaagttt cccatcagct 3720

ggggagcaag gtggtgaagc cggggctgct gagactggtg gcagcgctgg tgcaggagac 3780

ccaggaaagc agcaggctcc ggagaaacct ggagaagcta ctttgagttg tggcctcctt 3840

cagactgagc actgccttac ctccggggag gaagcttcta cctctgccct acgtgagtcc 3900

tgccaagctg agcaccccat ggccagctgc caggatgcct tgctgccagc cagagagctg 3960

ggtgggattc ccaggagcac catggatttt tctacacacc aggctgtccc agacccaaag 4020

gagctcctgc tgtctgggcc accagaagtg gctgctcctg acacccctta cctgcatgtc 4080

gacagtgctg cccagagagg agcagaagac agtggagtga aagctgtttc ctctgcagac 4140

cccagagctc ctggcgaaag cccctgtcct gtaggggagc ccccacttgc cttggaaaat 4200

gctgcctcct tgaagctgtt tgctggctcc ctcgcccccc tgttgcaacc aggagctgca 4260

ggtggggaaa tccctgcagt gcaagccagc agtggtagtc ccaaagccag aaccactgag 4320

ggaccagtgg actccatgcc atgcctggac cggatgccac ttctggccaa gggcaagcag 4380

gcaacagggg aagagaaagc agcaacagct ccaggtgcag gtgccaaggc cagtggggag 4440

ggcatggcag gtgatgcagc aggagagaca gagggcagca tggagaggat gggagagcct 4500

tcccaggacc caaagcaggg cacatcaggt ggtgtggaca caagctctga gcaaatcgcc 4560

accctcactg gcttcccaga cttcagggag cacatcgcca agatcttcga gaagcctgtg 4620

ctcggagccc tggccacacc tggagaaaag gcaggagctg ggaggagtgc agtgggtaaa 4680

gacctcacca ggccattggg cccagagaag cttctagatg ggcctccagg agtggatgtc 4740

acccttctcc ctgcacctcc tgctcgactc caggtggaga agaagcaaca gttggctgga 4800

gaggctgaga tttcccatct ggctctgcaa gatccagctt cagacaagct tctgggtcca 4860

gcagggctga cctgggagcg gaacttgcca ggtgccggtg tggggaagga gatggcaggt 4920

gtcccaccca cactgaggga agacgagagg ccagaggggc ctggggcagc ctggccaggc 4980

ctggaaggcc aggcttactc acagctggag aggagcaggc aggaattagc ttcaggtctt 5040

ccttcaccag cagctactca ggagctccct gtggagagag ctgctgcctt ccaggtggct 5100

ccccatagcc atggagaaga ggccgtggcc caagacagaa ttccttctgg aaagcagcac 5160

caggaaacat ctgcctgcga cagtccacat ggagaagatg gtcccgggga ctttgctcac 5220

acaggggttc caggacatgt gccaaggtcc acgtgtgccc cttctcctca gagggaggtt 5280

ttgactgtgc ctgaggccaa cagtgagccc tggacccttg acacgcttgg gggtgaaagg 5340

agacccggag tcactgctgg catcttggaa atgcgaaatg ccctgggcaa ccagagcacc 5400

cctgcaccac caactggaga agtggcagac actcccctgg agcctggcaa ggtggcaggc 5460

gctgctgggg aagcagaggg tgacatcacc ctgagcacag ctgagacaca ggcatgtgcg 5520

tccggtgatc tgcctgaagc aggtactacg aggacattct ccgttgtggc aggtgacttg 5580

gtgctgccag gaagctgtca ggacccagcc tgctctgaca aggctccggg gatggagggt 5640

acagctgccc ttcatgggga cagcccagcc aggccccagc aggctaagga gcagccaggg 5700

cctgagcgcc ccattccagc tggggatggg aaggtgtgcg tctcctcacc tccagagcct 5760

gacgaaactc acgacccgaa gctgcaacat ttggctccag aagagctcca cactgacaga 5820

gagagcccca ggcctggccc atccatgtta ccttcggttc ctaagaagga tgctccaaga 5880

gtcatggata aagtcacttc agatgagacc agaggtgcgg aaggaacaga aagttcacct 5940

gtggcagatg atatcatcca gcccgctgcc cccgcagacc tggaaagccc aaccttagct 6000

gcctcttcct accacggtga tgttgttggc caggtctcta cggatctgat agcccagagc 6060

atctccccag ctgctgccca tgcgggtctt cctccctcgg ctgcagaaca catagtttcg 6120

ccatctgccc cagctggtga cagagtagaa gcttccactc cctcctgccc agatccggcc 6180

aaggacctca gcaggagttc cgattctgaa gaggcatttg agaccccgga gtcaacgacc 6240

cctgtcaaag ctccgccagc tccaccccca ccaccccccg aagtcatccc agaacccgag 6300

gtcagcacac agccaccccc ggaagaacca ggatgtggtt ctgagacagt ccctgtccct 6360

gatggcccac ggagcgactc ggtggaagga agtcccttcc gtcccccgtc acactccttc 6420

tctgccgtct tcgatgaaga caagccgata gccagcagtg ggacttacaa cttggacttt 6480

gacaacattg agcttgtgga tacctttcag accttggagc ctcgtgcctc agacgctaag 6540

aatcaggagg gcaaagtgaa cacacggagg aagtccacgg attccgtccc catctctaag 6600

tctacactgt cccggtcgct cagcctgcaa gccagtgact ttgatggtgc ttcttcctca 6660

ggcaatcccg aggccgtggc ccttgcccca gatgcatata gcacgggttc cagcagtgct 6720

tctagtaccc ttaagcgaac taaaaaaccg aggccgcctt ccttaaaaaa gaaacagacc 6780

accaagaaac ccacagagac ccccccagtg aaggagacgc aacaggagcc agatgaagag 6840

agccttgtcc ccagtgggga gaatctagca tctgagacga aaacggaatc tgccaagacg 6900

gaaggtccta gcccagcctt attggaggag acgccccttg agcccgctgt ggggcccaaa 6960

gctgcctgcc ctctggactc agagagtgca gaaggggttg tccccccggc ttctggaggt 7020

ggcagagtgc agaactcacc ccctgtcggg aggaaaacgc tgcctcttac cacggccccg 7080

gaggcagggg aggtaacccc atcggatagc ggggggcaag aggactctcc agccaaaggg 7140

ctctccgtaa ggctggagtt tgactattct gaggacaaga gtagttggga caaccagcag 7200

gaaaaccccc ctcctaccaa aaagataggc aaaaagccag ttgccaaaat gcccctgagg 7260

aggccaaaga tgaaaaagac acccgagaaa cttgacaaca ctcctgcctc acctcccaga 7320

tcccctgctg aacccaatga catccccatt gctaaaggta cttacacctt tgatattgac 7380

aagtgggatg accccaattt taaccctttt tcttccacct caaaaatgca ggagtctccc 7440

aaactgcccc aacaatcata caactttgac ccagacacct gtgatgagtc cgttgacccc 7500

tttaagacat cctctaagac ccccagctca ccttctaaat ccccagcctc ctttgagatc 7560

ccagccagtg ctatggaagc caatggagtg gacggggatg ggctaaacaa gcccgccaag 7620

aagaagaaga cgcccctaaa gactgacaca tttagggtga aaaagtcgcc aaaacggtct 7680

cctctctctg atccaccttc ccaggacccc accccagctg ctacaccaga aacaccacca 7740

gtgatctctg cggtggtcca cgccacagat gaggaaaagc tggcggtcac caaccagaag 7800

tggacgtgca tgacagtgga cctagaggct gacaaacagg actacccgca gccctcggac 7860

ctgtccacct ttgtaaacga gaccaaattc agttcaccca ctgaggagtt ggattacaga 7920

aactcctatg aaattgaata tatggagaaa attggctcct ccttacctca ggacgacgat 7980

gccccgaaga agcaggcctt gtaccttatg tttgacactt ctcaggagag ccctgtcaag 8040

tcatctcccg tccgcatgtc agagtccccg acgccgtgtt cagggtcaag ttttgaagag 8100

actgaagccc ttgtgaacac tgctgcgaaa aaccagcatc ctgtcccacg aggactggcc 8160

cctaaccaag agtcacactt gcaggtgcca gagaaatcct cccagaagga gctggaggcc 8220

atgggcttgg gcaccccttc agaagcgatt gaaattacag ctcccgaggg ctcctttgcc 8280

tctgctgacg ccctcctcag caggctagct caccccgtct ctctctgtgg tgcacttgac 8340

tatctggagc ccgacttagc agaaaagaac cccccactat tcgctcagaa actccaggag 8400

gagttagagt ttgccatcat gcggatagaa gccctgaagc tggccaggca gattgctttg 8460

gcttcccgca gccaccagga tgccaagaga gaggctgctc acccaacaga cgtctccatc 8520

tccaaaacag ccttgtactc ccgcatcggg accgctgagg tggagaaacc tgcaggcctt 8580

ctgttccagc agcccgacct ggactctgcc ctccagatcg ccagagcaga gatcataacc 8640

aaggagagag aggtctcaga atggaaagat aaatatgaag aaagcaggcg ggaagtgatg 8700

gaaatgagga aaatagtggc cgagtatgag aagaccatcg ctcagatgat agaggacgaa 8760

cagagagaga agtcagtctc ccaccagacg gtgcagcagc tggttctgga gaaggagcaa 8820

gccctggccg acctgaactc cgtggagaag tctctggccg acctcttcag aagatatgag 8880

aagatgaagg aggtcctaga aggcttccgc aagaatgaag aggtgttgaa gagatgtgcg 8940

caggagtacc tgtcccgggt gaagaaggag gagcagaggt accaggccct gaaggtgcac 9000

gcggaggaga aactggacag ggccaatgct gagattgctc aggttcgagg caaggcccag 9060

caggagcaag ccgcccacca ggccagcctg cggaaggagc agctgcgagt ggacgccctg 9120

gaaaggacgc tggagcagaa gaataaagaa atagaagaac tcaccaagat ttgtgacgaa 9180

ctgattgcca aaatggggaa aagctaactc tgaaccgaat gttttggact taactgttgc 9240

gtgcaatatg accgtcggca cactgctgtt cctccagttc catggacagg ttctgttttc 9300

actttttcgt atgcactact gtatttcctt tctaaataaa attgatttga ttgtatgcag 9360

tactaaggag actatcagaa tttcttgcta ttggtttgca ttttcctagt ataattcata 9420

gcaagttgac ctcagagttc ctgtatcagg gagattgtct gattctctaa taaaagacac 9480

attgctgacc ttggccttgc cctttgtaca caagttccca gggtgagcag cttttggatt 9540

taatatgaac atgtacagcg tgcataggga ctcttgcctt aaggagtgta aacttgatct 9600

gcatttgctg atttgttttt aaaaaaacaa gaaatgcatg tttcaaataa aattctctat 9660

tgtaaataaa attttttctt tggatcttgg caaaaaaaaa aaaaaa 9706

<210> 156
<211> 13
<212> PRT
<213> Artificial Sequence

<220>
<223> Description of Artificial Sequence: Synthetic
      peptide

<400> 156
Ala Gln Ala Glu Ala Leu Ala Leu Gln Ala Ser Leu Arg
1               5                   10


<210> 157
<211> 6
<212> PRT
<213> Artificial Sequence

<220>
<223> Description of Artificial Sequence: Synthetic
      peptide

<400> 157
Phe Asp Pro Leu Leu Arg
1               5


<210> 158
<211> 1048
<212> PRT
<213> Artificial Sequence

<220>
<223> Description of Artificial Sequence: Synthetic
      polypeptide

<400> 158
   Met Gly Ala Pro Ala Cys Ala Leu Ala Leu Cys Val Ala Val Ala Ile
   1               5                   10                  15
   Val Ala Gly Ala Ser Ser Glu Ser Leu Gly Thr Glu Gln Arg Val Val
               20                  25                  30
   Gly Arg Ala Ala Glu Val Pro Gly Pro Glu Pro Gly Gln Gln Glu Gln
           35                  40                  45
   Leu Val Phe Gly Ser Gly Asp Ala Val Glu Leu Ser Cys Pro Pro Pro
       50                  55                  60
   Gly Gly Gly Pro Met Gly Pro Thr Val Trp Val Lys Asp Gly Thr Gly
   65                  70                  75                  80
   Leu Val Pro Ser Glu Arg Val Leu Val Gly Pro Gln Arg Leu Gln Val
                   85                  90                  95
   Leu Asn Ala Ser His Glu Asp Ser Gly Ala Tyr Ser Cys Arg Gln Arg
           100                 105                 110
   Leu Thr Gln Arg Val Leu Cys His Phe Ser Val Arg Val Thr Asp Ala
       115                 120                 125
   Pro Ser Ser Gly Asp Asp Glu Asp Gly Glu Asp Glu Ala Glu Asp Thr
       130                 135                 140
   Gly Val Asp Thr Gly Ala Pro Tyr Trp Thr Arg Pro Glu Arg Met Asp
   145                 150                 155                 160
   Lys Lys Leu Leu Ala Val Pro Ala Ala Asn Thr Val Arg Phe Arg Cys
                   165                 170                 175
   Pro Ala Ala Gly Asn Pro Thr Pro Ser Ile Ser Trp Leu Lys Asn Gly
           180                 185                 190
   Arg Glu Phe Arg Gly Glu His Arg Ile Gly Gly Ile Lys Leu Arg His
       195                 200                 205
   Gln Gln Trp Ser Leu Val Met Glu Ser Val Val Pro Ser Asp Arg Gly
       210                 215                 220
   Asn Tyr Thr Cys Val Val Glu Asn Lys Phe Gly Ser Ile Arg Gln Thr
   225                 230                 235                 240
   Tyr Thr Leu Asp Val Leu Glu Arg Ser Pro His Arg Pro Ile Leu Gln
                   245                 250                 255
   Ala Gly Leu Pro Ala Asn Gln Thr Ala Val Leu Gly Ser Asp Val Glu
           260                 265                 270
   Phe His Cys Lys Val Tyr Ser Asp Ala Gln Pro His Ile Gln Trp Leu
       275                 280                 285
   Lys His Val Glu Val Asn Gly Ser Lys Val Gly Pro Asp Gly Thr Pro
       290                 295                 300
   Tyr Val Thr Val Leu Lys Thr Ala Gly Ala Asn Thr Thr Asp Lys Glu
   305                 310                 315                 320
   Leu Glu Val Leu Ser Leu His Asn Val Thr Phe Glu Asp Ala Gly Glu
                   325                 330                 335
   Tyr Thr Cys Leu Ala Gly Asn Ser Ile Gly Phe Ser His His Ser Ala
           340                 345                 350
   Trp Leu Val Val Leu Pro Ala Glu Glu Glu Leu Val Glu Ala Asp Glu
           355                 360                 365
   Ala Gly Ser Val Tyr Ala Gly Ile Leu Ser Tyr Gly Val Gly Phe Phe
       370                 375                 380
   Leu Phe Ile Leu Val Val Ala Ala Val Thr Leu Cys Arg Leu Arg Ser
   385                 390                 395                 400
   Pro Pro Lys Lys Gly Leu Gly Ser Pro Thr Val His Lys Ile Ser Arg
                   405                 410                 415
   Phe Pro Leu Lys Arg Gln Val Ser Leu Glu Ser Asn Ala Ser Met Ser
               420                 425                 430
   Ser Asn Thr Pro Leu Val Arg Ile Ala Arg Leu Ser Ser Gly Glu Gly
           435                 440                 445
   Pro Thr Leu Ala Asn Val Ser Glu Leu Glu Leu Pro Ala Asp Pro Lys
       450                 455                 460
   Trp Glu Leu Ser Arg Ala Arg Leu Thr Leu Gly Lys Pro Leu Gly Glu
   465                 470                 475                 480
   Gly Cys Phe Gly Gln Val Val Met Ala Glu Ala Ile Gly Ile Asp Lys
                   485                 490                 495
   Asp Arg Ala Ala Lys Pro Val Thr Val Ala Val Lys Met Leu Lys Asp
               500                 505                 510
   Asp Ala Thr Asp Lys Asp Leu Ser Asp Leu Val Ser Glu Met Glu Met
           515                 520                 525
   Met Lys Met Ile Gly Lys His Lys Asn Ile Ile Asn Leu Leu Gly Ala
       530                 535                 540
   Cys Thr Gln Gly Gly Pro Leu Tyr Val Leu Val Glu Tyr Ala Ala Lys
   545                 550                 555                 560
   Gly Asn Leu Arg Glu Phe Leu Arg Ala Arg Arg Pro Pro Gly Leu Asp
                   565                 570                 575
   Tyr Ser Phe Asp Thr Cys Lys Pro Pro Glu Glu Gln Leu Thr Phe Lys
               580                 585                 590
   Asp Leu Val Ser Cys Ala Tyr Gln Val Ala Arg Gly Met Glu Tyr Leu
   595 600 605
   Ala Ser Gln Lys Cys Ile His Arg Asp Leu Ala Ala Arg Asn Val Leu
   610 615 620
   Val Thr Glu Asp Asn Val Met Lys Ile Ala Asp Phe Gly Leu Ala Arg
   625 630 635 640
   Asp Val His Asn Leu Asp Tyr Tyr Lys Lys Thr Thr Asn Gly Arg Leu
   645 650 655
   Pro Val Lys Trp Met Ala Pro Glu Ala Leu Phe Asp Arg Val Tyr Thr
   660 665 670
   His Gln Ser Asp Val Trp Ser Phe Gly Val Leu Leu Trp Glu Ile Phe
   675 680 685
   Thr Leu Gly Gly Ser Pro Tyr Pro Gly Ile Pro Val Glu Glu Leu Phe
   690 695 700
   Lys Leu Leu Lys Glu Gly His Arg Met Asp Lys Pro Ala Asn Cys Thr
   705 710 715 720
   His Asp Leu Tyr Met Ile Met Arg Glu Cys Trp His Ala Ala Pro Ser
   725 730 735
   Gln Arg Pro Thr Phe Lys Gln Leu Val Glu Asp Leu Asp Arg Val Leu
   740 745 750
   Thr Val Thr Ser Thr Asp Phe Lys Glu Ser Ala Leu Arg Lys Gln Ser
   755 760 765
   Leu Tyr Leu Lys Phe Asp Pro Leu Leu Arg Asp Ser Pro Gly Arg Pro
   770 775 780
   Val Pro Val Ala Thr Glu Thr Ser Ser Met His Gly Ala Asn Glu Thr
   785 790 795 800
   Pro Ser Gly Arg Pro Arg Glu Ala Lys Leu Val Glu Phe Asp Phe Leu
   805 810 815
   Gly Ala Leu Asp Ile Pro Val Pro Gly Pro Pro Pro Gly Val Pro Ala
   820 825 830
   Pro Gly Gly Pro Pro Leu Ser Thr Gly Pro Ile Val Asp Leu Leu Gln
   835 840 845
   Tyr Ser Gln Lys Asp Leu Asp Ala Val Val Lys Ala Thr Gln Glu Glu
       850                 855                 860
   Asn Arg Glu Leu Arg Ser Arg Cys Glu Glu Leu His Gly Lys Asn Leu
   865                 870                 875                 880
   Glu Leu Gly Lys Ile Met Asp Arg Phe Glu Glu Val Val Tyr Gln Ala
                   885                 890                 895
   Met Glu Glu Val Gln Lys Gln Lys Glu Leu Ser Lys Ala Glu Ile Gln
               900                 905                 910
   Lys Val Leu Lys Glu Lys Asp Gln Leu Thr Thr Asp Leu Asn Ser Met
           915                 920                 925
   Glu Lys Ser Phe Ser Asp Leu Phe Lys Arg Phe Glu Lys Gln Lys Glu
       930                 935                 940
   Val Ile Glu Gly Tyr Arg Lys Asn Glu Glu Ser Leu Lys Lys Cys Val
   945                 950                 955                 960
   Glu Asp Tyr Leu Ala Arg Ile Thr Gln Glu Gly Gln Arg Tyr Gln Ala
                   965                 970                 975
   Leu Lys Ala His Ala Glu Glu Lys Leu Gln Leu Ala Asn Glu Glu Ile
               980                 985                 990
   Ala Gln Val Arg Ser Lys Ala Gln  Ala Glu Ala Leu Ala  Leu Gln Ala
           995                 1000                1005
   Ser Leu  Arg Lys Glu Gln Met  Arg Ile Gln Ser Leu  Glu Lys Thr
       1010                1015                1020
   Val Glu  Gln Lys Thr Lys Glu  Asn Glu Glu Leu Thr  Arg Ile Cys
       1025                1030                1035
   Asp Asp  Leu Ile Ser Lys Met  Glu Lys Ile
       1040                1045

<210> 159
<211> 1027
<212> PRT
<213> Artificial Sequence

<220>
<223> Description of Artificial Sequence: Synthetic
      polypeptide

<400> 159
Met Gly Ala Pro Ala Cys Ala Leu Ala Leu Cys Val Ala Val Ala Ile

1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Val Ser Ala Gly Ser Gly Leu Val Pro Pro
        755                 760                 765

Ala Tyr Ala Pro Pro Pro Ala Val Pro Gly His Pro Ser Gly Arg Pro
    770                 775                 780

Arg Glu Ala Lys Leu Val Glu Phe Asp Phe Leu Gly Ala Leu Asp Ile
785                 790                 795                 800

Pro Val Pro Gly Pro Pro Pro Gly Val Pro Ala Pro Gly Gly Pro Pro
                805                 810                 815

Leu Ser Thr Gly Pro Ile Val Asp Leu Leu Gln Tyr Ser Gln Lys Asp
            820                 825                 830

Leu Asp Ala Val Val Lys Ala Thr Gln Glu Glu Asn Arg Glu Leu Arg
        835                 840                 845

Ser Arg Cys Glu Glu Leu His Gly Lys Asn Leu Glu Leu Gly Lys Ile
    850                 855                 860

Met Asp Arg Phe Glu Glu Val Val Tyr Gln Ala Met Glu Glu Val Gln
865                 870                 875                 880

Lys Gln Lys Glu Leu Ser Lys Ala Glu Ile Gln Lys Val Leu Lys Glu
                885                 890                 895

Lys Asp Gln Leu Thr Thr Asp Leu Asn Ser Met Glu Lys Ser Phe Ser
            900                 905                 910

Asp Leu Phe Lys Arg Phe Glu Lys Gln Lys Glu Val Ile Glu Gly Tyr
        915                 920                 925

Arg Lys Asn Glu Glu Ser Leu Lys Lys Cys Val Glu Asp Tyr Leu Ala
    930                 935                 940

Arg Ile Thr Gln Glu Gly Gln Arg Tyr Gln Ala Leu Lys Ala His Ala
945                 950                 955                 960

Glu Glu Lys Leu Gln Leu Ala Asn Glu Glu Ile Ala Gln Val Arg Ser
                965                 970                 975

Lys Ala Gln Ala Glu Ala Leu Ala Leu Gln Ala Ser Leu Arg Lys Glu
            980                 985                 990

Gln Met Arg Ile Gln Ser Leu Glu  Lys Thr Val Glu Gln  Lys Thr Lys
        995                 1000                1005

Glu Asn  Glu Glu Leu Thr Arg  Ile Cys Asp Asp Leu  Ile Ser Lys

1010                1015                1020

Met Glu  Lys Ile
    1025

<210> 160
<211> 984
<212> PRT
<213> Artificial Sequence

<220>
<223> Description of Artificial Sequence: Synthetic
      polypeptide

<400> 160
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly

435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Val Pro Gly Pro Pro Pro Gly Val Pro Ala
        755                 760                 765

Pro Gly Gly Pro Pro Leu Ser Thr Gly Pro Ile Val Asp Leu Leu Gln
    770                 775                 780

Tyr Ser Gln Lys Asp Leu Asp Ala Val Val Lys Ala Thr Gln Glu Glu
785                 790                 795                 800

Asn Arg Glu Leu Arg Ser Arg Cys Glu Glu Leu His Gly Lys Asn Leu
                805                 810                 815

Glu Leu Gly Lys Ile Met Asp Arg Phe Glu Glu Val Val Tyr Gln Ala
            820                 825                 830

Met Glu Glu Val Gln Lys Gln Lys Glu Leu Ser Lys Ala Glu Ile Gln
        835                 840                 845

Lys Val Leu Lys Glu Lys Asp Gln Leu Thr Thr Asp Leu Asn Ser Met
    850                 855                 860

Glu Lys Ser Phe Ser Asp Leu Phe Lys Arg Phe Glu Lys Gln Lys Glu
865                 870                 875                 880

Val Ile Glu Gly Tyr Arg Lys Asn Glu Glu Ser Leu Lys Lys Cys Val
                885                 890                 895

Glu Asp Tyr Leu Ala Arg Ile Thr Gln Glu Gly Gln Arg Tyr Gln Ala
            900                 905                 910

Leu Lys Ala His Ala Glu Glu Lys Leu Gln Leu Ala Asn Glu Glu Ile
        915                 920                 925

Ala Gln Val Arg Ser Lys Ala Gln Ala Glu Ala Leu Ala Leu Gln Ala
    930                 935                 940

Ser Leu Arg Lys Glu Gln Met Arg Ile Gln Ser Leu Glu Lys Thr Val
945                 950                 955                 960

Glu Gln Lys Thr Lys Glu Asn Glu Glu Leu Thr Arg Ile Cys Asp Asp
                965                 970                 975

Leu Ile Ser Lys Met Glu Lys Ile
            980

<210> 161
<211> 949
<212> PRT
<213> Artificial Sequence

<220>
<223> Description of Artificial Sequence: Synthetic
      polypeptide

<400> 161
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Val Lys Ala Thr Gln Glu Glu Asn Arg Glu
        755                 760                 765

Leu Arg Ser Arg Cys Glu Glu Leu His Gly Lys Asn Leu Glu Leu Gly
    770                 775                 780

Lys Ile Met Asp Arg Phe Glu Glu Val Val Tyr Gln Ala Met Glu Glu
785                 790                 795                 800

Val Gln Lys Gln Lys Glu Leu Ser Lys Ala Glu Ile Gln Lys Val Leu
                805                 810                 815

Lys Glu Lys Asp Gln Leu Thr Thr Asp Leu Asn Ser Met Glu Lys Ser
            820                 825                 830

Phe Ser Asp Leu Phe Lys Arg Phe Glu Lys Gln Lys Glu Val Ile Glu
        835                 840                 845

Gly Tyr Arg Lys Asn Glu Glu Ser Leu Lys Lys Cys Val Glu Asp Tyr
    850                 855                 860

Leu Ala Arg Ile Thr Gln Glu Gly Gln Arg Tyr Gln Ala Leu Lys Ala
865                 870                 875                 880

His Ala Glu Glu Lys Leu Gln Leu Ala Asn Glu Glu Ile Ala Gln Val
                885                 890                 895

Arg Ser Lys Ala Gln Ala Glu Ala Leu Ala Leu Gln Ala Ser Leu Arg
            900                 905                 910

Lys Glu Gln Met Arg Ile Gln Ser Leu Glu Lys Thr Val Glu Gln Lys

915                 920                 925

Thr Lys Glu Asn Glu Glu Leu Thr Arg Ile Cys Asp Asp Leu Ile Ser
    930                 935                 940

Lys Met Glu Lys Ile
945

<210> 162
<211> 20
<212> DNA
<213> Artificial Sequence

<220>
<223> Description of Artificial Sequence: Synthetic
      primer

<400> 162
gtaacctgcg ggagtttctg                                                   20

<210> 163
<211> 20
<212> DNA
<213> Artificial Sequence

<220>
<223> Description of Artificial Sequence: Synthetic
      primer

<400> 163
acaccaggtc cttgaaggtg                                                   20

<210> 164
<211> 20
<212> DNA
<213> Artificial Sequence

<220>
<223> Description of Artificial Sequence: Synthetic
      primer

<400> 164
cctgagggac agtcctggta                                                   20

<210> 165
<211> 20
<212> DNA
<213> Artificial Sequence

<220>
<223> Description of Artificial Sequence: Synthetic
      primer

<400> 165
agtgctccca agaaatcgaa                                                   20

<210> 166
<211> 23
<212> DNA
<213> Artificial Sequence

<220>
<223> Description of Artificial Sequence: Synthetic
      primer

<400> 166
cgtgaagatg ctgaaagacg atg                                              23


<210> 167
<211> 21
<212> DNA
<213> Artificial Sequence

<220>
<223> Description of Artificial Sequence: Synthetic
      primer

<400> 167
aaacgcttga agaggtcgga g                                                21


<210> 168
<211> 20
<212> DNA
<213> Artificial Sequence

<220>
<223> Description of Artificial Sequence: Synthetic
      primer

<400> 168
atgctagcag gggtctctga                                                  20


<210> 169
<211> 20
<212> DNA
<213> Artificial Sequence

<220>
<223> Description of Artificial Sequence: Synthetic
      primer

<400> 169
cccttccaga acacctttca                                                  20


<210> 170
<211> 18
<212> DNA
<213> Artificial Sequence

<220>
<223> Description of Artificial Sequence: Synthetic
      primer

<400> 170
atgatcatgc gggagtgc                                                    18

<210> 171
<211> 20
<212> DNA
<213> Artificial Sequence

<220>
<223> Description of Artificial Sequence: Synthetic
      primer

<400> 171
gggggtcgaa cttgaggtat                                                   20


<210> 172
<211> 20
<212> DNA
<213> Artificial Sequence

<220>
<223> Description of Artificial Sequence: Synthetic
      primer

<400> 172
cgcaggcttt ttgtagtgag                                                   20


<210> 173
<211> 18
<212> DNA
<213> Artificial Sequence

<220>
<223> Description of Artificial Sequence: Synthetic
      primer

<400> 173
tgtaggcgcg aaaggaag                                                     18


<210> 174
<211> 20
<212> DNA
<213> Artificial Sequence

<220>
<223> Description of Artificial Sequence: Synthetic
      primer

<400> 174
gaactcatcc ggacccctat                                                   20


<210> 175
<211> 20
<212> DNA
<213> Artificial Sequence

<220>
<223> Description of Artificial Sequence: Synthetic
      primer

<400> 175
gctttcccca ttgcacttta                                                    20


<210> 176
<211> 20
<212> DNA
<213> Artificial Sequence

<220>
<223> Description of Artificial Sequence: Synthetic
      primer

<400> 176
gaggagagaa gcacgtggag                                                    20


<210> 177
<211> 20
<212> DNA
<213> Artificial Sequence

<220>
<223> Description of Artificial Sequence: Synthetic
      primer

<400> 177
ggcagacgtg tgaggtgtaa                                                    20


<210> 178
<211> 20
<212> DNA
<213> Artificial Sequence

<220>
<223> Description of Artificial Sequence: Synthetic
      primer

<400> 178
gtgatcagca gctggactgt                                                    20


<210> 179
<211> 18
<212> DNA
<213> Artificial Sequence

<220>
<223> Description of Artificial Sequence: Synthetic
      primer

<400> 179
gagcctgggc atggatct                                                      18


<210> 180
<211> 20
<212> DNA
<213> Artificial Sequence

<220>
<223> Description of Artificial Sequence: Synthetic

primer

<400> 180
agaggtgacc accaatcagc                                                    20


<210> 181
<211> 20
<212> DNA
<213> Artificial Sequence

<220>
<223> Description of Artificial Sequence: Synthetic
      primer

<400> 181
cgtgtcccac acagagacag                                                    20


<210> 182
<211> 21
<212> DNA
<213> Artificial Sequence

<220>
<223> Description of Artificial Sequence: Synthetic
      oligonucleotide

<400> 182
tgcgtcgtgg agaacaagtt t                                                  21


<210> 183
<211> 21
<212> DNA
<213> Artificial Sequence

<220>
<223> Description of Artificial Sequence: Synthetic
      oligonucleotide

<400> 183
gttccactgc aaggtgtaca g                                                  21


<210> 184
<211> 21
<212> DNA
<213> Artificial Sequence

<220>
<223> Description of Artificial Sequence: Synthetic
      oligonucleotide

<400> 184
gcacaacctc gactactaca a                                                  21


<210> 185
<211> 822
<212> PRT
<213> Homo sapiens

<400> 185

<210> 186
<211> 5917
<212> DNA
<213> Homo sapiens

<400> 186
agatgcaggg gcgcaaacgc caaaggagac caggctgtag gaagagaagg gcagagcgcc      60

ggacagctcg gcccgctccc cgtcctttgg ggccgcggct ggggaactac aaggcccagc     120

aggcagctgc agggggcgga ggcggaggag ggaccagcgc gggtgggagt gagagagcga     180

gccctcgcgc cccgccggcg catagcgctc ggagcgctct tgcggccaca ggcgcggcgt     240

cctcggcggc gggcggcagc tagcgggagc cgggacgccg gtgcagccgc agcgcgcgga     300

ggaacccggg tgtgccggga gctgggcggc cacgtccgga cgggaccgag acccctcgta     360

gcgcattgcg gcgacctcgc cttccccggc cgcgagcgcg ccgctgcttg aaaagccgcg     420

gaacccaagg acttttctcc ggtccgagct cggggcgccc cgcagggcgc acggtacccg     480

tgctgcagtc gggcacgccg cggcgccggg gcctccgcag ggcgatggag cccggtctgc     540

aaggaaagtg aggcgccgcc gctgcgttct ggaggagggg ggcacaaggt ctggagaccc     600

cgggtggcgg acgggagccc tccccccgcc ccgcctccgg ggcaccagct ccggctccat     660

tgttcccgcc cgggctggag gcgccgagca ccgagcgccg ccgggagtcg agcgccggcc     720

gcggagctct tgcgaccccg ccaggacccg aacagagccc gggggcggcg ggccggagcc     780

ggggacgcgg gcacacgccc gctcgcacaa gccacggcgg actctcccga ggcggaacct     840

ccacgccgag cgagggtcag tttgaaaagg aggatcgagc tcactgtgga gtatccatgg     900

agatgtggag ccttgtcacc aacctctaac tgcagaactg ggatgtggag ctggaagtgc     960

ctcctcttct gggctgtgct ggtcacagcc acactctgca ccgctaggcc gtccccgacc    1020

ttgcctgaac aagcccagcc ctggggagcc cctgtggaag tggagtcctt cctggtccac    1080

cccggtgacc tgctgcagct tcgctgtcgg ctgcgggacg atgtgcagag catcaactgg    1140

ctgcgggacg gggtgcagct ggcggaaagc aaccgcaccc gcatcacagg ggaggaggtg 1200

gaggtgcagg actccgtgcc cgcagactcc ggcctctatg cttgcgtaac cagcagcccc 1260

tcgggcagtg acaccaccta cttctccgtc aatgtttcag atgctctccc ctcctcggag 1320

gatgatgatg atgatgatga ctcctcttca gaggagaaag aaacagataa caccaaacca 1380

aaccgtatgc ccgtagctcc atattggaca tccccagaaa agatggaaaa gaaattgcat 1440

gcagtgccgg ctgccaagac agtgaagttc aaatgccctt ccagtgggac cccaaacccc 1500

acactgcgct ggttgaaaaa tggcaaagaa ttcaaacctg accacagaat tggaggctac 1560

aaggtccgtt atgccacctg gagcatcata atggactctg tggtgccctc tgacaagggc 1620

aactacacct gcattgtgga gaatgagtac ggcagcatca accacacata ccagctggat 1680

gtcgtggagc ggtcccctca ccggcccatc ctgcaagcag ggttgcccgc caacaaaaca 1740

gtggccctgg gtagcaacgt ggagttcatg tgtaaggtgt acagtgaccc gcagccgcac 1800

atccagtggc taaagcacat cgaggtgaat gggagcaaga ttggcccaga caacctgcct 1860

tatgtccaga tcttgaagac tgctggagtt aataccaccg acaaagagat ggaggtgctt 1920

cacttaagaa atgtctcctt tgaggacgca ggggagtata cgtgcttggc gggtaactct 1980

atcggactct cccatcactc tgcatggttg accgttctgg aagccctgga agagaggccg 2040

gcagtgatga cctcgcccct gtacctggag atcatcatct attgcacagg ggccttcctc 2100

atctcctgca tggtggggtc ggtcatcgtc tacaagatga agagtggtac caagaagagt 2160

gacttccaca gccagatggc tgtgcacaag ctggccaaga gcatccctct gcgcagacag 2220

gtaacagtgt ctgctgactc cagtgcatcc atgaactctg gggttcttct ggttcggcca 2280

tcacggctct cctccagtgg gactcccatg ctagcagggg tctctgagta tgagcttccc 2340

gaagaccctc gctgggagct gcctcgggac agactggtct taggcaaacc cctgggagag 2400

ggctgctttg ggcaggtggt gttggcagag gctatcgggc tggacaagga caaacccaac 2460

cgtgtgacca aagtggctgt gaagatgttg aagtcggacg caacagagaa agacttgtca 2520

gacctgatct cagaaatgga gatgatgaag atgatcggga agcataagaa tatcatcaac 2580

ctgctggggg cctgcacgca ggatggtccc ttgtatgtca tcgtggagta tgcctccaag 2640

ggcaacctgc gggagtacct gcaggcccgg aggcccccag ggctggaata ctgctacaac 2700

cccagccaca acccagagga gcagctctcc tccaaggacc tggtgtcctg cgcctaccag 2760

gtggcccgag gcatggagta tctggcctcc aagaagtgca tacaccgaga cctggcagcc 2820

aggaatgtcc tggtgacaga ggacaatgtg atgaagatag cagactttgg cctcgcacgg 2880

gacattcacc acatcgacta ctataaaaag acaaccaacg gccgactgcc tgtgaagtgg 2940

atggcacccg aggcattatt tgaccggatc tacacccacc agagtgatgt gtggtctttc 3000

ggggtgctcc tgtgggagat cttcactctg ggcggctccc cataccccgg tgtgcctgtg 3060

gaggaacttt tcaagctgct gaaggagggt caccgcatgg acaagcccag taactgcacc 3120

aacgagctgt acatgatgat gcgggactgc tggcatgcag tgccctcaca gagacccacc 3180

ttcaagcagc tggtggaaga cctggaccgc atcgtggcct tgacctccaa ccaggagtac 3240

ctggacctgt ccatgcccct ggaccagtac tcccccagct ttcccgacac ccggagctct 3300

acgtgctcct caggggagga ttccgtcttc tctcatgagc cgctgcccga ggagccctgc 3360

ctgccccgac acccagccca gcttgccaat ggcggactca aacgccgctg actgccaccc 3420

acacgccctc cccagactcc accgtcagct gtaaccctca cccacagccc ctgctgggcc 3480

caccacctgt ccgtccctgt cccctttcct gctggcagga gccggctgcc taccaggggc 3540

cttcctgtgt ggcctgcctt caccccactc agctcacctc tccctccacc tcctctccac 3600

ctgctggtga gaggtgcaaa gaggcagatc tttgctgcca gccacttcat cccctcccag 3660

atgttggacc aacacccctc cctgccacca ggcactgcct ggagggcagg gagtgggagc 3720

caatgaacag gcatgcaagt gagagcttcc tgagctttct cctgtcggtt tggtctgttt 3780

tgccttcacc cataagcccc tcgcactctg gtggcaggtg ccttgtcctc agggctacag 3840

cagtagggag gtcagtgctt cgtgcctcga ttgaaggtga cctctgcccc agataggtgg 3900

tgccagtggc ttattaattc cgatactagt ttgctttgct gaccaaatgc ctggtaccag 3960

aggatggtga ggcgaaggcc aggttggggg cagtgttgtg gccctggggc ccagccccaa 4020

actgggggct ctgtatatag ctatgaagaa aacacaaagt gtataaatct gagtatatat 4080

ttacatgtct ttttaaaagg gtcgttacca gagatttacc catcgggtaa gatgctcctg 4140

gtggctggga ggcatcagtt gctatatatt aaaaacaaaa aagaaaaaaa aggaaaatgt 4200

ttttaaaaag gtcatatatt ttttgctact tttgctgttt tattttttta aattatgttc 4260

taaacctatt ttcagtttag gtccctcaat aaaaattgct gctgcttcat ttatctatgg 4320

gctgtatgaa aagggtggga atgtccactg gaaagaaggg acacccacgg gccctggggc 4380

taggtctgtc ccgagggcac cgcatgctcc cggcgcaggt tccttgtaac ctcttcttcc 4440

taggtcctgc acccagacct cacgacgcac ctcctgcctc tccgctgctt ttggaaagtc 4500

agaaaaagaa gatgtctgct tcgagggcag gaaccccatc catgcagtag aggcgctggg 4560

cagagagtca aggcccagca gccatcgacc atggatggtt tcctccaagg aaaccggtgg 4620

ggttgggctg ggaggggggc acctacctag gaatagccac ggggtagagc tacagtgatt 4680

aagaggaaag caagggcgcg gttgctcacg cctgtaatcc cagcactttg ggacaccgag 4740

gtgggcagat cacttcaggt caggagtttg agaccagcct ggccaactta gtgaaacccc 4800

atctctacta aaaatgcaaa aattatccag gcatggtggc acacgcctgt aatcccagct 4860

ccacaggagg ctgaggcaga atcccttgaa gctgggaggc ggaggttgca gtgagccgag 4920

attgcgccat tgcactccag cctgggcaac agagaaaaca aaaaggaaaa caaatgatga 4980

aggtctgcag aaactgaaac ccagacatgt gtctgccccc tctatgtggg catggttttg 5040

ccagtgcttc taagtgcagg agaacatgtc acctgaggct agttttgcat tcaggtccct 5100

ggcttcgttt cttgttggta tgcctcccca gatcgtcctt cctgtatcca tgtgaccaga 5160

ctgtatttgt tgggactgtc gcagatcttg gcttcttaca gttcttcctg tccaaactcc 5220

atcctgtccc tcaggaacgg ggggaaaatt ctccgaatgt ttttggtttt ttggctgctt 5280

ggaatttact tctgccacct gctggtcatc actgtcctca ctaagtggat tctggctccc 5340

ccgtacctca tggctcaaac taccactcct cagtcgctat attaaagctt atattttgct 5400

ggattactgc taaatacaaa agaaagttca atatgttttc atttctgtag ggaaaatggg 5460

attgctgctt taaatttctg agctagggat tttttggcag ctgcagtgtt ggcgactatt 5520

gtaaaattct ctttgtttct ctctgtaaat agcacctgct aacattacaa tttgtattta 5580

tgtttaaaga aggcatcatt tggtgaacag aactaggaaa tgaattttta gctcttaaaa 5640

gcatttgctt tgagaccgca caggagtgtc tttccttgta aaacagtgat gataatttct 5700

gccttggccc taccttgaag caatgttgtg tgaagggatg aagaatctaa aagtcttcat 5760

aagtccttgg gagaggtgct agaaaaatat aaggcactat cataattaca gtgatgtcct 5820

tgctgttact actcaaatca cccacaaatt tccccaaaga ctgcgctagc tgtcaaataa 5880

aagacagtga aattgacctg aaaaaaaaaa aaaaaaa 5917

<210> 187
<211> 76
<212> DNA
<213> Homo sapiens

<400> 187
gtgctggcat gccgcgccct cccagaggcc caccttcaag cagctggtgg aggacctgga 60

ccgtgtcctt accgtg 76

<210> 188
<211> 76
<212> DNA
<213> Homo sapiens

<400> 188
atgcgggagt gctggcatga cgcgccctcc cagaggccca ccttcaagca gctggtggag 60

gacctggacc gtgtcc 76

<210> 189
<211> 76
<212> DNA
<213> Homo sapiens

<400> 189
agctggtgga ggacctggac cgtgtcctta ccgtgacgtc caccgacgtg agtgctggct     60

ctggcctggt gccacc                                                     76


<210> 190
<211> 76
<212> DNA
<213> Homo sapiens

<400> 190
cccttaaaac aactcgttcc ctcagaccac acacaagaca gttcaagagg gactcaagga     60

cttacaggaa tgtcca                                                     76


<210> 191
<211> 76
<212> DNA
<213> Homo sapiens

<400> 191
aaccaaaggc tcagaccccc aggaatagaa aatataggcc cttaaaacaa ctcgttccct     60

cagaccacac acaaga                                                     76


<210> 192
<211> 76
<212> DNA
<213> Homo sapiens

<400> 192
tcaaggactt acaggaatgt ccagtgctcc caagaaatcg aactccacaa gcttggcttc     60

ccgcgcacgt cctgag                                                     76


<210> 193
<211> 76
<212> DNA
<213> Homo sapiens

<400> 193
ggacttacag gaatgtccag tgctcccaag aaatcgaact ccacaagctt ggcttcccgc     60

ggacgtcctg agggat                                                     76


<210> 194
<211> 76
<212> DNA
<213> Homo sapiens

<400> 194
cagaccacac acaagacagt tcaagaggga ctcaaggact tacaggaatg tccagtgctc     60

ccaagaaatc gaactc                                                     76


<210> 195
<211> 76

<212> DNA
<213> Homo sapiens

<400> 195
cttaccgtga cgtccaccga cgtgagtgct ggctctggcc tggtgccacc cgcctatgcc 60

cctcccctgc ccttag 76


<210> 196
<211> 76
<212> DNA
<213> Homo sapiens

<400> 196
aaacttgagg tataaggact gcttcctcaa ggccgactcc ttaaactggg gacaagaggg 60

caagtgatca ggtctg 76


<210> 197
<211> 76
<212> DNA
<213> Homo sapiens

<400> 197
aacttgaggt ataaggactg cttcctcaag gccgactcct taaactgggg acaagagggc 60

aagtgatcag gtctga 76


<210> 198
<211> 76
<212> DNA
<213> Homo sapiens

<400> 198
gcccgcaggt acatgatcat gcgggagtgc tggcatgccg cgccctccca gaggcccacc 60

ttcaagcagc tggtgg 76


<210> 199
<211> 76
<212> DNA
<213> Homo sapiens

<400> 199
accgtgacgt ccaccgacgt gagtgctggc tctggcctgg tgcgacccgc cgatctctct 60

cccctgtcct tttcct 76


<210> 200
<211> 76
<212> DNA
<213> Homo sapiens

<400> 200
tgggaggtgg cgggggccgg gggggggagg tgtgcaggtg agctccctgg cccttggccc 60

cctgccctct gggggg 76

<210> 201
<211> 76
<212> DNA
<213> Homo sapiens

<400> 201
   ctgggaatgg tggtgtctcg ggcagggttg tgggtgaccg ggggtgggag ggtgcggggg 60
   accggggggg ggaggg 76


<210> 202
<211> 76
<212> DNA
<213> Homo sapiens

<400> 202
agcgccctgc ccgcaggtac atgatcatgc gggagtgctg gcatgccgcg ccctcccaga 60

ggcccacctt caagca 76


<210> 203
<211> 76
<212> DNA
<213> Homo sapiens

<400> 203
gccaacgcca tgcccaggcc ggagagtccc ggggaggctg ctggtgggca gctgactgcg 60

gggacactgg gtggaa 76


<210> 204
<211> 76
<212> DNA
<213> Homo sapiens

<400> 204
aggccaccag aggccaacgc catgcccagg ccggagagtc ccggggaggc tgctggtggg 60

gaggcgaacg cgggga 76


<210> 205
<211> 76
<212> DNA
<213> Homo sapiens

<400> 205
tgcccaggcc ggagagtccc ggggcggctg ctggggggga gctgactggg ggggcactgg 60

gggggagacc cgggcc 76


<210> 206
<211> 76
<212> DNA
<213> Homo sapiens

<400> 206
taggccctta aaacaactcg ttccctcaga ccacacacaa gacagttcaa gagggactca 60

aggacttaca ggaatg                                                    76

<210> 207
<211> 76
<212> DNA
<213> Homo sapiens

<400> 207
actcaaggac ttacaggaat gtccagtgct cccaagaaat cgaactccac aagcttggct      60

tcccgcggac gtcctg                                                    76

<210> 208
<211> 76
<212> DNA
<213> Homo sapiens

<400> 208
aggcccttaa aacaactcgt tccctcagac cacacacaag acagttcaag agggactcaa      60

ggacttacag gaatgt                                                    76

<210> 209
<211> 76
<212> DNA
<213> Homo sapiens

<400> 209
gccctcccag aggcccacct tcaagcagct ggtggaggac ctggaccgtg tccttaccgt      60

gacgtccacc gacgtg                                                    76

<210> 210
<211> 76
<212> DNA
<213> Homo sapiens

<400> 210
gccgcgccct cccagaggcc caccttcaag cagctggtgg aggacctgga ccgtgtcctt      60

accgtgacgt ccaccg                                                    76

<210> 211
<211> 76
<212> DNA
<213> Homo sapiens

<400> 211
ggtggaggac ctggaccgtg accttaccgg gacgtccacc gacgggagtg ctggctctgg      60

cctggtgcca cccgcc                                                    76

<210> 212
<211> 76
<212> DNA
<213> Homo sapiens

<400> 212
gacgtccacc gacgtgagtg ctggctctgg cctggtgcca cccgcctatg cccctccccc     60

tgccgtcccc ggccat                                                     76


<210> 213
<211> 76
<212> DNA
<213> Homo sapiens

<400> 213
tgtccttacc gtgacgtcca ccgacgtgag tgctggctct ggcctggtgc cacccgccta     60

tgcccctccc cctgcc                                                     76


<210> 214
<211> 76
<212> DNA
<213> Homo sapiens

<400> 214
tacctgctgg tctcggtggc cacgggcact ggtctaccag ggctgtccct ccggaggggg     60

tcaaacttga gggata                                                     76


<210> 215
<211> 76
<212> DNA
<213> Homo sapiens

<400> 215
ctggaccgtg tccttaccgt gacgtccacc gacgtgagtg ctggctctgg cctggtgcca     60

cccgcccatg cccctc                                                     76


<210> 216
<211> 76
<212> DNA
<213> Homo sapiens

<400> 216
tgtccttacc gtgacgtcca ccgacgtgag tgctggctct ggcctggtgc cacccgccta     60

tgcccctccc ctgccc                                                     76


<210> 217
<211> 76
<212> DNA
<213> Homo sapiens

<400> 217
aaaagattta agtttagatc tttaatatac ctagaacggt ggctgtaacc agcaaggcag     60

gagccctttg tgttgg                                                     76


<210> 218

<211> 76
<212> DNA
<213> Homo sapiens

<400> 218
tgggtcaaac ttgaggtata aggactgctt cctcaaggcc gactccttat actggggaca 60

agagggcaag tgatca 76


<210> 219
<211> 76
<212> DNA
<213> Homo sapiens

<400> 219
gagtgctggc tctggcctgg tgccacccgc ctatgcccct ccccctggcg tccccggcca 60

tcctgccccc cagagt 76


<210> 220
<211> 76
<212> DNA
<213> Homo sapiens

<400> 220
gagtgctggc tctggcctgg tgccacccgc ctatgcccct ccccctgccg tccccggcca 60

tcctgccccc cagagt 76


<210> 221
<211> 76
<212> DNA
<213> Homo sapiens

<400> 221
gccaacgcca tgcccaggcc ggagagtccc ggggaggctg ctggtgggga gctgacttcg 60

gggacactgg ggggaa 76


<210> 222
<211> 76
<212> DNA
<213> Homo sapiens

<400> 222
catgcgggag tgctggcatg gcgcgccctc ccagcggccc accttcaagc agctggtggg 60

ggacctggac cgtgtc 76


<210> 223
<211> 76
<212> DNA
<213> Homo sapiens

<400> 223
acgtgagtgc tggctctggc ctggtgccac ccgcctatgc ccctccccct gccgtccccg 60

gccatcctgc ccccca 76

<210> 224
<211> 76
<212> DNA
<213> Homo sapiens

<400> 224
ccctcccaga ggcccacctt caagcagctg gtggaggacc tggaccgtgt ccttaccgtg 60

acgtccaccg acgtga 76


<210> 225
<211> 76
<212> DNA
<213> Artificial Sequence

<220>
<223> Description of Artificial Sequence: Synthetic
      oligonucleotide

<400> 225
ggacttacag gaatgtccag tgctcccaag aaatcgaact ccacaagctt ggcttcccgc 60

ggacgtcctg agggat 76


<210> 226
<211> 76
<212> DNA
<213> Artificial Sequence

<220>
<223> Description of Artificial Sequence: Synthetic
      oligonucleotide

<400> 226
catccctcag gacgtccgcg ggaagccaag cttgtggagt tcgatttctt gggagcactg 60

gacattcctg taagtc 76


<210> 227
<211> 76
<212> DNA
<213> Artificial Sequence

<220>
<223> Description of Artificial Sequence: Synthetic
      oligonucleotide

<400> 227
catccctcag gacgtccgcg ggaagccaag cttgtggagt tcgatttctt gggagcactg 60

gacattcctg taagtc 76


<210> 228
<211> 76
<212> DNA
<213> Artificial Sequence

<220>
<223> Description of Artificial Sequence: Synthetic
      oligonucleotide

<400> 228
catccctcag gacgtccgcg ggaagccaag cttgtggagt tcgatttctt gggagcactg        60

gacattcctg taagtc                                                        76


<210> 229
<211> 76
<212> DNA
<213> Artificial Sequence

<220>
<223> Description of Artificial Sequence: Synthetic
      oligonucleotide

<400> 229
gccatccctc aggacgtccg cgggaagcca agcttgtgga gttcgatttc ttgggagcac        60

tggacattcc tgtaag                                                        76


<210> 230
<211> 76
<212> DNA
<213> Artificial Sequence

<220>
<223> Description of Artificial Sequence: Synthetic
      oligonucleotide

<400> 230
ccggccatcc ctcaggacgt ccgcgggaag ccaagcttgt ggagttcgat ttcttgggag        60

cactggacat tcctgt                                                        76


<210> 231
<211> 76
<212> DNA
<213> Artificial Sequence

<220>
<223> Description of Artificial Sequence: Synthetic
      oligonucleotide

<400> 231
caggaatgtc cagtgctacc aagaaatcga actccacaag cttgggttcc cgcggacgtc        60

ctccgggatg gccgtg                                                        76


<210> 232
<211> 76
<212> DNA
<213> Artificial Sequence

<220>
<223> Description of Artificial Sequence: Synthetic
      oligonucleotide

<400> 232
caggaatgtc cagtgctccc aagaaatcga actccacaag cttggcttcc cgcggacgtc     60

ctgagggatg gccggg                                                     76


<210> 233
<211> 76
<212> DNA
<213> Artificial Sequence

<220>
<223> Description of Artificial Sequence: Synthetic
      oligonucleotide

<400> 233
tccccggcca tccctcagga cgtccgcggg aagccaagct tgtggagttc gatttcttgg     60

gagcactgga cattcc                                                     76


<210> 234
<211> 76
<212> DNA
<213> Artificial Sequence

<220>
<223> Description of Artificial Sequence: Synthetic
      oligonucleotide


<220>
<221> modified_base
<222> (21)..(21)
<223> a, c, t, g, unknown or other

<400> 234
tccccggcca tccctcagga ngtccgcggg aagccaagct tgtggagttc gatttcttgg     60

gagcactgga cattcc                                                     76


<210> 235
<211> 76
<212> DNA
<213> Artificial Sequence

<220>
<223> Description of Artificial Sequence: Synthetic
      oligonucleotide

<400> 235
gtccccggcc atccctcagg acgtccgcgg gaagccaagc ttgtggagtt cgatttcttg     60

ggagcactgg acattc                                                     76


<210> 236
<211> 76
<212> DNA
<213> Artificial Sequence

<220>
<223> Description of Artificial Sequence: Synthetic
      oligonucleotide

<400> 236
ccgtccccgg ccatccctca ggacgtccgc gggaagccaa gcttgtggag ttcgatttct        60

tgggagcact ggacat                                                        76


<210> 237
<211> 76
<212> DNA
<213> Artificial Sequence

<220>
<223> Description of Artificial Sequence: Synthetic
      oligonucleotide

<400> 237
tgctcccaag aaatcgaact ccacaagctt ggcttcccgc ggacgtcctg agggatggcc        60

ggggacggca ggggga                                                        76


<210> 238
<211> 76
<212> DNA
<213> Artificial Sequence

<220>
<223> Description of Artificial Sequence: Synthetic
      oligonucleotide

<400> 238
   aagaaatcga actccacaag cttggcttcc cgcggacgtc ctgagggatg gccggggacg        60
   gcagggggag gggcat                                                         76


<210> 239
<211> 76
<212> DNA
<213> Artificial Sequence

<220>
<223> Description of Artificial Sequence: Synthetic
      oligonucleotide

<400> 239
aaatcgaact ccacaagctt ggcttcccgc ggacgtcctg agggatggcc ggggacggca        60

gggggagggg catagg                                                        76


<210> 240
<211> 76
<212> DNA
<213> Artificial Sequence

<220>
<223> Description of Artificial Sequence: Synthetic
      oligonucleotide

<400> 240
ctggcctggt gccacccgcc tatgcccctc cccctgccgt ccccggccat ccctcaggac 60

gtccgcggga agccaa 76


<210> 241
<211> 76
<212> DNA
<213> Artificial Sequence

<220>
<223> Description of Artificial Sequence: Synthetic
      oligonucleotide

<400> 241
tcgtcccgcg gacttcctga tggatcgccg gggacggcag ggggaggggc ataggcgtgt 60

ggcaccaggc cagctc 76


<210> 242
<211> 76
<212> DNA
<213> Artificial Sequence

<220>
<223> Description of Artificial Sequence: Synthetic
      oligonucleotide


<220>
<221> modified_base
<222> (37)..(37)
<223> a, c, t, g, unknown or other

<400> 242
cttcccgcgg acgtcctgag ggatggccgg ggacggnagg gggaggggca taggcgggtg 60

gcaccaggcc agagcc 76


<210> 243
<211> 76
<212> DNA
<213> Artificial Sequence

<220>
<223> Description of Artificial Sequence: Synthetic
      oligonucleotide

<400> 243
gtgctggctc tggcctggtg ccacccgcct atgcccctcc ccctgccgtc cccggccatc 60

cctcaggacg tccgcg 76


<210> 244
<211> 76
<212> DNA
<213> Artificial Sequence

<220>
<223> Description of Artificial Sequence: Synthetic
      oligonucleotide

<400> 244
gagggatggc cggggacggc agggggaggg gcataggcgg gtggcaccag gccagagcca      60

gcactcacgt cggtgg                                                      76


<210> 245
<211> 76
<212> DNA
<213> Artificial Sequence

<220>
<223> Description of Artificial Sequence: Synthetic
      oligonucleotide

<400> 245
ggacaagagg gcaagtgatc aggtctgact gccatcccct aacacacaca gggggctaa      60

gggcagggga ggggca                                                      76


<210> 246
<211> 76
<212> DNA
<213> Artificial Sequence

<220>
<223> Description of Artificial Sequence: Synthetic
      oligonucleotide

<400> 246
ggacaagagg gcaagtgatc aggtctgact gccatcccct aacacacaca gggggctaa      60

gggcagggga ggggca                                                      76


<210> 247
<211> 76
<212> DNA
<213> Artificial Sequence

<220>
<223> Description of Artificial Sequence: Synthetic
      oligonucleotide

<400> 247
atgcccctcc cctgccctta gcccccctgt gtgtgttagg ggatggcagt cagacctgat      60

cacttgccct cttgtc                                                      76


<210> 248
<211> 76
<212> DNA
<213> Artificial Sequence

<220>
<223> Description of Artificial Sequence: Synthetic
      oligonucleotide

<400> 248
gcctatgccc ctcccctgcc cttagccccc ctgtgtgtgt taggggatgg cagtcagacc        60

tgatcacttg ccctct                                                        76


<210> 249
<211> 76
<212> DNA
<213> Artificial Sequence

<220>
<223> Description of Artificial Sequence: Synthetic
      oligonucleotide

<400> 249
gtgccacccg cctatgcccc tcccctgccc ttagcccccc tgtgtgtgtt aggggatggc        60

agtcagacct gatcac                                                        76


<210> 250
<211> 76
<212> DNA
<213> Artificial Sequence

<220>
<223> Description of Artificial Sequence: Synthetic
      oligonucleotide

<400> 250
tgactgccat cccctaacac acacaggggg gctaagggca ggggaggggc ataggcgggg        60

ggcaccaggc cagagc                                                        76


<210> 251
<211> 76
<212> DNA
<213> Artificial Sequence

<220>
<223> Description of Artificial Sequence: Synthetic
      oligonucleotide

<400> 251
tgactgccat cccctaacac acacaggggg gctaagggca ggggaggggc ataggcgggg        60

ggcaccaggc cagagc                                                        76


<210> 252
<211> 76
<212> DNA
<213> Artificial Sequence

<220>
<223> Description of Artificial Sequence: Synthetic
      oligonucleotide

<400> 252
   tgccatcccc taacacacac aggggggcta agggcagggg aggggcatag gcggggggca        60
   ccaggacaga ggcagc                                                    76

<210> 253
<211> 76
<212> DNA
<213> Artificial Sequence

<220>
<223> Description of Artificial Sequence: Synthetic
      oligonucleotide

<400> 253
cacacagggg ggctaagggc aggggagggg cataggcggg ggggaccagg cccgagccag      60

cactcacgtc gggggg                                                    76

<210> 254
<211> 76
<212> DNA
<213> Artificial Sequence

<220>
<223> Description of Artificial Sequence: Synthetic
      oligonucleotide

<400> 254
gacgtccacc gacgtgagtg ctggctctgg cctggtgcca cccgcctatg cccctcccct      60

gcccttagac cccctg                                                    76

<210> 255
<211> 76
<212> DNA
<213> Artificial Sequence

<220>
<223> Description of Artificial Sequence: Synthetic
      oligonucleotide

<400> 255
cttaccgtga cgtccaccga cgtgagtgct ggctctggcc tggtgccacc cgcctatgcc      60

cctcccctgc ccttag                                                    76

<210> 256
<211> 76
<212> DNA
<213> Artificial Sequence

<220>
<223> Description of Artificial Sequence: Synthetic
      oligonucleotide

<400> 256
tgtccttacc gtgacgtcca ccgacgtgag tgctggctct ggcctggtgc cacccgccta      60

tgcccctccc ctgccc                                                    76

<210> 257
<211> 76
<212> DNA
<213> Artificial Sequence

<220>
<223> Description of Artificial Sequence: Synthetic
      oligonucleotide

<400> 257
cgggggtggg agtgtgcggg tgaccggggg tgggagtgtg caggtgacct ccctggccct      60

tagcccctg cactct                                                      76


<210> 258
<211> 76
<212> DNA
<213> Artificial Sequence

<220>
<223> Description of Artificial Sequence: Synthetic
      oligonucleotide

<400> 258
   cgggtgaccg ggggagggag tgtgcagggg acctccctgg cccttagccc cctgcactct      60
   ggggggcagg atggcc                                                      76


<210> 259
<211> 76
<212> DNA
<213> Artificial Sequence

<220>
<223> Description of Artificial Sequence: Synthetic
      oligonucleotide

<400> 259
ggagtgtgca ggtgacctcc ctggccctta gccccctgca ctctgggggg caggatggcc      60

ggggacggca ggggga                                                      76


<210> 260
<211> 76
<212> DNA
<213> Artificial Sequence

<220>
<223> Description of Artificial Sequence: Synthetic
      oligonucleotide

<400> 260
ggggaggctg ctggtgggca gctgactgcg gggacactgg gaggaagcct ggaccctcag      60

cgaacttcgc ccagcc                                                      76


<210> 261
<211> 76

<212> DNA
<213> Artificial Sequence

<220>
<223> Description of Artificial Sequence: Synthetic
      oligonucleotide

<400> 261
acagcctggg cacagaggtg gctgtgcgaa ggtcgctgag ggtccaggct tccacccagt      60

gtccccgcag tcagct                                                      76


<210> 262
<211> 76
<212> DNA
<213> Artificial Sequence

<220>
<223> Description of Artificial Sequence: Synthetic
      oligonucleotide

<400> 262
tgactgcggg gacactgggt ggaagcctgg accctcagcg accttcgcac agccacctct      60

gtgcccaggc tgtgcc                                                      76


<210> 263
<211> 76
<212> DNA
<213> Artificial Sequence

<220>
<223> Description of Artificial Sequence: Synthetic
      oligonucleotide

<400> 263
cggggacact gggtggaagc ctggaccctc agcgaccttc gcacagccac ctctgtgccc      60

aggctgtgcc ccagaa                                                      76


<210> 264
<211> 76
<212> DNA
<213> Artificial Sequence

<220>
<223> Description of Artificial Sequence: Synthetic
      oligonucleotide

<400> 264
ggggacactg ggtggaagcc tggaccctca gcgaccttcg cacagccacc tctgtggcca      60

ggctgtgcca cagaag                                                      76


<210> 265
<211> 76
<212> DNA
<213> Artificial Sequence

<220>
<223> Description of Artificial Sequence: Synthetic
      oligonucleotide

<400> 265
ggacactggg tggaagcctg gaccctcagc gaccttcgca cagccacctc tgtgcccagg        60

ctgtgcccca gaaggc                                                        76


<210> 266
<211> 76
<212> DNA
<213> Artificial Sequence

<220>
<223> Description of Artificial Sequence: Synthetic
      oligonucleotide

<400> 266
gaagcctgga ccctcagcga ccttcgcaca gccacctctg tgccccggct gtgccccagc        60

cggcccgccc cacacc                                                        76


<210> 267
<211> 76
<212> DNA
<213> Artificial Sequence

<220>
<223> Description of Artificial Sequence: Synthetic
      oligonucleotide

<400> 267
gaagcctgga ccctcagcga ccttcgcaca gccacctctg tgcccaggct gtgccccaga        60

aggcccgccc cacacc                                                        76


<210> 268
<211> 76
<212> DNA
<213> Artificial Sequence

<220>
<223> Description of Artificial Sequence: Synthetic
      oligonucleotide

<400> 268
tggaccctca gcgaccttcg cacagccacc tctgtgccca ggctgtgccc cagaaggccc        60

gccccacacc tcagca                                                        76


<210> 269
<211> 76
<212> DNA
<213> Artificial Sequence

<220>
<223> Description of Artificial Sequence: Synthetic
      oligonucleotide

<400> 269
gaccctcagc gaccttcgca cagccacctc tgtgcccagg ctgtgcccca gaaggcccgc 60

cccacacctc agcact 76


<210> 270
<211> 76
<212> DNA
<213> Artificial Sequence

<220>
<223> Description of Artificial Sequence: Synthetic
      oligonucleotide

<400> 270
tcagcgacct tcgcacagcc acctctgtgc ccaggctgtg ccccagaagg cccgccccac 60

acctcagcac tctggg 76


<210> 271
<211> 76
<212> DNA
<213> Artificial Sequence

<220>
<223> Description of Artificial Sequence: Synthetic
      oligonucleotide

<400> 271
ccttcgcaca gccacctctg tgcccaggct gtgccccaga aggcccgccc cacacctcag 60

cactctgggg ggcagg 76


<210> 272
<211> 76
<212> DNA
<213> Artificial Sequence

<220>
<223> Description of Artificial Sequence: Synthetic
      oligonucleotide

<400> 272
gacgtccacc gacgtgagtg ctggctctgg cctggtgcca cccgcctatg cccctccccc 60

tgccgtcccc ggccat 76


<210> 273
<211> 76
<212> DNA
<213> Artificial Sequence

<220>
<223> Description of Artificial Sequence: Synthetic
      oligonucleotide

<400> 273
caagagggac tcaaggactt acaggaatgt ccagtgctcc caagaaatcg aactccacaa 60

gcttggcttc ccgcgg                                                    76

<210> 274
<211> 76
<212> DNA
<213> Artificial Sequence

<220>
<223> Description of Artificial Sequence: Synthetic
      oligonucleotide

<400> 274
caagagggac tcaaggactt acaggaatgt ccagtgctcc caagaaatcg aactccacaa      60

gcttggcttc ccgcgg                                                    76

<210> 275
<211> 76
<212> DNA
<213> Artificial Sequence

<220>
<223> Description of Artificial Sequence: Synthetic
      oligonucleotide

<400> 275
ataggccctt aaaacaactc gttccctcag accacacaca agacagttca agagggactc      60

aaggacttac aggaat                                                    76

<210> 276
<211> 76
<212> DNA
<213> Artificial Sequence

<220>
<223> Description of Artificial Sequence: Synthetic
      oligonucleotide

<400> 276
tcaagaggga ctcaaggact tacaggaatg tccagtgctc ccaagaaatc gaactccaca      60

agcttggctt cccgcg                                                    76

<210> 277
<211> 76
<212> DNA
<213> Artificial Sequence

<220>
<223> Description of Artificial Sequence: Synthetic
      oligonucleotide

<400> 277
accacacaca agacagttca agagggactc aaggacttac aggaatgtcc agtgctccca      60

agaaatcgaa ctccac                                                    76

<210> 278
<211> 76
<212> DNA
<213> Artificial Sequence

<220>
<223> Description of Artificial Sequence: Synthetic
      oligonucleotide

<400> 278
gagctggcct ggtgccacac gcctatgccc ctccccctgc cgtccccggc gatccatcag      60

gaagtccgcg ggacga                                                      76


<210> 279
<211> 76
<212> DNA
<213> Artificial Sequence

<220>
<223> Description of Artificial Sequence: Synthetic
      oligonucleotide

<400> 279
ccaccgacgt gagtgctggc tctggcctgg tgccacccgc ctatgcccct ccccctgccg      60

tccccggcca tccctc                                                      76


<210> 280
<211> 76
<212> DNA
<213> Artificial Sequence

<220>
<223> Description of Artificial Sequence: Synthetic
      oligonucleotide

<400> 280
caagagcctc agacagtgca tgagggaccc gagacagtgc ggcgagggaa cagcacagcg      60

gccccatgcc cccaac                                                      76


<210> 281
<211> 76
<212> DNA
<213> Artificial Sequence

<220>
<223> Description of Artificial Sequence: Synthetic
      oligonucleotide

<400> 281
caagagcctc agacagtgca tgagggaccc gagacagtgc ggcgagggaa cagcacaggg      60

gccccatgcc cccaac                                                      76


<210> 282
<211> 76

<212> DNA
<213> Artificial Sequence

<220>
<223> Description of Artificial Sequence: Synthetic
      oligonucleotide

<400> 282
cgttccctca gaccacacac aagacagttc aagagggact caaggactta caggaatgtc      60

cagtgctccc aagaga                                                      76


<210> 283
<211> 76
<212> DNA
<213> Artificial Sequence

<220>
<223> Description of Artificial Sequence: Synthetic
      oligonucleotide

<400> 283
ccaggaatag aaaatatagg cccttaaaac aactcgttcc ctcagaccac acacaagaca      60

gttcaagagg gactca                                                      76


<210> 284
<211> 76
<212> DNA
<213> Artificial Sequence

<220>
<223> Description of Artificial Sequence: Synthetic
      oligonucleotide


<220>
<221> modified_base
<222> (40)..(40)
<223> a, c, t, g, unknown or other

<400> 284
ggctctggcc tggtgccacc cgcctatgcc cctccccctn ccgtccccgg ccatccctca      60

ggacgtccgc gggaag                                                      76


<210> 285
<211> 76
<212> DNA
<213> Artificial Sequence

<220>
<223> Description of Artificial Sequence: Synthetic
      oligonucleotide

<400> 285
gccctgcccg caggtacatg atcatgcggg agtgctggca tgccgcgccc tcccagaggc      60

ccaccttcaa gcagct                                                      76

<210> 286
<211> 76
<212> DNA
<213> Artificial Sequence

<220>
<223> Description of Artificial Sequence: Synthetic
      oligonucleotide

<400> 286
ctggcatgcc gcgccctccc agaggcccac ctttaagcag ctggtagagg gcctggaccg      60

tgtccttacc gtgacg                                                      76


<210> 287
<211> 76
<212> DNA
<213> Artificial Sequence

<220>
<223> Description of Artificial Sequence: Synthetic
      oligonucleotide

<400> 287
taaaacaact cgttccctca gaccacacac aagacagttc aagagggact caaggactta      60

caggaatgtc cagtgc                                                      76


<210> 288
<211> 76
<212> DNA
<213> Artificial Sequence

<220>
<223> Description of Artificial Sequence: Synthetic
      oligonucleotide

<400> 288
cacggccatc ccggaggacg tccgcgggaa cccaagcttg tggagttcga tttcttggta      60

gcactggaca ttcctg                                                      76


<210> 289
<211> 76
<212> DNA
<213> Artificial Sequence

<220>
<223> Description of Artificial Sequence: Synthetic
      oligonucleotide

<400> 289
tccccctgcc gtccccggcc atccctcagg acgtccgcgg gaagccaagc ttgtggagtt      60

cgatttcttg ggagca                                                      76


<210> 290
<211> 76

<212> DNA
<213> Artificial Sequence

<220>
<223> Description of Artificial Sequence: Synthetic
      oligonucleotide

<400> 290
agaccacaca caagacagtt caagagggac tcaaggactt acaggaatgt ccagtgctcc      60

caagaaatcg aactcc                                                      76


<210> 291
<211> 76
<212> DNA
<213> Artificial Sequence

<220>
<223> Description of Artificial Sequence: Synthetic
      oligonucleotide

<400> 291
cccggccatc cctcaggacg tccgcgggaa gccaagcttg tggagttcga tttcttggga      60

gcactggaca ttcctg                                                      76


<210> 292
<211> 76
<212> DNA
<213> Artificial Sequence

<220>
<223> Description of Artificial Sequence: Synthetic
      oligonucleotide

<400> 292
ggcatgccgc gccctcccag aggcccacct tcaagcagct ggtggaggac ctggaccgtg      60

tccttaccgt gacgtc                                                      76


<210> 293
<211> 76
<212> DNA
<213> Artificial Sequence

<220>
<223> Description of Artificial Sequence: Synthetic
      oligonucleotide

<400> 293
ggcatgccgc gccctcccag aggcccacct tcaagcagct ggtggaggac ctggaccgtg      60

tccttaccgt gacgtc                                                      76


<210> 294
<211> 76
<212> DNA
<213> Artificial Sequence

<220>
<223> Description of Artificial Sequence: Synthetic
      oligonucleotide

<400> 294
cggcgcacat acctgctggt ctcggtggcc acgggcactg gtctaccagg actgtccctc      60

aggagggggt caaact                                                      76


<210> 295
<211> 76
<212> DNA
<213> Artificial Sequence

<220>
<223> Description of Artificial Sequence: Synthetic
      oligonucleotide

<400> 295
atacctgctg gtctcggtgg ccacgggcac tggtctacca ggactgtccc tcaggagggg      60

gtcaaacttg aggtat                                                      76


<210> 296
<211> 76
<212> DNA
<213> Artificial Sequence

<220>
<223> Description of Artificial Sequence: Synthetic
      oligonucleotide

<400> 296
aggtataagg actgcttcct caaggccgac tccttaaact ggggacaaga gggcaagtga      60

tcaggtctga ctgcca                                                      76


<210> 297
<211> 76
<212> DNA
<213> Artificial Sequence

<220>
<223> Description of Artificial Sequence: Synthetic
      oligonucleotide

<400> 297
ggaggacctg gactgtgtcc ttaccgtgac gtccaccgac gtgagtgctg gctctggcct      60

ggtgccaccc gcctat                                                      76


<210> 298
<211> 76
<212> DNA
<213> Artificial Sequence

<220>
<223> Description of Artificial Sequence: Synthetic
      oligonucleotide

<400> 298
ggaggacctg gaccgtgtcc ttaccgtgac gtccaccgac gtgagtgctg gctctggcct     60

ggtgccaccc gcctat                                                     76

<210> 299
<211> 76
<212> DNA
<213> Artificial Sequence

<220>
<223> Description of Artificial Sequence: Synthetic
      oligonucleotide

<400> 299
caagcagctg gtggaggacc tggaccgtgt ccttaccgtg acgtccaccg acgtgagtgc     60

tggctctggc ctggtg                                                     76

<210> 300
<211> 76
<212> DNA
<213> Artificial Sequence

<220>
<223> Description of Artificial Sequence: Synthetic
      oligonucleotide

<400> 300
accttcaagc agctggtgga ggacctggac cgtgtcctta ccgtgacgtc caccgacgtg     60

agtgctggct ctggcc                                                     76

<210> 301
<211> 76
<212> DNA
<213> Artificial Sequence

<220>
<223> Description of Artificial Sequence: Synthetic
      oligonucleotide

<400> 301
caaacttgag gtataaggac tgcttcctca aggccgactc cttaaactgg ggacaagagg     60

gcaagtgatc aggtct                                                     76

<210> 302
<211> 76
<212> DNA
<213> Artificial Sequence

<220>
<223> Description of Artificial Sequence: Synthetic
      oligonucleotide

<400> 302
tacctgctgg tctcggtggc cacgggcact ggtctaccag ggctgtccct ccggaggggg     60

tcaaacttga gggata                                                    76

<210> 303
<211> 76
<212> DNA
<213> Artificial Sequence

<220>
<223> Description of Artificial Sequence: Synthetic
      oligonucleotide

<400> 303
aacttgaggt ataaggactg cttcctcaag gccgactcct taaactgggg acaagagggc      60

aagtgatcag gtctga                                                    76

<210> 304
<211> 76
<212> DNA
<213> Artificial Sequence

<220>
<223> Description of Artificial Sequence: Synthetic
      oligonucleotide

<400> 304
agctggtgga ggacctggac cgtgtcctta ccgtgacgtc caccgacgtg agtgctggct      60

ctggcctggt gccacc                                                    76

<210> 305
<211> 76
<212> DNA
<213> Artificial Sequence

<220>
<223> Description of Artificial Sequence: Synthetic
      oligonucleotide

<400> 305
gcgccctccc agaggcccac cttcaagcag ctggtggagg acctggaccg tgtccttacc      60

gtgacgtcca ccgacg                                                    76

<210> 306
<211> 76
<212> DNA
<213> Artificial Sequence

<220>
<223> Description of Artificial Sequence: Synthetic
      oligonucleotide

<400> 306
gcgggagtgc tggcatgccg cgccctccca gaggcccacc ttcaagcagc tggtggagga      60

cctggaccgt gtcctt                                                    76

<210> 307
<211> 76
<212> DNA
<213> Artificial Sequence

<220>
<223> Description of Artificial Sequence: Synthetic
      oligonucleotide

<400> 307
cctccactgg gtcctcaggg gtgggggtcc ctccggggct gggcggggga gggactggca      60

ggcctgcagg ggggtt                                                      76


<210> 308
<211> 76
<212> DNA
<213> Artificial Sequence

<220>
<223> Description of Artificial Sequence: Synthetic
      oligonucleotide

<400> 308
tcacggcagc aagaaccaca ctcactgctg caaggccacc agaggccaac gccatgccca      60

ggccggagag tcccgg                                                      76


<210> 309
<211> 76
<212> DNA
<213> Artificial Sequence

<220>
<223> Description of Artificial Sequence: Synthetic
      oligonucleotide

<400> 309
tacatgatca tgcgggaggg ctggcatgcc gcgccctccc agaggcccac cttcaagcag      60

ctggtggagg gccggg                                                      76


<210> 310
<211> 76
<212> DNA
<213> Artificial Sequence

<220>
<223> Description of Artificial Sequence: Synthetic
      oligonucleotide

<400> 310
ggtgggaagc ggcggggctc actcctgagc gccctgcccg cagggacatg atcatgcggg      60

ggtgctggcc ttgcgg                                                      76


<210> 311
<211> 76

<212> DNA
<213> Artificial Sequence

<220>
<223> Description of Artificial Sequence: Synthetic
      oligonucleotide

<400> 311
gcgccctccc agaggcccac cttcaagcag ctggtggagg acctggaccg tgtccttacc      60

gtgacgtcca ccgacg                                                      76


<210> 312
<211> 76
<212> DNA
<213> Artificial Sequence

<220>
<223> Description of Artificial Sequence: Synthetic
      oligonucleotide

<400> 312
cctgcccccc agagtgctga ggtgtggggc gggccttctg gggcacagcc tgggcacaga      60

ggtggctgtg cgaagg                                                      76


<210> 313
<211> 76
<212> DNA
<213> Artificial Sequence

<220>
<223> Description of Artificial Sequence: Synthetic
      oligonucleotide

<400> 313
gcaggtacat gatcatgcgg gagtgccggc atttcgggac cttccctcgg gccaccctct      60

tccggttgtt gtgggc                                                      76


<210> 314
<211> 76
<212> DNA
<213> Artificial Sequence

<220>
<223> Description of Artificial Sequence: Synthetic
      oligonucleotide

<400> 314
gcaggtacat gatcatgcgg gagtgctggc atgccgcgcc ctccccgagg accaccttcc      60

agcagccggg ggaggg                                                      76


<210> 315
<211> 76
<212> DNA
<213> Artificial Sequence

<220>
<223> Description of Artificial Sequence: Synthetic
      oligonucleotide

<400> 315
cccgaataag gtgggaagcg gcggggctca ctcctgagcg ccctgaccgc aggtacatga        60

gcatgcggga gtggcg                                                        76


<210> 316
<211> 76
<212> DNA
<213> Artificial Sequence

<220>
<223> Description of Artificial Sequence: Synthetic
      oligonucleotide

<400> 316
   cgtgtcctta ccgtgacgtc caccgacgtg agtgctggct ctggcctggt gccacccgcc        60
   tatgcccctc cccctg                                                         76


<210> 317
<211> 76
<212> DNA
<213> Artificial Sequence

<220>
<223> Description of Artificial Sequence: Synthetic
      oligonucleotide

<400> 317
acatgatcat gcgggagtgc tggcatgccg cgccccccca gaggcccacc ttcaagcagc        60

tggtggagga cctgga                                                        76


<210> 318
<211> 76
<212> DNA
<213> Artificial Sequence

<220>
<223> Description of Artificial Sequence: Synthetic
      oligonucleotide

<400> 318
gccttctggg gcacagcctg ggcacagagg tggctgtgcg aaggtcgctg agggtccagg        60

cttccaccca gtgtcc                                                        76


<210> 319
<211> 27
<212> DNA
<213> Artificial Sequence

<220>
<223> Description of Artificial Sequence: Synthetic
      oligonucleotide

<220>
<221> CDS
<222> (2)..(25)

<400> 319
t ccg gat gaa ctg gtc cct gtc ctt cg                                    27
  Pro Asp Glu Leu Val Pro Val Leu
  1               5


<210> 320
<211> 8
<212> PRT
<213> Artificial Sequence

<220>
<223> Description of Artificial Sequence: Synthetic
      peptide

<400> 320
Pro Asp Glu Leu Val Pro Val Leu
1               5


<210> 321
<211> 26
<212> DNA
<213> Artificial Sequence

<220>
<223> Description of Artificial Sequence: Synthetic
      oligonucleotide


<220>
<221> CDS
<222> (1)..(24)

<400> 321
ctg atc gac ctg gat gtc ccc gtc ga                                      26
Leu Ile Asp Leu Asp Val Pro Val
1               5


<210> 322
<211> 8
<212> PRT
<213> Artificial Sequence

<220>
<223> Description of Artificial Sequence: Synthetic
      peptide

<400> 322
Leu Ile Asp Leu Asp Val Pro Val
1               5

<210> 323
<211> 26
<212> DNA
<213> Artificial Sequence

<220>
<223> Description of Artificial Sequence: Synthetic
      oligonucleotide

<220>
<221> CDS
<222> (3)..(26)

<400> 323
at gct gtc aga ggg ttt tgt gat gaa                                    26
   Ala Val Arg Gly Phe Cys Asp Glu
   1               5

<210> 324
<211> 8
<212> PRT
<213> Artificial Sequence

<220>
<223> Description of Artificial Sequence: Synthetic
      peptide

<400> 324
Ala Val Arg Gly Phe Cys Asp Glu
1               5

<210> 325
<211> 27
<212> DNA
<213> Artificial Sequence

<220>
<223> Description of Artificial Sequence: Synthetic
      oligonucleotide

<220>
<221> CDS
<222> (3)..(26)

<400> 325
ac tac gat aaa aga cat aac cag tgc g                                  27
   Tyr Asp Lys Arg His Asn Gln Cys
   1               5

<210> 326
<211> 8
<212> PRT
<213> Artificial Sequence

<220>
<223> Description of Artificial Sequence: Synthetic
      peptide

<400> 326
Tyr Asp Lys Arg His Asn Gln Cys
1               5

<210> 327
<211> 176
<212> DNA
<213> Artificial Sequence

<220>
<223> Description of Artificial Sequence: Synthetic
      polynucleotide

<400> 327
cgggggtggc cccccctcgg gggacagcgc atgcccgctg cgcaccatca agagagtcca      60

gttcggagtc ctgagtccgg atgaactggt ccctgtcctt cgaatggtgg aaggtgatac     120

catctatgat tactgctggt attctctgat gtcctcagcc cagccagaca cctcct         176


<210> 328
<211> 59
<212> PRT
<213> Artificial Sequence

<220>
<223> Description of Artificial Sequence: Synthetic
      polypeptide


<220>
<221> MOD_RES
<222> (59)..(59)
<223> Any amino acid

<400> 328
Gly Gly Gly Pro Pro Ser Gly Asp Ser Ala Cys Pro Leu Arg Thr Ile
1               5                   10                  15


Lys Arg Val Gln Phe Gly Val Leu Ser Pro Asp Glu Leu Val Pro Val
            20                  25                  30


Leu Arg Met Val Glu Gly Asp Thr Ile Tyr Asp Tyr Cys Trp Tyr Ser
        35                  40                  45


Leu Met Ser Ser Ala Gln Pro Asp Thr Ser Xaa
    50                  55


<210> 329
<211> 90
<212> DNA
<213> Artificial Sequence

<220>
<223> Description of Artificial Sequence: Synthetic
      oligonucleotide

<400> 329
tggtccctgt ccttcgaatg gtggaaggtg ataccatcta tgattactgc tggtattctc      60

tgatgtcctc agcccagcca gacacctcct                                      90

<210> 330
<211> 90
<212> DNA
<213> Artificial Sequence

<220>
<223> Description of Artificial Sequence: Synthetic
      oligonucleotide

<400> 330
ctggtccctg tccttcgaat ggtggaaggt gataccatct atgattactg ctggtattct      60

ctgatgtcct cagcccggcc agacacctcc                                        90


<210> 331
<211> 90
<212> DNA
<213> Artificial Sequence

<220>
<223> Description of Artificial Sequence: Synthetic
      oligonucleotide

<400> 331
actggtccct gtccttcgaa tggtggaagg tgataccatc tatgattact gctggtattc      60

tctgatgtcc tcagcccagc cagacaccac                                        90


<210> 332
<211> 90
<212> DNA
<213> Artificial Sequence

<220>
<223> Description of Artificial Sequence: Synthetic
      oligonucleotide

<400> 332
aactggtccc tgtccttcga atggtggaag gtgataccat ctatgattac tgctggtatt      60

ctctgatgtc ctcagcccag ccagacacct                                        90


<210> 333
<211> 90
<212> DNA
<213> Artificial Sequence

<220>
<223> Description of Artificial Sequence: Synthetic
      oligonucleotide

<400> 333
gaactggtcc ctgtccttcg aatggtggaa ggtgatacca tctatgatta ctgctggtat      60

tctctgatgt cctcagccca gccagacacc                                        90


<210> 334

<211> 90
<212> DNA
<213> Artificial Sequence

<220>
<223> Description of Artificial Sequence: Synthetic
      oligonucleotide

<400> 334
tgaactggtc cctgtccttc gaatggtgga aggtgatacc atctatgatt actgctggta        60

ttctctgatg tcctcagccc agccagacac                                          90


<210> 335
<211> 91
<212> DNA
<213> Artificial Sequence

<220>
<223> Description of Artificial Sequence: Synthetic
      oligonucleotide

<400> 335
atgaactggt ccctgtcctt cgaatggtgg aaggtgatac catctatgat tactgctgta        60

ttctctgatg tcctcagccc agccagacac c                                        91


<210> 336
<211> 90
<212> DNA
<213> Artificial Sequence

<220>
<223> Description of Artificial Sequence: Synthetic
      oligonucleotide

<400> 336
gatgaactgg tccctgtcct tcgaatggtg gaaggtgata ccatctatga ttactgctgg        60

tattctctga tgtcctcagc ccagccagac                                          90


<210> 337
<211> 90
<212> DNA
<213> Artificial Sequence

<220>
<223> Description of Artificial Sequence: Synthetic
      oligonucleotide

<400> 337
ggatgaactg gtccctgtcc ttcgaatggt ggaaggtgat accatctatg attactgctg        60

gtattctctg atgtcctcag cccagccaga                                          90


<210> 338
<211> 90
<212> DNA
<213> Artificial Sequence

<220>
<223> Description of Artificial Sequence: Synthetic
      oligonucleotide

<400> 338
cggatgaact ggtccctgtc cttcgaatgg tggaaggtga taccatctat gattactgct        60

ggtattctct gatgtcctca gcccagccag                                          90


<210> 339
<211> 90
<212> DNA
<213> Artificial Sequence

<220>
<223> Description of Artificial Sequence: Synthetic
      oligonucleotide

<400> 339
ccggatgaac tggtccctgt ccttcgaatg gtggaaggtg ataccatcta tgattactgc        60

tggtattctc tgatgtcctc agcccagcca                                          90


<210> 340
<211> 90
<212> DNA
<213> Artificial Sequence

<220>
<223> Description of Artificial Sequence: Synthetic
      oligonucleotide

<400> 340
tccggatgaa ctggtccctg tccttcgaat ggtggaaggt gataccatct atgattactg        60

ctggtattct ctgatgtcct cagcccagcc                                          90


<210> 341
<211> 90
<212> DNA
<213> Artificial Sequence

<220>
<223> Description of Artificial Sequence: Synthetic
      oligonucleotide

<400> 341
gtccggagga actggtccct gtccttcgaa tggtggaagg tgataccatc tatgattgct        60

gctggtattc tctgatgtcc tcagcccagc                                          90


<210> 342
<211> 90
<212> DNA
<213> Artificial Sequence

<220>
<223> Description of Artificial Sequence: Synthetic

oligonucleotide

<400> 342
agtccggatg aactggtccc tgtccttcga atggtggaag gtgataccat ctatgattac 60

tgctggtatt ctctgatgtc ctcagcccag 90


<210> 343
<211> 90
<212> DNA
<213> Artificial Sequence

<220>
<223> Description of Artificial Sequence: Synthetic
      oligonucleotide

<400> 343
gagtccggat gaactggtcc ctgtccttcg aatggtggaa ggtgatacca tctatgatta 60

ctgctggtat tctctgatgt cctcagcccg 90


<210> 344
<211> 90
<212> DNA
<213> Artificial Sequence

<220>
<223> Description of Artificial Sequence: Synthetic
      oligonucleotide

<400> 344
tgagtccgga tgaactggtc cctgtccttc gaatggtgga aggtgatacc atctatgatt 60

actgctggta ttctctgatg tcctcagccc 90


<210> 345
<211> 90
<212> DNA
<213> Artificial Sequence

<220>
<223> Description of Artificial Sequence: Synthetic
      oligonucleotide

<400> 345
ctgagtccgg atgaactggt ccctgtcctt cgaatggtgg aaggtgatac catctatgat 60

tactgctggt attctctgat gtcctcagcc 90


<210> 346
<211> 90
<212> DNA
<213> Artificial Sequence

<220>
<223> Description of Artificial Sequence: Synthetic
      oligonucleotide

<400> 346

cctgagtccg gatgaactgg tccctgtcct tcgaatggtg gaaggtgata ccatctatga 60

ttactgctgg tattctctga tgtcctcagc 90

<210> 347
<211> 90
<212> DNA
<213> Artificial Sequence

<220>
<223> Description of Artificial Sequence: Synthetic
      oligonucleotide

<400> 347
tcctgagtcc ggatgaactg gtccctgtcc ttcgaatggt ggaaggtgat accatctatg 60

attactgctg gtattctctg atgtcctcag 90

<210> 348
<211> 90
<212> DNA
<213> Artificial Sequence

<220>
<223> Description of Artificial Sequence: Synthetic
      oligonucleotide

<400> 348
gtcctgagtc cggatgaact ggtccctgtc cttcgaatgg tggaaggtga taccatctat 60

gattactgct ggtattctct gatgtcctca 90

<210> 349
<211> 90
<212> DNA
<213> Artificial Sequence

<220>
<223> Description of Artificial Sequence: Synthetic
      oligonucleotide

<400> 349
agtcctgagt ccggatgaac tggtccctgt ccttcgaatg gtggaaggtg ataccatcta 60

tgattactgc tggtattctc tgatgtcctc 90

<210> 350
<211> 90
<212> DNA
<213> Artificial Sequence

<220>
<223> Description of Artificial Sequence: Synthetic
      oligonucleotide

<400> 350
gagtcctgag tccggatgaa ctggtccctg tccttcgaat ggtggaaggt gataccatct 60

atgattactg ctggtattct ctgatgtcct 90

<210> 351
<211> 90
<212> DNA
<213> Artificial Sequence

<220>
<223> Description of Artificial Sequence: Synthetic
      oligonucleotide

<400> 351
ggagtcctga gtccggatga actggtccct gtccttcgaa tggtggaagg tgataccatc      60

tatgattact gctggtattc tctgatgtcc                                      90


<210> 352
<211> 90
<212> DNA
<213> Artificial Sequence

<220>
<223> Description of Artificial Sequence: Synthetic
      oligonucleotide

<400> 352
cggagtcctg agtccggatg aactggtccc tgtccttcga atggtggaag gtgataccat      60

ctatgattac tgctggtatt ctctgatgtc                                      90


<210> 353
<211> 90
<212> DNA
<213> Artificial Sequence

<220>
<223> Description of Artificial Sequence: Synthetic
      oligonucleotide

<400> 353
tcggagtcct gagtccggat gaactggtcc ctgtccttcg aatggtggaa ggtgatacca      60

tctatgatta ctgctggtat tctctgatgt                                      90


<210> 354
<211> 90
<212> DNA
<213> Artificial Sequence

<220>
<223> Description of Artificial Sequence: Synthetic
      oligonucleotide

<400> 354
ttcggagtcc tgagtccgga tgaactggtc cctgtccttc gaatggtgga aggtgatacc      60

atctatgatt actgctggta ttctctgatg                                      90


<210> 355

<211> 90
<212> DNA
<213> Artificial Sequence

<220>
<223> Description of Artificial Sequence: Synthetic
      oligonucleotide

<400> 355
gttcggagtc ctgagtccgg atgaactggt ccctgtcctt cgaatggtgg aaggtgatac      60

catctatgat tactgctggt attctctgat                                        90


<210> 356
<211> 90
<212> DNA
<213> Artificial Sequence

<220>
<223> Description of Artificial Sequence: Synthetic
      oligonucleotide

<400> 356
cagttcggag tcctgagtcc ggatgaactg gtccctgtcc ttcgaatggt ggaaggtgat      60

accatctatg attactgctg gtattctctg                                        90


<210> 357
<211> 90
<212> DNA
<213> Artificial Sequence

<220>
<223> Description of Artificial Sequence: Synthetic
      oligonucleotide

<400> 357
ccagttcgga gtcctgagtc cggatgaact ggtccctgtc cttcgaatgg tggaaggtga      60

taccatctat gattactgct ggtattctct                                        90


<210> 358
<211> 90
<212> DNA
<213> Artificial Sequence

<220>
<223> Description of Artificial Sequence: Synthetic
      oligonucleotide

<400> 358
tccagttcgg agtcctgagt ccggatgaac tggtccctgt ccttcgaatg gtggaaggtg      60

ataccatcta tgattactgc tggtattctc                                        90


<210> 359
<211> 90
<212> DNA
<213> Artificial Sequence

<220>
<223> Description of Artificial Sequence: Synthetic
      oligonucleotide

<400> 359
gtccagttcg gagtcctgag tccggatgaa ctggtccctg tccttcgaat ggtggaaggt      60

gataccatct atgattactg ctggtattct                                        90


<210> 360
<211> 90
<212> DNA
<213> Artificial Sequence

<220>
<223> Description of Artificial Sequence: Synthetic
      oligonucleotide

<400> 360
gagtccagtt cggagtcctg agtccggatg aactggtccc tgtccttcga atggtggaag      60

gtgataccat ctatgattac tgctggtatt                                        90


<210> 361
<211> 90
<212> DNA
<213> Artificial Sequence

<220>
<223> Description of Artificial Sequence: Synthetic
      oligonucleotide

<400> 361
agagtccagt tcggagtcct gagtccggat gaactggtcc ctgtccttcg aatggtggaa      60

ggtgatacca tctatgatta ctgctggtat                                        90


<210> 362
<211> 90
<212> DNA
<213> Artificial Sequence

<220>
<223> Description of Artificial Sequence: Synthetic
      oligonucleotide

<400> 362
gagagtccag ttcggagtcc tgagtccgga tgaactggtc cctgtccttc gaatggtgga      60

aggtgatacc atctatgatt actgctggta                                        90


<210> 363
<211> 90
<212> DNA
<213> Artificial Sequence

<220>
<223> Description of Artificial Sequence: Synthetic

oligonucleotide

<400> 363
agagagtcca gttcggagtc ctgagtccgg atgaactggt ccctgtcctt cgaatggtgg 60

aaggtgatac catctatgat tactgctggt 90


<210> 364
<211> 90
<212> DNA
<213> Artificial Sequence

<220>
<223> Description of Artificial Sequence: Synthetic
      oligonucleotide

<400> 364
aagagagtcc agttcggagt cctgagtccg gatgaactgg tccctgtcct tcgaatggtg 60

gaaggggata ccatctatga ttactgccgg 90


<210> 365
<211> 90
<212> DNA
<213> Artificial Sequence

<220>
<223> Description of Artificial Sequence: Synthetic
      oligonucleotide

<400> 365
caagagagtc cagttcggag tcctgagtcc ggatgaactg gtccctgtcc ttcgaatggt 60

ggaaggtgat accatctatg attactgctg 90


<210> 366
<211> 90
<212> DNA
<213> Artificial Sequence

<220>
<223> Description of Artificial Sequence: Synthetic
      oligonucleotide

<400> 366
tcaagagagt ccagttcgga gtcctgagtc cggatgaact ggtccctgtc cttcgaatgg 60

tggaaggtga taccatctat gattactgct 90


<210> 367
<211> 90
<212> DNA
<213> Artificial Sequence

<220>
<223> Description of Artificial Sequence: Synthetic
      oligonucleotide

<400> 367

atcaagagag tccagttcgg agtcctgagt ccggatgaac tggtccctgt ccttcgaatg 60

gtggaaggtg ataccatcta tgattactgc 90

<210> 368
<211> 90
<212> DNA
<213> Artificial Sequence

<220>
<223> Description of Artificial Sequence: Synthetic
      oligonucleotide

<400> 368
caccatcaag agagtccagt tcggagtcct gagtccggat gaactggtcc ctgtccttcg 60

aatggtggaa ggtgatacca tctatgatta 90

<210> 369
<211> 90
<212> DNA
<213> Artificial Sequence

<220>
<223> Description of Artificial Sequence: Synthetic
      oligonucleotide

<400> 369
cgcaccatca agagagtcca gttcggagtc ctgagtccgg atgaactggt ccctgtcctt 60

cgaatggtgg aaggtgatac catctatgat 90

<210> 370
<211> 90
<212> DNA
<213> Artificial Sequence

<220>
<223> Description of Artificial Sequence: Synthetic
      oligonucleotide

<400> 370
gcgcaccatc aagagagtcc agttcggagt cctgagtccg gatgaactgg tccctgtcct 60

tcgaatggtg gaaggtgata ccatctatga 90

<210> 371
<211> 90
<212> DNA
<213> Artificial Sequence

<220>
<223> Description of Artificial Sequence: Synthetic
      oligonucleotide

<400> 371
gcccgctgcg caccatcaag agagtccagt tcggagtcct gagtccggat gaactggtcc 60

ctgtccttcg aatggtggaa ggtgatacca 90

<210> 372
<211> 90
<212> DNA
<213> Artificial Sequence

<220>
<223> Description of Artificial Sequence: Synthetic
      oligonucleotide

<400> 372
cgcatgcccg ctgcgcacca tcaagagagt ccagttcgga gtcctgagtc cggatgaact      60

ggtccctgtc cttcgaatgg tggaaggtga                                        90


<210> 373
<211> 90
<212> DNA
<213> Artificial Sequence

<220>
<223> Description of Artificial Sequence: Synthetic
      oligonucleotide

<400> 373
gcgcatgccc gctgcgcacc atcaagagag tccagttcgg agtcctgagt ccggatgaac      60

tggtccctgt ccttcgaatg gtggaaggtg                                        90


<210> 374
<211> 90
<212> DNA
<213> Artificial Sequence

<220>
<223> Description of Artificial Sequence: Synthetic
      oligonucleotide

<400> 374
agcgcatgcc cgctgcgcac catcaagaga gtccagttcg gagtcctgag tccggatgaa      60

ctggtccctg tccttcgaat ggtggaaggt                                        90


<210> 375
<211> 90
<212> DNA
<213> Artificial Sequence

<220>
<223> Description of Artificial Sequence: Synthetic
      oligonucleotide

<400> 375
cagcgcatgc ccgctgcgca ccatcaagag agtccagttc ggagtcctga gtccggatga      60

actggtccct gtccttcgaa tggtggaagg                                        90


<210> 376

<211> 90
<212> DNA
<213> Artificial Sequence

<220>
<223> Description of Artificial Sequence: Synthetic
      oligonucleotide

<400> 376
gggacagcgc atgcccgctg cgcaccatca agagagtcca gttcggagtc ctgagtccgg      60

atgaactggt ccctgtcctt cgaatggtgg                                      90


<210> 377
<211> 90
<212> DNA
<213> Artificial Sequence

<220>
<223> Description of Artificial Sequence: Synthetic
      oligonucleotide

<400> 377
cgggggacag cgcatgcccg ctgcgcacca tcaagagagt ccagttcgga gtcctgagtc      60

cggatgaact ggtccctgtc cttcgaatgg                                      90


<210> 378
<211> 90
<212> DNA
<213> Artificial Sequence

<220>
<223> Description of Artificial Sequence: Synthetic
      oligonucleotide

<400> 378
cccccctcgg gggacagcgc atgcccgctg cgcaccatca agagagtcca gttcggagtc      60

ctgagtccgg atgaactggt ccctgtcctt                                      90


<210> 379
<211> 90
<212> DNA
<213> Artificial Sequence

<220>
<223> Description of Artificial Sequence: Synthetic
      oligonucleotide

<400> 379
ccccccctcg ggggacagcg catgcccgct gcgcaccatc aagagagtcc agttcggagt      60

cctgagtccg gatgaactgg tccctgtcct                                      90


<210> 380
<211> 90
<212> DNA
<213> Artificial Sequence

<220>
<223> Description of Artificial Sequence: Synthetic
      oligonucleotide

<400> 380
ggccccccct cgggggacag cgcatgcccg ctgcgcacca tcaagagagt ccagttcgga      60

gtcctgagtc cggatgaact ggtccctgtc                                        90


<210> 381
<211> 90
<212> DNA
<213> Artificial Sequence

<220>
<223> Description of Artificial Sequence: Synthetic
      oligonucleotide

<400> 381
ggggtggccc cccctcgggg gacagcgcat gcccgctgcg caccatcaag agagtccagt      60

tcggagtcct gagtccggat gaactggtcc                                        90


<210> 382
<211> 90
<212> DNA
<213> Artificial Sequence

<220>
<223> Description of Artificial Sequence: Synthetic
      oligonucleotide

<400> 382
cgggggtggc cccccctcgg gggacagcgc atgcccgctg cgcaccatca agagagtcca      60

gttcggagtc ctgagtccgg atgaactggt                                        90


<210> 383
<211> 170
<212> DNA
<213> Artificial Sequence

<220>
<223> Description of Artificial Sequence: Synthetic
      polynucleotide


<220>
<221> CDS
<222> (2)..(169)

<400> 383
g cag ctg gac tgt gcc ttg gac cta atg agg cgc ctg cct ccc cag caa      49
  Gln Leu Asp Cys Ala Leu Asp Leu Met Arg Arg Leu Pro Pro Gln Gln
  1               5                   10                  15

atc gag aaa aac ctc agc gac ctg atc gac ctg gat gtc ccc gtt gag        97
Ile Glu Lys Asn Leu Ser Asp Leu Ile Asp Leu Asp Val Pro Val Glu
            20                  25                  30

gcc ctc acc acg gtg aag cca tac tgc aat gag atc cat gcc cag gct     145
Ala Leu Thr Thr Val Lys Pro Tyr Cys Asn Glu Ile His Ala Gln Ala
        35                  40                  45

caa ctg tgg ctc aag aga gac ccc a                                   170
Gln Leu Trp Leu Lys Arg Asp Pro
    50                  55

<210> 384
<211> 56
<212> PRT
<213> Artificial Sequence

<220>
<223> Description of Artificial Sequence: Synthetic
      polypeptide

<400> 384
Gln Leu Asp Cys Ala Leu Asp Leu Met Arg Arg Leu Pro Pro Gln Gln
1               5                   10                  15

Ile Glu Lys Asn Leu Ser Asp Leu Ile Asp Leu Asp Val Pro Val Glu
            20                  25                  30

Ala Leu Thr Thr Val Lys Pro Tyr Cys Asn Glu Ile His Ala Gln Ala
        35                  40                  45

Gln Leu Trp Leu Lys Arg Asp Pro
    50                  55

<210> 385
<211> 90
<212> DNA
<213> Artificial Sequence

<220>
<223> Description of Artificial Sequence: Synthetic
      oligonucleotide

<400> 385
tggatgtccc cgtgggggcc ctcaccacgg tgaagccata ctgcaatgag atccatgccc      60

aggctcaact gtggctcaag agagacccca                                      90

<210> 386
<211> 90
<212> DNA
<213> Artificial Sequence

<220>
<223> Description of Artificial Sequence: Synthetic
      oligonucleotide

<400> 386
ctggatctcc ccgtcgagcc cctccccacg gtgaagccat actgcaatga gatccatgcc      60

caggctcaac tgtggctcaa gagagacccc                                    90

<210> 387
<211> 90
<212> DNA
<213> Artificial Sequence

<220>
<223> Description of Artificial Sequence: Synthetic
      oligonucleotide

<400> 387
caaggatgtc cccgttgagg ccctcaccac ggtgaagcca tactgcaatg agatccatgc      60

ccaggctcaa ctgtggctca agagagacct                                    90

<210> 388
<211> 90
<212> DNA
<213> Artificial Sequence

<220>
<223> Description of Artificial Sequence: Synthetic
      oligonucleotide

<400> 388
acctggatgt ccccgtcgag gccctcccca cggtgaagcc atactgcaat gagatccatg      60

cccaggctca actgtggctc aagagagacc                                    90

<210> 389
<211> 90
<212> DNA
<213> Artificial Sequence

<220>
<223> Description of Artificial Sequence: Synthetic
      oligonucleotide

<400> 389
gccctggatg tccccgtcga ggccctcacc acggtgaagc catactgcaa tgagatccat      60

gcccaggctc aactgtggct caagagagac                                    90

<210> 390
<211> 90
<212> DNA
<213> Artificial Sequence

<220>
<223> Description of Artificial Sequence: Synthetic
      oligonucleotide

<400> 390
ccaccttgat gtccccgtcg aggccctcac cacggtgaag ccatactgca atgagatcca      60

tgcccaggct caactgtggc tcaagagaga                                    90

<210> 391
<211> 90
<212> DNA
<213> Artificial Sequence

<220>
<223> Description of Artificial Sequence: Synthetic
      oligonucleotide

<400> 391
gcgacctgga tgtccccgtc gaggccctcc ccacggtgaa gccatactgc aatgagatcc      60

atgcccaggc tcaactgtgg ctcaagagag                                        90


<210> 392
<211> 90
<212> DNA
<213> Artificial Sequence

<220>
<223> Description of Artificial Sequence: Synthetic
      oligonucleotide

<400> 392
gatcgacctg gatgtccccg tcgaggccct caccacggtg aagccatact gcaatgagat      60

ccatgcccag gctcaactgt ggctcaagag                                        90


<210> 393
<211> 90
<212> DNA
<213> Artificial Sequence

<220>
<223> Description of Artificial Sequence: Synthetic
      oligonucleotide

<400> 393
acctgatcga cctggatgtc cccgtcgagg ccctcaccac ggtgaagcca tactgcaatg      60

agatccatgc ccaggctcaa ctgtggctca                                        90


<210> 394
<211> 90
<212> DNA
<213> Artificial Sequence

<220>
<223> Description of Artificial Sequence: Synthetic
      oligonucleotide

<400> 394
cgacctgatc gacctggatg tccccgtcga ggccctcacc acggtgaagc catactgcaa      60

tgagatccat gcccaggctc aactgtggct                                        90


<210> 395
<211> 90
<212> DNA

<213> Artificial Sequence

<220>
<223> Description of Artificial Sequence: Synthetic
      oligonucleotide

<400> 395
ctcagcgacc tgatcgacct ggatgtcccc gtcgaggccc tcaccacggt gaagccatac      60

tgcaatgaga tccatgccca ggctcaactg                                      90


<210> 396
<211> 90
<212> DNA
<213> Artificial Sequence

<220>
<223> Description of Artificial Sequence: Synthetic
      oligonucleotide

<400> 396
aaaacctcag cgacctgatc gacctggatg tccccgtcga ggccctcacc acggtgaagc      60

catactgcaa tgagatccat gcccaggctc                                      90


<210> 397
<211> 90
<212> DNA
<213> Artificial Sequence

<220>
<223> Description of Artificial Sequence: Synthetic
      oligonucleotide

<400> 397
gaaaaacctc agcgacctga tcgacctgga tgtccccgtc gaggccctca ccacggtgaa      60

gccatactgc aatgagatcc atgcccaggc                                      90


<210> 398
<211> 90
<212> DNA
<213> Artificial Sequence

<220>
<223> Description of Artificial Sequence: Synthetic
      oligonucleotide

<400> 398
agaaaaacct cagcgacctg atcgacctgg atgtccccgt cgaggccctc accacggtga      60

agccatactg caatgagatc catgcccagg                                      90


<210> 399
<211> 90
<212> DNA
<213> Artificial Sequence

<220>

<223> Description of Artificial Sequence: Synthetic
      oligonucleotide

<400> 399
cgagaaaaac ctcagcgacc tgatcgacct ggatgtcccc gtcgaggccc tcaccacggt      60

gaagccatac tgcaatgaga tccatgccca                                        90


<210> 400
<211> 90
<212> DNA
<213> Artificial Sequence

<220>
<223> Description of Artificial Sequence: Synthetic
      oligonucleotide

<400> 400
tcgagaaaaa cctcagcgac ctgatcgacc tggatgtccc cgtcgaggcc ctcaccacgg      60

tgaagccata ctgcaatgag atccatgccc                                        90


<210> 401
<211> 90
<212> DNA
<213> Artificial Sequence

<220>
<223> Description of Artificial Sequence: Synthetic
      oligonucleotide

<400> 401
atcgagaaaa acctcagcga cctgatcgac ctggatgtcc ccgtcgaggc cctcaccacg      60

gtgaagccat actgcaatga gatccatgcc                                        90


<210> 402
<211> 90
<212> DNA
<213> Artificial Sequence

<220>
<223> Description of Artificial Sequence: Synthetic
      oligonucleotide

<400> 402
aatcgagaaa aacctcagcg acctgatcga cctggatgtc cccgtcgagg ccctcaccac      60

ggtgaagcca tactgcaatg agatccatgc                                        90


<210> 403
<211> 90
<212> DNA
<213> Artificial Sequence

<220>
<223> Description of Artificial Sequence: Synthetic
      oligonucleotide

<400> 403
aaatcgaaaa aaacctcagc gacctgatcg acctggatgg ccccggcgag gccctcacca 60

cggtgaagcc atactgcaat gagatccatg 90


<210> 404
<211> 90
<212> DNA
<213> Artificial Sequence

<220>
<223> Description of Artificial Sequence: Synthetic
      oligonucleotide

<400> 404
caaatcgaga aaaacctcag cgacctgatc gacctggatg tccccgtcga ggccctcacc 60

acggtgaagc catactgcaa tgagatccat 90


<210> 405
<211> 90
<212> DNA
<213> Artificial Sequence

<220>
<223> Description of Artificial Sequence: Synthetic
      oligonucleotide

<400> 405
gcaaatcgag aaaaacctca gcgacctgat cgacctggat gtccccgtcg aggccctcac 60

cacggtgaag ccatactgca atgagatcca 90


<210> 406
<211> 90
<212> DNA
<213> Artificial Sequence

<220>
<223> Description of Artificial Sequence: Synthetic
      oligonucleotide

<400> 406
agcaaatcga gaaaaacctc agcgacctga tcgacctgga tgtccccgtc gaggccctca 60

ccacggtgaa gccatactgc aatgagatcc 90


<210> 407
<211> 90
<212> DNA
<213> Artificial Sequence

<220>
<223> Description of Artificial Sequence: Synthetic
      oligonucleotide

<400> 407
cagcaaatcg agaaaaacct cagcgacctg atcgacctgg atgtccccgt cgaggccctc 60

accacggtga agccatactg caatgagatc                                    90

<210> 408
<211> 90
<212> DNA
<213> Artificial Sequence

<220>
<223> Description of Artificial Sequence: Synthetic
      oligonucleotide

<400> 408
ccccagcaaa tcgagaaaaa cctcagcgac ctgatcgacc tggatgtccc cgtcgaggcc      60

ctcaccacgg tgaagccata ctgcaatgag                                    90

<210> 409
<211> 90
<212> DNA
<213> Artificial Sequence

<220>
<223> Description of Artificial Sequence: Synthetic
      oligonucleotide

<400> 409
ctccccagca aatcgagaaa aacctcagcg acctgatcga cctggatgtc cccgtcgagg      60

ccctcaccac ggtgaagcca tactgcaatg                                    90

<210> 410
<211> 90
<212> DNA
<213> Artificial Sequence

<220>
<223> Description of Artificial Sequence: Synthetic
      oligonucleotide

<400> 410
cctccccagc aaatcgagaa aaacctcagc gacctgatcg acctggatgt ccccgtcgag      60

gccctcacca cggtgaagcc atactgcaat                                    90

<210> 411
<211> 90
<212> DNA
<213> Artificial Sequence

<220>
<223> Description of Artificial Sequence: Synthetic
      oligonucleotide

<400> 411
gcctccccag caaatcgaga aaaacctcag cgacctgatc gacctggatg tccccgtcga      60

ggccctcacc acggtgaagc catactgcaa                                    90

<210> 412
<211> 90
<212> DNA
<213> Artificial Sequence

<220>
<223> Description of Artificial Sequence: Synthetic
      oligonucleotide

<400> 412
tgcctcccca gcaaatcgag aaaaacctca gcgacccgat cgacctggat gtccccgtcg      60

aggccctcac cacggtgaag ccatactgca                                        90


<210> 413
<211> 90
<212> DNA
<213> Artificial Sequence

<220>
<223> Description of Artificial Sequence: Synthetic
      oligonucleotide

<400> 413
ctgcctcccc agcaaatcga gaaaaacctc agcgacctga tcgacctgga tgtccccgtc      60

gaggccctca ccacggtgaa gccatactgc                                        90


<210> 414
<211> 90
<212> DNA
<213> Artificial Sequence

<220>
<223> Description of Artificial Sequence: Synthetic
      oligonucleotide

<400> 414
cctgcctccc cagcaaatcg agaaaaacct cagcgacctg atcgacctgg atgtccccgt      60

cgaggccctc accacggtga agccatactg                                        90


<210> 415
<211> 90
<212> DNA
<213> Artificial Sequence

<220>
<223> Description of Artificial Sequence: Synthetic
      oligonucleotide

<400> 415
cgcctgcctc cccagcaaat cgagaaaaac ctcagcgacc tgatcgacct ggatgtcccc      60

gtcgaggccc tcaccacggt gaagccatac                                        90


<210> 416
<211> 90
<212> DNA

<213> Artificial Sequence

<220>
<223> Description of Artificial Sequence: Synthetic
      oligonucleotide

<400> 416
ggcgcctgcc tccccagcaa atcgagaaaa acctcagcga cctgatcgac ctggatgtcc      60

ccgtcgaggc cctcaccacg gtgaagccat                                        90


<210> 417
<211> 90
<212> DNA
<213> Artificial Sequence

<220>
<223> Description of Artificial Sequence: Synthetic
      oligonucleotide

<400> 417
gcggtgcctg cctccccagc aaatcgagaa aaacctcagc gacctgatcg acctggatgt      60

ccccgtcgag gccctcacca cggtgaagcc                                        90


<210> 418
<211> 90
<212> DNA
<213> Artificial Sequence

<220>
<223> Description of Artificial Sequence: Synthetic
      oligonucleotide

<400> 418
tgaggcgcct gcctccccag caaatcgaga aaaacctcag cgacctgatc gacctggatg      60

tccccgtcga ggccctcacc acggtgaagc                                        90


<210> 419
<211> 90
<212> DNA
<213> Artificial Sequence

<220>
<223> Description of Artificial Sequence: Synthetic
      oligonucleotide

<400> 419
atgaggcgcc tgcctcccca gcaaatcgag aaaaacctca gcgacctgat cgacctggat      60

gtccccgtcg aggccctcac cacggtgaag                                        90


<210> 420
<211> 90
<212> DNA
<213> Artificial Sequence

<220>

<223> Description of Artificial Sequence: Synthetic
      oligonucleotide

<400> 420
aatgaggcgc ctgcctcccc agcaaatcga gaaaaacctc agcgacctga tcgacctgga      60

tgtccccgtc gaggccctca ccacggtgaa                                        90


<210> 421
<211> 90
<212> DNA
<213> Artificial Sequence

<220>
<223> Description of Artificial Sequence: Synthetic
      oligonucleotide

<400> 421
taatgaggcg cctgcctccc cagcaaatcg agaaaaacct cagcgacctg atcgacctgg      60

atgtccccgt cgaggccctc accacggtga                                        90


<210> 422
<211> 90
<212> DNA
<213> Artificial Sequence

<220>
<223> Description of Artificial Sequence: Synthetic
      oligonucleotide

<400> 422
gacctaatga ggcgcctgcc tccccagcaa atcgagaaaa acctcagcga cctgatcgac      60

ctggatgtcc ccgtcgaggc cctcaccacg                                        90


<210> 423
<211> 90
<212> DNA
<213> Artificial Sequence

<220>
<223> Description of Artificial Sequence: Synthetic
      oligonucleotide

<400> 423
ttggacctaa tgaggcgcct gcctccccag caaatcgaga aaaacctcag cgacctgatc      60

gacctggatg tccccgtcga ggccctcacc                                        90


<210> 424
<211> 90
<212> DNA
<213> Artificial Sequence

<220>
<223> Description of Artificial Sequence: Synthetic
      oligonucleotide

<400> 424
ctgggaccta aggaggcgcc tgcctcccca gcaaatcgag aaaaacctca gcgacctgat     60

cgacctggat gtccccgtcg aggccctcac                                      90


<210> 425
<211> 90
<212> DNA
<213> Artificial Sequence

<220>
<223> Description of Artificial Sequence: Synthetic
      oligonucleotide

<400> 425
gccttggacc taatgaggcg cctgcctccc cagcaaatcg agaaaaacct cagcgacctg     60

atcgacctgg atgtccccgt cgaggccctc                                      90


<210> 426
<211> 90
<212> DNA
<213> Artificial Sequence

<220>
<223> Description of Artificial Sequence: Synthetic
      oligonucleotide

<400> 426
gtgccttgga cctaatgagg cgcctgcctc cccagcaaat cgagaaaaac ctcagcgacc     60

tgatcgacct ggatgtcccc gtcgaggccc                                      90


<210> 427
<211> 90
<212> DNA
<213> Artificial Sequence

<220>
<223> Description of Artificial Sequence: Synthetic
      oligonucleotide

<400> 427
actgggcctg gggcctaatg aggcgcctgc ctccccagca aatcgagaaa aacctcagcg     60

acctgatcga cctggatgtc cccgtcgagg                                      90


<210> 428
<211> 90
<212> DNA
<213> Artificial Sequence

<220>
<223> Description of Artificial Sequence: Synthetic
      oligonucleotide

<400> 428
gactgtgcct gggccctaag gaggcgcctg cctccccagc aaatcgagaa aaacctcagc     60

gacctgatcg acctggatgt ccccgtcgag                                    90

<210> 429
<211> 90
<212> DNA
<213> Artificial Sequence

<220>
<223> Description of Artificial Sequence: Synthetic
      oligonucleotide

<400> 429
catggactgt gccttggacc taatgaggcg cctgcctccc cagcaaatcg agaaaaacct      60

cagcgacctg atcgacctgg atgtccccga                                    90

<210> 430
<211> 90
<212> DNA
<213> Artificial Sequence

<220>
<223> Description of Artificial Sequence: Synthetic
      oligonucleotide

<400> 430
gctggactgt gccttggacc taatgaggcc ccttcctccc cagcaaatcg agaaaaacct      60

cagcgacctg atcgacctgg atgtcccccg                                    90

<210> 431
<211> 91
<212> DNA
<213> Artificial Sequence

<220>
<223> Description of Artificial Sequence: Synthetic
      oligonucleotide

<400> 431
agctggactg tgccttggac ctaatgaggc gcctgcctcc ccagcaaatc gagaaaaacc      60

ccagcgacct gatcgacctg gatgtccccg g                                  91

<210> 432
<211> 91
<212> DNA
<213> Artificial Sequence

<220>
<223> Description of Artificial Sequence: Synthetic
      oligonucleotide

<400> 432
gcagctggac tgtgccttgg acctaatgag gcgcctgcct ccccagcaaa tcgagaaaaa      60

cctcagcgac ctgatcgacc tggatgtccc c                                  91

<210> 433
<211> 176
<212> DNA
<213> Artificial Sequence

<220>
<223> Description of Artificial Sequence: Synthetic
      polynucleotide

<400> 433
ggagtgggtt aatgcattaa tccttaagaa taaactgaaa gtgcgaactg cctatccgtc      60

attgagactt attcatgctg tcagagggtt ttgtgatgaa ggaacctgta cagataaagc     120

caatattctg tatgcctggg cgagaaatgc tccccctacc cggctcccca aaggtg         176


<210> 434
<211> 59
<212> PRT
<213> Artificial Sequence

<220>
<223> Description of Artificial Sequence: Synthetic
      polypeptide


<220>
<221> MOD_RES
<222> (59)..(59)
<223> Any amino acid

<400> 434
Glu Trp Val Asn Ala Leu Ile Leu Lys Asn Lys Leu Lys Val Arg Thr
1               5                   10                  15


Ala Tyr Pro Ser Leu Arg Leu Ile His Ala Val Arg Gly Phe Cys Asp
            20                  25                  30


Glu Gly Thr Cys Thr Asp Lys Ala Asn Ile Leu Tyr Ala Trp Ala Arg
        35                  40                  45


Asn Ala Pro Pro Thr Arg Leu Pro Lys Gly Xaa
    50                  55


<210> 435
<211> 90
<212> DNA
<213> Artificial Sequence

<220>
<223> Description of Artificial Sequence: Synthetic
      oligonucleotide

<400> 435
ggttttgtga tgaaggaacc tgtacagata aagccaatat tctgtatgcc tgggcgagaa      60

atgctccccc tacccggctc cccaaaggtg                                      90

<210> 436
<211> 90
<212> DNA
<213> Artificial Sequence

<220>
<223> Description of Artificial Sequence: Synthetic
      oligonucleotide

<400> 436
tggttttgtg atgaaggaac ctgtacagat aaagccaata ttctgtatgc ctgggcgaga      60

aatgctcccc ctacccggct ccccaaaggt                                        90


<210> 437
<211> 90
<212> DNA
<213> Artificial Sequence

<220>
<223> Description of Artificial Sequence: Synthetic
      oligonucleotide

<400> 437
ctggttttgt gatgaaggaa cctgtacaga taaagccaat attctgtatg cctgggcgag      60

aaatgctccc cctacgcggc tccccaaagg                                        90


<210> 438
<211> 90
<212> DNA
<213> Artificial Sequence

<220>
<223> Description of Artificial Sequence: Synthetic
      oligonucleotide

<400> 438
gagggttttg tgatgaagga acctgtacag ataaagccaa tattctgtat gcctgggcga      60

gaaatgctcc ccctacccgg ctccccaaag                                        90


<210> 439
<211> 91
<212> DNA
<213> Artificial Sequence

<220>
<223> Description of Artificial Sequence: Synthetic
      oligonucleotide

<400> 439
gttactggtt ttgtgatgaa ggaacctgta cagataaagc caatattctg tatgcctggg      60

cgagaaatgc tccccctacc cggctcccca a                                      91


<210> 440
<211> 90

<212> DNA
<213> Artificial Sequence

<220>
<223> Description of Artificial Sequence: Synthetic
      oligonucleotide

<400> 440
gctgtcagag ggttttgtga tgaaggaacc tgtacagata aagccaatat tctgtatgcc      60

tgggcgagaa atgctccccc tacccggctc                                        90


<210> 441
<211> 91
<212> DNA
<213> Artificial Sequence

<220>
<223> Description of Artificial Sequence: Synthetic
      oligonucleotide

<400> 441
tgctgtcaga gggttttgtg atgaaggaac ctgtacagat aaagccaata ttctgtatgc      60

ctgggcgaga aatgctcccc ctacccggcc c                                      91


<210> 442
<211> 90
<212> DNA
<213> Artificial Sequence

<220>
<223> Description of Artificial Sequence: Synthetic
      oligonucleotide

<400> 442
atgctgtcag agggttttgt gatgaaggaa cctgtacaga taaagccaat attctgtatg      60

cctgggcgag aaatgctccc cctacccggc                                        90


<210> 443
<211> 90
<212> DNA
<213> Artificial Sequence

<220>
<223> Description of Artificial Sequence: Synthetic
      oligonucleotide

<400> 443
catgctgtca gagggttttg tgatgaagga gcctgtacag ataaagccaa tattctgtat      60

gcctgggcga gaaatgctcc ccctacccgg                                        90


<210> 444
<211> 90
<212> DNA
<213> Artificial Sequence

<220>
<223> Description of Artificial Sequence: Synthetic
      oligonucleotide

<400> 444
tcatgctgtc agagggtttt gtgatgaagg aacctgtaca gataaagcca atattctgta      60

tgcctgggcg agaaatgctc cccctacccg                                        90


<210> 445
<211> 90
<212> DNA
<213> Artificial Sequence

<220>
<223> Description of Artificial Sequence: Synthetic
      oligonucleotide

<400> 445
tattcatgct gtcagagggt tttgtgatga aggaacctgt acagataaag ccaatattct      60

gtatgcctgg gcgagaaatg ctccccctac                                        90


<210> 446
<211> 90
<212> DNA
<213> Artificial Sequence

<220>
<223> Description of Artificial Sequence: Synthetic
      oligonucleotide

<400> 446
cttattcatg ctgtcagagg gttttgtgat gaaggaacct gtacagataa agccaatatt      60

ctgtatgcct gggcgagaaa tactccccct                                        90


<210> 447
<211> 90
<212> DNA
<213> Artificial Sequence

<220>
<223> Description of Artificial Sequence: Synthetic
      oligonucleotide

<400> 447
acttattcat gctgtcagag ggttttgtga tgaaggaacc tgtacagata aagccaatat      60

tctgtatgcc tgggcgagaa atgctccccc                                        90


<210> 448
<211> 90
<212> DNA
<213> Artificial Sequence

<220>
<223> Description of Artificial Sequence: Synthetic
      oligonucleotide

<400> 448
gagacttatt catgctgtca gagggttttg tgatgaagga acctgtacag ataaagccaa 60

tattctgtat gcctgggcga gaaatgctcc 90

<210> 449
<211> 90
<212> DNA
<213> Artificial Sequence

<220>
<223> Description of Artificial Sequence: Synthetic
      oligonucleotide

<400> 449
tgagacttat tcatgctgtc agagggtttt gtgatgaagg aacctgtaca gataaagcca 60

atattctgta tgcctgggcg agaaatgctc 90

<210> 450
<211> 90
<212> DNA
<213> Artificial Sequence

<220>
<223> Description of Artificial Sequence: Synthetic
      oligonucleotide

<400> 450
ttgagactta ttcatgctgt cagagggttt tgtgatgaag gaacctgtac agataaagcc 60

aatattctgt atgcctgggc gagaaatgct 90

<210> 451
<211> 90
<212> DNA
<213> Artificial Sequence

<220>
<223> Description of Artificial Sequence: Synthetic
      oligonucleotide

<400> 451
gtcattgaga cttattcatg ctgtcagagg gttttgtgat gaaggaacct gtacagataa 60

agccaatatt ctgtatgcct gggcgagaaa 90

<210> 452
<211> 90
<212> DNA
<213> Artificial Sequence

<220>
<223> Description of Artificial Sequence: Synthetic
      oligonucleotide

<400> 452
ccgtcattga gacttattca tgctgtcaga gggttttgtg atgaaggaac ctgtacagat 60

aaagccaata ttctgtatgc ctgggcgaga                                    90

<210> 453
<211> 90
<212> DNA
<213> Artificial Sequence

<220>
<223> Description of Artificial Sequence: Synthetic
      oligonucleotide

<400> 453
tatccgtcat tgagacttat tcatgctgtc agagggtttt gtgatgaagg aacctgtaca      60

gataaagcca atattctgta tgcctgggcg                                    90

<210> 454
<211> 90
<212> DNA
<213> Artificial Sequence

<220>
<223> Description of Artificial Sequence: Synthetic
      oligonucleotide

<400> 454
ctatccgtca ttgagactta ttcatgctgt cagagggttt tgtgatgaag gaacctgtac      60

agataaagcc aatattctgt atgcctgggc                                    90

<210> 455
<211> 90
<212> DNA
<213> Artificial Sequence

<220>
<223> Description of Artificial Sequence: Synthetic
      oligonucleotide

<400> 455
cctatccgtc attgagactt attcatgctg tcagagggtt ttgtgatgaa ggaacctgta      60

cagataaagc caatattctg tatgcctggg                                    90

<210> 456
<211> 90
<212> DNA
<213> Artificial Sequence

<220>
<223> Description of Artificial Sequence: Synthetic
      oligonucleotide

<400> 456
gcctatccgt cattgagact tattcatgct gtcagagggt tttgtgatga aggaacctgt      60

acagataaag ccaatattct gtatgcctgg                                    90

<210> 457
<211> 90
<212> DNA
<213> Artificial Sequence

<220>
<223> Description of Artificial Sequence: Synthetic
      oligonucleotide

<400> 457
tgaaagtgcg aactgcctat ccgtcattga gacttattca tgctgtcaga gggttttgtg      60

atgaaggaac ctgtacagat aaagccaata                                      90


<210> 458
<211> 90
<212> DNA
<213> Artificial Sequence

<220>
<223> Description of Artificial Sequence: Synthetic
      oligonucleotide

<400> 458
ctgaaagtgc gaactgccta tccgtcattg agacttattc atgctgtcag agggttttgt      60

gatgaaggaa cctgtacaga taaagccaat                                      90


<210> 459
<211> 90
<212> DNA
<213> Artificial Sequence

<220>
<223> Description of Artificial Sequence: Synthetic
      oligonucleotide

<400> 459
aaactgaaag tgcgaactgc ctatccgtca ttgagactta ttcatgctgt cagagggttt      60

tgtgatgaag gaacctgtac agataaagcc                                      90


<210> 460
<211> 90
<212> DNA
<213> Artificial Sequence

<220>
<223> Description of Artificial Sequence: Synthetic
      oligonucleotide

<400> 460
taaactgaaa gtgcgaactg cctatccgtc attgagactt attcatgctg tcagagggtt      60

ttgtgatgaa ggaacctgta cagataaagc                                      90


<210> 461
<211> 90

<212> DNA
<213> Artificial Sequence

<220>
<223> Description of Artificial Sequence: Synthetic
      oligonucleotide

<400> 461
attaatcctt aagaataaac tgaaagtgcg aactgcctat ccgtcattga gacttattca      60

tgctgtcaga gggttttgtg atgaaggaac                                       90


<210> 462
<211> 90
<212> DNA
<213> Artificial Sequence

<220>
<223> Description of Artificial Sequence: Synthetic
      oligonucleotide

<400> 462
gcattaatcc ttaagaataa actgaaagtg cgaactgcct atccgtcatt gagacttatt      60

catgctgtca gagggttttg tgatgaagga                                       90


<210> 463
<211> 90
<212> DNA
<213> Artificial Sequence

<220>
<223> Description of Artificial Sequence: Synthetic
      oligonucleotide

<400> 463
ggagtgggtt aatgcattaa tccttaagaa taaactgaaa gtgcgaactg cctatccgtc      60

attgagactt attcatgctg tcagagggtt                                       90


<210> 464
<211> 167
<212> DNA
<213> Artificial Sequence

<220>
<223> Description of Artificial Sequence: Synthetic
      polynucleotide

<400> 464
acacgatttt gtcttgtgtt gccaagtcta cctgtgccat caacaacacc ctcattgctt      60

tcttcatttt gactacgata aaagacataa ccagtgcggt gcaatccaag cgaagaaaat     120

ccaagtaaac aagcaggact gcgacttgat acttggaaat gtgtgtg                   167


<210> 465
<211> 56
<212> PRT

<213> Artificial Sequence

<220>
<223> Description of Artificial Sequence: Synthetic
      polypeptide

<220>
<221> MOD_RES
<222> (56)..(56)
<223> Any amino acid

<400> 465
His Asp Phe Val Leu Cys Cys Gln Val Tyr Leu Cys His Gln Gln His
1               5                   10                  15

Pro His Cys Phe Leu His Phe Asp Tyr Asp Lys Arg His Asn Gln Cys
            20                  25                  30

Gly Ala Ile Gln Ala Lys Lys Ile Gln Val Asn Lys Gln Asp Cys Asp
        35                  40                  45

Leu Ile Leu Gly Asn Val Cys Xaa
    50                  55

<210> 466
<211> 90
<212> DNA
<213> Artificial Sequence

<220>
<223> Description of Artificial Sequence: Synthetic
      oligonucleotide

<400> 466
ataaaagaca taaccagtgc ggtgcaatcc aagcgaagaa aatccaagta aacaagcagg      60

actgcgactt gatacttgga aatgtgtgtg                                        90

<210> 467
<211> 90
<212> DNA
<213> Artificial Sequence

<220>
<223> Description of Artificial Sequence: Synthetic
      oligonucleotide

<400> 467
ttgctttctt cattttgact acgataaaag acataaccag tgcggtgcaa tccaagcgaa      60

gaaaatccaa gtaaacaagc aggactgcga                                        90

<210> 468
<211> 90
<212> DNA
<213> Artificial Sequence

<220>
<223> Description of Artificial Sequence: Synthetic
      oligonucleotide

<400> 468
cctcattgct ttcttcattt tgactacgat aaaagacata accagtgcgg tgcaatccaa      60

gcgaagaaaa tccaagtaaa caagcaggac                                        90


<210> 469
<211> 90
<212> DNA
<213> Artificial Sequence

<220>
<223> Description of Artificial Sequence: Synthetic
      oligonucleotide

<400> 469
ccctcattgc tttcttcatt ttgactacga taaaagacat aaccagtgcg gtgcaatcca      60

agcgaagaaa atccaagtaa acaagcagga                                        90


<210> 470
<211> 90
<212> DNA
<213> Artificial Sequence

<220>
<223> Description of Artificial Sequence: Synthetic
      oligonucleotide

<400> 470
caccctcatt gctttcttca ttttgactac gataaaagac ataaccagtg cggtgcaatc      60

caagcgaaga aaatccaagt aaacaagcag                                        90


<210> 471
<211> 90
<212> DNA
<213> Artificial Sequence

<220>
<223> Description of Artificial Sequence: Synthetic
      oligonucleotide

<400> 471
caacaccctc attgctttct tcattttgac tacgataaaa gacataacca gtgcggtgca      60

atccaagcga agaaaatcca agtaaacaag                                        90


<210> 472
<211> 90
<212> DNA
<213> Artificial Sequence

<220>
<223> Description of Artificial Sequence: Synthetic

oligonucleotide

<400> 472
caacaacacc ctcattgctt tcttcatttt gactacgata aaagacataa ccagtgcggt 60

gcaatccaag cgaagaaaat ccaagtaaac 90


<210> 473
<211> 90
<212> DNA
<213> Artificial Sequence

<220>
<223> Description of Artificial Sequence: Synthetic
      oligonucleotide

<400> 473
atcaacaaca ccctcattgc tttcttcatt ttgactacga taaaagacat aaccagtgcg 60

gtgcaatcca agcgaagaaa atccaagtaa 90


<210> 474
<211> 90
<212> DNA
<213> Artificial Sequence

<220>
<223> Description of Artificial Sequence: Synthetic
      oligonucleotide

<400> 474
catcaacaac accctcattg ctttcttcat tttgactacg ataaaagaca taaccagtgc 60

ggtgcaatcc aagcgaagaa aatccaagta 90


<210> 475
<211> 90
<212> DNA
<213> Artificial Sequence

<220>
<223> Description of Artificial Sequence: Synthetic
      oligonucleotide

<400> 475
gtgccatcaa caacaccctc attgctttct tcattttgac tacgataaaa gacataacca 60

gtgcggtgca atccaagcga agaaaatcca 90


<210> 476
<211> 90
<212> DNA
<213> Artificial Sequence

<220>
<223> Description of Artificial Sequence: Synthetic
      oligonucleotide

<400> 476

gtctacctgt gccatcaaca acaccctcat tgctttcttc attttgacta cgataaaaga 60

cataaccagt gcggtgcaat ccaagcgaag 90

<210> 477
<211> 90
<212> DNA
<213> Artificial Sequence

<220>
<223> Description of Artificial Sequence: Synthetic
      oligonucleotide

<400> 477
caagtctacc tgtgccatca acaacaccct cattgctttc ttcattttga ctacgataaa 60

agacataacc agtgcggtgc aatccaagcg 90

<210> 478
<211> 90
<212> DNA
<213> Artificial Sequence

<220>
<223> Description of Artificial Sequence: Synthetic
      oligonucleotide

<400> 478
ccaagtctac ctgtgccatc aacaacaccc tcattgcttt cttcattttg actacgataa 60

aagacataac cagtgcggtg caatccaagc 90

<210> 479
<211> 90
<212> DNA
<213> Artificial Sequence

<220>
<223> Description of Artificial Sequence: Synthetic
      oligonucleotide

<400> 479
gtcttgtgtt gccaagtcta cctgtgccat caacaacacc ctcattgctt tcttcatttt 60

gactacgata aaagacataa ccagtgcggt 90

<210> 480
<211> 90
<212> DNA
<213> Artificial Sequence

<220>
<223> Description of Artificial Sequence: Synthetic
      oligonucleotide

<400> 480
ttttcttgtg ttgccaagtc tacctgtgcc atcaacaaca ccctcattgc tttcttcatt 60

ttgactacga taaaagacat aaccagtgcg 90

<210> 481
<211> 90
<212> DNA
<213> Artificial Sequence

<220>
<223> Description of Artificial Sequence: Synthetic
      oligonucleotide

<400> 481
attttgtctt gtgttgccaa gtctacctgt gccatcaaca acaccctcat tgctttcttc      60

attttgacta cgataaaaga cataaccagt                                      90


<210> 482
<211> 90
<212> DNA
<213> Artificial Sequence

<220>
<223> Description of Artificial Sequence: Synthetic
      oligonucleotide

<400> 482
acacgatttt gtcttgtgtt gccaagtcta cctgtgccat caacaacacc ctcattgctt      60

tcttcatttt gactacgata aaagacataa                                      90


<210> 483
<211> 166
<212> DNA
<213> Artificial Sequence

<220>
<223> Description of Artificial Sequence: Synthetic
      polynucleotide

<400> 483
atttagagca cagtgatgag caagcagtaa taaagtctcc cttaaaatgc accctccttc      60

cacctggcca ggaatcagca ttgggaatgg taccacctcc cgaaaatgtc agaatgaatt     120

ctgttaattt caagaacatt ctacagtggg agtcacctgc ttttgc                   166


<210> 484
<211> 55
<212> PRT
<213> Artificial Sequence

<220>
<223> Description of Artificial Sequence: Synthetic
      polypeptide


<220>
<221> MOD_RES
<222> (55)..(55)
<223> Any amino acid

<400> 484
Leu Glu His Ser Asp Glu Gln Ala Val Ile Lys Ser Pro Leu Lys Cys
1               5                   10                  15

Thr Leu Leu Pro Pro Gly Gln Glu Ser Ala Leu Gly Met Val Pro Pro
            20                  25                  30

Pro Glu Asn Val Arg Met Asn Ser Val Asn Phe Lys Asn Ile Leu Gln
        35                  40                  45

Trp Glu Ser Pro Ala Phe Xaa
    50                  55

<210> 485
<211> 90
<212> DNA
<213> Artificial Sequence

<220>
<223> Description of Artificial Sequence: Synthetic
      oligonucleotide

<400> 485
agcattggga atggtaccac ctcccgaaaa tgtcagaatg aattctgtta atttcaagaa      60

cattctacag tgggagtcac ctgcttttgc                                        90

<210> 486
<211> 90
<212> DNA
<213> Artificial Sequence

<220>
<223> Description of Artificial Sequence: Synthetic
      oligonucleotide

<400> 486
gtcagcattg ggaatggtac cacctcccga aaatgtcaga atgaattctg ttaatttcaa      60

gaacattcta cagtgggagt cacctgcttt                                        90

<210> 487
<211> 90
<212> DNA
<213> Artificial Sequence

<220>
<223> Description of Artificial Sequence: Synthetic
      oligonucleotide

<400> 487
cagtaataaa gtctccctta aaatgcaccc tccttccacc tggccaggaa tcagcattgg      60

gaatggtacc acctcccgaa aatgtcagaa                                        90

<210> 488
<211> 90
<212> DNA
<213> Artificial Sequence

<220>
<223> Description of Artificial Sequence: Synthetic
      oligonucleotide

<400> 488
gtgatgagca agcagtaata aagtctccct taaaatgcac cctccttcca cctggccagg      60

aatcagcatt gggaatggta ccacctcccg                                      90


<210> 489
<211> 90
<212> DNA
<213> Artificial Sequence

<220>
<223> Description of Artificial Sequence: Synthetic
      oligonucleotide

<400> 489
acagtgatga gcaagcagta ataaagtctc ccttaaaatg caccctcctt ccacctggcc      60

aggaatcagc attgggaatg gtaccaccaa                                      90


<210> 490
<211> 90
<212> DNA
<213> Artificial Sequence

<220>
<223> Description of Artificial Sequence: Synthetic
      oligonucleotide

<400> 490
atttagagca cagtgatgag caagcagtaa taaagtctcc cttaaaatgc accctccttc      60

cacctggcca ggaatcagca ttgggaatgg                                      90


<210> 491
<211> 12
<212> PRT
<213> Artificial Sequence

<220>
<223> Description of Artificial Sequence: Synthetic
      peptide

<400> 491
Leu Gln Leu Ala Asn Glu Glu Ile Ala Gln Val Arg
1               5                   10


<210> 492
<211> 10
<212> PRT
<213> Artificial Sequence

<220>
<223> Description of Artificial Sequence: Synthetic
      peptide

<400> 492
Asp Gly Thr Gly Leu Val Pro Ser Glu Arg
1               5                   10


<210> 493
<211> 13
<212> PRT
<213> Artificial Sequence

<220>
<223> Description of Artificial Sequence: Synthetic
      peptide

<400> 493
Val Gly Pro Asp Gly Thr Pro Tyr Val Thr Val Leu Lys
1               5                   10


<210> 494
<211> 7
<212> PRT
<213> Artificial Sequence

<220>
<223> Description of Artificial Sequence: Synthetic
      peptide

<400> 494
Val Leu Val Gly Pro Gln Arg
1               5


<210> 495
<211> 23
<212> DNA
<213> Artificial Sequence

<220>
<223> Description of Artificial Sequence: Synthetic
      primer

<400> 495
cgtgaagatg ctgaaagacg atg                                              23


<210> 496
<211> 21
<212> DNA
<213> Artificial Sequence

<220>
<223> Description of Artificial Sequence: Synthetic
      primer

<400> 496
aaacgcttga agaggtcgga g                                                21

<210> 497
<211> 21
<212> DNA
<213> Artificial Sequence

<220>
<223> Description of Artificial Sequence: Synthetic
      primer

<400> 497
tgcctgtgga ggaacttttc a                                                21


<210> 498
<211> 20
<212> DNA
<213> Artificial Sequence

<220>
<223> Description of Artificial Sequence: Synthetic
      primer

<400> 498
cccaaactca gcagcctaag                                                  20


<210> 499
<211> 19
<212> DNA
<213> Artificial Sequence

<220>
<223> Description of Artificial Sequence: Synthetic
      primer

<400> 499
tggaccgtgt ccttaccgt                                                   19


<210> 500
<211> 19
<212> DNA
<213> Artificial Sequence

<220>
<223> Description of Artificial Sequence: Synthetic
      primer

<400> 500
ggtcctttgg ggtcctgct                                                   19


<210> 501
<211> 20
<212> DNA
<213> Artificial Sequence

<220>
<223> Description of Artificial Sequence: Synthetic
      primer

<400> 501

cctctttcag ctccaaggca                                                    20

<210> 502
<211> 21
<212> DNA
<213> Artificial Sequence

<220>
<223> Description of Artificial Sequence: Synthetic
      primer

<400> 502
tctaccagga ctgtccctca g                                                   21

<210> 503
<211> 20
<212> DNA
<213> Artificial Sequence

<220>
<223> Description of Artificial Sequence: Synthetic
      primer

<400> 503
gggagtctca tttgcaccgt                                                    20

<210> 504
<211> 20
<212> DNA
<213> Artificial Sequence

<220>
<223> Description of Artificial Sequence: Synthetic
      primer

<400> 504
ctgcatccag gtccttctgg                                                    20

<210> 505
<211> 20
<212> DNA
<213> Artificial Sequence

<220>
<223> Description of Artificial Sequence: Synthetic
      primer

<400> 505
ccagttccag gttcttcccg                                                    20

<210> 506
<211> 21
<212> DNA
<213> Artificial Sequence

<220>
<223> Description of Artificial Sequence: Synthetic
      primer

<400> 506
caacctcttc gaacctgtcc a                                                21

<210> 507
<211> 20
<212> DNA
<213> Artificial Sequence

<220>
<223> Description of Artificial Sequence: Synthetic
      primer

<400> 507
aagacgatgc cactgacaag                                                  20

<210> 508
<211> 24
<212> DNA
<213> Artificial Sequence

<220>
<223> Description of Artificial Sequence: Synthetic
      primer

<400> 508
cccagcaggt tgatgatgtt tttg                                             24

<210> 509
<211> 22
<212> DNA
<213> Artificial Sequence

<220>
<223> Description of Artificial Sequence: Synthetic
      primer

<400> 509
tccttctccg acctcttcaa gc                                               22

<210> 510
<211> 23
<212> DNA
<213> Artificial Sequence

<220>
<223> Description of Artificial Sequence: Synthetic
      primer

<400> 510
taatcctcca cgcacttctt cag                                              23

<210> 511
<211> 18
<212> DNA
<213> Artificial Sequence

<220>

<223> Description of Artificial Sequence: Synthetic
      primer

<400> 511
tacctggacc tgtcggcg                                                    18


<210> 512
<211> 18
<212> DNA
<213> Artificial Sequence

<220>
<223> Description of Artificial Sequence: Synthetic
      primer

<400> 512
tgggcaaaca cggagtcg                                                    18


<210> 513
<211> 25
<212> DNA
<213> Artificial Sequence

<220>
<223> Description of Artificial Sequence: Synthetic
      primer

<400> 513
ccacagacgc acaggattct aagtc                                            25


<210> 514
<211> 22
<212> DNA
<213> Artificial Sequence

<220>
<223> Description of Artificial Sequence: Synthetic
      primer

<400> 514
tgagttttcc agtccaaggg tg                                               22


<210> 515
<211> 30
<212> DNA
<213> Artificial Sequence

<220>
<223> Description of Artificial Sequence: Synthetic
      oligonucleotide

<400> 515
gtgacgtcca ccgactttaa ggagtcggcc                                       30


<210> 516
<211> 10
<212> PRT
<213> Artificial Sequence

<220>
<223> Description of Artificial Sequence: Synthetic
      peptide

<400> 516
Val Thr Ser Thr Asp Phe Lys Glu Ser Ala
1               5                   10


<210> 517
<211> 30
<212> DNA
<213> Artificial Sequence

<220>
<223> Description of Artificial Sequence: Synthetic
      oligonucleotide

<400> 517
gtgacgtcca ccgacgtaaa ggcgacacag                                        30


<210> 518
<211> 10
<212> PRT
<213> Artificial Sequence

<220>
<223> Description of Artificial Sequence: Synthetic
      peptide

<400> 518
Val Thr Ser Thr Asp Val Lys Ala Thr Gln
1               5                   10


<210> 519
<211> 30
<212> DNA
<213> Artificial Sequence

<220>
<223> Description of Artificial Sequence: Synthetic
      oligonucleotide

<400> 519
gcgaagaggg gctcggtggt gcagttggca                                        30


<210> 520
<211> 30
<212> DNA
<213> Artificial Sequence

<220>
<223> Description of Artificial Sequence: Synthetic
      oligonucleotide

<400> 520
cctcccgcca gcaggagccc ccagcagcct                                        30

<210> 521
<211> 30
<212> DNA
<213> Artificial Sequence

<220>
<223> Description of Artificial Sequence: Synthetic
      oligonucleotide

<400> 521
gactcagtgc agatgttccc tcgccgcact                                        30


<210> 522
<211> 30
<212> DNA
<213> Artificial Sequence

<220>
<223> Description of Artificial Sequence: Synthetic
      oligonucleotide

<400> 522
gcctgggcac agagggtgtc cgagagcagc                                        30


<210> 523
<211> 30
<212> DNA
<213> Artificial Sequence

<220>
<223> Description of Artificial Sequence: Synthetic
      oligonucleotide

<400> 523
ccacccgcct atgccaggca attccgcgaa                                        30


<210> 524
<211> 30
<212> DNA
<213> Artificial Sequence

<220>
<223> Description of Artificial Sequence: Synthetic
      oligonucleotide

<400> 524
tggacctgtc ggcgcaggag caacggcagc                                        30


<210> 525
<211> 30
<212> DNA
<213> Artificial Sequence

<220>
<223> Description of Artificial Sequence: Synthetic
      oligonucleotide

<400> 525
gagtgctggc tctggcccgg gactctccgg                                        30

<210> 526
<211> 30
<212> DNA
<213> Artificial Sequence

<220>
<223> Description of Artificial Sequence: Synthetic
      oligonucleotide

<400> 526
aggtggctgt gcgaaggtcg ctgagggtcc                                        30


<210> 527
<211> 31
<212> DNA
<213> Artificial Sequence

<220>
<223> Description of Artificial Sequence: Synthetic
      oligonucleotide

<400> 527
tgccgtcccc ggccatccct caggacgtcc g                                      31


<210> 528
<211> 2421
<212> DNA
<213> Homo sapiens

<400> 528
atgggcgccc ctgcctgcgc cctcgcgctc tgcgtggccg tggccatcgt ggccggcgcc       60

tcctcggagt ccttggggac ggagcagcgc gtcgtggggc gagcggcaga agtcccgggc      120

ccagagcccg gccagcagga gcagttggtc ttcggcagcg gggatgctgt ggagctgagc      180

tgtcccccgc ccgggggtgg tcccatgggg cccactgtct gggtcaagga tggcacaggg      240

ctggtgccct cggagcgtgt cctggtgggg ccccagcggc tgcaggtgct gaatgcctcc      300

cacgaggact ccggggccta cagctgccgg cagcggctca cgcagcgcgt actgtgccac      360

ttcagtgtgc gggtgacaga cgctccatcc tcgggagatg acgaagacgg ggaggacgag      420

gctgaggaca caggtgtgga cacaggggcc ccttactgga cacggcccga gcggatggac      480

aagaagctgc tggccgtgcc ggccgccaac accgtccgct tccgctgccc agccgctggc      540

aaccccactc cctccatctc ctggctgaag aacggcaggg agttccgcgg cgagcaccgc      600

attggaggca tcaagctgcg gcatcagcag tggagcctgg tcatggaaag cgtggtgccc      660

tcggaccgcg gcaactacac ctgcgtcgtg gagaacaagt ttggcagcat ccggcagacg      720

tacacgctgg acgtgctgga gcgctccccg caccggccca tcctgcaggc ggggctgccg      780

gccaaccaga cggcggtgct gggcagcgac gtggagttcc actgcaaggt gtacagtgac      840

gcacagcccc acatccagtg gctcaagcac gtggaggtga atggcagcaa ggtgggcccg      900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagtac 2280

ctggacctgt cggcgccttt cgagcagtac tccccgggtg gccaggacac ccccagctcc 2340

agctcctcag gggacgactc cgtgtttgcc cacgacctgc tgcccccggc cccacccagc 2400

agtgggggct cgcggacgtg a 2421

<210> 529
<211> 2517
<212> DNA
<213> Homo sapiens

<400> 529
atgagtctgc aggtcttaaa cgacaaaaat gtcagcaatg aaaaaaatac agaaaattgc 60

gacttcctgt tttcgccacc agaagttacc ggaagatcgt ctgttcttcg tgtgtcacag 120

aaagaaaatg tgccacccaa gaacctggcc aaagctatga aggtgacttt tcagacacct 180

ctgcgggatc cacagacgca caggattcta agtcctagca tggccagcaa acttgaggct 240

cctttcactc aggatgacac ccttggactg gaaaactcac acccggtctg gacacagaaa 300

gagaaccaac agctcatcaa ggaagtggat gccaaaacta ctcatggaat tctacagaaa 360

ccagtggagg ctgacaccga cctcctgggg gatgcaagcc cagcctttgg gagtggcagc 420

tccagcgagt ctggcccagg tgccctggct gacctggact gctcaagctc ttcccagagc 480

ccaggaagtt ctgagaacca aatggtgtct ccaggaaaag tgtctggcag ccctgagcaa 540

gccgtggagg aaaaccttag ttcctattcc ttagacagaa gagtgacacc cgcctctgag 600

accctagaag acccttgcag gacagagtcc cagcacaaag cggagactcc gcacggagcc 660

gaggaagaat gcaaagcgga gactccgcac ggagccgagg aggaatgccg gcacggtggg 720

gtctgtgctc ccgcagcagt ggccacttcg cctcctggtg caatccctaa ggaagcctgc 780

ggaggagcac ccctgcaggg tctgcctggc gaagccctgg gctgccctgc gggtgtgggc 840

acccccgtgc cagcagatgg cactcagacc cttacctgtg cacacacctc tgctcctgag 900

agcacagccc caaccaacca cctggtggct ggcagggcca tgaccctgag tcctcaggaa 960

gaagtggctg caggccaaat ggccagctcc tcgaggagcg gacctgtaaa actagaattt 1020

gatgtatctg atggcgccac cagcaaaagg gcacccccac caaggagact gggagagagg 1080

tccggcctca agcctccctt gaggaaagca gcagtgaggc agcaaaaggc cccgcaggag 1140

gtggaggagg acgacggtag gagcggagca ggagaggacc cccccatgcc agcttctcgg 1200

ggctcttacc acctcgactg ggacaaaatg gatgacccaa acttcatccc gttcggaggt 1260

gacaccaagt ctggttgcag tgaggcccag cccccagaaa gccctgagac caggctgggc 1320

cagccagcgg ctgaacagtt gcatgctggg cctgccacgg aggagccagg tccctgtctg 1380

agccagcagc tgcattcagc ctcagcggag gacacgcctg tggtgcagtt ggcagccgag 1440

accccaacag cagagagcaa ggagagagcc ttgaactctg ccagcacctc gcttcccaca 1500

agctgtccag gcagtgagcc agtgcccacc catcagcagg ggcagcctgc cttggagctg 1560

aaagaggaga gcttcagaga ccccgctgag gttctaggca cgggcgcgga ggtggattac 1620

ctggagcagt ttggaacttc ctcgtttaag gagtcggcct tgaggaagca gtccttatac 1680

ctcaagttcg accccctcct gagggacagt cctggtagac cagtgcccgt ggccaccgag 1740

accagcagca tgcacggtgc aaatgagact ccctcaggac gtccgcggga agccaagctt 1800

gtggagttcg atttcttggg agcactggac attcctgtgc caggcccacc cccaggtgtt 1860

cccgcgcctg ggggcccacc cctgtccacc ggacctatag tggacctgct ccagtacagc 1920

cagaaggacc tggatgcagt ggtaaaggcg acacaggagg agaaccggga gctgaggagc 1980

aggtgtgagg agctccacgg gaagaacctg gaactgggga agatcatgga caggttcgaa 2040

gaggttgtgt accaggccat ggaggaagtt cagaagcaga aggaactttc caaagctgaa 2100

atccagaaag ttctaaaaga aaaagaccaa cttaccacag atctgaactc catggagaag 2160

tccttctccg acctcttcaa gcgttttgag aaacagaaag aggtgatcga gggctaccgc 2220

aagaacgaag agtcactgaa gaagtgcgtg gaggattacc tggcaaggat cacccaggag 2280

ggccagaggt accaagccct gaaggcccac gcggaggaga agctgcagct ggcaaacgag 2340

gagatcgccc aggtccggag caaggcccag gcggaagcgt tggccctcca ggccagcctg 2400

aggaaggagc agatgcgcat ccagtcgctg gagaagacag tggagcagaa gactaaagag 2460

aacgaggagc tgaccaggat ctgcgacgac ctcatctcca agatggagaa gatctga 2517

<210> 530
<211> 2850
<212> DNA
<213> Artificial Sequence

<220>
<223> Description of Artificial Sequence: Synthetic
      polynucleotide

<400> 530
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgtaaag 2280

gcgacacagg aggagaaccg ggagctgagg agcaggtgtg aggagctcca cgggaagaac 2340

ctggaactgg ggaagatcat ggacaggttc gaagaggttg tgtaccaggc catggaggaa 2400

gttcagaagc agaaggaact ttccaaagct gaaatccaga aagttctaaa agaaaaagac 2460

caacttacca cagatctgaa ctccatggag aagtccttct ccgacctctt caagcgtttt 2520

gagaaacaga aagaggtgat cgagggctac cgcaagaacg aagagtcact gaagaagtgc 2580

gtggaggatt acctggcaag gatcacccag gagggccaga ggtaccaagc cctgaaggcc 2640

cacgcggagg agaagctgca gctggcaaac gaggagatcg cccaggtccg gagcaaggcc 2700

caggcggaag cgttggccct ccaggccagc ctgaggaagg agcagatgcg catccagtcg 2760

ctggagaaga cagtggagca gaagactaaa gagaacgagg agctgaccag gatctgcgac 2820

gacctcatct ccaagatgga gaagatctga 2850

<210> 531
<211> 3147
<212> DNA
<213> Artificial Sequence

<220>
<223> Description of Artificial Sequence: Synthetic
      polynucleotide

<400> 531
atgggcgccc ctgcctgcgc cctcgcgctc tgcgtggccg tggccatcgt ggccggcgcc      60

tcctcggagt ccttggggac ggagcagcgc gtcgtggggc gagcggcaga agtcccgggc     120

ccagagcccg gccagcagga gcagttggtc ttcggcagcg gggatgctgt ggagctgagc     180

tgtcccccgc ccgggggtgg tcccatgggg cccactgtct gggtcaagga tggcacaggg     240

ctggtgccct cggagcgtgt cctggtgggg ccccagcggc tgcaggtgct gaatgcctcc     300

cacgaggact ccggggccta cagctgccgg cagcggctca cgcagcgcgt actgtgccac     360

ttcagtgtgc gggtgacaga cgctccatcc tcgggagatg acgaagacgg ggaggacgag     420

gctgaggaca caggtgtgga cacaggggcc ccttactgga cacggcccga gcggatggac     480

aagaagctgc tggccgtgcc ggccgccaac accgtccgct tccgctgccc agccgctggc     540

aaccccactc cctccatctc ctggctgaag aacggcaggg agttccgcgg cgagcaccgc     600

attggaggca tcaagctgcg gcatcagcag tggagcctgg tcatggaaag cgtggtgccc     660

tcggaccgcg gcaactacac ctgcgtcgtg gagaacaagt ttggcagcat ccggcagacg     720

tacacgctgg acgtgctgga gcgctccccg caccggccca tcctgcaggc ggggctgccg     780

gccaaccaga cggcggtgct gggcagcgac gtggagttcc actgcaaggt gtacagtgac     840

gcacagcccc acatccagtg gctcaagcac gtggaggtga atggcagcaa ggtgggcccg     900

gacggcacac cctacgttac cgtgctcaag acggcgggcg ctaacaccac cgacaaggag     960

ctagaggttc tctccttgca caacgtcacc tttgaggacg ccggggagta cacctgcctg    1020

gcgggcaatt ctattgggtt ttctcatcac tctgcgtggc tggtggtgct gccagccgag    1080

gaggagctgg tggaggctga cgaggcgggc agtgtgtatg caggcatcct cagctacggg    1140

gtgggcttct tcctgttcat cctggtggtg gcggctgtga cgctctgccg cctgcgcagc    1200

ccccccaaga aaggcctggg ctcccccacc gtgcacaaga tctcccgctt cccgctcaag    1260

cgacaggtgt ccctggagtc caacgcgtcc atgagctcca acacaccact ggtgcgcatc    1320

gcaaggctgt cctcagggga gggccccacg ctggccaatg tctccgagct cgagctgcct    1380

gccgacccca aatgggagct gtctcgggcc cggctgaccc tgggcaagcc ccttggggag    1440

ggctgcttcg gccaggtggt catggcggag gccatcggca ttgacaagga ccgggccgcc    1500

aagcctgtca ccgtagccgt gaagatgctg aaagacgatg ccactgacaa ggacctgtcg    1560

gacctggtgt ctgagatgga gatgatgaag atgatcggga aacacaaaaa catcatcaac    1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgactttaag 2280

gagtcggcct tgaggaagca gtccttatac ctcaagttcg accccctcct gagggacagt 2340

cctggtagac cagtgcccgt ggccaccgag accagcagca tgcacggtgc aaatgagact 2400

ccctcaggac gtccgcggga agccaagctt gtggagttcg atttcttggg agcactggac 2460

attcctgtgc caggcccacc cccaggtgtt cccgcgcctg ggggcccacc cctgtccacc 2520

ggacctatag tggacctgct ccagtacagc cagaaggacc tggatgcagt ggtaaaggcg 2580

acacaggagg agaaccggga gctgaggagc aggtgtgagg agctccacgg gaagaacctg 2640

gaactgggga agatcatgga caggttcgaa gaggttgtgt accaggccat ggaggaagtt 2700

cagaagcaga aggaactttc caaagctgaa atccagaaag ttctaaaaga aaaagaccaa 2760

cttaccacag atctgaactc catggagaag tccttctccg acctcttcaa gcgttttgag 2820

aaacagaaag aggtgatcga gggctaccgc aagaacgaag agtcactgaa gaagtgcgtg 2880

gaggattacc tggcaaggat cacccaggag ggccagaggt accaagccct gaaggcccac 2940

gcggaggaga agctgcagct ggcaaacgag gagatcgccc aggtccggag caaggcccag 3000

gcggaagcgt tggccctcca ggccagcctg aggaaggagc agatgcgcat ccagtcgctg 3060

gagaagacag tggagcagaa gactaaagag aacgaggagc tgaccaggat ctgcgacgac 3120

ctcatctcca agatggagaa gatctga 3147

<210> 532
<211> 2955
<212> DNA
<213> Artificial Sequence

<220>
<223> Description of Artificial Sequence: Synthetic
      polynucleotide

<400> 532
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgtgcca 2280

ggcccacccc caggtgttcc cgcgcctggg ggcccacccc tgtccaccgg acctatagtg 2340

gacctgctcc agtacagcca gaaggacctg gatgcagtgg taaaggcgac acaggaggag 2400

aaccgggagc tgaggagcag gtgtgaggag ctccacggga agaacctgga actggggaag 2460

atcatggaca ggttcgaaga ggttgtgtac caggccatgg aggaagttca gaagcagaag 2520

gaactttcca aagctgaaat ccagaaagtt ctaaaagaaa aagaccaact taccacagat 2580

ctgaactcca tggagaagtc cttctccgac ctcttcaagc gttttgagaa acagaaagag 2640

gtgatcgagg gctaccgcaa gaacgaagag tcactgaaga agtgcgtgga ggattacctg 2700

gcaaggatca cccaggaggg ccagaggtac caagccctga aggcccacgc ggaggagaag 2760

ctgcagctgg caaacgagga gatcgcccag gtccggagca aggcccaggc ggaagcgttg 2820

gccctccagg ccagcctgag gaaggagcag atgcgcatcc agtcgctgga gaagacagtg 2880

gagcagaaga ctaaagagaa cgaggagctg accaggatct gcgacgacct catctccaag 2940

atggagaaga tctga 2955

<210> 533
<211> 3330
<212> DNA
<213> Artificial Sequence

<220>
<223> Description of Artificial Sequence: Synthetic
      polynucleotide

<400> 533
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagaga 2280

gccttgaact ctgccagcac ctcgcttccc acaagctgtc caggcagtga gccagtgccc 2340

acccatcagc aggggcagcc tgccttggag ctgaaagagg agagcttcag agaccccgct 2400

gaggttctag gcacgggcgc ggaggtggat tacctggagc agtttggaac ttcctcgttt 2460

aaggagtcgg ccttgaggaa gcagtcctta tacctcaagt tcgaccccct cctgagggac 2520

agtcctggta gaccagtgcc cgtggccacc gagaccagca gcatgcacgg tgcaaatgag 2580

actccctcag gacgtccgcg ggaagccaag cttgtggagt tcgatttctt gggagcactg 2640

gacattcctg tgccaggccc acccccaggt gttcccgcgc ctgggggccc acccctgtcc 2700

accggaccta tagtggacct gctccagtac agccagaagg acctggatgc agtggtaaag 2760

gcgacacagg aggagaaccg ggagctgagg agcaggtgtg aggagctcca cgggaagaac 2820

ctggaactgg ggaagatcat ggacaggttc gaagaggttg tgtaccaggc catggaggaa 2880

gttcagaagc agaaggaact ttccaaagct gaaatccaga aagttctaaa agaaaaagac 2940

caacttacca cagatctgaa ctccatggag aagtccttct ccgacctctt caagcgtttt 3000

gagaaacaga aagaggtgat cgagggctac cgcaagaacg aagagtcact gaagaagtgc 3060

gtggaggatt acctggcaag gatcacccag gagggccaga ggtaccaagc cctgaaggcc 3120

cacgcggagg agaagctgca gctggcaaac gaggagatcg cccaggtccg gagcaaggcc 3180

caggcggaag cgttggccct ccaggccagc ctgaggaagg agcagatgcg catccagtcg 3240

ctggagaaga cagtggagca gaagactaaa gagaacgagg agctgaccag gatctgcgac 3300

gacctcatct ccaagatgga gaagatctga 3330

<210> 534
<211> 2780
<212> DNA
<213> Artificial Sequence

<220>
<223> Description of Artificial Sequence: Synthetic
      polynucleotide

<400> 534
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagtac 2280

ctggacctgt cggcgccttt cgagcagtac tccccgggtg gccaggacac ccccagaagc 2340

agaaggaact ttccaaagct gaaatccaga aagttctaaa agaaaaagac caacttacca 2400

cagatctgaa ctccatggag aagtccttct ccgacctctt caagcgtttt gagaaacaga 2460

aagaggtgat cgagggctac cgcaagaacg aagagtcact gaagaagtgc gtggaggatt 2520

acctggcaag gatcacccag gagggccaga ggtaccaagc cctgaaggcc cacgcggagg 2580

agaagctgca gctggcaaac gaggagatcg cccaggtccg gagcaaggcc caggcggaag 2640

cgttggccct ccaggccagc ctgaggaagg agcagatgcg catccagtcg ctggagaaga 2700

cagtggagca gaagactaaa gagaacgagg agctgaccag gatctgcgac gacctcatct 2760

ccaagatgga gaagatctga 2780

<210> 535
<211> 3135
<212> DNA
<213> Artificial Sequence

<220>
<223> Description of Artificial Sequence: Synthetic
      polynucleotide

<400> 535
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagtac 2280

ctggacctgt cggcgcagga gcaacggcag cctaccctcc agccacaggg ctgctgcctt 2340

gctggttaca gccaccgttt ctctagcatg cacggtgcaa atgagactcc ctcaggacgt 2400

ccgcgggaag ccaagcttgt ggagttcgat ttcttgggag cactggacat tcctgtgcca 2460

ggcccacccc caggtgttcc cgcgcctggg ggcccacccc tgtccaccgg acctatagtg 2520

gacctgctcc agtacagcca gaaggacctg gatgcagtgg taaaggcgac acaggaggag 2580

aaccgggagc tgaggagcag gtgtgaggag ctccacggga agaacctgga actggggaag 2640

atcatggaca ggttcgaaga ggttgtgtac caggccatgg aggaagttca gaagcagaag 2700

gaactttcca aagctgaaat ccagaaagtt ctaaaagaaa aagaccaact taccacagat 2760

ctgaactcca tggagaagtc cttctccgac ctcttcaagc gttttgagaa acagaaagag 2820

gtgatcgagg gctaccgcaa gaacgaagag tcactgaaga agtgcgtgga ggattacctg 2880

gcaaggatca cccaggaggg ccagaggtac caagccctga aggcccacgc ggaggagaag 2940

ctgcagctgg caaacgagga gatcgcccag gtccggagca aggcccaggc ggaagcgttg 3000

gccctccagg ccagcctgag gaaggagcag atgcgcatcc agtcgctgga gaagacagtg 3060

gagcagaaga ctaaagagaa cgaggagctg accaggatct gcgacgacct catctccaag 3120

atggagaaga tctga 3135

<210> 536
<211> 3396
<212> DNA
<213> Artificial Sequence

<220>
<223> Description of Artificial Sequence: Synthetic
      polynucleotide

<400> 536
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500
aagcctgtca ccgtagccgt gaagatgctg aaagacgatg ccactgacaa ggacctgtcg 1560
gacctggtgt ctgagatgga gatgatgaag atgatcggga aacacaaaaa catcatcaac 1620
ctgctgggcg cctgcacgca gggcgggccc ctgtacgtgc tggtggagta cgcggccaag 1680
ggtaacctgc gggagtttct gcgggcgcgg cggcccccgg gcctggacta ctccttcgac 1740
acctgcaagc cgcccgagga gcagctcacc ttcaaggacc tggtgtcctg tgcctaccag 1800
gtggcccggg gcatggagta cttggcctcc cagaagtgca tccacaggga cctggctgcc 1860
cgcaatgtgc tggtgaccga ggacaacgtg atgaagatcg cagacttcgg gctggcccgg 1920
gacgtgcaca acctcgacta ctacaagaag acgaccaacg gccggctgcc cgtgaagtgg 1980
atggcgcctg aggccttgtt tgaccgagtc tacactcacc agagtgacgt ctggtccttt 2040
ggggtcctgc tctgggagat cttcacgctg gggggctccc cgtaccccgg catccctgtg 2100
gaggagctct tcaagctgct gaaggagggc caccgcatgg acaagcccgc caactgcaca 2160
cacgacctgt acatgatcat gcgggagtgc tggcatgccg cgccctccca gaggcccacc 2220
ttcaagcagc tggtggagga cctggaccgt gtccttaccg tgacgtccac cgacgagtac 2280
ctggacctgt cggcgcctgt ggtgcagttg gcagccgaga ccccaacagc agagagcaag 2340
gagagagcct tgaactctgc cagcacctcg cttcccacaa gctgtccagg cagtgagcca 2400
gtgcccaccc atcagcaggg gcagcctgcc ttggagctga aagaggagag cttcagagac 2460
cccgctgagg ttctaggcac gggcgcggag gtggattacc tggagcagtt tggaacttcc 2520
tcgtttaagg agtcggcctt gaggaagcag tccttatacc tcaagttcga ccccctcctg 2580
agggacagtc ctggtagacc agtgcccgtg gccaccgaga ccagcagcat gcacggtgca 2640
aatgagactc cctcaggacg tccgcgggaa gccaagcttg tggagttcga tttcttggga 2700
gcactggaca ttcctgtgcc aggcccaccc ccaggtgttc ccgcgcctgg gggcccaccc 2760
ctgtccaccg gacctatagt ggacctgctc cagtacagcc agaaggacct ggatgcagtg 2820
gtaaaggcga cacaggagga gaaccgggag ctgaggagca ggtgtgagga gctccacggg 2880
aagaacctgg aactggggaa gatcatggac aggttcgaag aggttgtgta ccaggccatg 2940
gaggaagttc agaagcagaa ggaactttcc aaagctgaaa tccagaaagt tctaaaagaa 3000
aaagaccaac ttaccacaga tctgaactcc atggagaagt ccttctccga cctcttcaag 3060
cgttttgaga aacagaaaga ggtgatcgag ggctaccgca agaacgaaga gtcactgaag 3120
aagtgcgtgg aggattacct ggcaaggatc acccaggagg gccagaggta ccaagccctg 3180
aaggcccacg cggaggagaa gctgcagctg gcaaacgagg agatcgccca ggtccggagc 3240
aaggcccagg cggaagcgtt ggccctccag gccagcctga ggaaggagca gatgcgcatc 3300

cagtcgctgg agaagacagt ggagcagaag actaaagaga acgaggagct gaccaggatc 3360

tgcgacgacc tcatctccaa gatggagaag atctga 3396

<210> 537
<211> 3504
<212> DNA
<213> Artificial Sequence

<220>
<223> Description of Artificial Sequence: Synthetic
      polynucleotide

<400> 537
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagtac 2280

ctggacctgt cggcgccttt cgagcagtac tccccgggtg gccaggacac ccccagctcc 2340

agctcctcag gggacgtgcg tgagccaccg cacccggcgt ttcctaggtc agcggaggac 2400

acgcctgtgg tgcagttggc agccgagacc ccaacagcag agagcaagga gagagccttg 2460

aactctgcca gcacctcgct tcccacaagc tgtccaggca gtgagccagt gcccacccat 2520

cagcaggggc agcctgcctt ggagctgaaa gaggagagct tcagagaccc cgctgaggtt 2580

ctaggcacgg gcgcggaggt ggattacctg gagcagtttg gaacttcctc gtttaaggag 2640

tcggccttga ggaagcagtc cttatacctc aagttcgacc ccctcctgag ggacagtcct 2700

ggtagaccag tgcccgtggc caccgagacc agcagcatgc acggtgcaaa tgagactccc 2760

tcaggacgtc cgcgggaagc caagcttgtg gagttcgatt tcttgggagc actggacatt 2820

cctgtgccag gcccaccccc aggtgttccc gcgcctgggg gcccacccct gtccaccgga 2880

cctatagtgg acctgctcca gtacagccag aaggacctgg atgcagtggt aaaggcgaca 2940

caggaggaga accgggagct gaggagcagg tgtgaggagc tccacgggaa gaacctggaa 3000

ctggggaaga tcatggacag gttcgaagag gttgtgtacc aggccatgga ggaagttcag 3060

aagcagaagg aactttccaa agctgaaatc cagaaagttc taaaagaaaa agaccaactt 3120

accacagatc tgaactccat ggagaagtcc ttctccgacc tcttcaagcg ttttgagaaa 3180

cagaaagagg tgatcgaggg ctaccgcaag aacgaagagt cactgaagaa gtgcgtggag 3240

gattacctgg caaggatcac ccaggagggc cagaggtacc aagccctgaa ggcccacgcg 3300

gaggagaagc tgcagctggc aaacgaggag atcgcccagg tccggagcaa ggcccaggcg 3360

gaagcgttgg ccctccaggc cagcctgagg aaggagcaga tgcgcatcca gtcgctggag 3420

aagacagtgg agcagaagac taaagagaac gaggagctga ccaggatctg cgacgacctc 3480

atctccaaga tggagaagat ctga 3504

<210> 538
<211> 3555
<212> DNA
<213> Artificial Sequence

<220>
<223> Description of Artificial Sequence: Synthetic
      polynucleotide

<400> 538
atgggcgccc ctgcctgcgc cctcgcgctc tgcgtggccg tggccatcgt ggccggcgcc 60

tcctcggagt ccttggggac ggagcagcgc gtcgtggggc gagcggcaga agtcccgggc 120

ccagagcccg gccagcagga gcagttggtc ttcggcagcg gggatgctgt ggagctgagc 180

tgtcccccgc ccgggggtgg tcccatgggg cccactgtct gggtcaagga tggcacaggg 240

ctggtgccct cggagcgtgt cctggtgggg ccccagcggc tgcaggtgct gaatgcctcc 300

cacgaggact ccggggccta cagctgccgg cagcggctca cgcagcgcgt actgtgccac 360

ttcagtgtgc gggtgacaga cgctccatcc tcgggagatg acgaagacgg ggaggacgag 420

gctgaggaca caggtgtgga cacaggggcc ccttactgga cacggcccga gcggatggac 480

aagaagctgc tggccgtgcc ggccgccaac accgtccgct tccgctgccc agccgctggc 540

aaccccactc cctccatctc ctggctgaag aacggcaggg agttccgcgg cgagcaccgc 600

attggaggca tcaagctgcg gcatcagcag tggagcctgg tcatggaaag cgtggtgccc 660

tcggaccgcg gcaactacac ctgcgtcgtg gagaacaagt ttggcagcat ccggcagacg 720

tacacgctgg acgtgctgga gcgctccccg caccggccca tcctgcaggc ggggctgccg 780

gccaaccaga cggcggtgct gggcagcgac gtggagttcc actgcaaggt gtacagtgac 840

gcacagcccc acatccagtg gctcaagcac gtggaggtga atggcagcaa ggtgggcccg 900

gacggcacac cctacgttac cgtgctcaag acggcgggcg ctaacaccac cgacaaggag 960

ctagaggttc tctccttgca caacgtcacc tttgaggacg ccggggagta cacctgcctg 1020

gcgggcaatt ctattgggtt ttctcatcac tctgcgtggc tggtggtgct gccagccgag 1080

gaggagctgg tggaggctga cgaggcgggc agtgtgtatg caggcatcct cagctacggg 1140

gtgggcttct tcctgttcat cctggtggtg gcggctgtga cgctctgccg cctgcgcagc 1200

ccccccaaga aaggcctggg ctcccccacc gtgcacaaga tctcccgctt cccgctcaag 1260

cgacaggtgt ccctggagtc caacgcgtcc atgagctcca acacaccact ggtgcgcatc 1320

gcaaggctgt cctcagggga gggccccacg ctggccaatg tctccgagct cgagctgcct 1380

gccgacccca aatgggagct gtctcgggcc cggctgaccc tgggcaagcc ccttggggag 1440

ggctgcttcg gccaggtggt catggcggag gccatcggca ttgacaagga ccgggccgcc 1500

aagcctgtca ccgtagccgt gaagatgctg aaagacgatg ccactgacaa ggacctgtcg 1560

gacctggtgt ctgagatgga gatgatgaag atgatcggga aacacaaaaa catcatcaac 1620

ctgctgggcg cctgcacgca gggcgggccc ctgtacgtgc tggtggagta cgcggccaag 1680

ggtaacctgc gggagtttct gcgggcgcgg cggcccccgg gcctggacta ctccttcgac 1740

acctgcaagc cgcccgagga gcagctcacc ttcaaggacc tggtgtcctg tgcctaccag 1800

gtggcccggg gcatggagta cttggcctcc cagaagtgca tccacaggga cctggctgcc 1860

cgcaatgtgc tggtgaccga ggacaacgtg atgaagatcg cagacttcgg gctggcccgg 1920

gacgtgcaca acctcgacta ctacaagaag acgaccaacg gccggctgcc cgtgaagtgg 1980

atggcgcctg aggccttgtt tgaccgagtc tacactcacc agagtgacgt ctggtccttt 2040

ggggtcctgc tctgggagat cttcacgctg gggggctccc cgtaccccgg catccctgtg 2100

gaggagctct tcaagctgct gaaggagggc caccgcatgg acaagcccgc caactgcaca 2160

cacgacctgt acatgatcat gcgggagtgc tggcatgccg cgccctccca gaggcccacc 2220

ttcaagcagc tggtggagga cctggaccgt gtccttaccg tgacgtccac cgacgagtac 2280

ctggacctgt cggcgccttt cgagcagtac tccccgggtg gccaggacac cccagaaagc 2340

cctgagacca ggctgggcca gccagcggct gaacagttgc atgctgggcc tgccacggag 2400

gagccaggtc cctgtctgag ccagcagctg cattcagcct cagcggagga cacgcctgtg 2460

gtgcagttgg cagccgagac cccaacagca gagagcaagg agagagcctt gaactctgcc 2520

agcacctcgc ttcccacaag ctgtccaggc agtgagccag tgcccaccca tcagcagggg 2580

cagcctgcct tggagctgaa agaggagagc ttcagagacc ccgctgaggt tctaggcacg 2640

ggcgcggagg tggattacct ggagcagttt ggaacttcct cgtttaagga gtcggccttg 2700

aggaagcagt ccttatacct caagttcgac cccctcctga gggacagtcc tggtagacca 2760

gtgcccgtgg ccaccgagac cagcagcatg cacggtgcaa atgagactcc ctcaggacgt 2820

ccgcgggaag ccaagcttgt ggagttcgat ttcttgggag cactggacat tcctgtgcca 2880

ggcccacccc caggtgttcc cgcgcctggg ggcccacccc tgtccaccgg acctatagtg 2940

gacctgctcc agtacagcca gaaggacctg gatgcagtgg taaaggcgac acaggaggag 3000

aaccgggagc tgaggagcag gtgtgaggag ctccacggga agaacctgga actggggaag 3060

atcatggaca ggttcgaaga ggttgtgtac caggccatgg aggaagttca gaagcagaag 3120

gaactttcca aagctgaaat ccagaaagtt ctaaaagaaa aagaccaact taccacagat 3180

ctgaactcca tggagaagtc cttctccgac ctcttcaagc gttttgagaa acagaaagag 3240

gtgatcgagg gctaccgcaa gaacgaagag tcactgaaga agtgcgtgga ggattacctg 3300

gcaaggatca cccaggaggg ccagaggtac caagccctga aggcccacgc ggaggagaag 3360

ctgcagctgg caaacgagga gatcgcccag gtccggagca aggcccaggc ggaagcgttg 3420

gccctccagg ccagcctgag gaaggagcag atgcgcatcc agtcgctgga gaagacagtg 3480

gagcagaaga ctaaagagaa cgaggagctg accaggatct gcgacgacct catctccaag 3540

atggagaaga tctga 3555

<210> 539
<211> 949
<212> PRT
<213> Artificial Sequence

<220>
<223> Description of Artificial Sequence: Synthetic
      polypeptide

<400> 539
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Val Lys Ala Thr Gln Glu Glu Asn Arg Glu
        755                 760                 765

Leu Arg Ser Arg Cys Glu Glu Leu His Gly Lys Asn Leu Glu Leu Gly
    770                 775                 780

Lys Ile Met Asp Arg Phe Glu Glu Val Val Tyr Gln Ala Met Glu Glu
785                 790                 795                 800

Val Gln Lys Gln Lys Glu Leu Ser Lys Ala Glu Ile Gln Lys Val Leu
                805                 810                 815

Lys Glu Lys Asp Gln Leu Thr Thr Asp Leu Asn Ser Met Glu Lys Ser
            820                 825                 830

Phe Ser Asp Leu Phe Lys Arg Phe Glu Lys Gln Lys Glu Val Ile Glu
        835                 840                 845

Gly Tyr Arg Lys Asn Glu Glu Ser Leu Lys Lys Cys Val Glu Asp Tyr
    850                 855                 860

Leu Ala Arg Ile Thr Gln Glu Gly Gln Arg Tyr Gln Ala Leu Lys Ala
865                 870                 875                 880

His Ala Glu Glu Lys Leu Gln Leu Ala Asn Glu Glu Ile Ala Gln Val
                885                 890                 895

Arg Ser Lys Ala Gln Ala Glu Ala Leu Ala Leu Gln Ala Ser Leu Arg
            900                 905                 910

Lys Glu Gln Met Arg Ile Gln Ser Leu Glu Lys Thr Val Glu Gln Lys

915                 920                 925

Thr Lys Glu Asn Glu Glu Leu Thr Arg Ile Cys Asp Asp Leu Ile Ser
    930                 935                 940

Lys Met Glu Lys Ile
945

<210> 540
<211> 1048
<212> PRT
<213> Artificial Sequence

<220>
<223> Description of Artificial Sequence: Synthetic
      polypeptide

<400> 540
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser

420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Phe Lys Glu Ser Ala Leu Arg Lys Gln Ser
        755                 760                 765

Leu Tyr Leu Lys Phe Asp Pro Leu Leu Arg Asp Ser Pro Gly Arg Pro
    770                 775                 780

Val Pro Val Ala Thr Glu Thr Ser Ser Met His Gly Ala Asn Glu Thr
785                 790                 795                 800

Pro Ser Gly Arg Pro Arg Glu Ala Lys Leu Val Glu Phe Asp Phe Leu
                805                 810                 815

Gly Ala Leu Asp Ile Pro Val Pro Gly Pro Pro Pro Gly Val Pro Ala
            820                 825                 830

Pro Gly Gly Pro Pro Leu Ser Thr Gly Pro Ile Val Asp Leu Leu Gln
        835                 840                 845

Tyr Ser Gln Lys Asp Leu Asp Ala Val Val Lys Ala Thr Gln Glu Glu
    850                 855                 860

Asn Arg Glu Leu Arg Ser Arg Cys Glu Glu Leu His Gly Lys Asn Leu
865                 870                 875                 880

Glu Leu Gly Lys Ile Met Asp Arg Phe Glu Glu Val Val Tyr Gln Ala
                885                 890                 895

Met Glu Glu Val Gln Lys Gln Lys Glu Leu Ser Lys Ala Glu Ile Gln
            900                 905                 910

Lys Val Leu Lys Glu Lys Asp Gln Leu Thr Thr Asp Leu Asn Ser Met
        915                 920                 925

Glu Lys Ser Phe Ser Asp Leu Phe Lys Arg Phe Glu Lys Gln Lys Glu
    930                 935                 940

Val Ile Glu Gly Tyr Arg Lys Asn Glu Glu Ser Leu Lys Lys Cys Val
945                 950                 955                 960

Glu Asp Tyr Leu Ala Arg Ile Thr Gln Glu Gly Gln Arg Tyr Gln Ala
                965                 970                 975

Leu Lys Ala His Ala Glu Glu Lys Leu Gln Leu Ala Asn Glu Glu Ile
            980                 985                 990

Ala Gln Val Arg Ser Lys Ala Gln Ala Glu Ala Leu Ala Leu Gln Ala
        995                 1000                1005

Ser Leu Arg Lys Glu Gln Met Arg Ile Gln Ser Leu Glu Lys Thr
    1010                1015                1020

Val Glu Gln Lys Thr Lys Glu Asn Glu Glu Leu Thr Arg Ile Cys
    1025                1030                1035

Asp Asp Leu Ile Ser Lys Met Glu Lys Ile
    1040                1045

<210> 541
<211> 984
<212> PRT
<213> Artificial Sequence

<220>
<223> Description of Artificial Sequence: Synthetic
polypeptide

<400> 541
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Val Pro Gly Pro Pro Pro Gly Val Pro Ala
        755                 760                 765

Pro Gly Gly Pro Pro Leu Ser Thr Gly Pro Ile Val Asp Leu Leu Gln
    770                 775                 780

Tyr Ser Gln Lys Asp Leu Asp Ala Val Val Lys Ala Thr Gln Glu Glu
785                 790                 795                 800

Asn Arg Glu Leu Arg Ser Arg Cys Glu Glu Leu His Gly Lys Asn Leu
                805                 810                 815

Glu Leu Gly Lys Ile Met Asp Arg Phe Glu Glu Val Val Tyr Gln Ala
            820                 825                 830

Met Glu Glu Val Gln Lys Gln Lys Glu Leu Ser Lys Ala Glu Ile Gln

835                 840                 845

Lys Val Leu Lys Glu Lys Asp Gln Leu Thr Thr Asp Leu Asn Ser Met
    850                 855                 860

Glu Lys Ser Phe Ser Asp Leu Phe Lys Arg Phe Glu Lys Gln Lys Glu
865                 870                 875                 880

Val Ile Glu Gly Tyr Arg Lys Asn Glu Glu Ser Leu Lys Lys Cys Val
                885                 890                 895

Glu Asp Tyr Leu Ala Arg Ile Thr Gln Glu Gly Gln Arg Tyr Gln Ala
            900                 905                 910

Leu Lys Ala His Ala Glu Glu Lys Leu Gln Leu Ala Asn Glu Glu Ile
        915                 920                 925

Ala Gln Val Arg Ser Lys Ala Gln Ala Glu Ala Leu Ala Leu Gln Ala
    930                 935                 940

Ser Leu Arg Lys Glu Gln Met Arg Ile Gln Ser Leu Glu Lys Thr Val
945                 950                 955                 960

Glu Gln Lys Thr Lys Glu Asn Glu Glu Leu Thr Arg Ile Cys Asp Asp
                965                 970                 975

Leu Ile Ser Lys Met Glu Lys Ile
            980

<210> 542
<211> 1109
<212> PRT
<213> Artificial Sequence

<220>
<223> Description of Artificial Sequence: Synthetic
      polypeptide

<400> 542
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu

305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Arg Ala Leu Asn Ser Ala Ser Thr Ser
        755                 760                 765

Leu Pro Thr Ser Cys Pro Gly Ser Glu Pro Val Pro Thr His Gln Gln
    770                 775                 780

Gly Gln Pro Ala Leu Glu Leu Lys Glu Glu Ser Phe Arg Asp Pro Ala
785                 790                 795                 800

Glu Val Leu Gly Thr Gly Ala Glu Val Asp Tyr Leu Glu Gln Phe Gly
                805                 810                 815

Thr Ser Ser Phe Lys Glu Ser Ala Leu Arg Lys Gln Ser Leu Tyr Leu
            820                 825                 830

Lys Phe Asp Pro Leu Leu Arg Asp Ser Pro Gly Arg Pro Val Pro Val
        835                 840                 845

Ala Thr Glu Thr Ser Ser Met His Gly Ala Asn Glu Thr Pro Ser Gly
    850                 855                 860

Arg Pro Arg Glu Ala Lys Leu Val Glu Phe Asp Phe Leu Gly Ala Leu
865                 870                 875                 880

Asp Ile Pro Val Pro Gly Pro Pro Pro Gly Val Pro Ala Pro Gly Gly
                885                 890                 895

Pro Pro Leu Ser Thr Gly Pro Ile Val Asp Leu Leu Gln Tyr Ser Gln
            900                 905                 910

Lys Asp Leu Asp Ala Val Val Lys Ala Thr Gln Glu Glu Asn Arg Glu
        915                 920                 925

Leu Arg Ser Arg Cys Glu Glu Leu His Gly Lys Asn Leu Glu Leu Gly
    930                 935                 940

Lys Ile Met Asp Arg Phe Glu Glu Val Val Tyr Gln Ala Met Glu Glu
945                 950                 955                 960

Val Gln Lys Gln Lys Glu Leu Ser Lys Ala Glu Ile Gln Lys Val Leu
                965                 970                 975

Lys Glu Lys Asp Gln Leu Thr Thr Asp Leu Asn Ser Met Glu Lys Ser
            980                 985                 990

Phe Ser Asp Leu Phe Lys Arg Phe Glu Lys Gln Lys Glu Val Ile Glu
        995                 1000                1005

Gly Tyr Arg Lys Asn Glu Glu Ser Leu Lys Lys Cys Val Glu Asp
    1010                1015                1020

Tyr Leu Ala Arg Ile Thr Gln Glu Gly Gln Arg Tyr Gln Ala Leu
    1025                1030                1035

Lys Ala His Ala Glu Glu Lys Leu Gln Leu Ala Asn Glu Glu Ile
    1040                1045                1050

Ala Gln Val Arg Ser Lys Ala Gln Ala Glu Ala Leu Ala Leu Gln
    1055                1060                1065

Ala Ser Leu Arg Lys Glu Gln Met Arg Ile Gln Ser Leu Glu Lys
    1070                1075                1080

Thr Val Glu Gln Lys Thr Lys Glu Asn Glu Glu Leu Thr Arg Ile
    1085                1090                1095

Cys Asp Asp Leu Ile Ser Lys Met Glu Lys Ile
    1100                1105

<210> 543
<211> 926
<212> PRT
<213> Artificial Sequence

<220>
<223> Description of Artificial Sequence: Synthetic
      polypeptide

<400> 543
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr

            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Lys Gln Lys Glu Leu
    770                 775                 780

Ser Lys Ala Glu Ile Gln Lys Val Leu Lys Glu Lys Asp Gln Leu Thr
785                 790                 795                 800

Thr Asp Leu Asn Ser Met Glu Lys Ser Phe Ser Asp Leu Phe Lys Arg
                805                 810                 815

Phe Glu Lys Gln Lys Glu Val Ile Glu Gly Tyr Arg Lys Asn Glu Glu
            820                 825                 830

Ser Leu Lys Lys Cys Val Glu Asp Tyr Leu Ala Arg Ile Thr Gln Glu
        835                 840                 845

Gly Gln Arg Tyr Gln Ala Leu Lys Ala His Ala Glu Glu Lys Leu Gln
    850                 855                 860

Leu Ala Asn Glu Glu Ile Ala Gln Val Arg Ser Lys Ala Gln Ala Glu
865                 870                 875                 880

Ala Leu Ala Leu Gln Ala Ser Leu Arg Lys Glu Gln Met Arg Ile Gln
                885                 890                 895

Ser Leu Glu Lys Thr Val Glu Gln Lys Thr Lys Glu Asn Glu Glu Leu
            900                 905                 910

Thr Arg Ile Cys Asp Asp Leu Ile Ser Lys Met Glu Lys Ile
        915                 920                 925

<210> 544
<211> 1043
<212> PRT
<213> Artificial Sequence

<220>
<223> Description of Artificial Sequence: Synthetic
      polypeptide

<400> 544
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His

195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Gln Glu Gln
        755                 760                 765

Arg Gln Pro Thr Leu Gln Pro Gln Gly Cys Cys Leu Ala Gly Tyr Ser
    770                 775                 780

His Arg Ser Ser Met His Gly Ala Asn Glu Thr Pro Ser Gly Arg Pro
785                 790                 795                 800

Arg Glu Ala Lys Leu Val Glu Phe Asp Phe Leu Gly Ala Leu Asp Ile
                805                 810                 815

Pro Val Pro Gly Pro Pro Pro Gly Val Pro Ala Pro Gly Gly Pro Pro
            820                 825                 830

Leu Ser Thr Gly Pro Ile Val Asp Leu Leu Gln Tyr Ser Gln Lys Asp
        835                 840                 845

Leu Asp Ala Val Val Lys Ala Thr Gln Glu Glu Asn Arg Glu Leu Arg
    850                 855                 860

Ser Arg Cys Glu Glu Leu His Gly Lys Asn Leu Glu Leu Gly Lys Ile
865                 870                 875                 880

Met Asp Arg Phe Glu Glu Val Val Tyr Gln Ala Met Glu Glu Val Gln
                885                 890                 895

Lys Gln Lys Glu Leu Ser Lys Ala Glu Ile Gln Lys Val Leu Lys Glu
            900                 905                 910

Lys Asp Gln Leu Thr Thr Asp Leu Asn Ser Met Glu Lys Ser Phe Ser
        915                 920                 925

Asp Leu Phe Lys Arg Phe Glu Lys Gln Lys Glu Val Ile Glu Gly Tyr
    930                 935                 940

Arg Lys Asn Glu Glu Ser Leu Lys Lys Cys Val Glu Asp Tyr Leu Ala
945                 950                 955                 960

Arg Ile Thr Gln Glu Gly Gln Arg Tyr Gln Ala Leu Lys Ala His Ala
                965                 970                 975

Glu Glu Lys Leu Gln Leu Ala Asn Glu Glu Ile Ala Gln Val Arg Ser
            980                 985                 990

Lys Ala Gln Ala Glu Ala Leu Ala  Leu Gln Ala Ser Leu  Arg Lys Glu
        995                 1000                 1005

Gln Met  Arg Ile Gln Ser Leu  Glu Lys Thr Val Glu  Gln Lys Thr
    1010                 1015                 1020

Lys Glu  Asn Glu Glu Leu Thr  Arg Ile Cys Asp Asp  Leu Ile Ser
    1025                 1030                 1035

Lys Met  Glu Lys Ile
    1040

<210> 545
<211> 1131
<212> PRT
<213> Artificial Sequence

<220>
<223> Description of Artificial Sequence: Synthetic
      polypeptide

<400> 545
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu

610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Val Val
        755                 760                 765

Gln Leu Ala Ala Glu Thr Pro Thr Ala Glu Ser Lys Glu Arg Ala Leu
    770                 775                 780

Asn Ser Ala Ser Thr Ser Leu Pro Thr Ser Cys Pro Gly Ser Glu Pro
785                 790                 795                 800

Val Pro Thr His Gln Gln Gly Gln Pro Ala Leu Glu Leu Lys Glu Glu
                805                 810                 815

Ser Phe Arg Asp Pro Ala Glu Val Leu Gly Thr Gly Ala Glu Val Asp
            820                 825                 830

Tyr Leu Glu Gln Phe Gly Thr Ser Ser Phe Lys Glu Ser Ala Leu Arg
        835                 840                 845

Lys Gln Ser Leu Tyr Leu Lys Phe Asp Pro Leu Leu Arg Asp Ser Pro
    850                 855                 860

Gly Arg Pro Val Pro Val Ala Thr Glu Thr Ser Ser Met His Gly Ala
865                 870                 875                 880

Asn Glu Thr Pro Ser Gly Arg Pro Arg Glu Ala Lys Leu Val Glu Phe
                885                 890                 895

Asp Phe Leu Gly Ala Leu Asp Ile Pro Val Pro Gly Pro Pro Pro Gly
            900                 905                 910

Val Pro Ala Pro Gly Gly Pro Pro Leu Ser Thr Gly Pro Ile Val Asp
        915                 920                 925

Leu Leu Gln Tyr Ser Gln Lys Asp Leu Asp Ala Val Val Lys Ala Thr
    930                 935                 940

Gln Glu Glu Asn Arg Glu Leu Arg Ser Arg Cys Glu Glu Leu His Gly
945                 950                 955                 960

Lys Asn Leu Glu Leu Gly Lys Ile Met Asp Arg Phe Glu Glu Val Val
                965                 970                 975

Tyr Gln Ala Met Glu Glu Val Gln Lys Gln Lys Glu Leu Ser Lys Ala
            980                 985                 990

Glu Ile Gln Lys Val Leu Lys Glu  Lys Asp Gln Leu Thr  Thr Asp Leu
        995                 1000                 1005

Asn Ser  Met Glu Lys Ser Phe  Ser Asp Leu Phe Lys  Arg Phe Glu
    1010                 1015                 1020

Lys Gln  Lys Glu Val Ile Glu  Gly Tyr Arg Lys Asn  Glu Glu Ser
    1025                 1030                 1035

Leu Lys  Lys Cys Val Glu Asp  Tyr Leu Ala Arg Ile  Thr Gln Glu
    1040                 1045                 1050

Gly Gln  Arg Tyr Gln Ala Leu  Lys Ala His Ala Glu  Glu Lys Leu
    1055                 1060                 1065

Gln Leu  Ala Asn Glu Glu Ile  Ala Gln Val Arg Ser  Lys Ala Gln
    1070                 1075                 1080

Ala Glu  Ala Leu Ala Leu Gln  Ala Ser Leu Arg Lys  Glu Gln Met
    1085                 1090                 1095

Arg Ile  Gln Ser Leu Glu Lys  Thr Val Glu Gln Lys  Thr Lys Glu
    1100                 1105                 1110

Asn Glu Glu Leu Thr Arg Ile Cys Asp Asp Leu Ile Ser Lys Met
    1115                1120                1125

Glu Lys Ile
    1130

<210> 546
<211> 1166
<212> PRT
<213> Artificial Sequence

<220>
<223> Description of Artificial Sequence: Synthetic
polypeptide

<220>
<221> MOD_RES
<222> (795)..(795)
<223> Any amino acid

<400> 546
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg

405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
        660                 665                 670

His Gln Ser Asp Val Trp Ser Phe Gly Val Leu Leu Trp Glu Ile Phe
    675                 680                 685

Thr Leu Gly Gly Ser Pro Tyr Pro Gly Ile Pro Val Glu Glu Leu Phe
690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
        740                 745                 750

Thr Val Thr Ser Thr Asp Glu Tyr Leu Asp Leu Ser Ala Pro Phe Glu
    755                 760                 765

Gln Tyr Ser Pro Gly Gly Gln Asp Thr Pro Ser Ser Ser Ser Ser Gly
770                 775                 780

Val Arg Glu Pro Pro His Pro Ala Phe Pro Xaa Ser Ala Glu Asp Thr
785                 790                 795                 800

Pro Val Val Gln Leu Ala Ala Glu Thr Pro Thr Ala Glu Ser Lys Glu
                805                 810                 815

Arg Ala Leu Asn Ser Ala Ser Thr Ser Leu Pro Thr Ser Cys Pro Gly
        820                 825                 830

Ser Glu Pro Val Pro Thr His Gln Gln Gly Gln Pro Ala Leu Glu Leu
    835                 840                 845

Lys Glu Glu Ser Phe Arg Asp Pro Ala Glu Val Leu Gly Thr Gly Ala
850                 855                 860

Glu Val Asp Tyr Leu Glu Gln Phe Gly Thr Ser Ser Phe Lys Glu Ser
865                 870                 875                 880

Ala Leu Arg Lys Gln Ser Leu Tyr Leu Lys Phe Asp Pro Leu Leu Arg
                885                 890                 895

Asp Ser Pro Gly Arg Pro Val Pro Val Ala Thr Glu Thr Ser Ser Met
        900                 905                 910

His Gly Ala Asn Glu Thr Pro Ser Gly Arg Pro Arg Glu Ala Lys Leu
        915                 920                 925

Val Glu Phe Asp Phe Leu Gly Ala Leu Asp Ile Pro Val Pro Gly Pro
    930                 935                 940

Pro Pro Gly Val Pro Ala Pro Gly Gly Pro Pro Leu Ser Thr Gly Pro
945                 950                 955                 960

Ile Val Asp Leu Leu Gln Tyr Ser Gln Lys Asp Leu Asp Ala Val Val
                965                 970                 975

Lys Ala Thr Gln Glu Glu Asn Arg Glu Leu Arg Ser Arg Cys Glu Glu
            980                 985                 990

Leu His Gly Lys Asn Leu Glu Leu Gly Lys Ile Met Asp Arg Phe Glu
        995                 1000                1005

Glu Val Val Tyr Gln Ala Met Glu Glu Val Gln Lys Gln Lys Glu
    1010                1015                1020

Leu Ser Lys Ala Glu Ile Gln Lys Val Leu Lys Glu Lys Asp Gln
    1025                1030                1035

Leu Thr Thr Asp Leu Asn Ser Met Glu Lys Ser Phe Ser Asp Leu
    1040                1045                1050

Phe Lys Arg Phe Glu Lys Gln Lys Glu Val Ile Glu Gly Tyr Arg
    1055                1060                1065

Lys Asn Glu Glu Ser Leu Lys Lys Cys Val Glu Asp Tyr Leu Ala
    1070                1075                1080

Arg Ile Thr Gln Glu Gly Gln Arg Tyr Gln Ala Leu Lys Ala His
    1085                1090                1095

Ala Glu Glu Lys Leu Gln Leu Ala Asn Glu Glu Ile Ala Gln Val
    1100                1105                1110

Arg Ser Lys Ala Gln Ala Glu Ala Leu Ala Leu Gln Ala Ser Leu
    1115                1120                1125

Arg Lys Glu Gln Met Arg Ile Gln Ser Leu Glu Lys Thr Val Glu
    1130                1135                1140

Gln Lys Thr Lys Glu Asn Glu Glu Leu Thr Arg Ile Cys Asp Asp
    1145                1150                1155

Leu Ile  Ser Lys Met Glu Lys  Ile
    1160                1165

<210> 547
<211> 1184
<212> PRT
<213> Artificial Sequence

<220>
<223> Description of Artificial Sequence: Synthetic
      polypeptide

<400> 547
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe

690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Glu Ser Pro Glu Thr Arg
    770                 775                 780

Leu Gly Gln Pro Ala Ala Glu Gln Leu His Ala Gly Pro Ala Thr Glu
785                 790                 795                 800

Glu Pro Gly Pro Cys Leu Ser Gln Gln Leu His Ser Ala Ser Ala Glu
                805                 810                 815

Asp Thr Pro Val Val Gln Leu Ala Ala Glu Thr Pro Thr Ala Glu Ser
            820                 825                 830

Lys Glu Arg Ala Leu Asn Ser Ala Ser Thr Ser Leu Pro Thr Ser Cys
        835                 840                 845

Pro Gly Ser Glu Pro Val Pro Thr His Gln Gln Gly Gln Pro Ala Leu
    850                 855                 860

Glu Leu Lys Glu Glu Ser Phe Arg Asp Pro Ala Glu Val Leu Gly Thr
865                 870                 875                 880

Gly Ala Glu Val Asp Tyr Leu Glu Gln Phe Gly Thr Ser Ser Phe Lys
                885                 890                 895

Glu Ser Ala Leu Arg Lys Gln Ser Leu Tyr Leu Lys Phe Asp Pro Leu
            900                 905                 910

Leu Arg Asp Ser Pro Gly Arg Pro Val Pro Val Ala Thr Glu Thr Ser
        915                 920                 925

Ser Met His Gly Ala Asn Glu Thr Pro Ser Gly Arg Pro Arg Glu Ala
    930                 935                 940

Lys Leu Val Glu Phe Asp Phe Leu Gly Ala Leu Asp Ile Pro Val Pro
945                 950                 955                 960

Gly Pro Pro Pro Gly Val Pro Ala Pro Gly Gly Pro Pro Leu Ser Thr
                965                 970                 975

Gly Pro Ile Val Asp Leu Leu Gln Tyr Ser Gln Lys Asp Leu Asp Ala
            980                 985                 990

Val Val Lys Ala Thr Gln Glu Glu  Asn Arg Glu Leu Arg  Ser Arg Cys
        995                 1000                 1005

Glu Glu  Leu His Gly Lys Asn  Leu Glu Leu Gly Lys  Ile Met Asp
    1010                 1015                 1020

Arg Phe  Glu Glu Val Val Tyr  Gln Ala Met Glu Glu  Val Gln Lys
    1025                 1030                 1035

Gln Lys  Glu Leu Ser Lys Ala  Glu Ile Gln Lys Val  Leu Lys Glu
    1040                 1045                 1050

Lys Asp  Gln Leu Thr Thr Asp  Leu Asn Ser Met Glu  Lys Ser Phe
    1055                 1060                 1065

Ser Asp  Leu Phe Lys Arg Phe  Glu Lys Gln Lys Glu  Val Ile Glu
    1070                 1075                 1080

Gly Tyr  Arg Lys Asn Glu Glu  Ser Leu Lys Lys Cys  Val Glu Asp
    1085                 1090                 1095

Tyr Leu  Ala Arg Ile Thr Gln  Glu Gly Gln Arg Tyr  Gln Ala Leu
    1100                 1105                 1110

Lys Ala  His Ala Glu Glu Lys  Leu Gln Leu Ala Asn  Glu Glu Ile
    1115                 1120                 1125

Ala Gln  Val Arg Ser Lys Ala  Gln Ala Glu Ala Leu  Ala Leu Gln
    1130                 1135                 1140

Ala Ser  Leu Arg Lys Glu Gln  Met Arg Ile Gln Ser  Leu Glu Lys
    1145                 1150                 1155

Thr Val  Glu Gln Lys Thr Lys  Glu Asn Glu Glu Leu  Thr Arg Ile
    1160                 1165                 1170

Cys Asp  Asp Leu Ile Ser Lys  Met Glu Lys Ile
    1175                 1180

<210> 548
<211> 22
<212> DNA
<213> Artificial Sequence

<220>
<223> Description of Artificial Sequence: Synthetic
      primer

<400> 548
cttcggggag cagcgatgcg ac                                                22


<210> 549
<211> 22
<212> DNA
<213> Artificial Sequence

<220>
<223> Description of Artificial Sequence: Synthetic
      primer

<400> 549
ctgtccatcc agaggaggag ta                                                22


<210> 550
<211> 32
<212> DNA
<213> Artificial Sequence

<220>
<223> Description of Artificial Sequence: Synthetic
      oligonucleotide

<400> 550
cgtgacgtcc accgacttta aggagtcggc ct                                     32


<210> 551
<211> 32
<212> DNA
<213> Artificial Sequence

<220>
<223> Description of Artificial Sequence: Synthetic
      oligonucleotide

<400> 551
cgtgacgtcc accgacgtaa aggcgacaca gg                                     32


<210> 552
<211> 32
<212> DNA
<213> Artificial Sequence

<220>
<223> Description of Artificial Sequence: Synthetic
      oligonucleotide

<400> 552

tgcgaagagg ggctcggtgg tgcagttggc ag                                    32

<210> 553
<211> 32
<212> DNA
<213> Artificial Sequence

<220>
<223> Description of Artificial Sequence: Synthetic
      oligonucleotide

<400> 553
gcctcccgcc agcaggagcc cccagcagcc tt                                    32

<210> 554
<211> 32
<212> DNA
<213> Artificial Sequence

<220>
<223> Description of Artificial Sequence: Synthetic
      oligonucleotide

<400> 554
agactcagtg cagatgttcc ctcgccgcac tg                                    32

<210> 555
<211> 32
<212> DNA
<213> Artificial Sequence

<220>
<223> Description of Artificial Sequence: Synthetic
      oligonucleotide

<400> 555
agcctgggca cagagggtgt ccgagagcag cc                                    32

<210> 556
<211> 32
<212> DNA
<213> Artificial Sequence

<220>
<223> Description of Artificial Sequence: Synthetic
      oligonucleotide

<400> 556
gccacccgcc tatgccaggc aattccgcga ag                                    32

<210> 557
<211> 32
<212> DNA
<213> Artificial Sequence

<220>
<223> Description of Artificial Sequence: Synthetic
      oligonucleotide

<400> 557
ctggacctgt cggcgcagga gcaacggcag cc                                32

<210> 558
<211> 32
<212> DNA
<213> Artificial Sequence

<220>
<223> Description of Artificial Sequence: Synthetic
      oligonucleotide

<400> 558
gagtgctggc tctggcccgg gactctccgg cc                                32

<210> 559
<211> 32
<212> DNA
<213> Artificial Sequence

<220>
<223> Description of Artificial Sequence: Synthetic
      oligonucleotide

<400> 559
gaggtggctg tgcgaaggtc gctgagggtc ca                                32

<210> 560
<211> 33
<212> DNA
<213> Artificial Sequence

<220>
<223> Description of Artificial Sequence: Synthetic
      oligonucleotide

<400> 560
ctgccgtccc cggccatccc tcaggacgtc cgc                               33

## Description

This application claims the benefit of and priority to U.S. Provisional Patent Application No. 62/096311, filed on December 23, 2014, the content of which is hereby incorporated by reference in its entirety.

All patents, patent applications and publications cited herein are hereby incorporated by reference in their entirety. The disclosures of these publications in their entireties are hereby incorporated by reference into this application.

This patent disclosure contains material that is subject to copyright protection. The copyright owner has no objection to the facsimile reproduction by anyone of the patent document or the patent disclosure as it appears in the U.S. Patent and Trademark Office patent file or records, but otherwise reserves any and all copyright rights.

### GOVERNMENT SUPPORT

This invention was made with government support under Grant Nos. R01CA101644, R01CA085628, R01CA131126, and R01CA178546 awarded by the National Cancer Institute and R01NS061776 awarded by the National Institute of Neurological Disorders and Stroke. The Government has certain rights in the invention.

### SEQUENCE LISTING

The instant application contains a Sequence Listing which has been submitted electronically in ASCII format on compact disc and on paper as a separate part of the description and is hereby incorporated by reference in its entirety. Said ASCII copy, created on December 22, 2015, is named 19240.984WO2_SL.txt and is 470,083 bytes in size. The sequence listing forms part of the application. Sequences 1-494 are also described in WO2014/018673, the contents of which are hereby incorporated by reference in its entirety.

### BACKGROUND OF THE INVENTION

Glioblastoma multiforme (GBM) is the most common form of brain cancer and among the most incurable and lethal of all human cancers. The current standard of care includes surgery, chemotherapy, and radiation therapy. However, the prognosis of GBM remains uniformly poor. There are few available targeted therapies and none that specifically target GBM.

The target population of GBM patients who may carry FGFR-TACC gene fusions and would benefit from targeted inhibition of FGFR kinase activity is estimated to correspond to 6,000 patients per year worldwide.

### SUMMARY OF THE INVENTION

The invention is based, at least in part, on the discovery of a highly expressed class of gene fusions in GBM, which join the tyrosine kinase domain of FGFR genes to the TACC domain of TACC1 or TACC3. The invention is based, at least in part, on the finding that FGFR-TACC fusions identify a subset of GBM patients who will benefit from targeted inhibition of the tyrosine kinase activity of FGFR. Identification of fusions of FGFR and TACC genes in glioblastoma patients and other subjects afflicted with a gene-fusion associated cancer (such as an epithelial cancer) are useful therapeutic targets.

The invention is also based, at least in part, on the discovery of gene fusions joining the tyrosine kinase domain of FGFR genes to the TACC domain of TACC1 or TACC3 in grade II and III glioma. The invention is based, at least in part, on the finding that FGFR-TACC fusions identify a subset of grade II and III glioma patients who will benefit from targeted inhibition of the tyrosine kinase activity of FGFR. Identification of fusions of FGFR and TACC genes in glioma patients are useful therapeutic targets.

An aspect of the invention provides for a purified fusion protein comprising a tyrosine kinase domain of an FGFR protein fused to a polypeptide that constitutively activates the tyrosine kinase domain of the FGFR protein. In one embodiment, the FGFR protein is FGFR1, FGFR2, FGFR3, or FGR4. In another embodiment, the purified fusion protein is essentially free of other human proteins.

An aspect of the invention provides for a purified fusion protein comprising a transforming acidic coiled-coil (TACC) domain fused to a polypeptide with a tyrosine kinase domain, wherein the TACC domain constitutively activates the tyrosine kinase domain. In one embodiment, the TACC protein is TACC1, TACC2, or TACC3. In another embodiment, the purified fusion protein is essentially free of other human proteins.

An aspect of the invention provides for a purified fusion protein comprising the tyrosine kinase domain of an FGFR protein fused 5' to the TACC domain of a transforming acidic coiled-coil-containing (TACC) protein. In one embodiment, the FGFR protein is FGFR1, FGFR2, FGFR3, or FGR4. In another embodiment, the TACC protein is TACC1, TACC2, or TACC3. In another embodiment, the purified fusion protein is essentially free of other human proteins.

An aspect of the invention provides for a purified fusion protein encoded by an FGFR1-TACC1 nucleic acid, wherein FGFR1-TACC1 comprises a combination of exons 1-17 of FGFR1 located on human chromosome 8p11 spliced 5' to a combination of exons 7-13 of TACC1 located on human chromosome 8p11, wherein a genomic breakpoint occurs in any one of exons 1-17 of FGFR1 and any one of exons 7-13 of TACC1. In another embodiment, the purified fusion protein is essentially free of other human proteins.

An aspect of the invention provides for a purified fusion protein encoded by an FGFR2-TACC2 nucleic acid, wherein FGFR2-TACC2 comprises a combination of any exons 1-18 of FGFR2 located on human chromosome 10q26 spliced 5' to a combination of any exons 1-23 of TACC2 located on human chromosome 10q26. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of exons 1-16 of FGFR3 and any one of exons 8-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-18 of FGFR3 located on human chromosome 4p16 spliced 5' to a combination of exons 4-16 of TACC3 located on human chromosome 4p16, wherein a genomic breakpoint occurs in any one of exons 1-18 of FGFR3 and any one of exons 4-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of introns 1-16 of FGFR3 and any one of exons 8-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-18 of FGFR3 located on human chromosome 4p16 spliced 5' to a combination of exons 4-16 of TACC3 located on human chromosome 4p16, wherein a genomic breakpoint occurs in any one of introns 1-18 of FGFR3 and any one of exons 4-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of exons 1-16 of FGFR3 and any one of introns 7-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-18 of FGFR3 located on human chromosome 4p16 spliced 5' to a combination of exons 4-16 of TACC3 located on human chromosome 4p16, wherein a genomic breakpoint occurs in any one of exons 1-18 of FGFR3 and any one of introns 3-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of introns 1-16 of FGFR3 and any one of introns 7-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-18 of FGFR3 located on human chromosome 4p16 spliced 5' to a combination of exons 4-16 of TACC3 located on human chromosome 4p16, wherein a genomic breakpoint occurs in any one of introns 1-18 of FGFR3 and any one of introns 3-16 of TACC3. In another embodiment, the purified fusion protein is essentially free of other human proteins.

An aspect of the invention provides for a synthetic nucleic acid encoding the fusion proteins described above.

An aspect of the invention provides for a purified FGFR3-TACC3 fusion protein comprising SEQ ID NO: 79, 158, 159, 160, 161, 539, 540, 541, 542, 543, 544, 545, 546, 547. In another embodiment, the purified fusion protein is essentially free of other human proteins.

An aspect of the invention provides for a purified FGFR3-TACC3 fusion protein having a genomic breakpoint comprising at least 3 consecutive amino acids from amino acids 730-758 of SEQ ID NO: 90 and comprising at least 3 consecutive amino acids from amino acids 549-838 of SEQ ID NO: 92. In another embodiment, the purified fusion protein is essentially free of other human proteins.

An aspect of the invention provides for a purified FGFR3-TACC3 fusion protein having a genomic breakpoint comprising at least 3 consecutive amino acids from amino acids 730-781 of SEQ ID NO: 90 and comprising at least 3 consecutive amino acids from amino acids 432-838 of SEQ ID NO: 92. In another embodiment, the purified fusion protein is essentially free of other human proteins.

An aspect of the invention provides for a purified FGFR3-TACC3 fusion protein having a genomic breakpoint comprising SEQ ID NO: 78. In another embodiment, the purified fusion protein is essentially free of other human proteins.

An aspect of the invention provides for a purified FGFR3-TACC3 fusion protein having a genomic breakpoint comprising any one of SEQ ID NOS: 85, 86, 87, 89, 516, or 518. In another embodiment, the purified fusion protein is essentially free of other human proteins.

An aspect of the invention provides for a purified FGFR1-TACC1 fusion protein comprising SEQ ID NO: 150. In another embodiment, the purified fusion protein is essentially free of other human proteins.

An aspect of the invention provides for a purified FGFR1-TACC1 fusion protein having a genomic breakpoint comprising at least 3 consecutive amino acids from amino acids 746-762 of SEQ ID NO: 146 and comprising at least 3 consecutive amino acids from amino acids 572-590 of SEQ ID NO: 148. In another embodiment, the purified fusion protein is essentially free of other human proteins.

An aspect of the invention provides for a purified FGFR1-TACC1 fusion protein having a genomic breakpoint comprising at least 3 consecutive amino acids from amino acids 746-762 of SEQ ID NO: 146 and comprising at least 3 consecutive amino acids from amino acids 571-590 of SEQ ID NO: 148. In another embodiment, the purified fusion protein is essentially free of other human proteins.

An aspect of the invention provides for a purified FGFR1-TACC1 fusion protein having a genomic breakpoint comprising SEQ ID NO: 88. In another embodiment, the purified fusion protein is essentially free of other human proteins.

An aspect of the invention provides for a purified DNA encoding an FGFR3-TACC3 fusion protein comprising SEQ ID NO: 94, 530, 531, 532, 533, 534, 535, 536, 537, or 538. In another embodiment, the purified fusion protein is essentially free of other human proteins. An aspect of the invention provides for a purified cDNA encoding an FGFR3-TACC3 fusion protein comprising SEQ ID NO: 94, 530, 531, 532, 533, 534, 535, 536, 537, or 538.

An aspect of the invention provides for a synthetic nucleic acid encoding an FGFR3-TACC3 fusion protein having a genomic breakpoint comprising at least 9 consecutive in-frame nucleotides from nucleotides 2443-2530 of SEQ ID NO: 91 and comprising at least 9 consecutive in-frame nucleotides from nucleotides 1800-2847 of SEQ ID NO: 93.

An aspect of the invention provides for a synthetic nucleic acid encoding an FGFR3-TACC3 fusion protein having a genomic breakpoint comprising any one of SEQ ID NOS: 1-77, or 519-527.

An aspect of the invention provides for a synthetic nucleic acid encoding an FGFR1-TACC1 fusion protein comprising SEQ ID NO: 151.

An aspect of the invention provides for a synthetic nucleic acid encoding an FGFR1-TACC1 fusion protein having a genomic breakpoint comprising at least 9 consecutive in-frame nucleotides from nucleotides 3178-3228 of SEQ ID NO: 147 and comprising at least 9 consecutive in-frame nucleotides from nucleotides 2092-2794 of SEQ ID NO: 149.

An aspect of the invention provides for a synthetic nucleic acid encoding an FGFR1-TACC1 fusion protein having a genomic breakpoint comprising SEQ ID NO: 83.

An aspect of the invention provides for an antibody or antigen-binding fragment thereof, that specifically binds to a purified fusion protein comprising a tyrosine kinase domain of an FGFR protein fused to a polypeptide that constitutively activates the tyrosine kinase domain of the FGFR protein. In one embodiment, the FGFR protein is FGFR1, FGFR2, FGFR3, or FGFR4. In another embodiment, the fusion protein is an FGFR-TACC fusion protein. In a further embodiment, the FGFR-TACC fusion protein is FGFR1-TACC1, FGFR2-TACC2, or FGFR3-TACC3. In some embodiments, the FGFR1-TACC1 fusion protein comprises the amino acid sequence of SEQ ID NO: 150. In other embodiments, the FGFR3-TACC3 fusion protein comprises the amino acid sequence of SEQ ID NO: 79, 158, 159, 160, 161, 539, 540, 541, 542, 543, 544, 545, 546, or 547.

An aspect of the invention provides for a composition for decreasing in a subject the expression level or activity of a fusion protein comprising the tyrosine kinase domain of an FGFR protein fused to a polypeptide that constitutively activates the tyrosine kinase domain of the FGFR protein, the composition in an admixture of a pharmaceutically acceptable carrier comprising an inhibitor of the fusion protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the listed small molecules. In other embodiments, the small molecule that specifically binds to a FGFR protein comprises an oral pan-FGFR tyrosine kinase inhibitor. In other embodiments, the small molecule that specifically binds to a FGFR protein comprises JNJ-42756493.

An aspect of the invention provides for a method for decreasing in a subject in need thereof the expression level or activity of a fusion protein comprising the tyrosine kinase domain of an FGFR protein fused to a polypeptide that constitutively activates the tyrosine kinase domain of the FGFR protein. In one embodiment, the method comprises administering to the subject a therapeutic amount of a composition for decreasing the expression level or activity in a subject of a fusion protein comprising the tyrosine kinase domain of an FGFR protein fused to a polypeptide that constitutively activates the tyrosine kinase domain of the FGFR protein. In one embodiment, the method comprises obtaining a sample from the subject to determine the level of expression of an FGFR fusion molecule in the subject. In some embodiments, the sample is incubated with an agent that binds to an FGFR fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, immunostaining, or other antibody detection methods. In a further embodiment, the method comprises determining whether the fusion protein expression level or activity is decreased compared to fusion protein expression level or activity prior to administration of the composition, thereby decreasing the expression level or activity of the fusion protein. In one embodiment, the fusion protein is an FGFR-TACC fusion protein. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In one embodiment, the composition for decreasing the expression level or activity of a fusion protein comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the small molecules listed. In other embodiments, the small molecule that specifically binds to a FGFR protein comprises an oral pan-FGFR tyrosine kinase inhibitor. In other embodiments, the small molecule that specifically binds to a FGFR protein comprises JNJ-42756493. An aspect of the invention provides for a method for treating a gene-fusion associated cancer in a subject in need thereof, the method comprising administering to the subject an effective amount of a FGFR fusion molecule inhibitor. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In one embodiment, the gene-fusion associated cancer comprises bladder carcinoma, squamous lung carcinoma and head and neck carcinoma. In one embodiment, the gene-fusion associated cancer comprises glioma. In one embodiment, the gene-fusion associated cancer comprises grade II or III glioma. In one embodiment, the gene-fusion associated cancer comprises IDH wild-type grade II or III glioma. In one embodiment, the method comprises obtaining a sample from the subject to determine the level of expression of an FGFR fusion molecule in the subject. In some embodiments the sample from the subject is a tissue sample. In some embodiments, the sample is a paraffin-embedded tissue section. In some embodiments, the tissue sample from the subject is a tumor sample. In some embodiments, the sample is incubated with an agent that binds to an FGFR fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, immunostaining, or other antibody detection methods. In another embodiment, the FGFR fusion protein comprises an FGFR protein fused to a polypeptide that constitutively activates the tyrosine kinase domain of the FGFR protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the small molecules listed. In other embodiments, the small molecule that specifically binds to a FGFR protein comprises an oral pan-FGFR tyrosine kinase inhibitor. In other embodiments, the small molecule that specifically binds to a FGFR protein comprises JNJ-42756493. An aspect of the invention provides for a method of decreasing growth of a solid tumor in a subject in need thereof, the method comprising administering to the subject an effective amount of a FGFR fusion molecule inhibitor, wherein the inhibitor decreases the size of the solid tumor. In one embodiment, the solid tumor comprises glioblastoma multiforme, breast cancer, lung cancer, prostate cancer, or colorectal carcinoma. In one embodiment, the solid tumor comprises bladder carcinoma, squamous lung carcinoma and head and neck carcinoma. In one embodiment, the solid tumor comprises glioma. In one embodiment, the solid tumor comprises grade II or III glioma. In one embodiment, the solid tumor comprises IDH wild-type grade II or III glioma. In one embodiment, the method comprises obtaining a sample from the subject to determine the level of expression of an FGFR fusion molecule in the subject. In some embodiments the sample from the subject is a tissue sample. In some embodiments, the sample is a paraffin-embedded tissue section. In some embodiments, the tissue sample from the subject is a tumor sample. In some embodiments, the sample is incubated with an agent that binds to an FGFR fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, immunostaining, or other antibody detection methods. In another embodiment, the FGFR fusion protein comprises an FGFR protein fused to a polypeptide that constitutively activates the tyrosine kinase domain of the FGFR protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the small molecules listed. In other embodiments, the small molecule that specifically binds to a FGFR protein comprises an oral pan-FGFR tyrosine kinase inhibitor. In other embodiments, the small molecule that specifically binds to a FGFR protein comprises JNJ-42756493.

An aspect of the invention provides for a diagnostic kit for determining whether a sample from a subject exhibits a presence of a FGFR fusion, the kit comprising at least one oligonucleotide that specifically hybridizes to a FGFR fusion, or a portion thereof. In one embodiment, the oligonucleotides comprise a set of nucleic acid primers or *in situ* hybridization probes. In another embodiment, the oligonucleotide comprises SEQ ID NO: 162, 163, 164, 165, 166, 167, 168, 169, 495, 496, 497, 498, 499, 500, 501, 502, 503, 504, 505, 506, 507, 508, 509, 510, 511, 512, 513, 514 or a combination of the listed oligonucleotides. In one embodiment, the primers prime a polymerase reaction only when a FGFR fusion is present. In another embodiment, the determining comprises gene sequencing, selective hybridization, selective amplification, gene expression analysis, or a combination thereof. In a further embodiment, the FGFR-fusion is an FGFR-TACC fusion. In some embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a diagnostic kit for determining whether a sample from a subject exhibits a presence of a FGFR fusion protein, the kit comprising an antibody that specifically binds to a FGFR fusion protein comprising SEQ ID NO: 79, 85, 86, 87, 88, 89, 150, 158, 159, 160, 161, 516, 518, 539, 540, 541, 542, 543, 544, 545, 546, or 547 wherein the antibody will recognize the protein only when a FGFR fusion protein is present. In one embodiment, the antibody directed to and FGFR fusion comprising SEQ ID NO: 79, 85, 86, 87, 88, 89, 150, 158, 159, 160, 161, 516, 518, 539, 540, 541, 542, 543, 544, 545, 546, or 547. In a further embodiment, the FGFR-fusion is an FGFR-TACC fusion. In some embodiments, the FGFR is FGFR1, FGFR2, FGFR3, or FGFR4. In other embodiments, the FGFR-TACC fusion is FGFR1-TACC1, FGFR2-TACC2, or FGFR3-TACC3. In some embodiments the sample from the subject is a tissue sample. In some embodiments, the sample is a paraffin-embedded tissue section. In some embodiments, the tissue sample from the subject is a tumor sample.

An aspect of the invention provides for a method for detecting the presence of a FGFR fusion in a human subject. In one embodiment, the method comprises obtaining a biological sample from the human subject. In some embodiments the sample from the subject is a tissue sample. In some embodiments, the sample is a paraffin-embedded tissue section. In some embodiments, the tissue sample from the subject is a tumor sample. In some embodiments, the sample is incubated with an agent that binds to an FGFR fusion molecule, such as an antibody. In another embodiment, the detection or determination comprises protein expression analysis, for example by western blot analysis, ELISA, immunostaining or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. In other embodiments, the FGFR fusion molecule inhibitor comprises an oral pan-FGFR tyrosine kinase inhibitor. In other embodiments, the FGFR fusion molecule inhibitor comprises JNJ-42756493. In another embodiment, the method comprises detecting whether or not there is a FGFR fusion present in the subject. In one embodiment, the detecting comprises measuring FGFR fusion protein levels by ELISA using an antibody directed to SEQ ID NO: 79, 85, 86, 87, 88, 89, 150, 158, 159, 160, 161, 516, 518, 539, 540, 541, 542, 543, 544, 545, 546, or 547; western blot using an antibody directed to SEQ ID NO: 79, 85, 86, 87, 88, 89, 150, 158, 159, 160, 161, 516, 518, 539, 540, 541, 542, 543, 544, 545, 546, or 547; immunostaining using an antibody directed to SEQ ID NO: 79, 85, 86, 87, 88, 89, 150, 158, 159, 160, 161, 516, 518, 539, 540, 541, 542, 543, 544, 545, 546, or 547; mass spectroscopy, isoelectric focusing, or a combination of the listed methods. In some embodiments, the FGFR-fusion is an FGFR-TACC fusion. In other embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a method for detecting the presence of a FGFR fusion in a human subject. In one embodiment, the method comprises obtaining a biological sample from a human subject. In some embodiments, the sample is incubated with an agent that binds to an FGFR fusion molecule, such as a probe, a nucleic acid primer, and the like. In other embodiments, the detection or determination comprises nucleic acid sequencing, selective hybridization, selective amplification, gene expression analysis, or a combination thereof. In some embodiments, the method further comprises assessing whether to administer a FGFR fusion molecule inhibitor based on the expression pattern of the subject. In further embodiments, the method comprises administering a FGFR fusion molecule inhibitor to the subject. In another embodiment, the method comprises detecting whether or not there is a nucleic acid sequence encoding a FGFR fusion protein in the subject. In one embodiment, the nucleic acid sequence comprises any one of SEQ ID NOS: 1-77, 80-84, 95-145, 515, 517, 519-527, or 530-538. In another embodiment, the detecting comprises using hybridization, amplification, or sequencing techniques to detect a FGFR fusion. In a further embodiment, the amplification uses primers comprising SEQ ID NO: 162, 163, 164,165, 166, 167, 168, 169, 495, 496, 497, 498, 499, 500, 501, 502, 503, 504, 505, 506, 507, 508, 509, 510, 511, 512, 513, or 514. In some embodiments, the FGFR-fusion is an FGFR-TACC fusion. In other embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a method of initiating oncogenic transformation *in vitro.* The method comprises (a) transducing cells cultured *in vitro* with FGFR-TACC fusion DNA; and (b) determining whether the cells acquire the ability to grow in anchorage-independent conditions, form multi-layered foci, or a combination thereof.

An aspect of the invention provides for a method of initiating oncogenic transformation *in vivo.* The method comprises (a) transducing cells cultured *in vitro* with FGFR-TACC fusion DNA; (b) injecting a mouse with the transduced cells; and (c) determining whether a tumor grows in the mouse. In one embodiment, the injecting is a subcutaneous or intracranial injection.

An aspect of the invention provides a method of identifying a compound that decreases the oncogenic activity of a FGFR-TACC fusion. The method comprises (a) transducing a cell cultured *in vitro* with FGFR-TACC DNA; (b) contacting a cell with a ligand source for an effective period of time; and (c) determining whether the cells acquire the ability to grow in anchorage-independent conditions, form multi-layered foci, or a combination thereof, compared to cells cultured in the absence of the test compound.

In one embodiment, the method comprises contacting a sample from the subject with an antibody specific for a FGFR fusion molecule, and determining the presence of an immune complex. In another embodiment, the method comprises contacting a sample from the subject with an antibody specific for a FGFR molecule, or a TACC molecule, and determining the presence of an immune complex. In another embodiment, the antibody recognizes the FGFR3 C-terminal region, or the TACC3 N-terminal region, or a combination thereof. In another embodiment, the antibody recognizes the FGFR3 C-terminal region, or the TACC3 N-terminal region, or a combination thereof. In another embodiment, the method comprises contacting a sample from the subject with an antibody specific for a FGFR molecule, or a TACC molecule, or a FGFR fusion molecule, and determining the amount of an immune complex formed compared to the amount of immune complex formed in non-tumor cells or tissue, wherein an increased amount of an immune complex indicates the presence of an FGFR fusion.

In one embodiment, the method comprises contacting a sample from the subject with primers specific for a FGFR fusion molecule, and determining the presence of an PCR product. In another embodiment, the method comprises contacting a sample from the subject with primer specific for a FGFR molecule, or a TACC molecule, and determining the presence of a PCR product. In another embodiment, the primers recognizes the nucleic acids encoding a FGFR3 C-terminal region, or nucleic acids encoding a TACC3 N-terminal region, or a combination thereof. In another embodiment, the method comprises contacting a sample from the subject with primers specific for a FGFR molecule, or a TACC molecule, or a FGFR fusion molecule, and determining the amount of PCR product formed compared to the amount of PCR product formed in non-tumor cells or tissue, wherein an increased amount of PCR product indicates the presence of an FGFR fusion.

An aspect of the invention provides for a purified fusion protein comprising the tyrosine kinase domain of an FGFR protein fused to the TACC domain of a transforming acidic coiled-coil-containing (TACC) protein. In one embodiment, the FGFR protein is FGFR1, FGFR2, FGFR3, or FGFR4. In one embodiment, the TACC protein is TACC1, TACC2, or TACC3. In one embodiment, the fusion protein is FGFR1-TACC1, FGFR2-TACC2, or FGFR3-TACC3. In one embodiment, the fusion protein comprises SEQ ID NO: 79, SEQ ID NO: 158, SEQ ID NO: 159, SEQ ID NO: 160, SEQ ID NO: 161, SEQ ID NO: 539, SEQ ID NO: 540, SEQ ID NO: 541, SEQ ID NO: 542, SEQ ID NO: 543, SEQ ID NO: 545, SEQ ID NO: 546, or SEQ ID NO: 547. In one embodiment, the fusion protein has a breakpoint comprising at least 3 consecutive amino acids from amino acids 730-758 of SEQ ID NO: 90 and comprising at least 3 consecutive amino acids from amino acids 549-838 of SEQ ID NO: 92. In one embodiment, the fusion protein has a breakpoint comprising SEQ ID NO: 78, SEQ ID NO: 85, SEQ ID NO: 86, SEQ ID NO: 87, SEQ ID NO:89, SEQ ID NO: 516, or SEQ ID NO:518. In one embodiment, the fusion protein comprises SEQ ID NO: 150. In one embodiment, the fusion protein has a breakpoint comprising at least 3 consecutive amino acids from amino acids 746-762 of SEQ ID NO: 146 and comprising at least 3 consecutive amino acids from amino acids 572-590 of SEQ ID NO: 148. In one embodiment, the fusion protein has a breakpoint comprising SEQ ID NO: 88.

An aspect of the invention provides for a cDNA encoding a fusion protein comprising the tyrosine kinase domain of FGFR fused to the TACC domain of TACC. In one embodiment the FGFR is FGFR1, FGFR2, FGFR3, or FGFR4. In one embodiment, the TACC is TACC1, TACC2, or TACC3. In one embodiment, the fusion protein is FGFR1-TACC1, FGFR2-TACC2, or FGFR3-TACC3. In one embodiment, the cDNA comprises SEQ ID NO: 94, SEQ ID NO: 530, SEQ ID NO: 531, SEQ ID NO: 532, SEQ ID NO: 533, SEQ ID NO: 534, SEQ ID NO: 535, SEQ ID NO: 536, SEQ ID NO: 537 or SEQ ID NO: 538. In one embodiment, the cDNA has a breakpoint comprising at least 9 consecutive in-frame nucleotides from nucleotides 2443-2530 of SEQ ID NO: 91 and comprising at least 9 consecutive in-frame nucleotides from nucleotides 1800-2847 of SEQ ID NO: 93. In one embodiment, the cDNA has a breakpoint comprising any one of SEQ ID NOs: 1-77, or SEQ ID NOs: 519-527. In one embodiment, the cDNA comprises SEQ ID NO: 151. In one embodiment, the cDNA has a breakpoint comprising at least 9 consecutive in-frame nucleotides from nucleotides 3178-3228 of SEQ ID NO: 147 and comprising at least 9 consecutive in-frame nucleotides from nucleotides 2092-2794 of SEQ ID NO: 149. In one embodiment, the cDNA has a breakpoint comprising SEQ ID NO: 83. In one embodiment, the cDNA comprises a combination of exons 1-16 of FGFR3 spliced 5' to a combination of exons 8-16 of TACC3, wherein a breakpoint occurs in: a) any one of exons 1-16 of FGFR3 and any one of exons 8-16 of TACC3; b) any one of introns 1-16 of FGFR3 and any one of exons 8-16 of TACC3; c) any one of exons 1-16 of FGFR3 and any one of introns 7-16 of TACC3; or d) any one of introns 1-16 of FGFR3 and any one of introns 7-16 of TACC3. In one embodiment, the cDNA comprises a combination of exons 1-17 of FGFR1 spliced 5' to a combination of exons 7-13 of TACC1, wherein a breakpoint occurs in any one of exons 1-17 of FGFR3 and any one of exons 7-13 of TACC3. In one embodiment, the cDNA comprises a combination of exons 1-18 of FGFR2 spliced 5' to a combination of exons 1-23 of TACC2.

### BRIEF DESCRIPTION OF THE FIGURES

To conform to the requirements for PCT patent applications, many of the figures presented herein are black and white representations of images originally created in color. In the below descriptions and the examples, the colored plots, and images are described in terms of its appearance in black and white. The original color versions can be viewed in Singh et al., Science (2012), 337(6099):1231-5 (including the accompanying Supplementary Information available in the on-line version of the manuscript available on the Science web site) and in Di Stefano et al., Clin. Cancer Res. (2015), 21(14):3307-17 (including the accompanying Supplementary Information available in the on-line version of the manuscript). For the purposes of the PCT, the contents of Singh et al., Science (2012), 337(6099):1231-5 and Di Stefano et al., Clin. Cancer Res. (2015), 21(14):3307-17including the accompanying "Supplementary Information," are herein incorporated by reference.

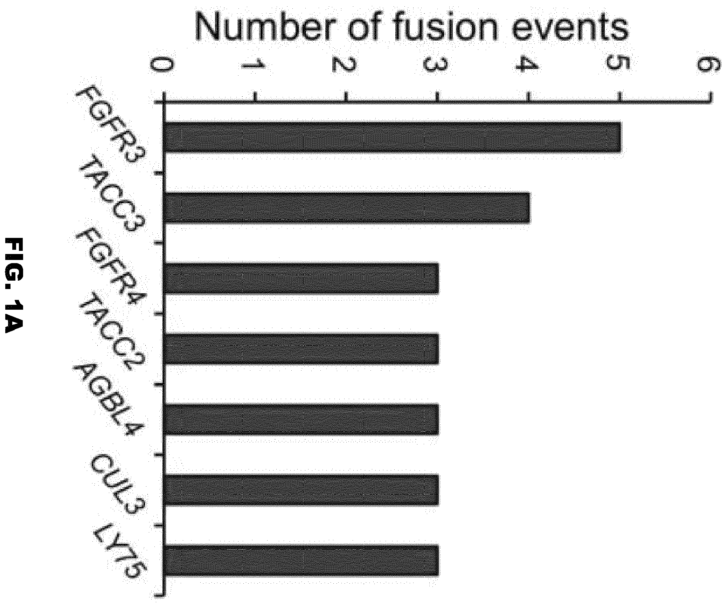

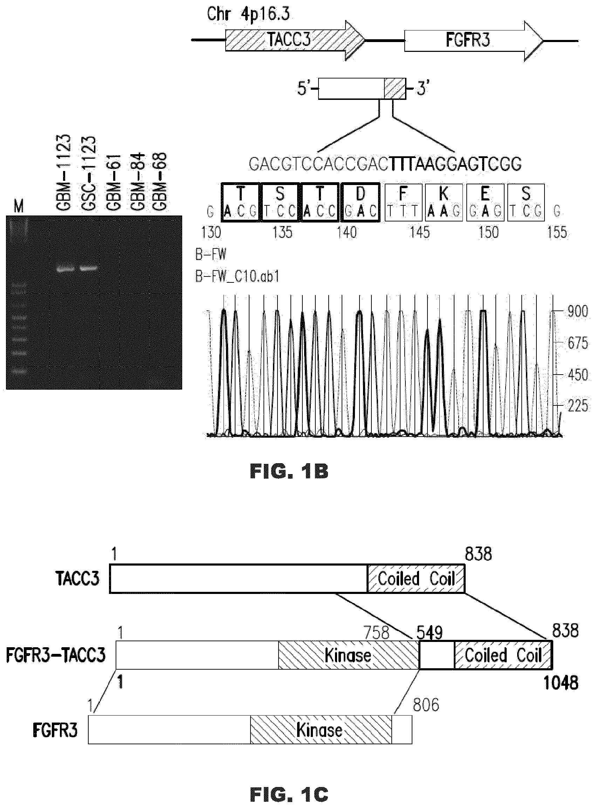

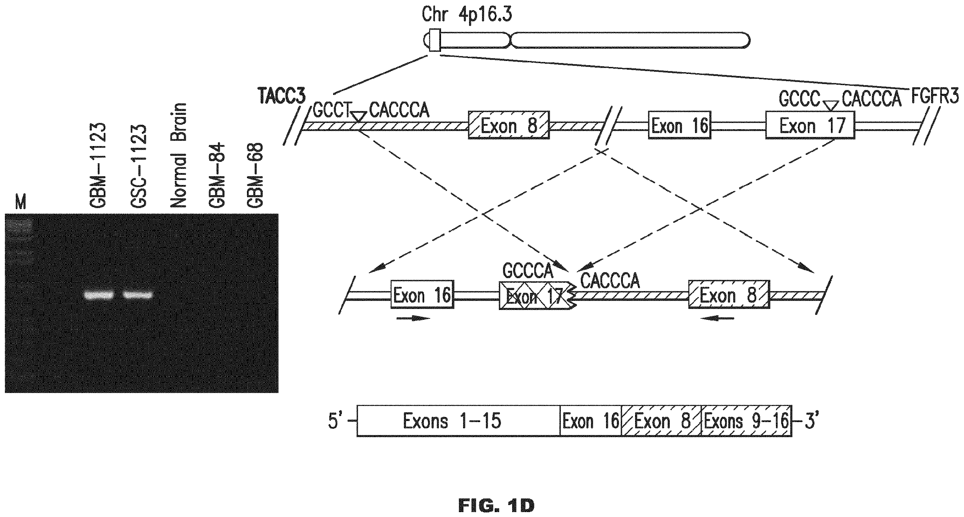

**FIG. 1A** is a graph that shows genes recurrently involved in gene fusions in TCGA. Only genes involved in at least three gene fusions across different samples are displayed. **FIG. 1B** shows an *FGFR3-TACC3* gene fusion identified by whole transcriptome sequencing of GSCs. On the left, *FGFR3-TACC3*-specific PCR from cDNA derived from GSCs and GBM is shown. On the right, Sanger sequencing chromatogram shows the reading frame at the breakpoint (SEQ ID NO: 80) and putative translation of the fusion protein (SEQ ID NO: 85) in the positive samples. **FIG. 1C** shows an *FGFR3-TACC3* gene fusion identified by whole transcriptome sequencing of GSCs. The fusion protein joins the tyrosine kinase domain of FGFR3 to the TACC domain of TACC3. **FIG. 1D** shows an *FGFR3-TACC3* gene fusion identified by whole transcriptome sequencing of GSCs. Genomic fusion of *FGFR3* exon 17 with intron 7 of *TACC3* is shown. In the fused mRNA, exon 16 of *FGFR3* is spliced 5' to exon 8 of *TACC3.* Filled arrows indicate the position of the fusion-genome primers, which generate fusion-specific PCR products in GSC-1123 and GBM-1123.

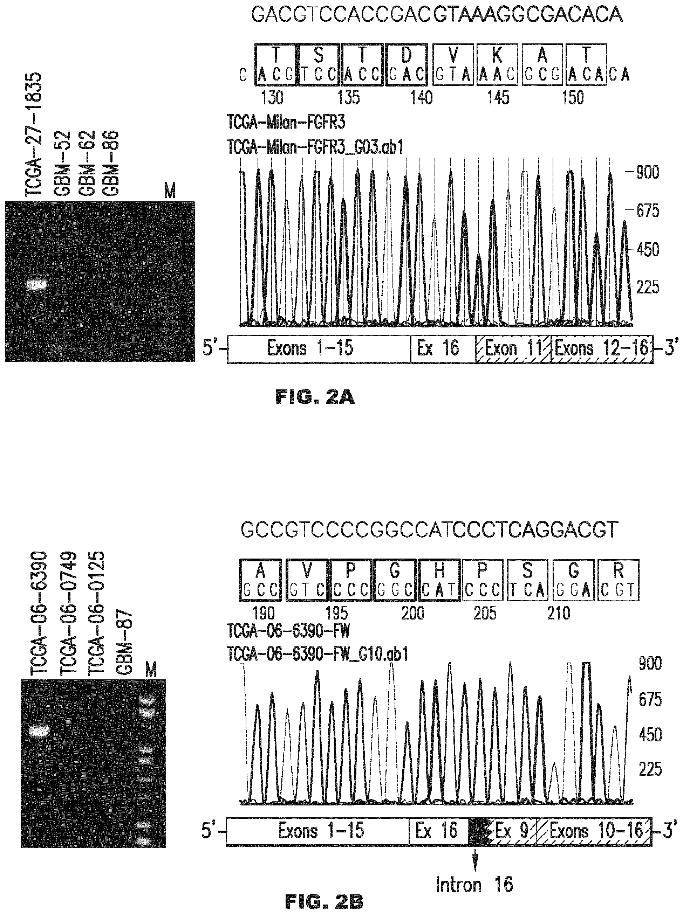

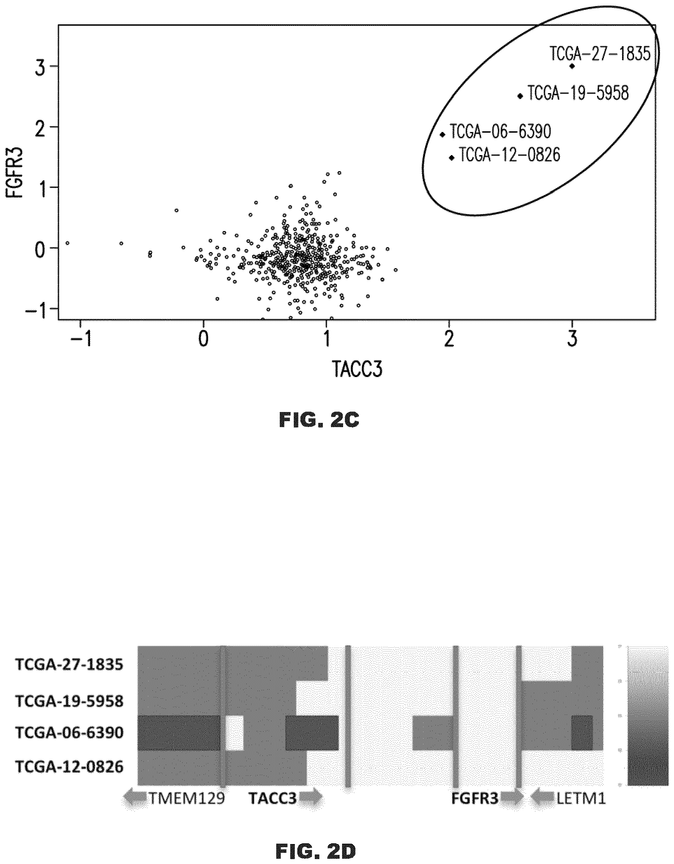

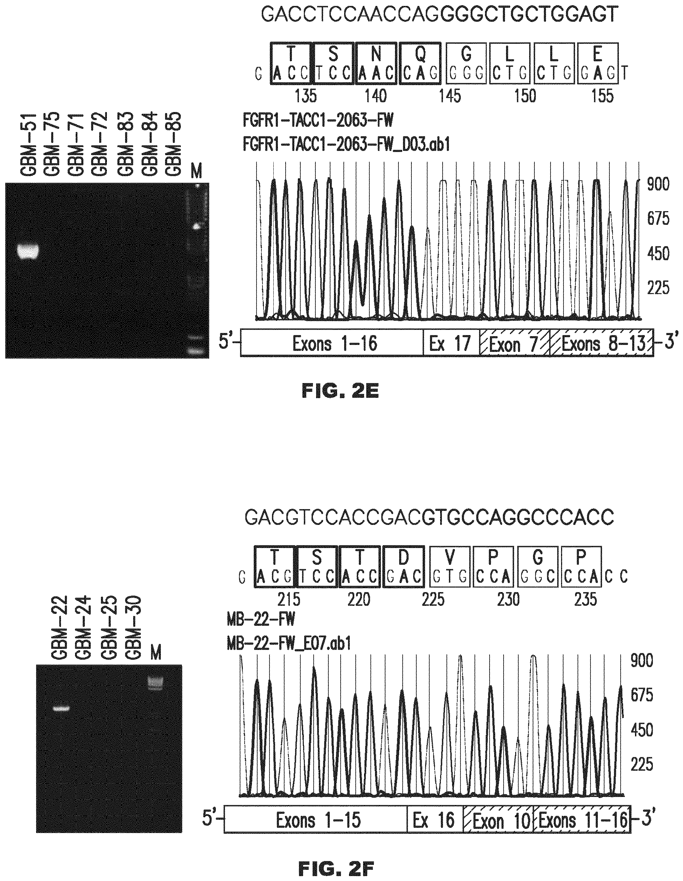

**FIG. 2A** and **FIG. 2B** show recurrent gene fusions between *FGFR* and TACC genes in GBM. On the left, a gel of FGFR-TACC-specific PCR is shown for *FGFR3-TACC3* from a GBM cDNA sample. On the right, Sanger sequencing chromatograms show the reading frame at the breakpoint (SEQ ID NO: 81 (FIG. 2B); SEQ ID NO: 82 (FIG. 2C) and putative translation of the fusion protein (SEQ ID NO: 86 (FIG. 2B); SEQ ID NO: 87 (FIG. 2D) in the positive samples. **FIG. 2C** shows recurrent gene fusions between *FGFR* and *TACC* genes in GBM. Co-outlier expression of *FGFR3* and *TACC3* in four GBM tumors from Atlas-TCGA is shown in the plot. **FIG. 2D** shows recurrent gene fusions between *FGFR* and *TACC* genes in GBM. CNV analysis shows microamplifications of the rearranged portions of the *FGFR3* and *TACC3* genes in the same four Atlas-TCGA GBM samples. **FIG. 2E-F** shows recurrent gene fusions between *FGFR* and *TACC* genes in GBM. On the left, a gel of *FGFR-TACC*-specific PCR is shown for *FGFR1-TACC1* (FIG. 2F) or *FGFR3-TACC3* (FIG. 2G) from a GBM cDNA sample. On the right, Sanger sequencing chromatograms show the reading frame at the breakpoint (SEQ ID NO: 83 (FIG. 2F); SEQ ID NO: 84 (FIG. 2G) and putative translation of the fusion protein (SEQ ID NO: 88 (FIG. 2F); SEQ ID NO: 89 (FIG. 2G) in the positive samples.

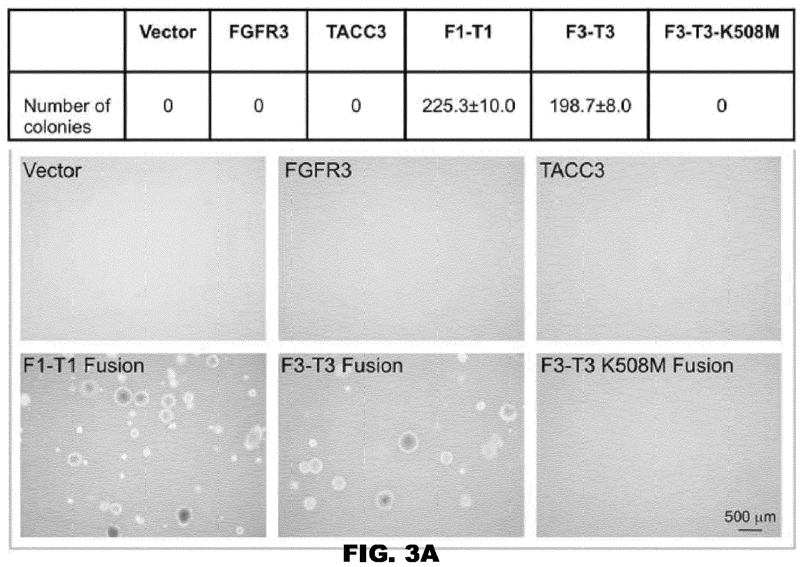

**FIG. 3A** shows transforming activity of FGFR-TACC fusion proteins. FGFR1-TACC1 and FGFR3-TACC3 induce anchorage-independent growth in Rat1A fibroblasts. The number of soft agar colonies was scored from triplicate samples 14 days after plating. Representative microphotographs are shown.

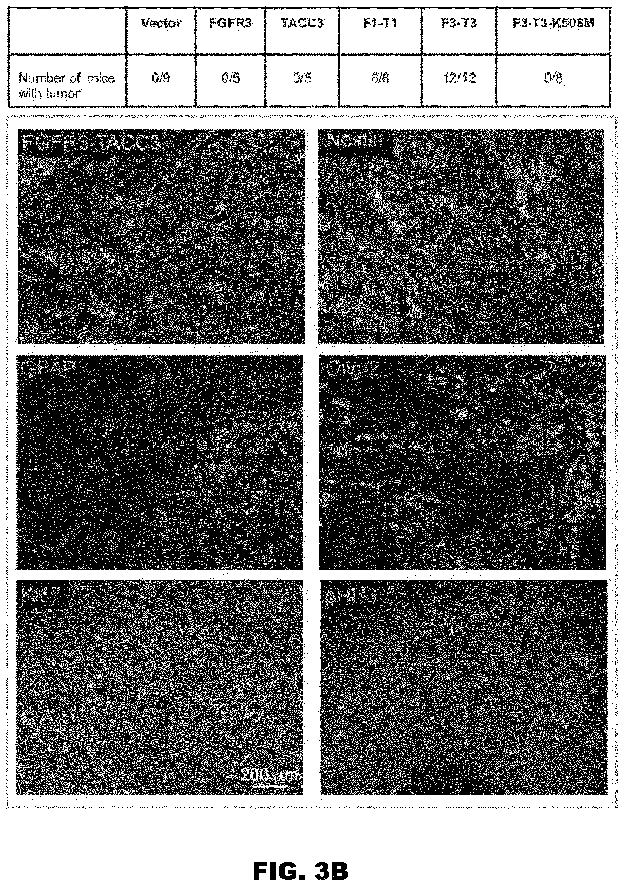

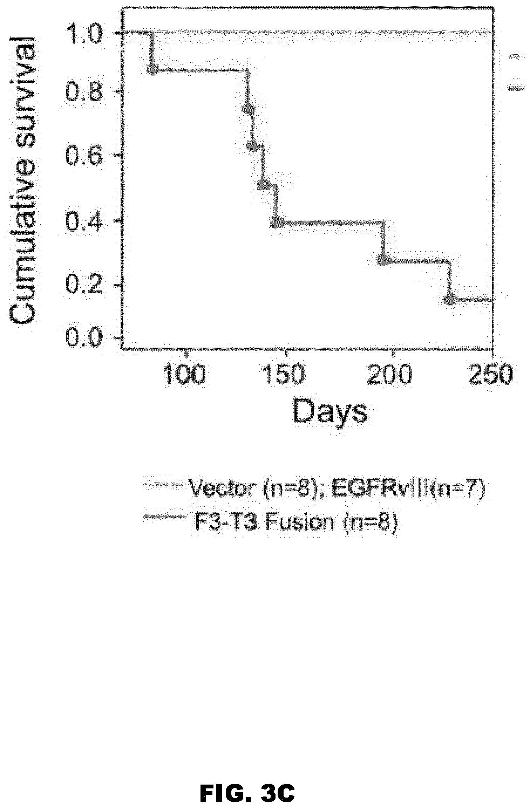

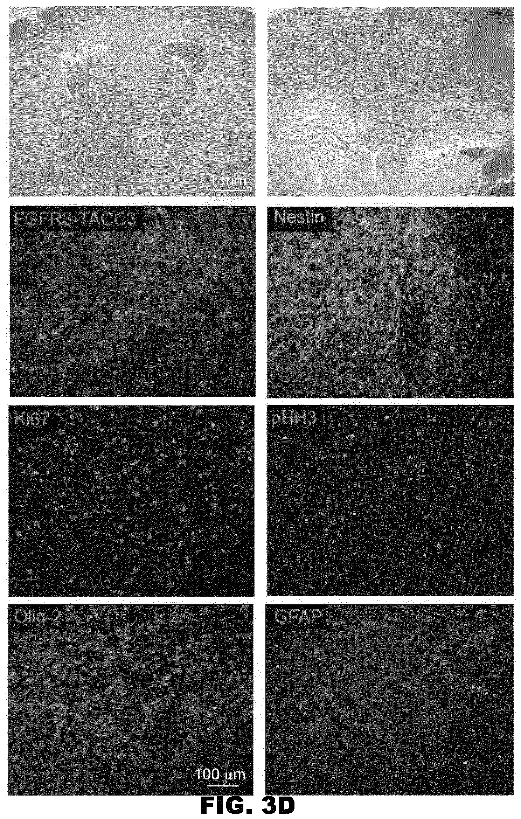

**FIG. 3B** are photomicrographs showing of immunofluoresence staining of tumors from mice injected with *Ink4A;Arf-*/*-* astrocytes expressing FGFR3-TACC3 showing positivity for glioma-specific (Nestin, Oig2 and GFAP) and proliferation markers (Ki67 and pHH3). Sub-cutaneous tumors were generated by *Ink4A;Arf-*/*-* astrocytes expressing FGFR-TACC fusions. **FIG. 3C** shows Kaplan-Meier survival curves of mice injected intracranially with pTomo-shp53 (n = 8) or pTomo-EGFRvIII-shp53 (n = 7) (green line; "light grey" in black and white image) and pTomo-FGFR3-TACC3-shp53 (n = 8, red line; "dark grey" in black and white image). Points on the curves indicate deaths (log-rank test, p = 0.00001, pTomo-shp53 vs. pTomo-FGFR3-TACC3-shp53). **FIG. 3D** shows representative photomicrographs of Hematoxylin and Eosin staining of advanced FGFR3-TACC3-shp53 generated tumors showing histological features of high-grade glioma. Of note is the high degree of infiltration of the normal brain by the tumor cells. Immunofluorescence staining shows that glioma and stem cell markers (Nestin, Olig2 and GFAP), the proliferation markers (Ki67 and pHH3) and the FGFR3-TACC3 protein are widely expressed in the FGFR3-TACC3-shp53 brain tumors. F1-T1: FGFR1-TACC1; F3-T3: FGFR3-TACC3; F3-T3-K508M: FGFR3-TACC3-K508M.

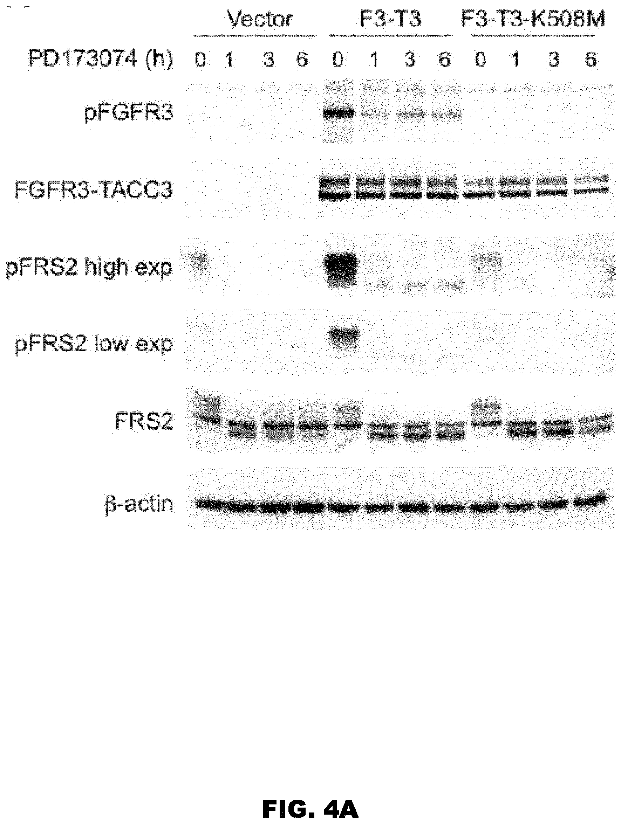

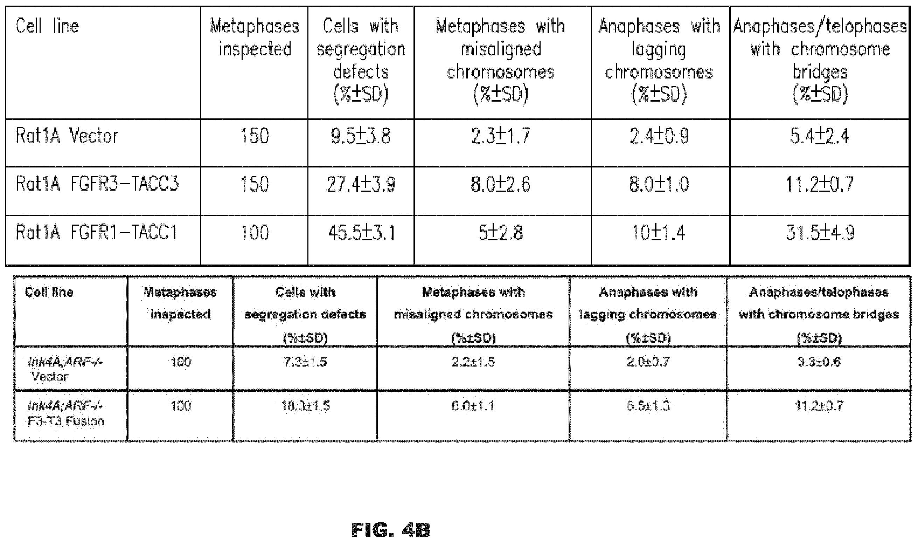

**FIG. 4A** shows that FGFR3-TACC3 localizes to spindle poles, delays mitotic progression and induces chromosome segregation defects and aneuploidy Constitutive auto-phosphorylation of FGFR3-TACC3 fusion. *Ink4A;Arf-*/*-* astrocytes transduced with empty lentivirus or a lentivirus expressing FGFR3-TACC3 or FGFR3-TACC3-K508M were left untreated (0) or treated with 100 nM of the FGFR inhibitor PD173074 for the indicated times. Phospho-proteins and total proteins were analyzed by Western blot using the indicated antibodies. **FIG. 4B** shows quantitative analysis of segregation defects in Rat1A expressing FGFR1-TACC1 and FGFR3-TACC3. F3-T3: FGFR3-TACC3; F3-T3-K508M: FGFR3-TACC3-K508M (top panel). FGFR3-TACC3 protein induces chromosomal mis-segregation, chromatid cohesion defects and defective spindle checkpoint. Quantitative analysis of metaphase spreads for chromosome segregation defects in *Ink4A;ARF-*/*-* astrocytes expressing vector control or FGFR3-TACC3 is shown in the bottom panel.

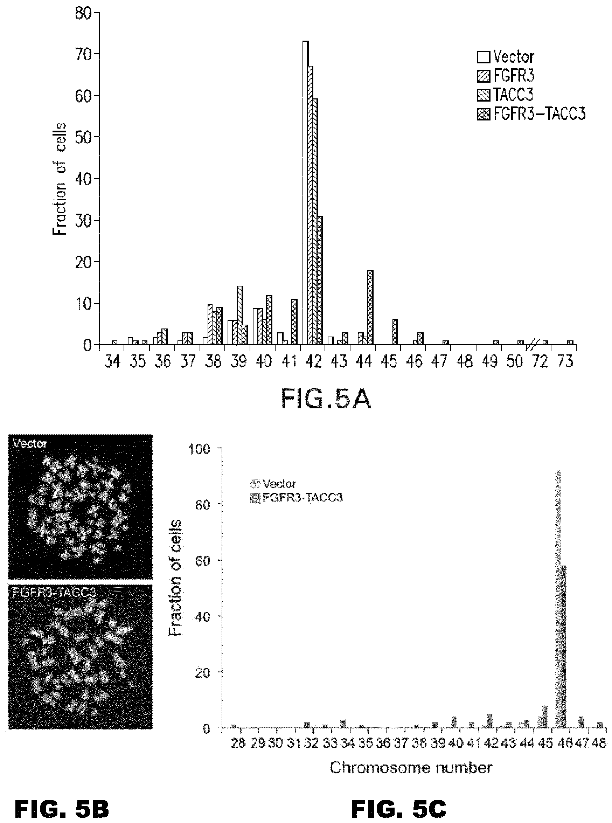

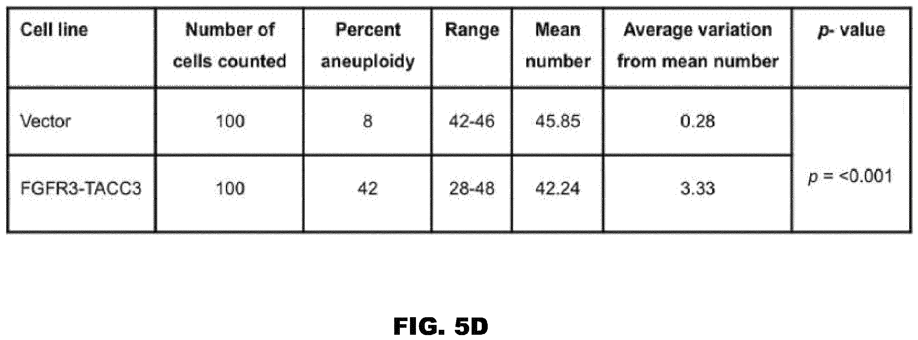

**FIG. 5A** shows karyotype analysis of Rat1A cells transduced with control, FGFR3, TACC3 or FGFR3-TACC3 expressing lentivirus. Distribution of chromosome counts of cells arrested in mitosis and analyzed for karyotypes using DAPI. Chromosomes were counted in 100 metaphase cells for each condition to determine the ploidy and the diversity of chromosome counts within the cell population. FGFR3-TACC3 fusion induces aneuploidy. **FIG. 5B** shows representative karyotypes and **FIG. 5C** shows distribution of chromosome counts of human astrocytes transduced with control or FGFR3-TACC3 expressing lentivirus. Chromosomes were counted in 100 metaphase cells for each condition to determine the ploidy and the diversity of chromosome counts within the cell population. **FIG. 5D** shows quantitative analysis of chromosome number in 100 metaphase cells for each condition to determine the ploidy and the diversity of chromosome counts within the cell population. (n=3 independent experiments).

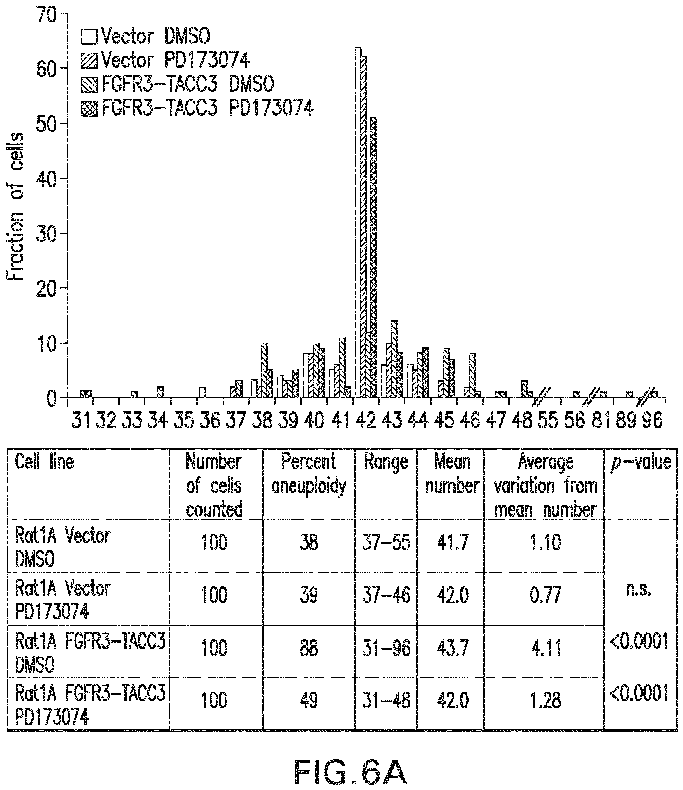

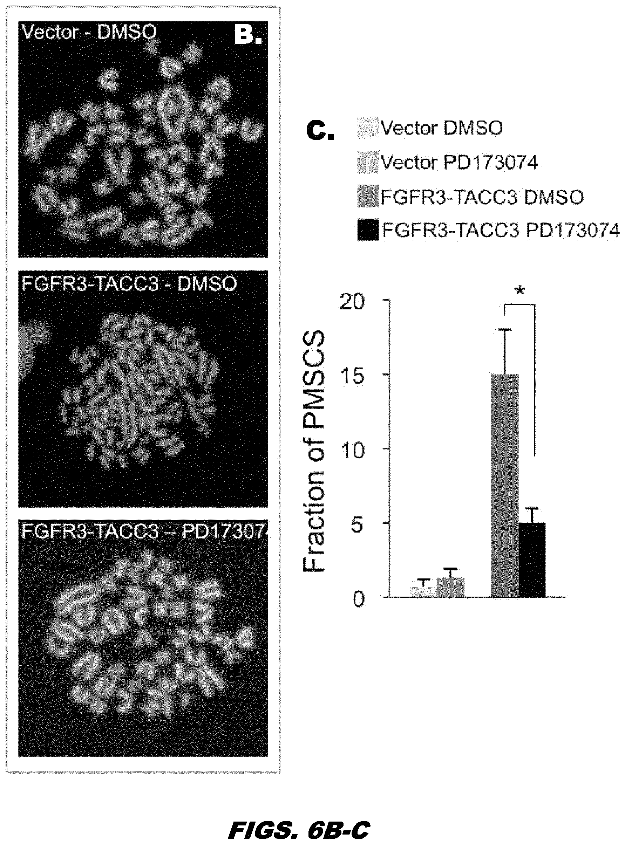

**FIG. 6A** shows inhibition of FGFR-TK activity corrects the aneuploidy initiated by FGFR3-TACC3. The upper panel is a karyotype analysis of Rat1A cells transduced with control or FGFR3-TACC3 lentivirus and treated with vehicle (DMSO) or PD173470 (100 nM) for five days. The lower panel shows the ploidy and the diversity of chromosome counts within the cell population were determined by quantitative analysis of chromosome number in 100 metaphase cells for each condition. **FIG. 6B** shows inhibition of FGFR-TK activity corrects the aneuploidy initiated by FGFR3-TACC3. Correction of premature sister chromatid separation (PMSCS) by PD173470 in cells expressing FGFR3-TACC3. Panels show representative metaphase spreads. DNA was stained by DAPI. **FIG. 6C** shows quantitative analysis of metaphases with loss of sister chromatid cohesion (n=3; p = 0.001, FGFR3-TACC3 treated with DMSO vs. FGFR3-TACC3 treated with PD173470).

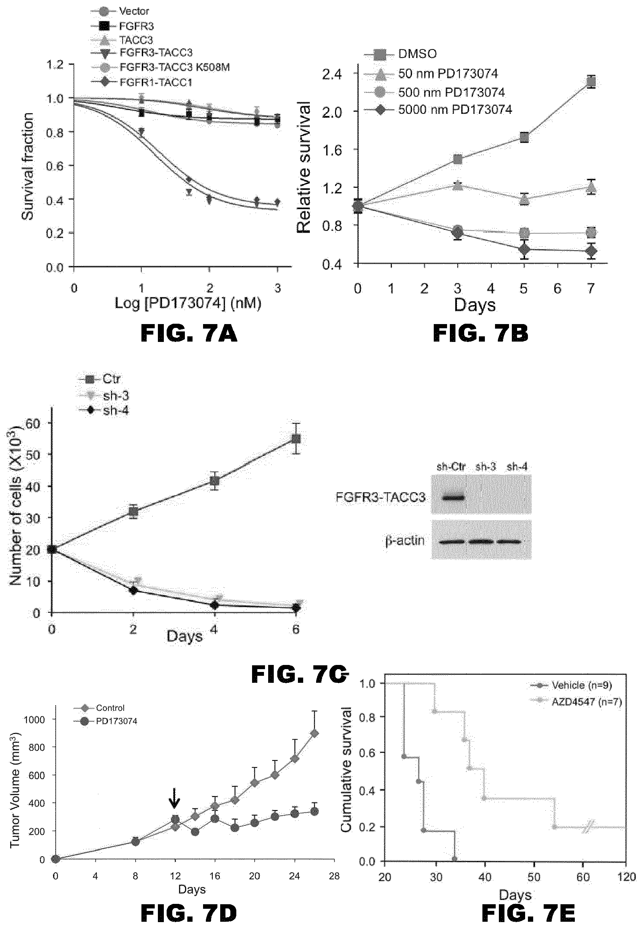

**FIG. 7A** shows inhibition of FGFR-TK activity suppresses tumor growth initiated by FGFR3-TACC3. Growth rate of Rat1A transduced with the indicated lentiviruses and treated for three days with increasing concentrations of PD173074. Cell growth was determined by the MTT assay. Data are presented as the means±standard error (n=4). **FIG. 7B** shows the growth rate of GSC-1123 treated with PD173470 at the indicated concentrations for the indicated times. Cell growth was determined by the MTT assay. Data are presented as the means±standard error (n=4). **FIG. 7C** shows the growth inhibitory effect of silencing FGFR3-TACC3 fusion. At the left, parallel cultures of GSC-1123 cells were transduced in triplicate. Rat1A cells expressing FGFR3-TACC3 fusion were transduced with lentivirus expressing a non-targeting shRNA (Ctr) or shRNA sequences targeting FGFR3 (sh2, sh3, sh4). Five days after infection cells were plated at density of 2X10⁴ cells/well in triplicate and the number of trypan blue excluding cells was scored at the indicated times. Infection with lentivirus expressing sh-3 and sh-4, the most efficient FGFR3 silencing sequences reverted the growth rate of FGFR3-TACC3 expressing cultures to levels comparable to those of Rat1A transduced with empty vector. Values are the means ± standard deviation (n = 3). At th right sided figure, GSC-1123 cells were transduced with lentivirus expressing a non-targeting shRNA (sh-Ctr) or lentivirus expressing sh-3 and sh-4 sequences targeting FGFR3. Western Blot analysis was performed on parallel cultures using the FGFR3 antibody to the detect FGFT3-TACC3 fusion protein. 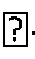 -actin is shown as a control for loading. **FIG. 7D** shows that the FGFR inhibitor PD173074 suppresses tumor growth of glioma sub-cutaneous xenografts generated by *Ink4A;Arf-*/*-* astrocytes expressing FGFR3-TACC3. After tumor establishment (200-300 mm³, arrow) mice were treated with vehicle or PD173074 (50 mg/kg) for 14 days. Values are mean tumor volumes ± standard error (n = 7 mice per group). **FIG. 7E** is a Kaplan-Meier analysis of glioma-bearing mice following orthotopic implantation of *Ink4A;Arf-*/*-* astrocytes transduced with FGFR3-TACC3. After tumor engraftment mice were treated with vehicle (n=9) or AZD4547 (50 mg/kg) (n=7) for 20 days (*p* = 0.001).

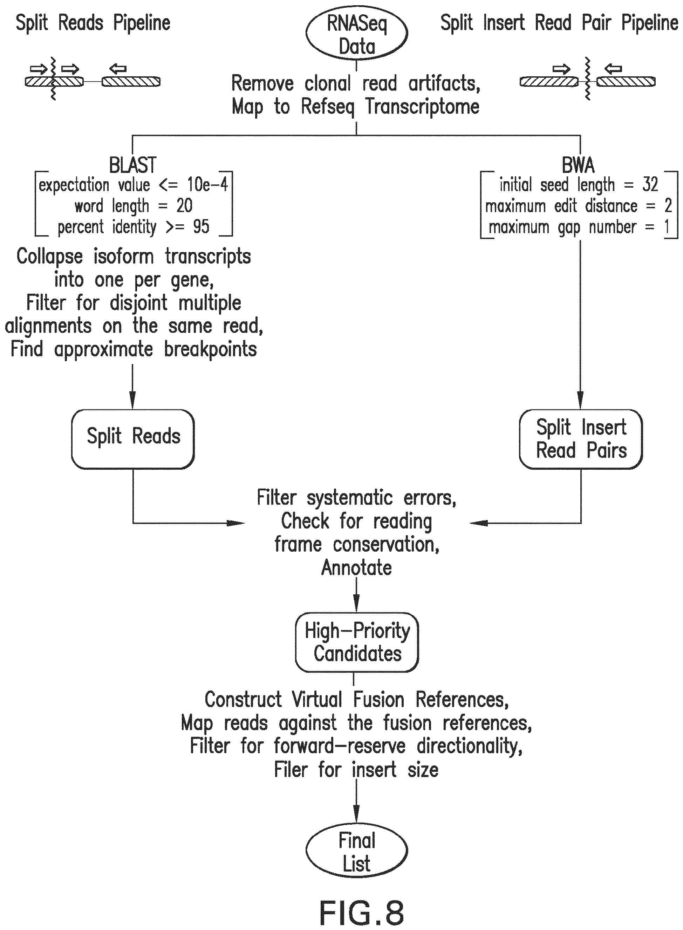

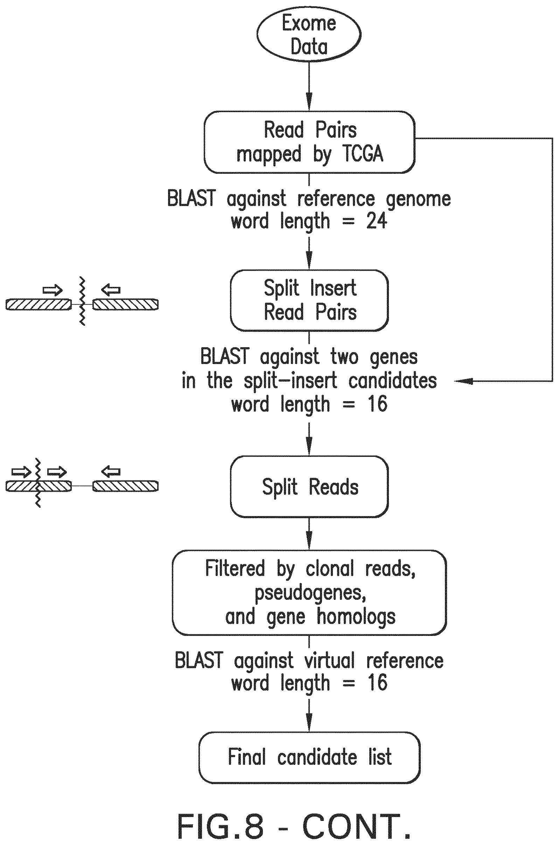

**FIG.** 8 shows a schematic of the TX-Fuse pipeline for the identification of fusion transcripts from RNA-Seq data generated from nine GSC cultures. The continued figure shows a schematic of the Exome-Fuse pipeline for the identification of gene fusion rearrangements from DNA exome sequences of 84 GBM TCGA tumor samples.

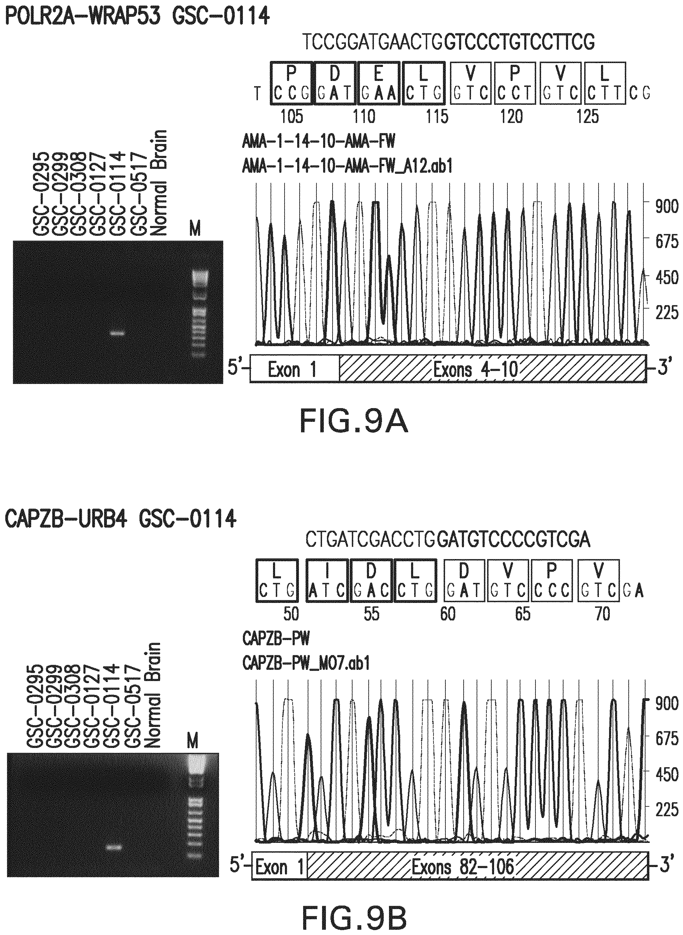

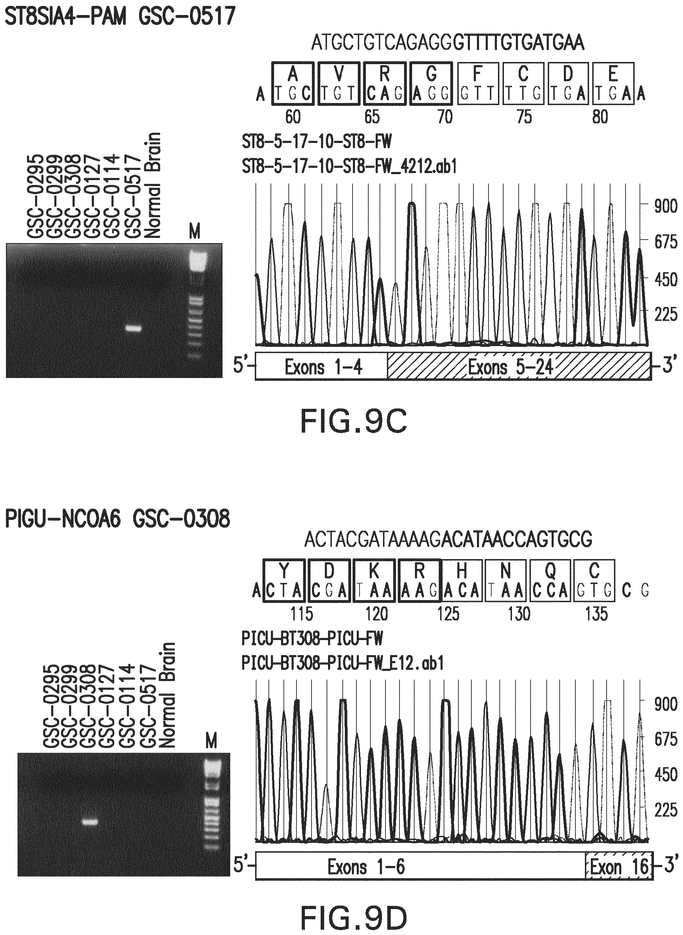

**FIGS. 9A-D** shows the validation of fusion transcripts identified by RNA-seq of nine GSCs. Sanger sequencing chromatograms show the reading frames at the breakpoint and putative translation of the fusion proteins in the positive samples (right side). The left side shows gels of RT-PCR conducted. **(****FIG. 9A**) POLR2A-WRAP53. DNA sequence disclosed as SEQ ID NO: 319 and protein sequence disclosed as SEQ ID NO: 320. (**FIG. 9B**) CAPZB-UBR4. DNA sequence disclosed as SEQ ID NO: 321 and protein sequence disclosed as SEQ ID NO: 322. (**FIG. 9C**) ST8SIA4-PAM. DNA sequence disclosed as SEQ ID NO: 323 and protein sequence disclosed as SEQ ID NO: 324. (**FIG. 9D**) PIGU-NCOA6. DNA sequence disclosed as SEQ ID NO: 325 and protein sequence disclosed as SEQ ID NO: 326. Also (below), 6 split-reads (SEQ ID NOS 485-490, respectively, in order of appearance) are shown aligning on the breakpoint of the IFNAR2-IL10RB fusion (SEQ ID NO: 483). The predicted reading frame at the breakpoint is shown at the top with IFNAR2 sequences in red (left) and IL10RB in blue (right). Protein sequence disclosed as SEQ ID NO: 484.

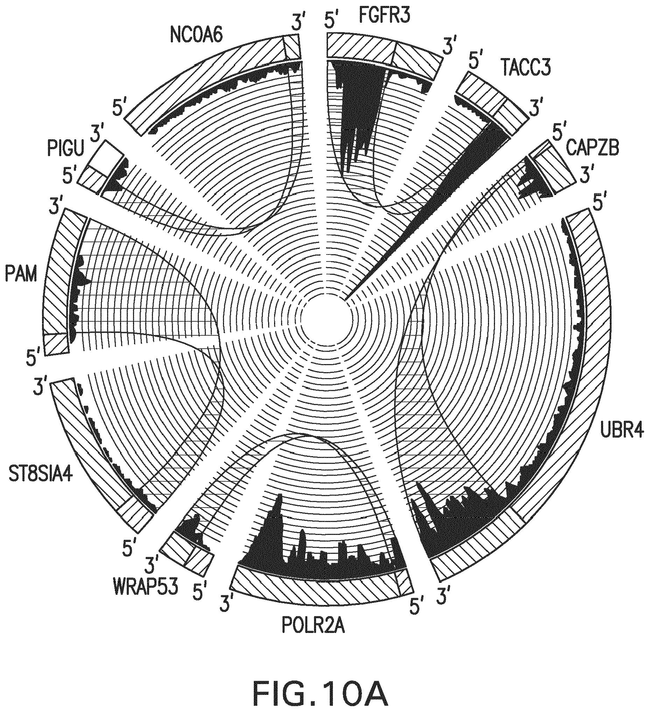

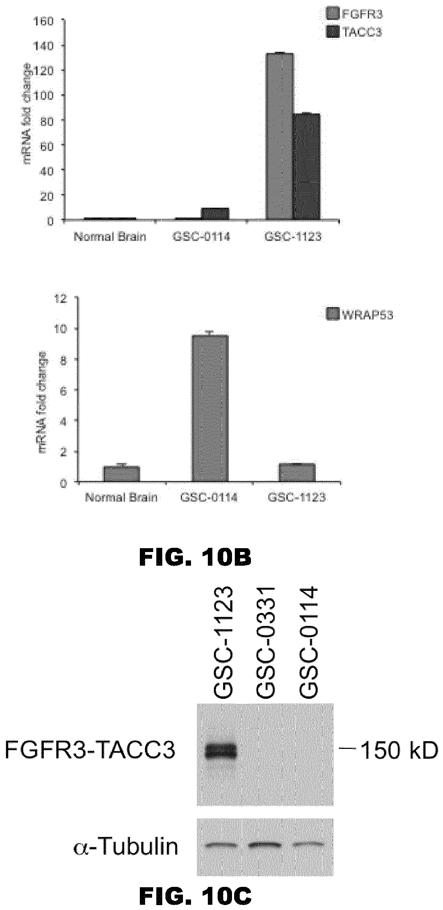

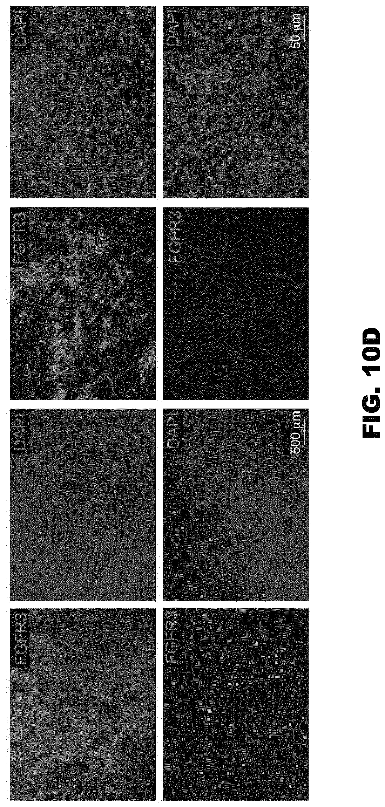

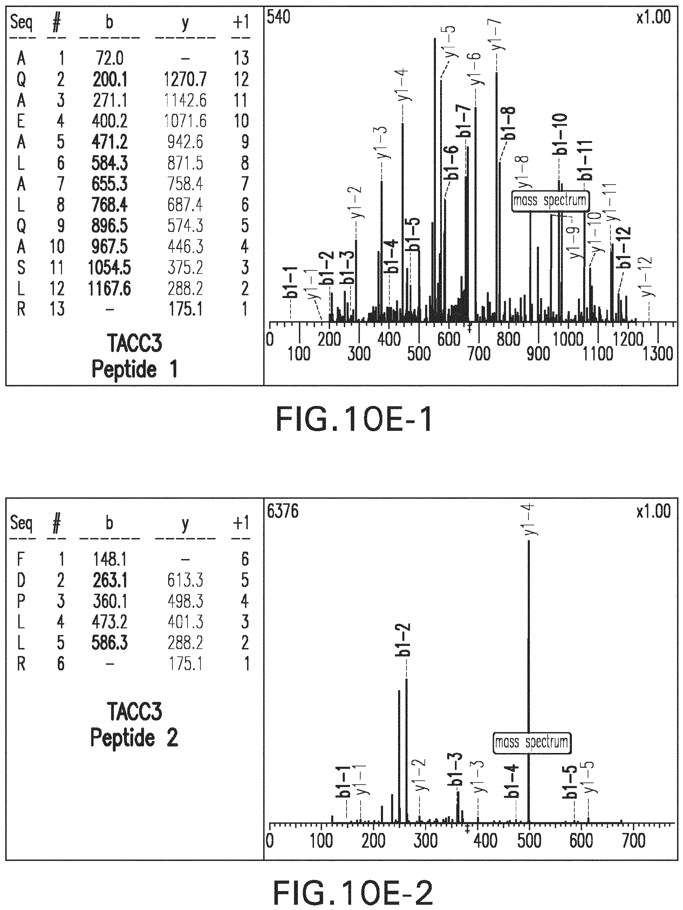

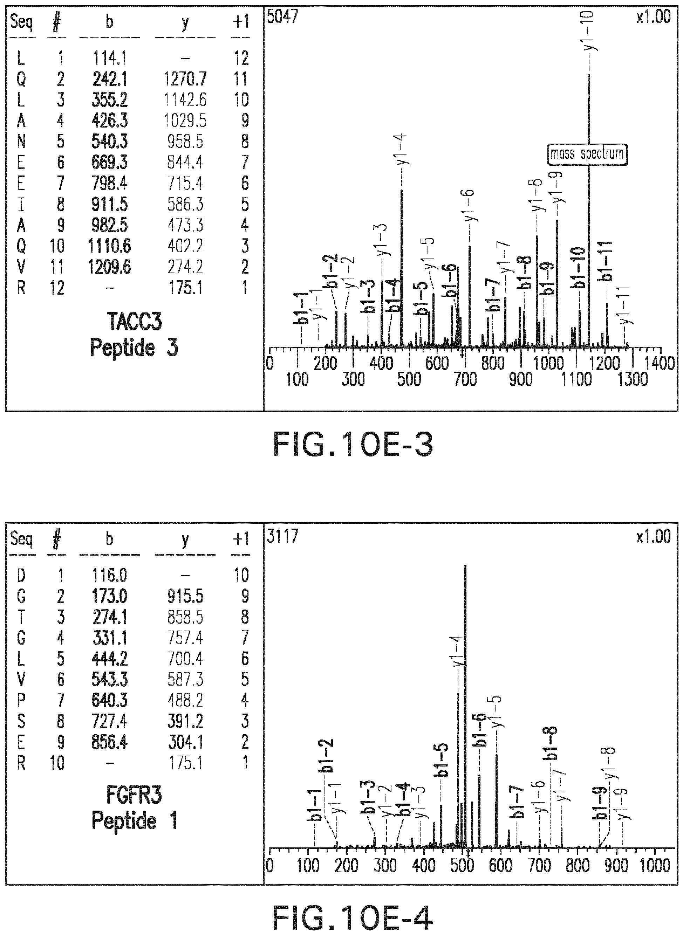

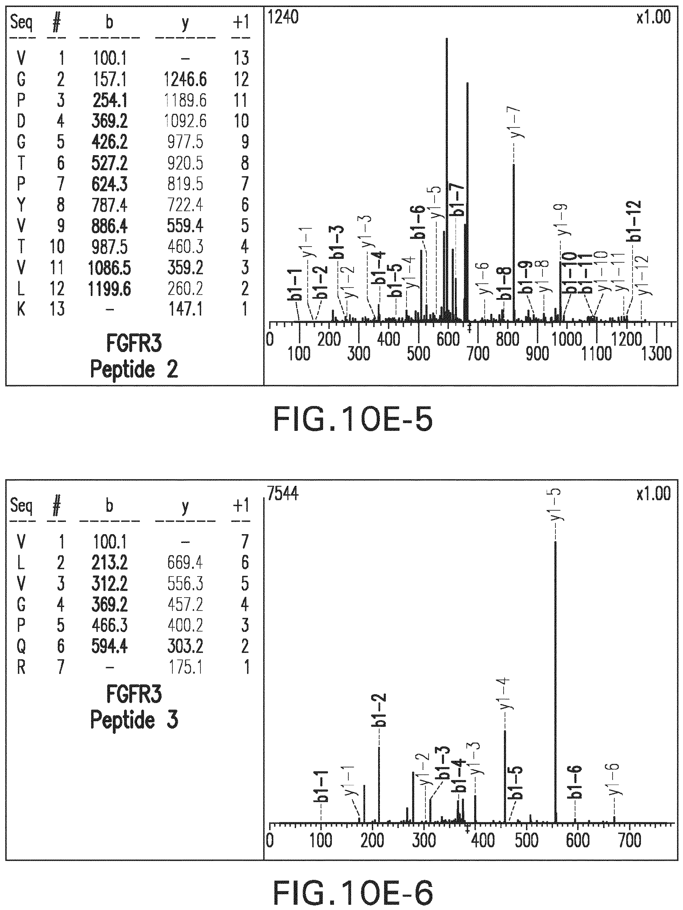

FIG. **10A** shows the analysis and validation of the expression of fused transcripts in GSCs and GBM sample. Expression measured by read depth from RNA-seq data. Light grey arcs indicate predicted components of transcripts fused together. Overall read depth (blue; "grey" in black and white image) and split insert depth (red; "dark grey" in black and white image) are depicted in the graph, with a 50-read increment and a maximum range of 1800 reads. Note the very high level of expression in the regions of the genes implicated in the fusion events, particularly for FGFR3-TACC3. **FIG. 10B** shows the analysis and validation of the expression of fused transcripts in GSCs and GBM sample. Top panel, qRT-PCR showing the very high expression of FGFR3 and TACC3 mRNA sequences included in the FGFR3-TACC3 fusion transcript in GSC-1123. Bottom panel, for comparison the expression of sequences of WRAP53 mRNA included in the POLR2A-WRAP53 fusion in GSC-0114 is also shown. **FIG. 10C** shows the expression of the FGFR3-TACC3 protein in GSC-1123 and GBM-1123. Western blot analysis with a monoclonal antibody, which recognizes the N-terminal region of human FGFR3 shows expression of a ∼150 kD protein in GSC-1123 but not in the GSC cultures GSC-0331 and GSC-0114, which lack the FGFR3-TACC3 rearrangement. **FIG. 10D** shows the analysis and validation of the expression of fused transcripts in GSCs and GBM sample. Immunostaining analysis with the FGFR3 antibody of the tumor GBM-1123 (top panel) and a GBM tumor lacking the FGFR3-TACC3 rearrangement. FGFR3 (red; "light grey" in black and white image), DNA (DAPI, blue; "grey" in black and white image). The pictures were taken at low (left) and high (right) magnification. **FIG. 10E1-E6** shows MS/MS analysis of the ∼150 kD fusion protein immunoprecipitated by the monoclonal anti-FGFR3 antibody from GSC-1123, identifying three unique peptides mapping to the FGFR3 (FGFR3 Peptide 1 (SEQ ID NO: 492), 2 (SEQ ID NO: 493), and 3 (SEQ ID NO: 494)) and three peptides mapping to the C-terminal region of TACC3 (TACC Peptide 1 (SEQ ID NO: 156), 2 (SEQ ID NO: 157), and 3 (SEQ ID NO: 491)).

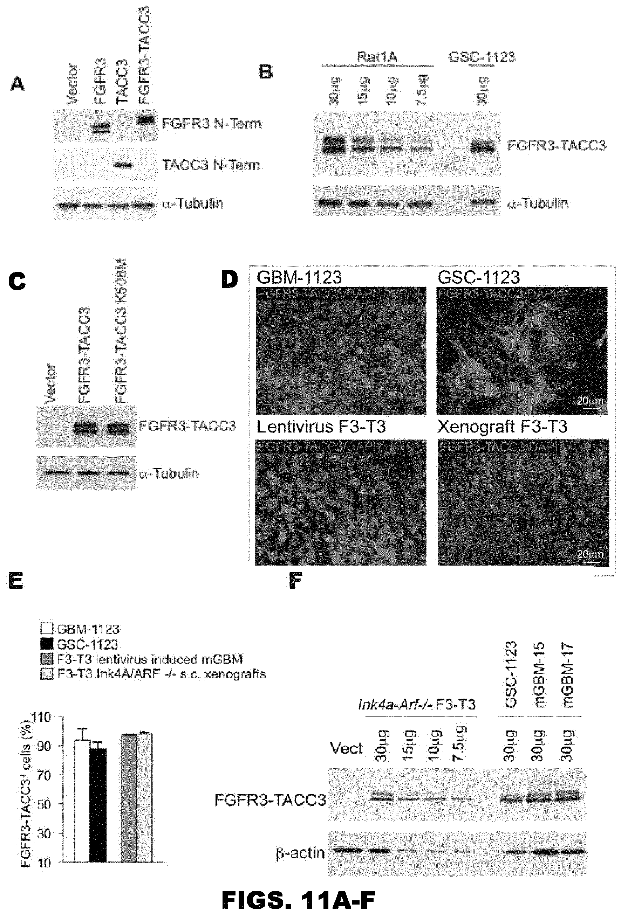

**FIGS. 11A-C** shows Rat1A cells transduced with control lentivirus or lentivurus expressing FGFR3, TACC3, FGFR3-TACC3 (**FIG. 11A**) that were analyzed by Western blot with an antibody recognizing the N-terminus of FGFR3 (included in the FGFR3-TACC3 fusion protein) or the N-terminus of TACC3 (not included in the FGFR3-TACC3 fusion protein). **FIG. 11B** shows quantitative Western blot analysis of endogenous FGFR3-TACC3 in GSC-1123 compared with lentivirally expressed FGFR3-TACC3 in Rat1A. **FIG. 11C** shows Western blot analysis of FGFR3-TACC3 and FGFR3-TACC3-K508M in Rat1A. α-tubulin is shown as a control for loading. **FIGS. 11D-F** shows expression analyses of FGFR3-TACC3 fusion construct (**FIG. 11D**) FGFR3 immunostaining of GBM-1123 (left, upper panel), BTSC1123 (right, upper panel), mouse GBM induced by FGFR3-TACC3 expressing lentivirus (left, lower panel), and sub-cutaneous xenograft of mouse astrocytes transformed by FGFR3-TACC3 fusion (right, lower panel); FGFR3-TACC3, red ("light grey" in black and white image); DNA (DAPI), blue ("grey" in black and white image). **FIG. 11E** shows quantification of FGFR3-TACC3 positive cells in the tumors and cultures of cells shown in **FIG. 11D. FIG. 11F** shows a quantitative Western blot analysis of ectopic FGFR3-TACC3 fusion protein in mouse astrocytes and FGFR3-TACC3 induced mouse GBM (mGBM-15 and mGBM-17) compared with the endogenous expression in GBM1123. β-actin is shown as a control for loading. F3-T3: FGFR3-TACC3. α-tubulin or β-actin is shown as a control for loading.

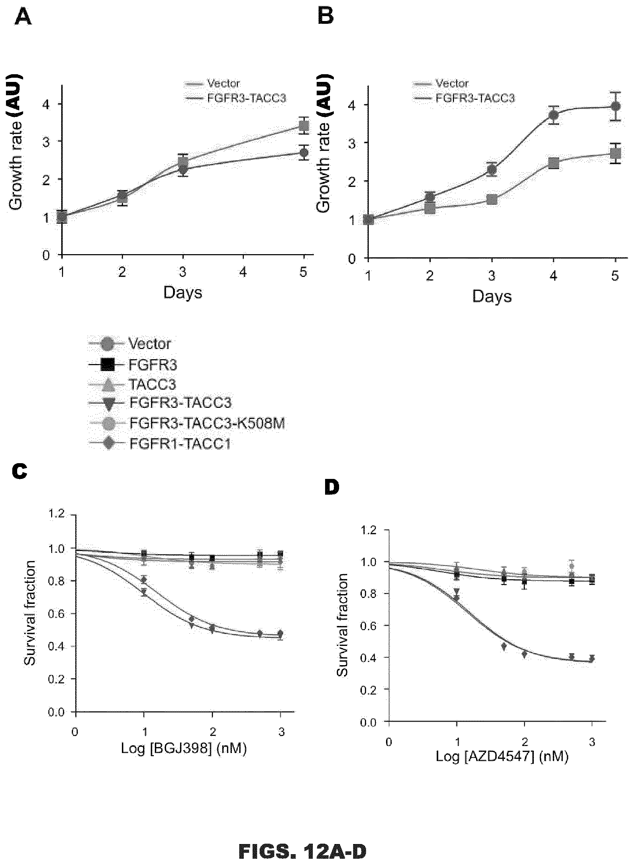

**FIGS. 12A-B** shows growth curves of human primary astrocytes transduced with lentivirus expressing FGFR3-TACC3 fusion or the empty vector. An analysis was conducted of FGFR3-TACC3 fusion mediated growth alteration and specific effect of RTK inhibitors on cells carrying FGFR-TACC fusions. **FIG. 12A** is a graph that shows cell proliferation of human primary astrocytes transduced with lentivirus expressing FGFR3-TACC3 fusion or the empty vector was determined by the MTT assay 7 days after infection (passage 1). Values are the means ± standard deviation (n = 4). p-value: 0.0033. **FIG. 12B** is a graph that shows cell proliferation of human primary astrocytes transduced with lentivirus expressing FGFR3-TACC3 fusion or the empty vector was determined by the MTT assay six weeks after the infection (passage 10). Values are the means±standard deviation (n=4). p-value: 0.0018.

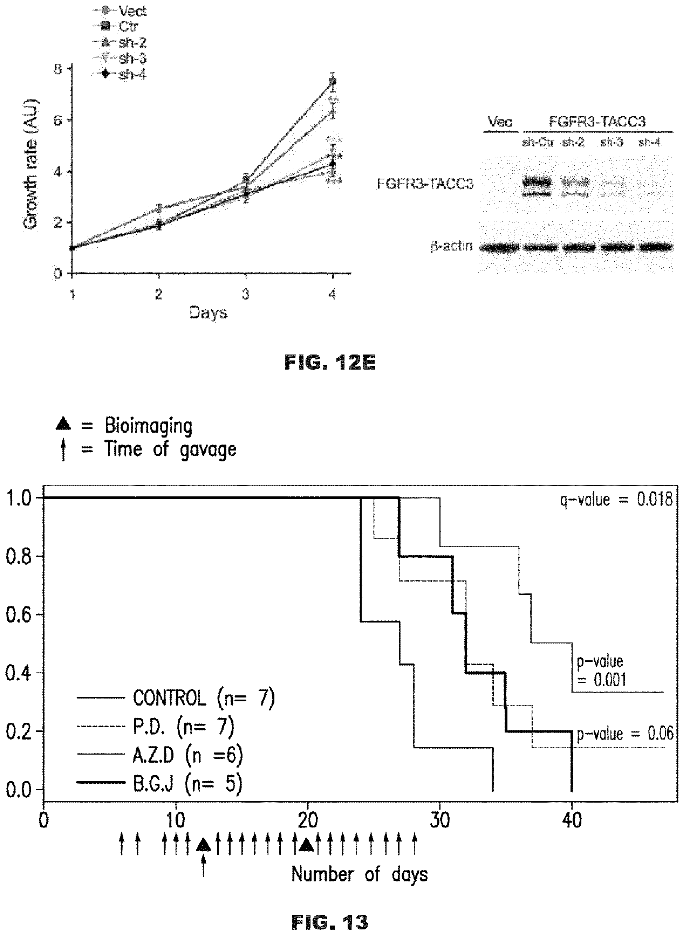

**FIGS. 12C-D** shows specific growth inhibitory effect by FGFR inhibitors on FGFR-TACC fusion expressing cells. Cell growth was determined by MTT assay. Rat1A cells transduced with the indicated lentivirus were treated for three days with BGJ398 **(****FIG. 12C****)** or AZD4547 **(****FIG. 12D****)** at the indicated concentration. Values are the means ± standard error (n = 4). **FIG. 12E** shows the growth inhibitory effect of silencing FGFR3-TACC3 fusion. **(left)** GSC-1123 cells were transduced in triplicate with lentivirus expressing a non-targeting shRNA (Ctr) or lentivirus expressing sh-3 and sh-4 sequences targeting FGFR3. Five days after infection cells were plated at density of 2X10⁴ cells/well in triplicate and the number of trypan blue excluding cells was scored at the indicated times. Values are the means ± standard deviation (n = 3). **(right)** Western Blot analysis was performed on parallel cultures collected five days after infection using the FGFR3 antibody to the detect FGFT3-TACC3 fusion protein. 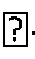 -actin is shown as a control for loading. (**: *p*-value = < 0.005; ***: *p*-value = < 0.0001).
**FIG. 13** shows a survival plot of cells treated with PD173074 , NVP-BGJ398, or AZD4547.

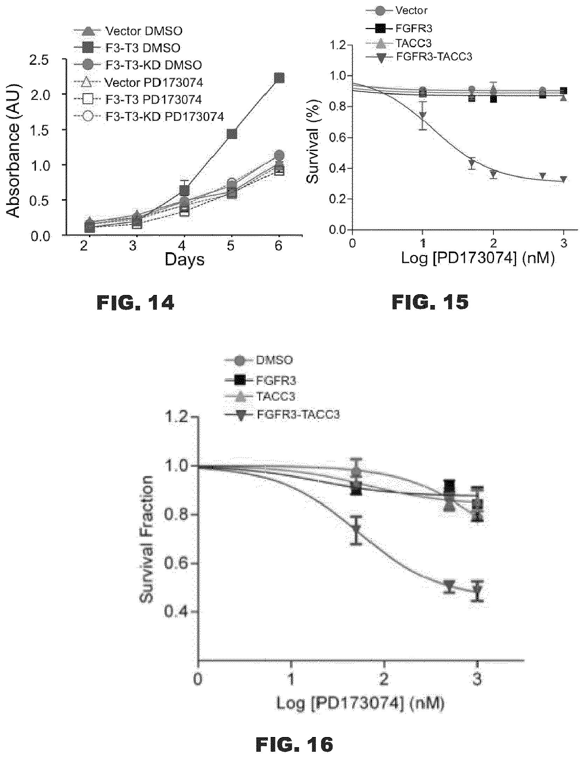

**FIG. 14** shows that inhibition of FGFR-TK activity corrects the aneuploidy and suppresses tumor growth initiated by FGFR3-TACC3. Short-term growth inhibition assays are shown of Rat1A transduced with the indicated lentivirus and treated with PD173470 at the indicated concentrations. Cells were treated for three days. Cell viability was determined by the MTT assay. Error bars show means ± standard error (n = 4).
**FIG. 15** is a growth inhibition assay of human astrocytes transduced with the indicated lentivirus and treated for four days with PD173470 at the indicated concentration. Cell viability was determined by the MTT assay. Error bars show means ± standard error (n = 4).
**FIG. 16** is a graph showing a growth inhibition assay of human astrocytes transduced with the indicated lentivirus and treated for four days with PD173470 at the indicated concentration. Cell viability was determined by the MTT assay. Error bars show means ± standard error (n = 4).

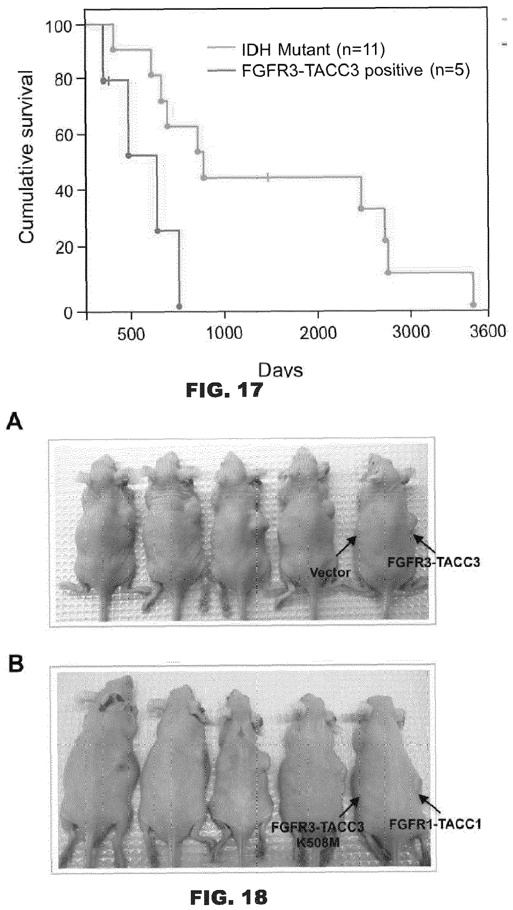

**FIG. 17** shows Kaplan-Meier analysis of IDH mutant and *FGFR3-TACC3* positive human GBM. Log rank test *p*-value: 0.0169.
**FIG. 18A-B** is a picture that shows tumor xenografts that were induced following sub-cutaneous injection of *Ink4A;Arf-*/*-* mouse astrocytes transduced with lentivirus expressing FGFR3-TACC3 (upper panel A, right flank) or FGFR1-TACC1 (lower panel B, right flank) fusion, but not with the empty vector (upper panel, left flank) or FGFR3-TACC3 carrying a K508M mutation in the kinase domain (FGFR3-TACC3-K508M; lower panel, left flank).

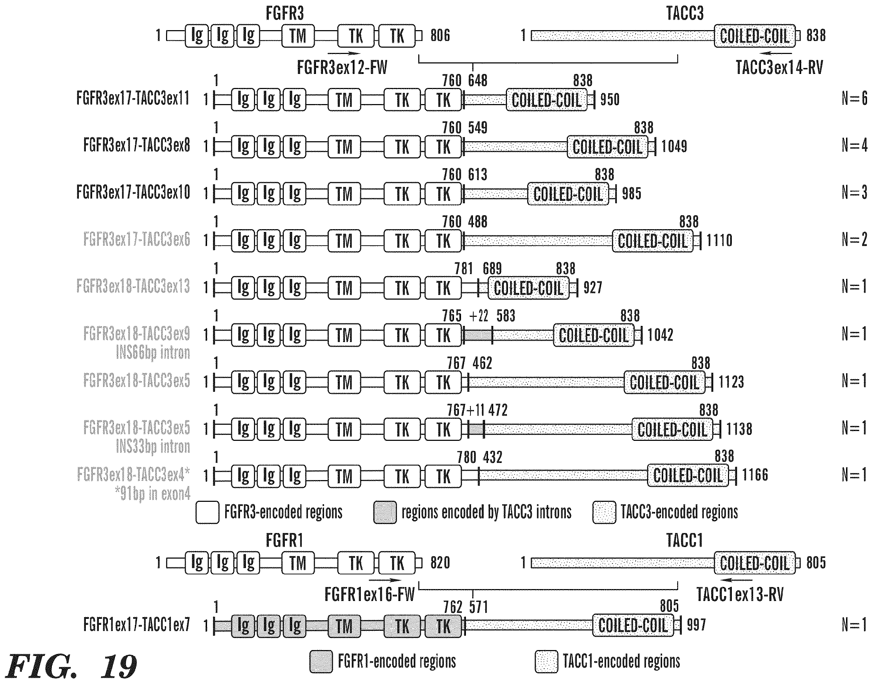

**FIG. 19** shows the structure of FGFR-TACC gene fusions identified by RT-PCR-Sanger sequencing (see also SEQ ID NOs: 530-547). Predicted FGFR-TACC fusion proteins encoded by the transcripts identified by RT-PCR. Regions corresponding to FGFR3 or TACC3 are shown in red or blue, respectively. FGFR1 and TACC1 corresponding regions are shown in yellow and green. On the left are indicated the FGFR and TACC exons joined in the fused mRNA; the presence of TACC3 introns is also reported when they are spliced in the fusion cDNA. On the right, the number of patients harboring the corresponding fusion variant is indicated. The novel transcripts discovered in this study are highlighted in red. Black arrows indicate the position of the primers used for the FGFR-TACC fusions screening.

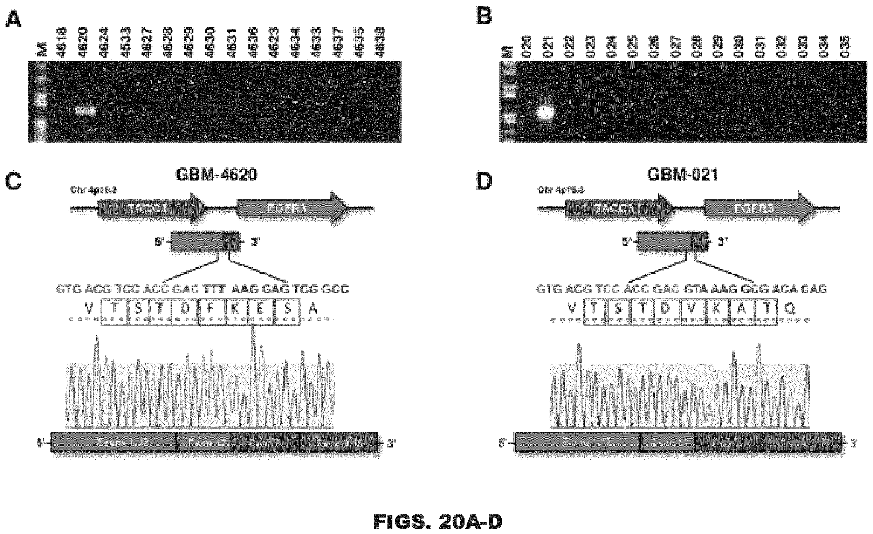

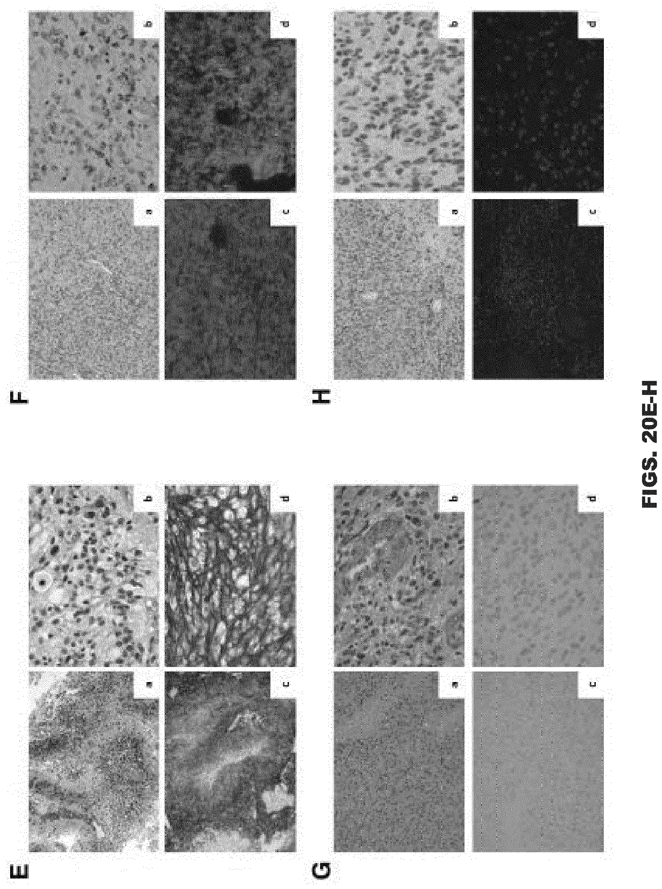

**FIGS. 20A-H** show the identification and immunostaining of FGFR3-TACC3-positive tumors. Results from RT-PCR screening in representative samples from the Pitié-Salpêtrière Hospital (A, C) and the Besta (B, D) datasets. M, DNA ladder. Schematic representation of the FGFR3-TACC3 fusion transcripts identified in samples GBM-4620 (C) and GBM-021 (D). The junction sequences on the mRNA (GBM-4620 (C) SEQ ID NO: 515; GBM-021 (D) SEQ ID NO: 517) and the reading frame and translation (GBM-4620 (C) SEQ ID NO: 516; GBM-021 (D) SEQ ID NO: 518) at the breakpoint are reported. Representative microphotographs of H&E and FGFR3 immunostaining in the FGFR3-TACC3 positive samples GBM-4620 (E) and GBM-021 (F) and two FGFR3-TACC3 negative samples (panels G and H); a, H&E, 10X magnification; b, H&E, 40X magnification; c, FGFR3, 10X magnification; d, FGFR3, 40X magnification. Figures 20C and 20D disclose chromatogram readings as SEQ ID NOS 550 and 551, respectively.

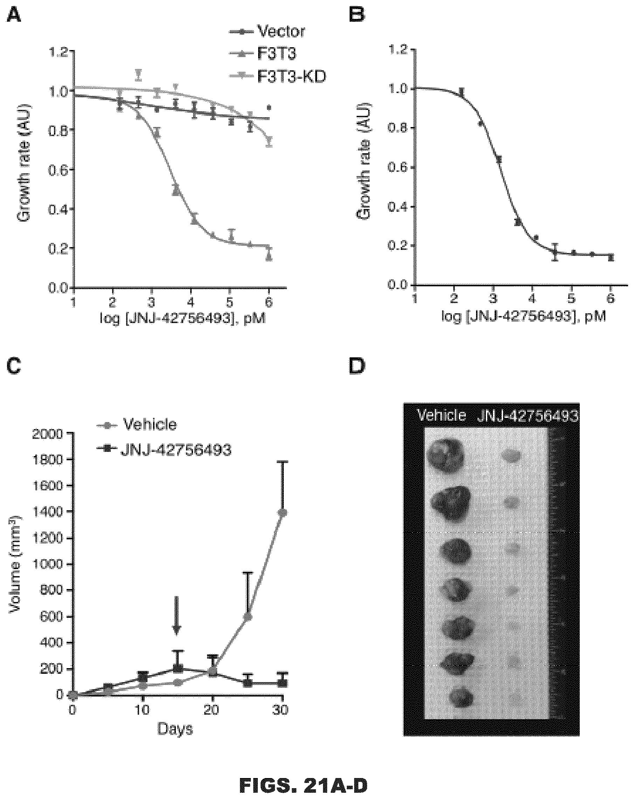

**FIGS. 21A-D** show pre-clinical evaluation of FGFR3-TACC3 inhibition by JNJ-42756493. (A) Mouse astrocytes expressing FGFR3-TACC3 (F3T3), FGFR3-TACC3-KD (F3T3-KD) or the empty vector (Vector) were treated with the indicated concentration of JNJ-42756493. Cell viability was determined by the MTT assay. Error bars show mean±SEM (n=6). (B) Survival analysis of GIC28 1123 treated with JNJ-42756493. (C) The FGFR-TK inhibitor JNJ-42756493 suppresses tumor growth of subcutaneous tumors generated by GIC-1123. After tumor establishment (arrow) mice were treated with vehicle or JNJ-42756493 (12 mg/kg) for 14 days. Values are mean tumor volumes±SD, (n=9 mice per group). P-value of the slope calculated from the treatment starting point (arrow) is 0.04. (D) Photograph showing the tumors dissected from vehicle or JNJ-42756493 treated mice after two weeks of treatment.

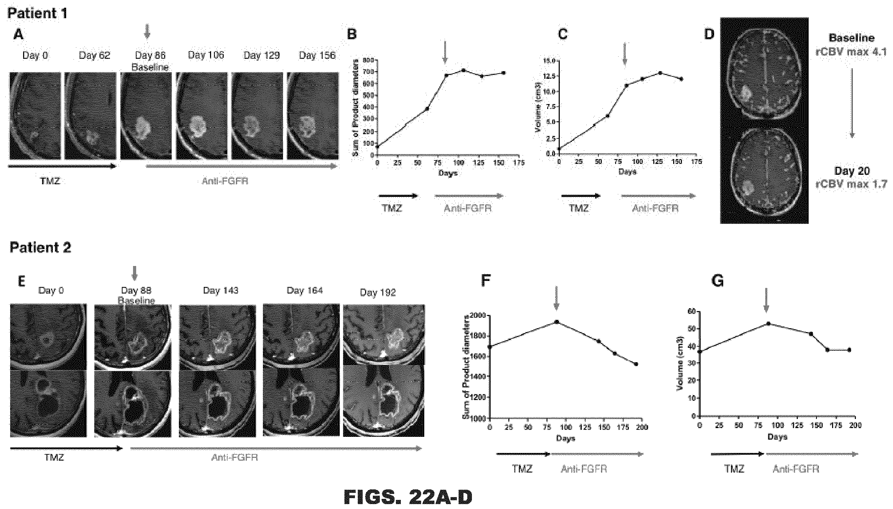

**FIGS. 22A-G** show baseline and post-treatment Magnetic Resonance Imaging (MRI) of patients treated with JNJ-42756493. Patient 1 (Panels A-D). (A) Post-gadolinium T1 weighted images show the target lesion on the right parietal lobe. The interval (days) from the beginning of follow-up is indicated above each MRI. (B) Analysis of sum of product diameters (SPD) before and during the anti-FGFR treatment (RANO criteria). (C) Analysis of tumor volume (cm3) before and during the anti-FGFR treatment. During anti-FGFR treatment a stabilization of the tumor was observed according to RANO criteria and volumetry. (D) Perfusion images at baseline and after 20 days of anti-FGFR treatment. rCBV (relative cerebral blood volume). Post-gadolinium T1 weighted images with color overlay of rCBV are shown. Patient 2 (Panels E-G). (E) Two different MRI slice levels of superior and middle part of the lesion are presented. (F) Analysis of sum of product diameters (SPD) before and during the anti-FGFR treatment. During the anti-FGFR treatment a reduction of 22% of tumor size was observed. (G) Volumetric evaluation showed a 28% tumor reduction. Vertical red arrow indicates the start of anti-FGFR treatment (baseline).

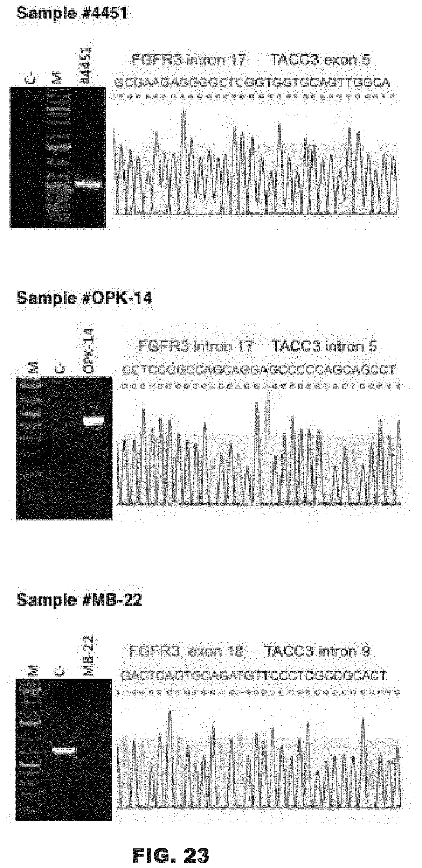

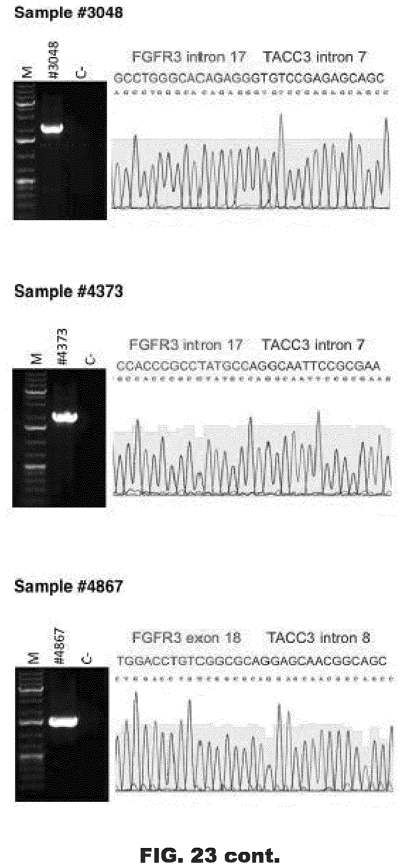

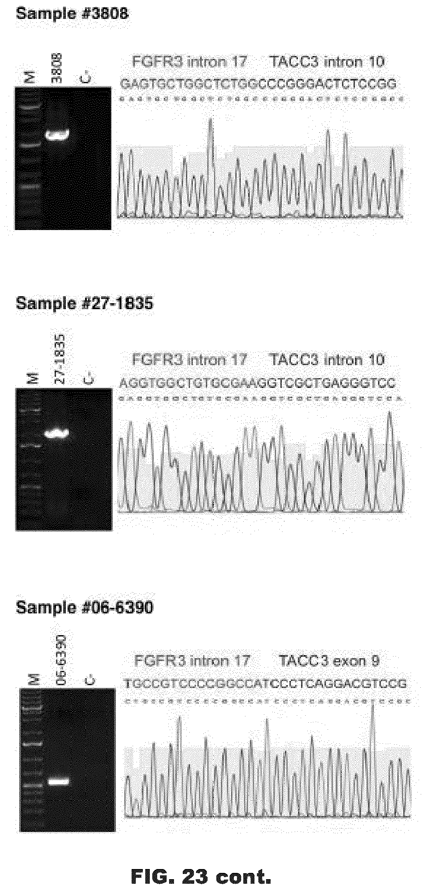

**FIG. 23** shows the genomic PCR images and Sanger sequences of FGFR3-TACC3 genomic breakpoints. Fusion specific PCR products and Sanger sequencing chromatograms showing the FGFR3-TACC3 genomic breakpoints (Sample #4451 SEQ ID NO:519; Sample #OPK-14 SEQ ID NO: 520; Sample #MB-22 SEQ ID NO: 521; Sample #3048 SEQ ID NO: 522; Sample #4373 SEQ ID NO: 523; Sample #4867 SEQ ID NO: 524; Sample #3808 SEQ ID NO: 525; Sample #27-1835 SEQ ID NO: 526; Sample #06-6390 SEQ ID NO: 527). The genomic sequences corresponding to FGFR3 and TACC3 are indicated in red or blue, respectively. M, DNA ladder; C-, Negative Control. Figure 23 discloses chromatogram readings as SEQ ID NOS 552-560, respectively, in order of appearance.

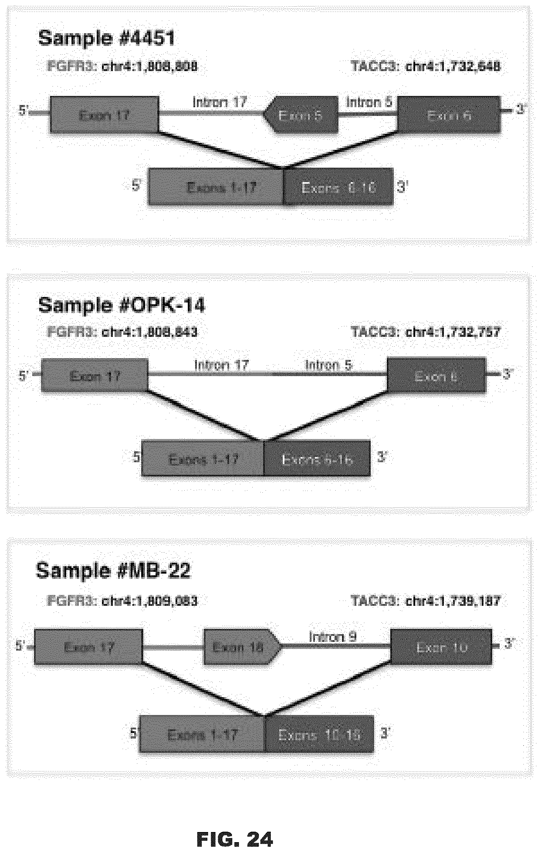

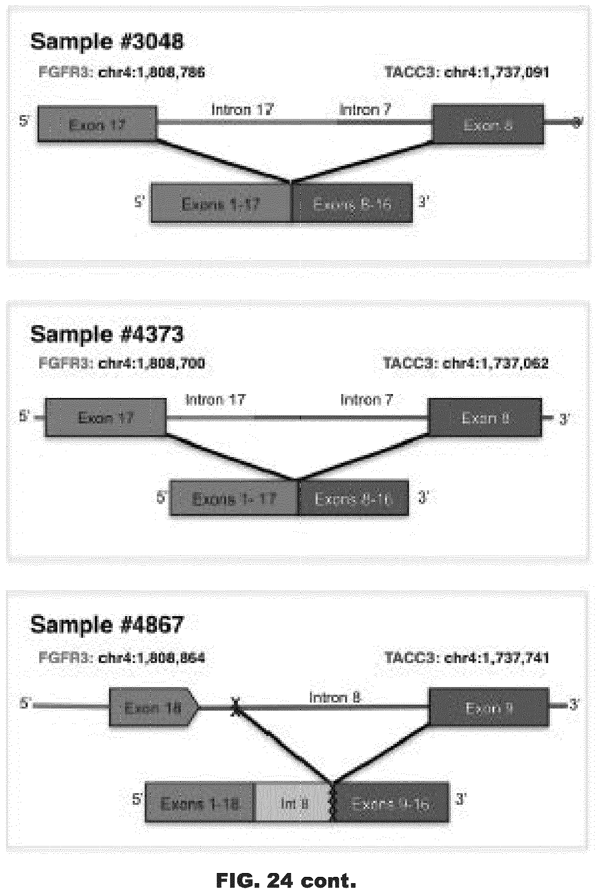

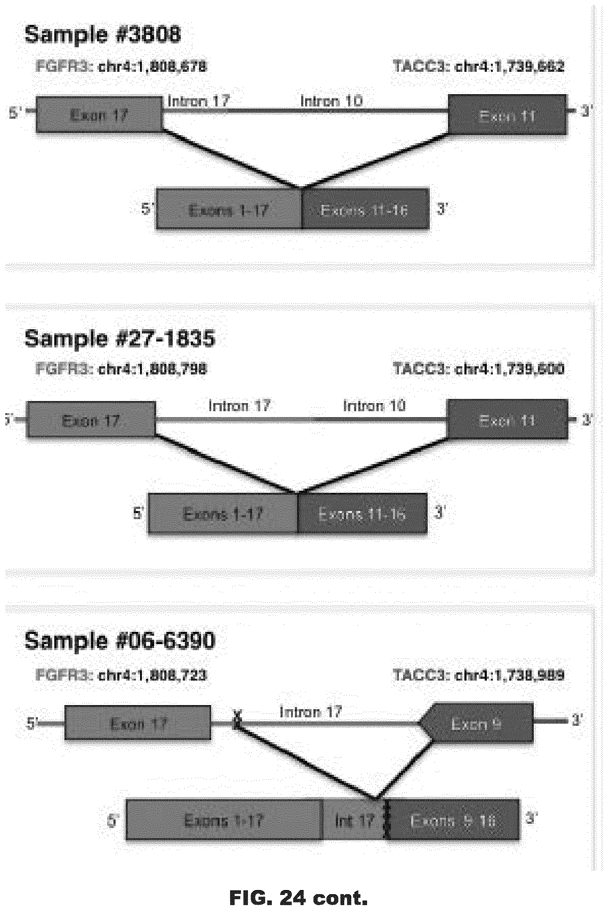

**FIG. 24** shows schematics of FGFR3-TACC3 genomic breakpoints. Schematic representation of the genomic fusions between FGFR3 and TACC3 compared to the corresponding mRNA. In red and blue are reported the regions belonging to FGFR3 and TACC3, respectively. The genomic breakpoint coordinates, according to the genome build GRCh37/hg19, are indicated above each fusion gene.

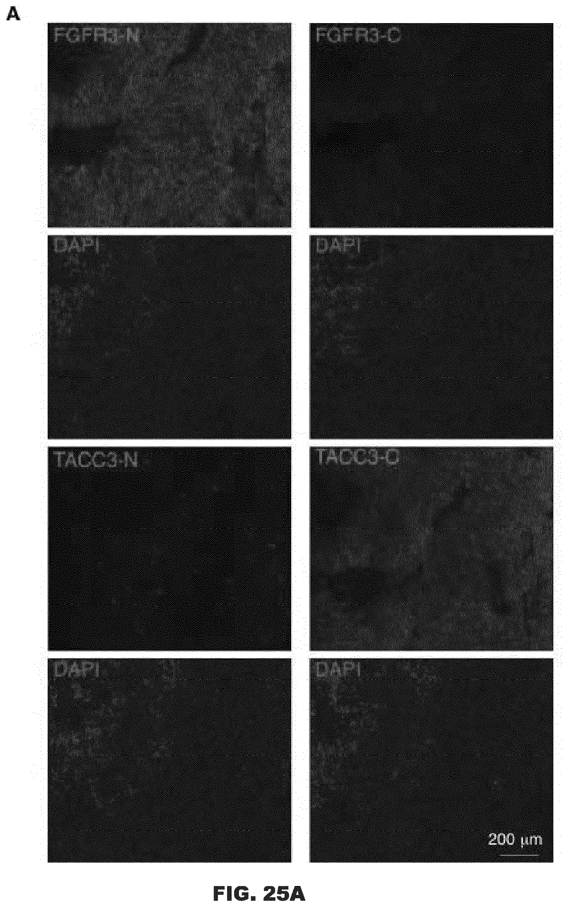

**FIGS. 25A-B** show evaluation of the expression of FGFR3-TACC3 fusion elements. (A) Microphotographs of immunofluorescence staining of a representative GBM harboring FGFR3- TACC3 fusion using antibodies that recognize the N- and C- termini of FGFR3 (FGFR3-N, FGFR3-C) and TACC3 (TACC3-N, TACC3-C), red. Nuclei are counterstained with DAPI, blue. (B) Quantitative RT-PCR of four representative GBM carrying FGFR3-TACC3 fusion and three negative controls using primer pairs that amplify FGFR3 and TACC3 regions included in or excluded from the fusion transcripts, as indicated in the diagram. OAW28: ovarian cystoadenocarcinoma cell line harboring wild type FGFR3 and TACC3 genes; GBM55 and GBM0822: GBM harboring wild type FGFR3 and TACC3 genes; GBM3808; GBM1133; GBM0826; GBM3048: GBM harboring FGFR3-TACC3 (F3-T3) fusion. Error bars are SD of triplicate samples.

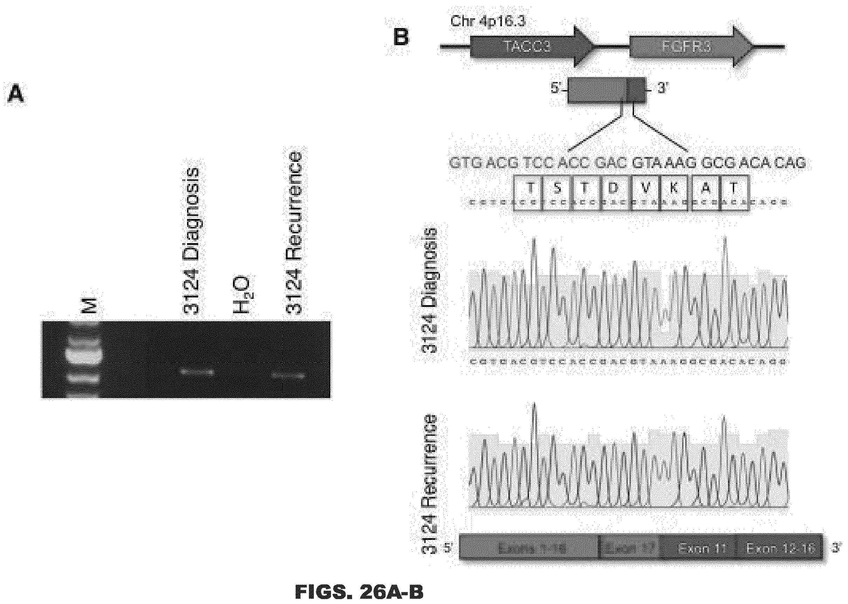

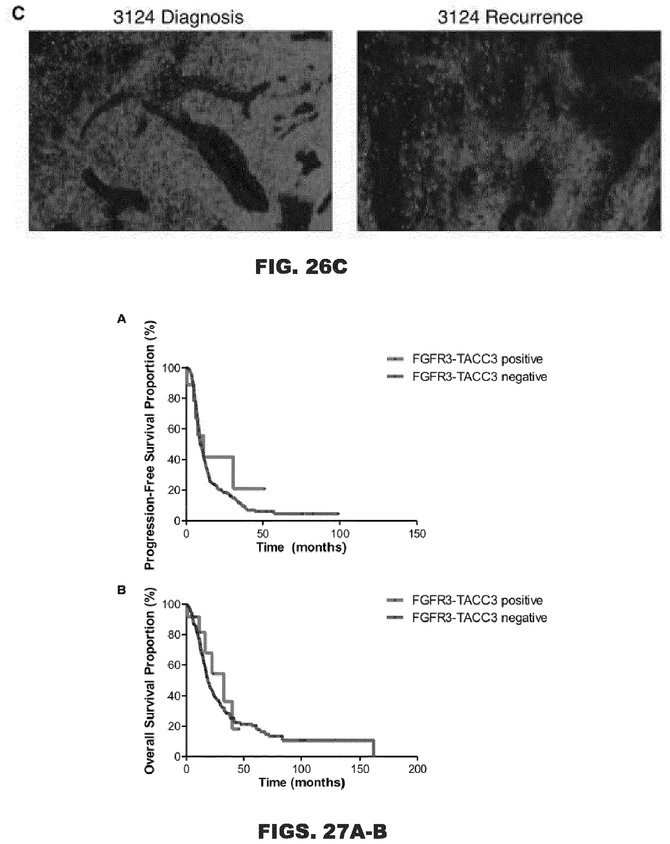

**FIGS 26A-C** show the FGFR3-TACC3 fusion gene and protein are retained in recurrent GBM. (FIG. 26A) FGFR3-TACC3 fusion specific RT-PCR product from untreated and recurrent GBM from patient #3124. (FIG. 26B) Sanger sequencing chromatogram showing the identical reading frame at the breakpoint (SEQ ID NO: 517) and the putative translation of the fusion protein (SEQ ID NO: 86) in the untreated and recurrent tumor from the same patient. The fused exons at mRNA level are shown. Regions corresponding to FGFR3 and TACC3 are indicated in red and blue, respectively. T, threonine; S, serine; D, aspartic acid; V, valine; K, lysine; A, alanine. (FIG. 26C) Representative microphotographs of FGFR3 immunofluorescence (IF) staining in both untreated and recurrent GBM. Blue staining, DAPI; Red staining, FGFR3. 10X Magnification. Figure 26B discloses chromatogram readings as SEQ ID NOS 551 and 551, respectively, in order of appearance **FIGS. 27A-B** show PFS and OS of FGFR3-TACC3-positive glioma patients. (A) Kaplan-Meier curves in IDH wild-type glioma patients don't show significant differences in Progression Free Survival (PFS) between FGFR3-TACC3 positive (N=12, median PFS=11.20 months) and FGFR3-TACC3 negative (N=274, Median PFS= 12.27 months) (P=0.85). (B) Kaplan-Meier curves in IDH wild-type glioma patients don't show significant differences in Overall Survival (OS) between FGFR3-TACC3 positive (N=12, Median OS=32.80 months) and FGFR3-TACC3 negative (N=326, Median OS=18.60 months) (P=0.6). In red FGFR3-TACC3 positive patients, in green FGFR3-TACC3 negative patients. Open circles represent censored patients.

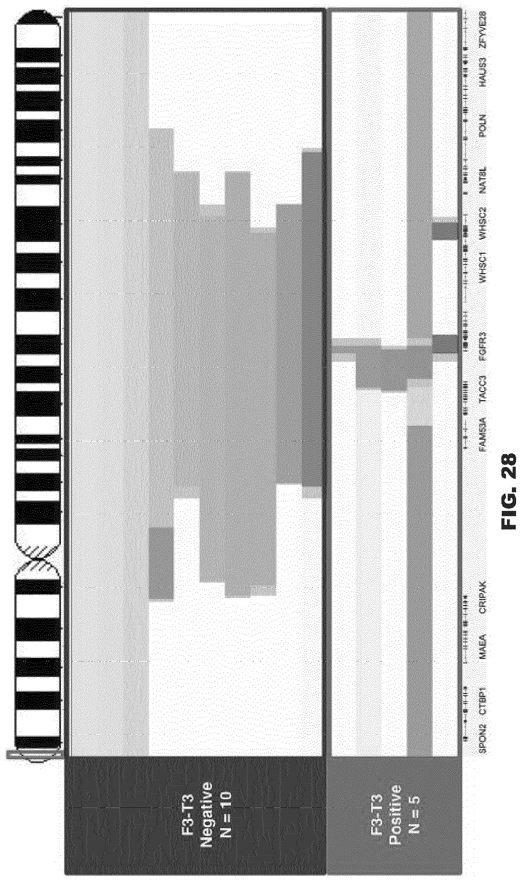

**FIG. 28** shows analysis of SNP6.0 arrays of GBM harboring CNVs of FGFR3 and TACC3 genomic loci. CNVs of the FGFR3/TACC3 genomic loci in "gain labeled" (LRR > 0.2) TCGA samples. The CNA magnitudes (expressed as log2 ratio) were classified using simple thresholds: deletion (x < -1), loss (-1 < x ≤ -0.2), gain (0.2 ≤ x < 1) or amplification (x > 1). Gains are in gradient of red, loss in gradient of blue. Samples with uniform gains/amplification of FGFR3 and TACC3 lack FGFR3-TACC3 fusions. Samples harboring FGFR3-TACC3 fusions (F3-T3) show microamplifications involving the first FGFR3 exons, which are spliced in the fusion gene.

### DETAILED DESCRIPTION OF THE INVENTION

Glioblastoma multiformes (GBMs) are the most common form of brain tumors in adults accounting for 12-15% of intracranial tumors and 50-60% of primary brain tumors. GBM is among the most lethal forms of human cancer. The history of successful targeted therapy of cancer largely coincides with the inactivation of recurrent and oncogenic gene fusions in hematological malignancies and recently in some types of epithelial cancer. GBM is among the most lethal and incurable forms of human cancer. Targeted therapies against common genetic alterations in GBM have not changed the dismal clinical outcome of the disease, most likely because they have systematically failed to eradicate the truly addicting oncoprotein activities of GBM. Recurrent chromosomal rearrangements resulting in the creation of oncogenic gene fusions have not been found in GBM.

GBM is among the most difficult forms of cancer to treat in humans (*1*). So far, the therapeutic approaches that have been tested against potentially important oncogenic targets in GBM have met limited success (2-4). Recurrent chromosomal translocations leading to production of oncogenic fusion proteins are viewed as initiating and addicting events in the pathogenesis of human cancer, thus providing the most desirable molecular targets for cancer therapy (*5, 6*). Recurrent and oncogenic gene fusions have not been found in GBM. Chromosomal rearrangements are hallmarks of hematological malignancies but recently they have also been uncovered in subsets of solid tumors (breast, prostate, lung and colorectal carcinoma) (*7, 8*). Important and successful targeted therapeutic interventions for patients whose tumors carry these rearrangements have stemmed from the discovery of functional gene fusions, especially when the translocations involve kinase-coding genes *(BCR-ABL, EML4-ALK) (9, 10).*

A hallmark of GBM is rampant chromosomal instability (CIN), which leads to aneuploidy (*11*). CIN and aneuploidy are early events in the pathogenesis of cancer (*12*). It has been suggested that genetic alterations targeting mitotic fidelity might be responsible for missegregation of chromosomes during mitosis, resulting in aneuploidy (*13, 14*).

Fibroblast growth factor receptors (FGFR) are transmembrane receptors that bind to members of the fibroblast growth factor family of proteins. The structure of the FGFRs consist of an extracellular ligand binding domain comprised of three Ig-like domains, a single transmembrane helix domain, and an intracellular domain with tyrosine kinase activity (Johnson, D.E., Williams, E. T. Structural and functional diversity in the FGF receptor multigene family. (1993) Adv. Cancer Res, 60:1-41).

Transforming acidic coiled-coiled protein (TACC) stabilize microtubules during mitosis by recruiting minispindles (Msps)/XMAP215 proteins to centrosomes. TACCs have been implicated in cancer.

From a medical perspective, the FGFR-TACC fusions provide the first "bona-fide" oncogenically addictive gene fusions in GBM whose identification has long been overdue in this disease.

Beside GBM, which features the highest grade of malignancy among glioma (grade IV), lower grade glioma which include grade II and grade III are a heterogeneous group of tumors in which specific molecular features are associated with divergent clinical outcome. The majority of grade II-III glioma (but only a small subgroup of GBM) harbor mutations in IDH genes (IDH1 or IDH2), which confer a more favorable clinical outcome. Conversely, the absence of IDH mutations is associated with the worst prognosis (5).

Described herein is the identification of FGFR-TACC gene fusions (mostly FGFR3-TACC3, and rarely FGFR1-TACC1) as the first example of highly oncogenic and recurrent gene fusions in GBM. The FGFR-TACC fusions that have been identified so far include the Tyrosine Kinase (TK) domain of FGFR and the coiled-coil domain of TACC proteins, both necessary for the oncogenic function of FGFR-TACC fusions. FGFR3-TACC3 fusions have been identified in pediatric and adult glioma, bladder carcinoma, squamous lung carcinoma and head and neck carcinoma, thus establishing FGFR-TACC fusions as one of the chromosomal translocation most frequently found across multiple types of human cancers (6-15).

Here, a screening method for FGFR-TACC fusions is reported that includes a RT-PCR assay designed to identify the known and novel FGFR3-TACC3 fusion transcripts, followed by confirmation of the inframe breakpoint by Sanger sequencing. Using this assay, a dataset of 584 GBM and 211 grade II and grade III gliomas has been analyzed. It was determined that brain tumors harboring FGFR-TACC fusions manifest strong and homogeneous intra-tumor expression of the FGFR3 and TACC3 component invariably included in the fusion protein, when analyzed by immunostaining. A significant clinical benefit following treatment with a specific inhibitor of FGFR-TK is reported in two GBM patients who harbored FGFR3-TACC3 rearrangement.

### DNA and AminoAcid Manipulation Methods and Purification Thereof

The practice of aspects of the present invention can employ, unless otherwise indicated, conventional techniques of cell biology, cell culture, molecular biology, transgenic biology, microbiology, recombinant DNA, and immunology, which are within the skill of the art. Such techniques are explained fully in the literature. *See*, e.g., Molecular Cloning A Laboratory Manual, 3rd Ed., ed. by Sambrook (2001), Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Pat. No: 4,683,195; Nucleic Acid Hybridization (B. D. Hames & S. J. Higgins eds. 1984); Transcription and Translation (B. D. Hames & S. J. Higgins eds. 1984); Culture Of Animal Cells (R. I. Freshney, Alan R. Liss, Inc., 1987); Immobilized Cells and Enzymes (IRL Press, 1986); B. Perbal, A Practical Guide To Molecular Cloning (1984); the series, Methods In Enzymology (Academic Press, Inc., N.Y.), specifically, Methods In Enzymology, Vols. 154 and 155 (Wu et al. eds.); Gene Transfer Vectors For Mammalian Cells (J. H. Miller and M. P. Calos eds., 1987, Cold Spring Harbor Laboratory); Immunochemical Methods In Cell And Molecular Biology (Caner and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986). All patents, patent applications and references cited herein are incorporated by reference in their entireties.

One skilled in the art can obtain a protein in several ways, which include, but are not limited to, isolating the protein via biochemical means or expressing a nucleotide sequence encoding the protein of interest by genetic engineering methods.

A protein is encoded by a nucleic acid (including, for example, genomic DNA, complementary DNA (cDNA), synthetic DNA, as well as any form of corresponding RNA). For example, it can be encoded by a recombinant nucleic acid of a gene. The proteins of the invention can be obtained from various sources and can be produced according to various techniques known in the art. For example, a nucleic acid that encodes a protein can be obtained by screening DNA libraries, or by amplification from a natural source. A protein can be a fragment or portion thereof. The nucleic acids encoding a protein can be produced via recombinant DNA technology and such recombinant nucleic acids can be prepared by conventional techniques, including chemical synthesis, genetic engineering, enzymatic techniques, or a combination thereof. For example, a fusion protein of the invention comprises a tyrosine kinase domain of an FGFR protein fused to a polypeptide that constitutively activates the tyrosine kinase domain of the FGFR protein. For example, a fusion protein of the invention comprises a transforming acidic coiled-coil (TACC) domain fused to a polypeptide with a tyrosine kinase domain, wherein the TACC domain constitutively activates the tyrosine kinase domain. An example of a FGFR1-TACC1 polypeptide has the amino acid sequence shown in SEQ ID NO: 150. An example of a FGFR3-TACC3 protein is the polypeptide encoded by the nucleic acid having the nucleotide sequence shown in SEQ ID NOs: 94, 530, 531, 532, 533, 534, 535, 536, 537, or 538. Examples of a FGFR3-TACC3 polypeptide has the amino acid sequence shown in SEQ ID NO: 79, 158, 159, 160, 161, 539, 540, 541, 542, 543, 544, 545, 546, or 547.

The Genbank ID for the FGFR3 gene is 2261. Three isoforms are listed for FGFGR3, e.g., having Genebank Accession Nos. NP_000133 (corresponding nucleotide sequence NM_000142); NP_001156685 (corresponding nucleotide sequence NM_001163213); NP_075254 (corresponding nucleotide sequence NM_022965).

SEQ ID NO: 90 is the FGFR3 Amino Acid Sequence, Transcript Variant 1 (NP_000133; 806 aa). The location of exons are 1-37, 38-126, 127-149, 150-205, 206-247, 248-310, 311-359, 360-422, 423-471, 472-511, 512-549, 550-612, 613-653, 654-676, 677-723, 724-758, 759-7806. Amino acids encoded by nucleotides spanning exons are E37, D127, G149, E247, A359, R471, D512, G549, V677, and L723.

SEQ ID NO: 91 is the FGFR3 Nucleotide Sequence, Transcript Variant 1 (NM_000142; 4304 bp).

SEQ ID NO: 528 is the FGFR3 wt cDNA Nucleotide Sequence corresponding to the coding sequence of FGFR3 (2421 bp) (NM_000142.4 NP_000133.1). The location of exons are 110-379, 446-615, 740-930, 1076-1266, 1413-1534, 1646-1836, 1960-2030, and 2169-2274.

The Genbank ID for the TACC3 gene is 10460. SEQ ID NO: 92 is the TACC3 Amino Acid Sequence (NP_006333) (838 aa). The location of exons are 1-54, 55-101, 102-462, 463-487, 488-531, 532-548, 549-583, 584-612, 613-647, 648-672, 673-688, 689-741, 742-777, 778-817. Amino acids encoded by nucleotides spanning exons are N102, Q462, V531, S583, G673, E688, and L777. Double underlining indicates K112, the amino acid encoded by the nucleotides 334-336 of SEQ ID NO: 529.

SEQ ID NO: 93 is the TACC3 Nucleotide Sequence (NM_006342) (2847 bp).

SEQ ID NO: 529 is the TACC3 wt cDNA Nucleotide Sequence corresponding to the coding sequence of TACC3 (2517 bp) (NM_006342.2, NP_006333.1). The location of exons are 163- 305, 1386-1461, 1592-1644, 1749-1836, 1942-2018, 2063-2223, and 2331-2451.

SEQ ID NO: 94 is the nucleotide sequence of FGFR3-TACC3.

SEQ ID NO: 530 is the nucleotide sequence (cDNA) of FGFR3ex17-TACC3ex11. The sequence corresponding to FGFR3 is 1-2274. The sequence corresponding to TACC3 is 2275-2850.

SEQ ID NO: 531 is the nucleotide sequence (cDNA) of FGFR3ex17-TACC3ex8. The sequence corresponding to FGFR3 is 1-2274. The sequence corresponding to TACC3 is 2275-3147.

SEQ ID NO: 532 is the nucleotide sequence (cDNA) of FGFR3ex17-TACC3ex10. The sequence corresponding to FGFR3 is 1-2274. The sequence corresponding to TACC3 is 2275-2955.

SEQ ID NO: 533 is the nucleotide sequence (cDNA) of FGFR3ex17-TACC3ex6. The sequence corresponding to FGFR3 is 1-2274. The sequence corresponding to TACC3 is 2275-3330.

SEQ ID NO: 534 is the nucleotide sequence (cDNA) of FGFR3ex18-TACC3ex13. The sequence corresponding to FGFR3 is 1-2336. The sequence corresponding to TACC3 is 2337-2780.

SEQ ID NO: 535 is the nucleotide sequence (cDNA) of FGFR3ex18-TACC3ex9_INS66BP. The sequence corresponding to FGFR3 is 1-2296. The sequence corresponding to TACC3 is 2363-3135. The sequence corresponding the the 66bp intronic insert is 2297-2362.

SEQ ID NO: 536 is the nucleotide sequence (cDNA) of FGFR3ex18-TACC3ex5. The sequence corresponding to FGFR3 is 1-2295. The sequence corresponding to TACC3 is 2296-3396.

SEQ ID NO: 537 is the nucleotide sequence (cDNA) of FGFR3ex18-TACC3ex5_INS33bp. The sequence corresponding to FGFR3 is 1-2355. The sequence corresponding to TACC3 is 2389-3503. The sequence corresponding the the 33bp intronic insert is 2356-2388.

SEQ ID NO: 538 is the nucleotide sequence (cDNA) of FGFR3ex18-TACC3ex4. The sequence corresponding to FGFR3 is 1-2331. The sequence corresponding to TACC3 is 2332-3555.

The Genbank ID for the FGFR1 gene is 2260. Eight isoforms are listed for FGFR1, e.g., having Genebank Accession Nos. NP_001167534 (corresponding nucleotide sequence NM_001174063); NP_001167535 (corresponding nucleotide sequence NM_001174064); NP_001167536 (corresponding nucleotide sequence NM_001174065); NP_001167537 (corresponding nucleotide sequence NM_001174066); NP_001167538 (corresponding nucleotide sequence NM_001174067); NP_056934 (corresponding nucleotide sequence NM_015850); NP_075593 (corresponding nucleotide sequence NM_023105); NP_075594 (corresponding nucleotide sequence NM_023106); NP_075598 (corresponding nucleotide sequence NM_023110).

SEQ ID NO: 146 is the FGFR1 Amino Acid Sequence for isoform 10, having Genebank Accession No. NP_001167534 (820 aa).

SEQ ID NO: 147 is the FGFR1 Nucleotide Sequence for isoform 10, having Genebank Accession No. NM_001174063 (5895 bp). The start codon is 943-945.

SEQ ID NO: 185 is the FGFR1 Amino Acid Sequence for isoform 1, having Genebank Accession No. NP_075598 (822 aa).

SEQ ID NO: 186 is the FGFR1 Nucleotide Sequence for isoform 1, having Genebank Accession No. NM_023110 (5917 bp).

The Genbank ID for the TACC1 gene is 6867. Three isoforms are listed for TACC1, e.g., having Genebank Accession Nos. NP_006274 (corresponding nucleotide sequence NM_001174063); NP_001167535 (corresponding nucleotide sequence NM_001174064); NP_001167536 (corresponding nucleotide sequence NM_001174065).

SEQ ID NO: 148 is the TACC1 Amino Acid Sequence for isoform 1, having Genebank Accession No. NP_006274 (805 aa).

SEQ ID NO: 149 is the TACC1 Nucleotide Sequence for isoform 1, having Genebank Accession No. NM_006283 (7802 bp).

SEQ ID NO: 150 is the amino acid sequence of the FGFR1-TACC1 fusion protein.

SEQ ID NO: 151 is the nucleotide sequence that encodes the FGFR1-TACC1 fusion protein (FGFR1 - 1-2286; TACC1 - 2287-2989.

The Genbank ID for the FGFR2 gene is 2263. Eight isoforms are listed for FGFR2, e.g., having Genebank Accession Nos. NP_000132 (corresponding nucleotide sequence NM_000141); NP_001138385 (corresponding nucleotide sequence NM_001144913); NP_001138386 (corresponding nucleotide sequence NM_001144914); NP_001138387 (corresponding nucleotide sequence NM_001144915); NP_001138388 (corresponding nucleotide sequence NM_001144916); NP_001138389 (corresponding nucleotide sequence NM_001144917); NP_001138390 (corresponding nucleotide sequence NM_001144918); NP_001138391 (corresponding nucleotide sequence NM_001144919); NP_075259 (corresponding nucleotide sequence NM_022970).

SEQ ID NO: 152 is the FGFR2 Amino Acid Sequence for isoform 1, having Genebank Accession No. NP_000132 (821 aa).

SEQ ID NO: 153 is the FGFR2 Nucleotide Sequence for isoform 1, having Genebank Accession No. NM_000141 (4654 bp).

The Genbank ID for the TACC2 gene is 10579. Four isoforms are listed for TACC2, e.g., having Genebank Accession Nos. NP_996744 (corresponding nucleotide sequence NM_206862); NP_996743 (corresponding nucleotide sequence NM_206861); NP_996742 (corresponding nucleotide sequence NM_206860); NP_008928 (corresponding nucleotide sequence NM_006997).

SEQ ID NO: 154 is the TACC2 Amino Acid Sequence for isoform a, having Genebank Accession No. NP_996744 (2948 aa).

SEQ ID NO: 155 is the TACC2 Nucleotide Sequence for isoform a, having Genebank Accession No. NM_206862 (9706 bp).

SEQ ID NO: 158 is the amino acid sequence of the FGFR3-TACC3-1 fusion protein. Position 1-758 corresponds to the FGFR3 protein. Position 759- 1048 corresponds to the TACC3 protein.

SEQ ID NO: 159 is the amino acid sequence of the FGFR3-TACC3-2 fusion protein. Position 1-758 corresponds to the FGFR3 protein. Position 759- 1027 corresponds to the TACC3 protein.

SEQ ID NO: 160 is the amino acid sequence of the FGFR3-TACC3-3 fusion protein. Position 1-758 corresponds to the FGFR3 protein. Position 759- 984 corresponds to the TACC3 protein.

SEQ ID NO: 161 is the amino acid sequence of the FGFR3-TACC3-4 fusion protein. Position 1-758 corresponds to the FGFR3 protein. Position 759- 949 corresponds to the TACC3 protein.

SEQ ID NO: 539 is the amino acid sequence of FGFR3ex17-TACC3ex11. The sequence corresponding to FGFR3 is 1-758 (underlined). The sequence corresponding to TACC3 is 759-949 (shaded gray):

MGAPACALALCVAVAIVAGASSESLGTEQRVVGRAAEVPGPEPGQQEQLVFGSGDAVELSCPPPGGGPMG
PTVWVKDGTGLVPSERVLVGPQRLQVLNASHEDSGAYSCRQRLTQRVLCHFSVRVTDAPSSGDDEDGEDE
AEDTGVDTGAPYWTRPERMDKKLLAVPAANTVRFRCPAAGNPTPSISWLKNGREFRGEHRIGGIKLRHQQ
WSLVMESVVPSDRGNYTCVVENKFGSIRQTYTLDVLERSPHRPILQAGLPANQTAVLGSDVEFHCKVYSD
AQPHIQWLKHVEVNGSKVGPDGTPYVTVLKTAGANTTDKELEVLSLHNVTFEDAGEYTCLAGNSIGFSHH
SAWLVVLPAEEELVEADEAGSVYAGILSYGVGFFLFILVVAAVTLCRLRSPPKKGLGSPTVHKISRFPLK
RQVSLESNASMSSNTPLVRIARLSSGEGPTLANVSELELPADPKWELSRARLTLGKPLGEGCFGQVVMAE
AIGIDKDRAAKPVTVAVKMLKDDATDKDLSDLVSEMEMMKMIGKHKNIINLLGACTQGGPLYVLVEYAAK
GNLREFLRARRPPGLDYSFDTCKPPEEQLTFKDLVSCAYQVARGMEYLASQKCIHRDLAARNVLVTEDNV
MKIADFGLARDVHNLDYYKKTTNGRLPVKWMAPEALFDRVYTHQSDVWSFGVLLWEIFTLGGSPYPGIPV
EELFKLLKEGHRMDKPANCTHDLYMIMRECWHAAPSQRPTFKQLVEDLDRVLTVTSTDVKATQEENRELR

SRCEELHGKNLELGKIMDRFEEVVYQAMEEVQKQKELSKAEIQKVLKEKDQLTTDLNSMEKSFSDLFKRF
EKQKEVIEGYRKNEESLKKCVEDYLARITQEGQRYQALKAHAEEKLQLANEEIAQVRSKAQAEALALQAS
LRKEQMRIQSLEKTVEQKTKENEELTRICDDLISKMEKI

SEQ ID NO: 540 is the amino acid sequence of FGFR3ex17-TACC3ex8. The sequence corresponding to FGFR3 is 1-758 (underlined). The sequence corresponding to TACC3 is 759-1048 (shaded gray):

MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGAP
YWTRPERMDKKLLAVPAANTVRFRCPAAGNPTPSISWLKNGREFRGEHRIGGIKLRHQQWSLVMESVVPSDRGNYT
CVVENKFGSIRQTYTLDVLERSPHRPILQAGLPANQTAVLGSDVEFHCKVYSDAQPHIQWLKHVEVNGSKVGPDGTP
YVTVLKTAGANTTDKELEVLSLHNVTFEDAGEYTCLAGNSIGFSHHSAWLVVLPAEEELVEADEAGSVYAGILSYGVGF
FLFILVVAAVTLCRLRSPPKKGLGSPTVHKISRFPLKRQVSLESNASMSSNTPLVRIARLSSGEGPTLANVSELELPADPK
WELSRARLTLGKPLGEGCFGQVVMAEAIGIDKDRAAKPVTVAVKMLKDDATDKDLSDLVSEMEMMKMIGKHKNII
NLLGACTQGGPLYVLVEYAAKGNLREFLRARRPPGLDYSFDTCKPPEEQLTFKDLVSCAYQVARGMEYLASQKCIHRD
LAARNVLVTEDNVMKIADFGLARDVHNLDYYKKTTNGRLPVKWMAPEALFDRVYTHQSDVWSFGVLLWEIFTLGGS
PYPGIPVEELFKLLKEGHRMDKPANCTHDLYMIMRECWHAAPSQRPTFKQLVEDLDRVLTVTSTDFKESALRKQSLYL
KFDPLLRDSPGRPVPVATETSSMHGANETPSGRPREAKLVEFDFLGALDIPVPGPPPGVPAPGGPPLSTGPIVDLLQYS
QKDLDAVVKATQEENRELRSRCEELHGKNLELGKIMDRFEEVVYQAMEEVQKQKELSKAEIQKVLKEKDQLTTDLNS
MEKSFSDLFKRFEKQKEVIEGYRKNEESLKKCVEDYLARITQEGQRYQALKAHAEEKLQLANEEIAQVRSKAQAEALAL
QASLRKEQMRIQSLEKTVEQKTKENEELTRICDDLISKMEKI

SEQ ID NO: 541 is the amino acid sequence of FGFR3ex17-TACC3ex10. The sequence corresponding to FGFR3 is 1-758 (underlined). The sequence corresponding to TACC3 is 759-984 (shaded gray):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE
RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDVPGPPPGVPAPGGPPLSTGPIVDLLQYSQKDLDAVVKATQEENRE
LRSRCEELHGKNLELGKIMDRFEEVVYQAMEEVQKQKELSKAEIQKVLKEKDQLTTDLNSMEKSFSDLFKRFEKQKEVIEGY
RKNEESLKKCVEDYLARITQEGQRYQALKAHAEEKLQLANEEIAQVRSKAQAEALALQASLRKEQMRIQSLEKTVEQKTKEN
EELTRICDDLISKMEKI

SEQ ID NO: 542 is the amino acid sequence of FGFR3ex17-TACC3ex6. The sequence corresponding to FGFR3 is 1-758 (underlined). The sequence corresponding to TACC3 is 759-1109 (shaded gray):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE
RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDERALNSASTSLPTSCPGSEPVPTHQQGQPALELKEESFRDPAEVLG
TGAEVDYLEQFGTSSFKESALRKQSLYLKFDPLLRDSPGRPVPVATETSSMHGANETPSGRPREAKLVEFDFLGALDIPVPGP
PPGVPAPGGPPLSTGPIVDLLQYSQKDLDAVVKATQEENRELRSRCEELHGKNLELGKIMDRFEEVVYQAMEEVQKQKELS
KAEIQKVLKEKDQLTTDLNSMEKSFSDLFKRFEKQKEVIEGYRKNEESLKKCVEDYLARITQEGQRYQALKAHAEEKLQLANE
EIAQVRSKAQAEALALQASLRKEQMRIQSLEKTVEQKTKENEELTRICDDLISKMEKI

SEQ ID NO: 543 is the amino acid sequence of FGFR3ex18-TACC3ex13. The sequence corresponding to FGFR3 is 1-779 (underlined). The sequence corresponding to TACC3 is 780-926 (shaded gray):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE
RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDEYLDLSAPFEQYSPGGQDTPSKQKELSKAEIQKVLKEKDQLTTDLN
SMEKSFSDLFKRFEKQKEVIEGYRKNEESLKKCVEDYLARITQEGQRYQALKAHAEEKLQLANEEIAQVRSKAQAEALALQA
SLRKEQMRIQSLEKTVEQKTKENEELTRICDDLISKMEKI

SEQ ID NO: 544 is the amino acid sequence of FGFR3ex18-TACC3ex9_INS66BP. The sequence corresponding to FGFR3 is 1-765 (underlined). The sequence corresponding to TACC3 is 788-1043 (shaded gray). The sequence corresponding the the 66bp intronic insert is 766-787 (double-underlined):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE
RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDEYLDLSAQEQRQPTLQPQGCCLAGYSHRSSMHGANETPSGRPRE
AKLVEFDFLGALDIPVPGPPPGVPAPGGPPLSTGPIVDLLQYSQKDLDAVVKATQEENRELRSRCEELHGKNLELGKIMDRF
EEVVYQAMEEVQKQKELSKAEIQKVLKEKDQLTTDLNSMEKSFSDLFKRFEKQKEVIEGYRKNEESLKKCVEDYLARITQEG
QRYQALKAHAEEKLQLANEEIAQVRSKAQAEALALQASLRKEQMRIQSLEKTVEQKTKENEELTRICDDLISKMEKI

SEQ ID NO: 545 is the amino acid sequence of FGFR3ex18-TACC3ex5. The sequence corresponding to FGFR3 is 1-765 (underlined). The sequence corresponding to TACC3 is 766-1131 (shaded gray):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE
RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDEYLDLSAPVVQLAAETPTAESKERALNSASTSLPTSCPGSEPVPTH
QQGQPALELKEESFRDPAEVLGTGAEVDYLEQFGTSSFKESALRKQSLYLKFDPLLRDSPGRPVPVATETSSMHGANETPSG
RPREAKLVEFDFLGALDIPVPGPPPGVPAPGGPPLSTGPIVDLLQYSQKDLDAVVKATQEENRELRSRCEELHGKNLELGKI
MDRFEEVVYQAMEEVQKQKELSKAEIQKVLKEKDQLTTDLNSMEKSFSDLFKRFEKQKEVIEGYRKNEESLKKCVEDYLARI
TQEGQRYQALKAHAEEKLQLANEEIAQVRSKAQAEALALQASLRKEQMRIQSLEKTVEQKTKENEELTRICDDLISKMEKI

SEQ ID NO: 546 is the amino acid sequence of FGFR3ex18-TACC3ex5_INS33bp. The sequence corresponding to FGFR3 is 1-784 (underlined). The sequence corresponding to TACC3 is 796-1166 (shaded gray). The sequence corresponding the the 33bp intronic insert is 785-795 (double underlined):

MGAPACALALCVAVAIVAGASSESLGTEQRVVGRAAEVPGPEPGQQEQLVFGSGDAVELSCPPPGGGPMGPTVWVKDG
TGLVPSERVLVGPQRLQVLNASHEDSGAYSCRQRLTQRVLCHFSVRVTDAPSSGDDEDGEDEAEDTGVDTGAPYWTRPE

RMDKKLLAVPAANTVRFRCPAAGNPTPSISWLKNGREFRGEHRIGGIKLRHQQWSLVMESVVPSDRGNYTCVVENKFGSI
RQTYTLDVLERSPHRPILQAGLPANQTAVLGSDVEFHCKVYSDAQPHIQWLKHVEVNGSKVGPDGTPYVTVLKTAGANTT
DKELEVLSLHNVTFEDAGEYTCLAGNSIGFSHHSAWLVVLPAEEELVEADEAGSVYAGILSYGVGFFLFILVVAAVTLCRLRSP
PKKGLGSPTVHKISRFPLKRQVSLESNASMSSNTPLVRIARLSSGEGPTLANVSELELPADPKWELSRARLTLGKPLGEGCFG
QVVMAEAIGIDKDRAAKPVTVAVKMLKDDATDKDLSDLVSEMEMMKMIGKHKNIINLLGACTQGGPLYVLVEYAAKGNL
REFLRARRPPGLDYSFDTCKPPEEQLTFKDLVSCAYQVARGMEYLASQKCIHRDLAARNVLVTEDNVMKIADFGLARDVH
NLDYYKKTTNGRLPVKWMAPEALFDRVYTHQSDVWSFGVLLWEIFTLGGSPYPGIPVEELFKLLKEGHRMDKPANCTHDL
YMIMRECWHAAPSQRPTFKQLVEDLDRVLTVTSTDEYLDLSAPFEQYSPGGQDTPSSSSSGVREPPHPAFPXSAEDTPVVQ
LAAETPTAESKERALNSASTSLPTSCPGSEPVPTHQQGQPALELKEESFRDPAEVLGTGAEVDYLEQFGTSSFKESALRKQSL
YLKFDPLLRDSPGRPVPVATETSSMHGANETPSGRPREAKLVEFDFLGALDIPVPGPPPGVPAPGGPPLSTGPIVDLLQYSQ
KDLDAVVKATQEENRELRSRCEELHGKNLELGKIMDRFEEVVYQAMEEVQKQKELSKAEIQKVLKEKDQLTTDLNSMEKSF
SDLFKRFEKQKEVIEGYRKNEESLKKCVEDYLARITQEGQRYQALKAHAEEKLQLANEEIAQVRSKAQAEALALQASLRKEQ
MRIQSLEKTVEQKTKENEELTRICDDLISKMEKI

SEQ ID NO: 547 is the amino acid sequence of FGFR3ex18-TACC3ex4. The sequence corresponding to FGFR3 is 1-777 (underlined). The sequence corresponding to TACC3 is 778-1184 (shaded gray).

MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGAP
YWTRPERMDKKLLAVPAANTVRFRCPAAGNPTPSISWLKNGREFRGEHRIGGIKLRHQQWSLVMESVVPSDRGNYT
CVVENKFGSIRQTYTLDVLERSPHRPILQAGLPANQTAVLGSDVEFHCKVYSDAQPHIQWLKHVEVNGSKVGPDGTP
YVTVLKTAGANTTDKELEVLSLHNVTFEDAGEYTCLAGNSIGFSHHSAWLVVLPAEEELVEADEAGSVYAGILSYGVGF
FLFILVVAAVTLCRLRSPPKKGLGSPTVHKISRFPLKRQVSLESNASMSSNTPLVRIARLSSGEGPTLANVSELELPADPK
WELSRARLTLGKPLGEGCFGQVVMAEAIGIDKDRAAKPVTVAVKMLKDDATDKDLSDLVSEMEMMKMIGKHKNII
NLLGACTQGGPLYVLVEYAAKGNLREFLRARRPPGLDYSFDTCKPPEEQLTFKDLVSCAYQVARGMEYLASQKCIHRD
LAARNVLVTEDNVMKIADFGLARDVHNLDYYKKTTNGRLPVKWMAPEALFDRVYTHQSDVWSFGVLLWEIFTLGGS
PYPGIPVEELFKLLKEGHRMDKPANCTHDLYMIMRECWHAAPSQRPTFKQLVEDLDRVLTVTSTDEYLDLSAPFEQYS
PGGQDTPESPETRLGQPAAEQLHAGPATEEPGPCLSQQLHSASAEDTPVVQLAAETPTAESKERALNSASTSLPTSCP
GSEPVPTHQQGQPALELKEESFRDPAEVLGTGAEVDYLEQFGTSSFKESALRKQSLYLKFDPLLRDSPGRPVPVATETS
SMHGANETPSGRPREAKLVEFDFLGALDIPVPGPPPGVPAPGGPPLSTGPIVDLLQYSQKDLDAVVKATQEENRELRS
RCEELHGKNLELGKIMDRFEEVVYQAMEEVQKQKELSKAEIQKVLKEKDQLTTDLNSMEKSFSDLFKRFEKQKEVIEGY
RKNEESLKKCVEDYLARITQEGQRYQALKAHAEEKLQLANEEIAQVRSKAQAEALALQASLRKEQMRIQSLEKTVEQK
TKENEELTRICDDLISKMEKI

The Genbank ID for the FGFR4 gene is 2264. Three isoforms are listed for FGFR4, e.g., having Genebank Accession Nos. NP_002002 (corresponding nucleotide sequence NM_002011); NP_075252 (corresponding nucleotide sequence NM_022963); NP_998812 (corresponding nucleotide sequence NM_213647).

As used herein, a "FGFR fusion molecule" can be a nucleic acid (e.g., synthetic, purified, and/or recombinant) which encodes a polypeptide corresponding to a fusion protein comprising a tyrosine kinase domain of an FGFR protein fused to a polypeptide that constitutively activates the tyrosine kinase domain of the FGFR protein, or a nucleic acid encoding a fusion protein comprising a transforming acidic coiled-coil (TACC) domain fused to a polypeptide with a tyrosine kinase domain, wherein the TACC domain constitutively activates the tyrosine kinase domain. It can also be a fusion protein comprising a tyrosine kinase domain of an FGFR protein fused to a polypeptide that constitutively activates the tyrosine kinase domain of the FGFR protein, or a fusion protein comprising a transforming acidic coiled-coil (TACC) domain fused to a polypeptide with a tyrosine kinase domain, wherein the TACC domain constitutively activates the tyrosine kinase domain. For example, a FGFR fusion molecule can include a FGFR1-TACC1 (e.g., comprising the amino acid sequence shown in SEQ ID NO: 150, or comprising the nucleic acid sequence shown in SEQ ID NO: 88), FGFR2-TACC2, FGFR3-TACC3 (e.g., comprising the amino acid sequence shown in SEQ ID NOS: 79, 158-161, or 539-547 or comprising the nucleic acid sequence shown in SEQ ID NOS: 80-82, 84, 94-145, 515, 517, 519-527, or 530-538), or other FGFR-TACC fusion proteins (e.g., an N-terminal fragment of FGFR4 containing its tyrosine kinase domain fused to a fragment of TACC1, TACC2, or TACC3). For example, a FGFR fusion molecule can include a FGFR1-containing fusion comprising the amino acid sequence corresponding to Genebank Accession no. NP_001167534, NP_001167535, NP_001167536, NP_001167537, NP_001167538, NP_056934, NP_075593, NP_075594, or NP_075598; or a FGFR1-containing fusion comprising the nucleotide sequence corresponding to Genebank Accession no. NM_001174063, NM_001174064, NM_001174065, NM_001174066, NM_001174067, NM_015850, NM_023105, NM_023106, or NM_023110. For example, a FGFR fusion molecule can include a FGFR2-containing fusion comprising the amino acid sequence corresponding to Genebank Accession no. NP_000132, NP_001138385, NP_001138386, NP_001138387, NP_001138388, NP_001138389, NP_001138390, NP_001138391, or NP_075259; or a FGFR2-containing fusion comprising the nucleotide sequence corresponding to Genebank Accession no. NM_000141, NM_001144913, NM_001144914, NM_001144915, NM_001144916, NM_001144917, NM_001144918, NM_001144919, or NM_022970. For example, a FGFR fusion molecule can include a FGFR3-containing fusion comprising the amino acid sequence corresponding to Genebank Accession no. NP_000133, NP_001156685, or NP_075254; or a FGFR3-containing fusion comprising the nucleotide sequence corresponding to Genebank Accession no. NM_000142, NM_001163213, or NM_022965. For example, a FGFR fusion molecule can include a FGFR4-containing fusion comprising the amino acid sequence corresponding to Genebank Accession no. NP_002002, NP_075252, or NP_998812; or a FGFR4-containing fusion comprising the nucleotide sequence corresponding to Genebank Accession no. NM_002011, NM_022963, or NM_213647. A FGFR fusion molecule can also include a tyrosine kinase domain of an FGFR protein fused to a protein encoded by any one of the genes listed in Table 7. A FGFR fusion molecule can include a variant of the above described examples, such as a fragment thereof. **Table 7**. Fusion Partners

| **gene** | **gene** | **gene** | **gene** |
|---|---|---|---|
| ABCA13 | C21orf29 | CAM KK1 | DNAJC6 |
| ABCC1 | CACNA1C | CAMSAP1 | DYRK3 |
| ABCC12 | CACNA1G | CAMTA1 | EIF2C2 |
| ABCC6 | CNTNAP4 | CAP2 | FAM184B |
| ABL1 | CUL3 | CCDC147 | FREM2 |
| ADAM 12 | DMD | CCDC158 | GDPD2 |
| ADCY10 | DUSP27 | CELF2 | GLI3 |
| ADCY2 | ECE1 | CILP | IL1RN |
| ADCY8 | EYS | CMYA5 | ISX |
| AGBL4 | FAM172A | COL14A1 | KIDINS220 |
| AHNAK | FAM184B | CORO7 | LRBA |
| ANXA7 | FGFR4 | CSMD2 | LY75 |
| AP4S1 | ITGAV | CUL3 | MDH2 |
| AQP2 | LRP1 | DDI2 | MMP12 |
| ARMC6 | LY75 | DEPDC5 | N4BP2L2 |
| ATP5B | MAPKAP1 | DEPDC7 | NCF2 |
| ATP6AP1L | MYT1 | DI10L | NCOR1 |
| ATP6V0D2 | NCF2 | DMD | NCRNA00157 |
| ATXN1 | NCOR1 | EDA | NRXN3 |
| BAHD1 | NHSL2 | EFHC1 | PARP16 |
| BBX | NKAIN2 | EFS | PLA2G2F |
| BCA10 | NR3C1 | EIF2C2 | PLEK2 |
| C15orf23 | NUP188 | ENTPD2 | PRKCH |
| C15orf33 | OSBPL10 | EYS | PTPRS |
| C21orf29 | PACSIN1 | FAM160A1 | ROBO1 |
| C2CD3 | PARP16 | MUSK | SASH3 |
| C6orf170 | PDZRN4 | NEUROG1 | SH3BP5 |
| C7orf44 | POLM | NHSL2 | SLC44A2 |
| CACNA1C | PPP1R3A | NR3C1 | SLC5A4 |
| CACNA1G | PSEN1 | ODZ1 | SNX5 |
| FAM168A | PTPRD | PCDH12 | SORCS2 |
| FAM172A | PTPRS | PLCL1 | SRRM1 |
| FAM192A | RALYL | PLEKHM3 | SSX3 |
| FAM19A2 | RERE | PLOD3 | STAG 2 |
| FBXL4 | RIMBP2 | PRKCH | STK24 |
| FH | RNF216 | PSEN1 | SURF6 |
| FREM2 | SDAD1 | SEPT5 | SYNPO2 |
| GAPVD1 | SEC14L3 | SLC44A2 | TAF1 |
| GLI3 | SH3RF3 | SNTA1 | TMEM80 |
| GPR182 | SLC9A1 | USP48 | TNFRSF10B |
| GSTA3 | SMOC2 | VSNL1 | TTYH1 |
| IGFBP3 | SNX5 | WDFY1 | UNC93B1 |
| ITGA9 | TACC2 | WISP2 | VSNL1 |
| ITGB2 | SRGAP1 | XRRA1 | XRCC4 |
| JOSD2 | SSX3 | LRRC4B | ZNF410 |
| KIDINS220 | SUMF1 | LRRK2 | TRIOBP |
| LAMA2 | SYNPO2 | MAPKAP1 | TTYH1 |
| LCLAT1 | TNFRSF10B | MST1R | LRBA |
| LIN9 | | | |

For example, a FGFR fusion molecule can include a FGFR3-containing fusion comprising the amino acid sequence corresponding to residues 1-760 of FGFR3 (e.g. SEQ ID NO: 90) fused to the amino acid sequence corresponding residues 648-838 of TACC3 (e.g. SEQ ID NO: 92). A FGFR fusion molecule can also include a FGFR3-containing fusion comprising the amino acid sequence corresponding to residues 1-760 of FGFR3 (e.g. SEQ ID NO: 90) fused to the amino acid sequence corresponding residues 549-838 of TACC3 (e.g. SEQ ID NO: 92). A FGFR fusion molecule can include a FGFR3-containing fusion comprising the amino acid sequence corresponding to residues 1-760 of FGFR3 (e.g. SEQ ID NO: 90) fused to the amino acid sequence corresponding residues 613-838 of TACC3 (e.g. SEQ ID NO: 92). A FGFR fusion molecule can include a FGFR3-containing fusion comprising the amino acid sequence corresponding to residues 1-760 of FGFR3 (e.g. SEQ ID NO: 90) fused to the amino acid sequence corresponding residues 488-838 of TACC3 (e.g. SEQ ID NO: 92). A FGFR fusion molecule can include a FGFR3-containing fusion comprising the amino acid sequence corresponding to residues 1-781 of FGFR3 (e.g. SEQ ID NO: 90) fused to the amino acid sequence corresponding residues 689-838 of TACC3 (e.g. SEQ ID NO: 92). A FGFR fusion molecule can include a FGFR3-containing fusion comprising the amino acid sequence corresponding to residues 1-765 of FGFR3 (e.g. SEQ ID NO: 90) fused to the amino acid sequence corresponding residues 583-838 of TACC3 (e.g. SEQ ID NO: 92). A FGFR fusion molecule can include a FGFR3-containing fusion comprising the amino acid sequence corresponding to residues 1-767 of FGFR3 (e.g. SEQ ID NO: 90) fused to the amino acid sequence corresponding residues 462-838 of TACC3 (e.g. SEQ ID NO: 92). A FGFR fusion molecule can include a FGFR3-containing fusion comprising the amino acid sequence corresponding to residues 1-767 of FGFR3 (e.g. SEQ ID NO: 90) fused to the amino acid sequence corresponding residues 472-838 of TACC3 (e.g. SEQ ID NO: 92). A FGFR fusion molecule can include a FGFR3-containing fusion comprising the amino acid sequence corresponding to residues 1-780 of FGFR3 (e.g. SEQ ID NO: 90) fused to the amino acid sequence corresponding residues 432-838 of TACC3 (e.g. SEQ ID NO: 92). A FGFR fusion molecule can include a FGFR1-containing fusion comprising the amino acid sequence corresponding to residues 1-762 of FGFR1 (e.g. SEQ ID NOS: 146, 185) fused to the amino acid sequence corresponding residues 571-805 of TACC1 (e.g. SEQ ID NO: 148). For example, a FGFR fusion molecule can include SEQ ID NOs: 539-543, 545, and 547. The alteration in a chromosome region occupied by a FGFR fusion molecule, e.g., a FGFR1-TACC1, FGFR2-TACC2, FGFR3-TACC3 or other FGFR-TACC nucleic acid, can result in amino acid substitutions, RNA splicing or processing, product instability, the creation of stop codons, production of oncogenic fusion proteins, frame-shift mutations, and/or truncated polypeptide production. A FGFR fusion molecule can include FGFR and TACC exons joined in the fused mRNA or cDNA. A FGFR fusion molecule can also include FGFR and TACC exons joined in the fused mRNA or cDNA along with the presence of FGFR or TACC introns that are spliced in the fusion cDNA. FGFR or TACC introns can encode amino acids of the FGFR fusion molecule. For example, a FGFR fusion molecule can include a FGFR3-containing fusion comprising the amino acid sequence corresponding to residues 1-765 of FGFR3 (e.g. SEQ ID NO: 90) fused to a 22 amino acid sequence encoded by a TACC3 intron fused to the amino acid sequence corresponding to residues 583-838 of TACC3 (e.g. SEQ ID NO: 92). For example, a FGFR fusion molecule can include a FGFR3-containing fusion comprising the amino acid sequence corresponding to residues 1-767 of FGFR3 (e.g. SEQ ID NO: 90) fused to a 11 amino acid sequence encoded by a TACC3 intron fused to the amino acid sequence corresponding to residues 472-838 of TACC3 (e.g. SEQ ID NO: 92). For example, a FGFR fusion molecule can include SEQ ID NOs: 544 and 546.

A FGFR fusion protein can also include a fusion protein encoded by an FGFR3-TACC3 nucleic acid, wherein FGFR3-TACC3 comprises a combination of introns and exons 1-18 of FGFR3 located on human chromosome 4p16 spliced 5' to a combination of introns and exons 4-16 of TACC3 located on human chromosome 4p16, wherein a genomic breakpoint occurs in any one of introns or exons 1-18 of FGFR3 and any one of introns or exons 3-16 of TACC3. For example, a FGFR fusion protein can also include a fusion protein encoded by an FGFR3-TACC3 nucleic acid, wherein the nucleic acid comprises exons 1-17 of FGFR3 located on human chromosome 4p16 spliced 5' to exons 11-16 of TACC3 located on human chromosome 4p16. In one embodiment, a genomic breakpoint occurs in intron 17 of FGFR3 and in intron 10 of TACC3. For example, a FGFR fusion protein can also include a fusion protein encoded by an FGFR3-TACC3 nucleic acid, wherein the nucleic acid comprises exons 1-17 of FGFR3 located on human chromosome 4p16 spliced 5' to exons 8-16 of TACC3 located on human chromosome 4p16. In one embodiment, a genomic breakpoint occurs in intron 17 of FGFR3 and in intron 7 of TACC3. For example, a FGFR fusion protein can also include a fusion protein encoded by an FGFR3-TACC3 nucleic acid, wherein the nucleic acid comprises exons 1-17 of FGFR3 located on human chromosome 4p16 spliced 5' to exons 10-16 of TACC3 located on human chromosome 4p16. In one embodiment, a genomic breakpoint occurs in exon 18 of FGFR3 and in intron 9 of TACC3. For example, a FGFR fusion protein can also include a fusion protein encoded by an FGFR3-TACC3 nucleic acid, wherein the nucleic acid comprises exons 1-17 of FGFR3 located on human chromosome 4p16 spliced 5' to exons 6-16 of TACC3 located on human chromosome 4p16. In one embodiment, a genomic breakpoint occurs in intron 17 of FGFR3 and in intron 5 of TACC3. In one embodiment, a genomic breakpoint occurs in intron 17 of FGFR3 and in exon 5 of TACC3. For example, a FGFR fusion protein can also include a fusion protein encoded by an FGFR3-TACC3 nucleic acid, wherein the nucleic acid comprises exons 1-18 of FGFR3 located on human chromosome 4p16 spliced 5' to exons 13-16 of TACC3 located on human chromosome 4p16. In one embodiment, a genomic breakpoint occurs in exon 18 of FGFR3 and in intron 12 or exon 13 of TACC3. For example, a FGFR fusion protein can also include a fusion protein encoded by an FGFR3-TACC3 nucleic acid, wherein the nucleic acid comprises exons 1-18 of FGFR3 located on human chromosome 4p16 spliced 5' to a portion of intron 8 of TACC3 and exons 9-16 of TACC3 located on human chromosome 4p16. In one embodiment, a genomic breakpoint occurs in exon 18 of FGFR3 and in intron 8 of TACC3. For example, a FGFR fusion protein can also include a fusion protein encoded by an FGFR3-TACC3 nucleic acid, wherein the nucleic acid comprises exons 1-18 of FGFR3 located on human chromosome 4p16 spliced 5' to exons 5-16 of TACC3 located on human chromosome 4p16. In one embodiment, a genomic breakpoint occurs in exon 18 of FGFR3 and in intron 4 or exon 5 of TACC3. For example, a FGFR fusion protein can also include a fusion protein encoded by an FGFR3-TACC3 nucleic acid, wherein the nucleic acid comprises exons 1-18 of FGFR3 located on human chromosome 4p16 spliced 5' to a portion of intron 4 of TACC 3 and exons 5-16 of TACC3 located on human chromosome 4p16. In one embodiment, a genomic breakpoint occurs in exon 18 of FGFR3 and in intron 4 or exon 5 of TACC3. For example, a FGFR fusion protein can also include a fusion protein encoded by an FGFR3-TACC3 nucleic acid, wherein the nucleic acid comprises exons 1-18 of FGFR3 located on human chromosome 4p16 spliced 5' to exons 4-16 of TACC3 located on human chromosome 4p16. In one embodiment, a genomic breakpoint occurs in exon 18 of FGFR3 and in intron 3 or exon 4 of TACC3. For example, a FGFR fusion protein can also include a fusion protein encoded by an FGFR3-TACC3 nucleic acid, wherein the nucleic acid comprises exons 1-17 of FGFR3 and a portion of intron 17 of FGFR3 located on human chromosome 4p16 spliced 5' to exons 9-16 of TACC3 located on human chromosome 4p16. In one embodiment, a genomic breakpoint occurs in intron 17 of FGFR3 and in exon 9 of TACC3. For example, a FGFR fusion protein can also include a fusion protein encoded by an FGFR1-TACC1 nucleic acid, wherein the nucleic acid comprises exons 1-17 of FGFR1 located on human chromosome 8p11 spliced 5' to exons 7-13 of TACC1 located on human chromosome 8p11. In one embodiment, a genomic breakpoint occurs in intron 17 or exon 17 of FGFR1 and in intron 6 or exon 7 of TACC1.

In one embodiment, the coordinates comprising FGFR3 translocations comprise chr4:1,795,039-1,810,599. In a further embodiment, the genomic breakpoint coordinate according to the genome build GRCh37/hg19 for FGFR3 is chr4:1,808,808, chr4:1,808,843, chr4:1,809,083, chr4:1,808,785, chr4:1,808,700, chr4:1,808,864, chr4:1,808,678, chr4:1, 808,798, or chr4:1,808,723. In a further embodiment, the coordinates comprising TACC3 translocations comprise chr4:1,723,217-1,746,905. In a further embodiment, the genomic breakpoint coordinate according to the genome build GRCh37/hg19 for TACC3 is chr4:1,732,648, chr4:1,732,757, chr4:1,739,187, chr4:1,737,091, chr4:1,737,062, chr4:1,737741, chr4:1,739,662, chr4:1,739,600, or chr4:1,738,989.

The nucleic acid can be any type of nucleic acid, including genomic DNA, complementary DNA (cDNA), recombinant DNA, synthetic or semi-synthetic DNA, as well as any form of corresponding RNA. A cDNA is a form of DNA artificially synthesized from a messenger RNA template and is used to produce gene clones. A synthetic DNA is free of modifications that can be found in cellular nucleic acids, including, but not limited to, histones and methylation. For example, a nucleic acid encoding a FGFR fusion molecule can comprise a recombinant nucleic acid encoding such a protein. The nucleic acid can be a non-naturally occurring nucleic acid created artificially (such as by assembling, cutting, ligating or amplifying sequences). It can be double-stranded or single-stranded.

The invention further provides for nucleic acids that are complementary to a FGFR fusion molecule. Complementary nucleic acids can hybridize to the nucleic acid sequence described above under stringent hybridization conditions. Non-limiting examples of stringent hybridization conditions include temperatures above 30°C, above 35°C, in excess of 42°C, and/or salinity of less than about 500 mM, or less than 200 mM. Hybridization conditions can be adjusted by the skilled artisan via modifying the temperature, salinity and/or the concentration of other reagents such as SDS or SSC.

According to the invention, protein variants can include amino acid sequence modifications. For example, amino acid sequence modifications fall into one or more of three classes: substitutional, insertional or deletional variants. Insertions can include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. These variants ordinarily are prepared by site-specific mutagenesis of nucleotides in the DNA encoding the protein, thereby producing DNA encoding the variant, and thereafter expressing the DNA in recombinant cell culture.

In one embodiment, a FGFR fusion molecule comprises a protein or polypeptide encoded by a nucleic acid sequence encoding a FGFR fusion molecule, such as the sequences shown in SEQ ID NOS: 80-82, 84, 94-145, 515, 517, 519-527, or 530-538. In some embodiments, the nucleic acid sequence encoding a FGFR fusion molecule is about 70%, about 75%, about 80%, about 85%, about 90%, about 93%, about 95%, about 97%, about 98%, or about 99% identical to SEQ ID NOS: 80-82, 84, 94-145, 515, 517, 519-527, or 530-538. In another embodiment, the polypeptide can be modified, such as by glycosylations and/or acetylations and/or chemical reaction or coupling, and can contain one or several non-natural or synthetic amino acids. An example of a FGFR fusion molecule is the polypeptide having the amino acid sequence shown in SEQ ID NOS: 79, 88, 150, 158-161, or 539-547. In some embodiments, the FGFR fusion molecule that is a polypeptide is about 70%, about 75%, about 80%, about 85%, about 90%, about 93%, about 95%, about 97%, about 98%, or about 99% identical to SEQ ID NOS: 79, 88, 150, 158-161, or 539-547. In another embodiment, a FGFR fusion molecule can be a fragment of a FGFR fusion protein. For example, the FGFR fusion molecule can encompass any portion of at least about 8 consecutive amino acids of SEQ ID NOS: 79, 88, 150, 158-161, or 539-547. The fragment can comprise at least about 10 amino acids, a least about 20 amino acids, at least about 30 amino acids, at least about 40 amino acids, a least about 50 amino acids, at least about 60 amino acids, or at least about 75 amino acids of SEQ ID NOS: 79, 88, 150, 158-161, or 539-547. Fragments include all possible amino acid lengths between about 8 and 100 about amino acids, for example, lengths between about 10 and 100 amino acids, between about 15 and 100 amino acids, between about 20 and 100 amino acids, between about 35 and 100 amino acids, between about 40 and 100 amino acids, between about 50 and 100 amino acids, between about 70 and 100 amino acids, between about 75 and 100 amino acids, or between about 80 and 100 amino acids. Fragments include all possible amino acid lengths between about 100 and 800 amino acids, for example, lengths between about 125 and 800 amino acids, between about 150 and 800 amino acids, between about 175 and 800 amino acids, between about 200 and 800 amino acids, between about 225 and 800 amino acids, between about 250 and 800 amino acids, between about 275 and 800 amino acids, between about 300 and 800 amino acids, between about 325 and 800 amino acids, between about 350 and 800 amino acids, between about 375 and 800 amino acids, between about 400 and 800 amino acids, between about 425 and 800 amino acids, between about 450 and 800 amino acids, between about 475 and 800 amino acids, between about 500 and 800 amino acids, between about 525 and 800 amino acids, between about 550 and 800 amino acids, between about 575 and 800 amino acids, between about 600 and 800 amino acids, between about 625 and 800 amino acids, between about 650 and 800 amino acids, between about 675 and 800 amino acids, between about 700 and 800 amino acids, between about 725 and 800 amino acids, between about 750 and 800 amino acids, or between about 775 and 800 amino acids.

Chemical Synthesis. Nucleic acid sequences encoding a FGFR fusion molecule can be synthesized, in whole or in part, using chemical methods known in the art. Alternatively, a polypeptide can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer).

Optionally, polypeptides fragments can be separately synthesized and combined using chemical methods to produce a full-length molecule. For example, these methods can be utilized to synthesize a fusion protein of the invention. In one embodiment, the fusion protein comprises a tyrosine kinase domain of an FGFR protein fused to a polypeptide that constitutively activates the tyrosine kinase domain of the FGFR protein. In another embodiment, a fusion protein comprises a transforming acidic coiled-coil (TACC) domain fused to a polypeptide with a tyrosine kinase domain, wherein the TACC domain constitutively activates the tyrosine kinase domain. An example of a fusion protein is the FGFR1-TACC1 polypeptide, which comprises the amino acid sequence shown in SEQ ID NO: 150. An example of a fusion protein is the FGFR3-TACC3 polypeptide, which has the amino acid sequence comprising SEQ ID NO: 79, 158, 159, 160, 161,539, 540, 541, 542, 543, 544, 545, 546, or 547.

Obtaining, Purifying and Detecting FGFR fusion molecules. A polypeptide encoded by a nucleic acid, such as a nucleic acid encoding a FGFR fusion molecule, or a variant thereof, can be obtained by purification from human cells expressing a protein or polypeptide encoded by such a nucleic acid. Non-limiting purification methods include size exclusion chromatography, ammonium sulfate fractionation, ion exchange chromatography, affinity chromatography, and preparative gel electrophoresis.

A synthetic polypeptide can be substantially purified via high performance liquid chromatography (HPLC), such as ion exchange chromatography (IEX-HPLC). The composition of a synthetic polypeptide, such as a FGFR fusion molecule, can be confirmed by amino acid analysis or sequencing.

Other constructions can also be used to join a nucleic acid sequence encoding a polypeptide/protein of the claimed invention to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Including cleavable linker sequences (i.e., those specific for Factor Xa or enterokinase (Invitrogen, San Diego, Calif.)) between the purification domain and a polypeptide encoded by a nucleic acid of the invention also can be used to facilitate purification. For example, the skilled artisan can use an expression vector encoding 6 histidine residues that precede a thioredoxin or an enterokinase cleavage site in conjunction with a nucleic acid of interest. The histidine residues facilitate purification by immobilized metal ion affinity chromatography, while the enterokinase cleavage site provides a means for purifying the polypeptide encoded by, for example, an FGFR1-TACC1, FGFR2-TACC2, FGFR3-TACC3, other FGFR-TACC, FGFR-containing, or TACC containing nucleic acid.

Host cells which contain a nucleic acid encoding a FGFR fusion molecule, and which subsequently express the same, can be identified by various procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a nucleic acid encoding a FGFR fusion molecule can be detected by DNA-DNA or DNA-RNA hybridization or amplification using probes or fragments of nucleic acids encoding the same. In one embodiment, a nucleic acid fragment of a FGFR fusion molecule can encompass any portion of at least about 8 consecutive nucleotides of SEQ ID NOS: 80-82, 84, 94-145, 515, 517, 519-527, or 530-538. In another embodiment, the fragment can comprise at least about 10 consecutive nucleotides, at least about 15 consecutive nucleotides, at least about 20 conseutive nucleotides, or at least about 30 consecutive nucleotides of SEQ ID NOS: 80-82, 84, 94-145, 515, 517, 519-527, 530-538. Fragments can include all possible nucleotide lengths between about 8 and about 100 nucleotides, for example, lengths between about 15 and about 100 nucleotides, or between about 20 and about 100 nucleotides. Nucleic acid amplification-based assays involve the use of oligonucleotides selected from sequences encoding a FGFR fusion molecule nucleic acid, or FGFR fusion molecule nucleic acid to detect transformants which contain a nucleic acid encoding a protein or polypeptide of the same. Protocols are known in the art for detecting and measuring the expression of a polypeptide encoded by a nucleic acid, such as a nucleic acid encoding a FGFR fusion molecule, using either polyclonal or monoclonal antibodies specific for the polypeptide. Non-limiting examples include enzyme-linked immunosorbent assay (ELISA), radioimmunoassay (RIA), immunostaining, and fluorescence activated cell sorting (FACS). A two-site, monoclonal-based immunoassay using monoclonal antibodies reactive to two non-interfering epitopes on a polypeptide encoded by a nucleic acid, such as a nucleic acid encoding a FGFR fusion molecule, can be used, or a competitive binding assay can be employed.

Labeling and conjugation techniques are known by those skilled in the art and can be used in various nucleic acid and amino acid assays. Methods for producing labeled hybridization or PCR probes for detecting sequences related to nucleic acid sequences encoding a protein, such as FGFR fusion molecule, include, but are not limited to, oligolabeling, nick translation, end-labeling, or PCR amplification using a labeled nucleotide. Alternatively, nucleic acid sequences, such as nucleic acids encoding a FGFR fusion molecule, can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, and/or magnetic particles.

A fragment can be a fragment of a protein, such as a FGFR fusion protein. For example, a fragment of a FGFR fusion molecule can encompass any portion of at least about 8 consecutive amino acids of SEQ ID NOS: 79, 88, 150, 158-161, or 539-547. The fragment can comprise at least about 10 consecutive amino acids, at least about 20 consecutive amino acids, at least about 30 consecutive amino acids, at least about 40 consecutive amino acids, a least about 50 consecutive amino acids, at least about 60 consecutive amino acids, at least about 70 consecutive amino acids, at least about 75 consecutive amino acids, at least about 80 consecutive amino acids, at least about 85 consecutive amino acids, at least about 90 consecutive amino acids, at least about 95 consecutive amino acids, at least about 100 consecutive amino acids, at least about 200 consecutive amino acids, at least about 300 consecutive amino acids, at least about 400 consecutive amino acids, at least about 500 consecutive amino acids, at least about 600 consecutive amino acids, at least about 700 consecutive amino acids, or at least about 800 consecutive amino acids of SEQ ID NOS: 79, 88, 150, 158-161, or 539-547. Fragments include all possible amino acid lengths between about 8 and 100 about amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids.

### Cell Transfection

Host cells transformed with a nucleic acid sequence of interest can be cultured under conditions suitable for the expression and recovery of the protein from cell culture. The polypeptide produced by a transformed cell can be secreted or contained intracellularly depending on the sequence and/or the vector used. Expression vectors containing a nucleic acid sequence, such as a nucleic acid encoding a FGFR fusion molecule, can be designed to contain signal sequences which direct secretion of soluble polypeptide molecules encoded by the nucleic acid. Cell transfection and culturing methods are described in more detail below.

A eukaryotic expression vector can be used to transfect cells in order to produce proteins encoded by nucleotide sequences of the vector, e.g. those encoding a FGFR fusion molecule. Mammalian cells can contain an expression vector (for example, one that contains a nucleic acid encoding a fusion protein comprising a tyrosine kinase domain of an FGFR protein fused to a polypeptide that constitutively activates the tyrosine kinase domain of the FGFR protein, or a nucleic acid encoding a fusion protein comprises a transforming acidic coiled-coil (TACC) domain fused to a polypeptide with a tyrosine kinase domain, wherein the TACC domain constitutively activates the tyrosine kinase domain) via introducing the expression vector into an appropriate host cell via methods known in the art.

A host cell strain can be chosen for its ability to modulate the expression of the inserted sequences or to process the expressed polypeptide encoded by a nucleic acid, in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, Va. 20110-2209) and can be chosen to ensure the correct modification and processing of the foreign protein.

An exogenous nucleic acid can be introduced into a cell via a variety of techniques known in the art, such as lipofection, microinjection, calcium phosphate or calcium chloride precipitation, DEAE-dextran-mediated transfection, or electroporation. Electroporation is carried out at approximate voltage and capacitance to result in entry of the DNA construct(s) into cells of interest (such as glioma cells (cell line SF188), neuroblastoma cells (cell lines IMR-32, SK-N-SH, SH-F and SH-N), astrocytes and the like). Other transfection methods also include modified calcium phosphate precipitation, polybrene precipitation, liposome fusion, and receptor-mediated gene delivery.

Cells that will be genetically engineered can be primary and secondary cells obtained from various tissues, and include cell types which can be maintained and propagated in culture. Non-limiting examples of primary and secondary cells include epithelial cells, neural cells, endothelial cells, glial cells, fibroblasts, muscle cells (such as myoblasts) keratinocytes, formed elements of the blood (e.g., lymphocytes, bone marrow cells), and precursors of these somatic cell types.

Vertebrate tissue can be obtained by methods known to one skilled in the art, such a punch biopsy or other surgical methods of obtaining a tissue source of the primary cell type of interest. In one embodiment, a punch biopsy or removal (e.g., by aspiration) can be used to obtain a source of cancer cells (for example, glioma cells, neuroblastoma cells, and the like). A mixture of primary cells can be obtained from the tissue, using methods readily practiced in the art, such as explanting or enzymatic digestion (for examples using enzymes such as pronase, trypsin, collagenase, elastase dispase, and chymotrypsin). Biopsy methods have also been described in United States Patent No. 7,419,661 and PCT application publication WO 2001/32840, and each are hereby incorporated by reference.

Primary cells can be acquired from the individual to whom the genetically engineered primary or secondary cells are administered. However, primary cells can also be obtained from a donor, other than the recipient, of the same species. The cells can also be obtained from another species (for example, rabbit, cat, mouse, rat, sheep, goat, dog, horse, cow, bird, or pig). Primary cells can also include cells from a purified vertebrate tissue source grown attached to a tissue culture substrate (for example, flask or dish) or grown in a suspension; cells present in an explant derived from tissue; both of the aforementioned cell types plated for the first time; and cell culture suspensions derived from these plated cells. Secondary cells can be plated primary cells that are removed from the culture substrate and replated, or passaged, in addition to cells from the subsequent passages. Secondary cells can be passaged one or more times. These primary or secondary cells can contain expression vectors having a gene that encodes a FGFR fusion molecule.

### Cell Culturing

Various culturing parameters can be used with respect to the host cell being cultured. Appropriate culture conditions for mammalian cells are well known in the art (Cleveland WL, et al., J Immunol Methods, 1983, 56(2): 221-234) or can be determined by the skilled artisan (see, for example, Animal Cell Culture: A Practical Approach 2nd Ed., Rickwood, D. and Hames, B. D., eds. (Oxford University Press: New York, 1992)). Cell culturing conditions can vary according to the type of host cell selected. Commercially available medium can be utilized. Non-limiting examples of medium include, for example, Minimal Essential Medium (MEM, Sigma, St. Louis, Mo.); Dulbecco's Modified Eagles Medium (DMEM, Sigma); Ham's F10 Medium (Sigma); HyClone cell culture medium (HyClone, Logan, Utah); RPMI-1640 Medium (Sigma); and chemically-defined (CD) media, which are formulated for various cell types, e.g., CD-CHO Medium (Invitrogen, Carlsbad, Calif.).

The cell culture media can be supplemented as necessary with supplementary components or ingredients, including optional components, in appropriate concentrations or amounts, as necessary or desired. Cell culture medium solutions provide at least one component from one or more of the following categories: (1) an energy source, usually in the form of a carbohydrate such as glucose; (2) all essential amino acids, and usually the basic set of twenty amino acids plus cysteine; (3) vitamins and/or other organic compounds required at low concentrations; (4) free fatty acids or lipids, for example linoleic acid; and (5) trace elements, where trace elements are defined as inorganic compounds or naturally occurring elements that can be required at very low concentrations, usually in the micromolar range.

The medium also can be supplemented electively with one or more components from any of the following categories: (1) salts, for example, magnesium, calcium, and phosphate; (2) hormones and other growth factors such as, serum, insulin, transferrin, and epidermal growth factor; (3) protein and tissue hydrolysates, for example peptone or peptone mixtures which can be obtained from purified gelatin, plant material, or animal byproducts; (4) nucleosides and bases such as, adenosine, thymidine, and hypoxanthine; (5) buffers, such as HEPES; (6) antibiotics, such as gentamycin or ampicillin; (7) cell protective agents, for example pluronic polyol; and (8) galactose. In one embodiment, soluble factors can be added to the culturing medium.

The mammalian cell culture that can be used with the present invention is prepared in a medium suitable for the type of cell being cultured. In one embodiment, the cell culture medium can be any one of those previously discussed (for example, MEM) that is supplemented with serum from a mammalian source (for example, fetal bovine serum (FBS)). In another embodiment, the medium can be a conditioned medium to sustain the growth of host cells.

Three-dimensional cultures can be formed from agar (such as Gey's Agar), hydrogels (such as matrigel, agarose, and the like; Lee et al., (2004) Biomaterials 25: 2461-2466) or polymers that are cross-linked. These polymers can comprise natural polymers and their derivatives, synthetic polymers and their derivatives, or a combination thereof. Natural polymers can be anionic polymers, cationic polymers, amphipathic polymers, or neutral polymers. Non-limiting examples of anionic polymers can include hyaluronic acid, alginic acid (alginate), carageenan, chondroitin sulfate, dextran sulfate, and pectin. Some examples of cationic polymers, include but are not limited to, chitosan or polylysine. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73). Examples of amphipathic polymers can include, but are not limited to collagen, gelatin, fibrin, and carboxymethyl chitin. Non-limiting examples of neutral polymers can include dextran, agarose, or pullulan. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73).

Cells to be cultured can harbor introduced expression vectors, such as plasmids. The expression vector constructs can be introduced via transformation, microinjection, transfection, lipofection, electroporation, or infection. The expression vectors can contain coding sequences, or portions thereof, encoding the proteins for expression and production. Expression vectors containing sequences encoding the produced proteins and polypeptides, as well as the appropriate transcriptional and translational control elements, can be generated using methods well known to and practiced by those skilled in the art. These methods include synthetic techniques, *in vitro* recombinant DNA techniques, and *in vivo* genetic recombination which are described in J. Sambrook et al., 2001, Molecular Cloning, A Laboratory Manual, Cold Spring Harbor Press, Plainview, N.Y. and in F. M. Ausubel et al., 1989, Current Protocols in Molecular Biology, John Wiley & Sons, New York, N.Y.

### FGFR Fusion Molecule Inhibitors

The invention provides methods for use of compounds that decrease the expression level or activity of a FGFR fusion molecule in a subject. In addition, the invention provides methods for using compounds for the treatment of a gene-fusion associated cancer. In one embodiment, the gene-fusion associated cancer is an epithelial cancer. In one embodiment, the gene-fusion associated cancer comprises glioblastoma multiforme, breast cancer, lung cancer, prostate cancer, or colorectal carcinoma.

As used herein, a "FGFR fusion molecule inhibitor" refers to a compound that interacts with a FGFR fusion molecule of the invention and modulates its activity and/or its expression. For example, the compound can decrease the activity or expression of a FGFR fusion molecule. The compound can be an antagonist of a FGFR fusion molecule (e.g., a FGFR fusion molecule inhibitor). Some non-limiting examples of FGFR fusion molecule inhibitors include peptides (such as peptide fragments comprising a FGFR fusion molecule, or antibodies or fragments thereof), small molecules, and nucleic acids (such as siRNA or antisense RNA specific for a nucleic acid comprising a FGFR fusion molecule). Antagonists of a FGFR fusion molecule decrease the amount or the duration of the activity of an FGFR fusion protein. In one embodiment, the fusion protein comprises a tyrosine kinase domain of an FGFR protein fused to a polypeptide that constitutively activates the tyrosine kinase domain of the FGFR protein (e.g., FGFR1-TACC1, FGFR2-TACC2, FGFR3-TACC3 or other FGFR-TACC), or a fusion protein comprises a transforming acidic coiled-coil (TACC) domain fused to a polypeptide with a tyrosine kinase domain, wherein the TACC domain constitutively activates the tyrosine kinase domain. Antagonists include proteins, nucleic acids, antibodies, small molecules, or any other molecule which decrease the activity of a FGFR fusion molecule.

The term "modulate," as it appears herein, refers to a change in the activity or expression of a FGFR fusion molecule. For example, modulation can cause a decrease in protein activity, binding characteristics, or any other biological, functional, or immunological properties of a FGFR fusion molecule, such as an FGFR fusion protein.

In one embodiment, a FGFR fusion molecule inhibitor can be a peptide fragment of a FGFR fusion protein that binds to the protein itself.

For example, the FGFR fusion polypeptide can encompass any portion of at least about 8 consecutive amino acids of SEQ ID NOS: 79, 88, 150, 158-161, or 539-547. The fragment can comprise at least about 10 consecutive amino acids, at least about 20 consecutive amino acids, at least about 30 consecutive amino acids, at least about 40 consecutive amino acids, a least about 50 consecutive amino acids, at least about 60 consecutive amino acids, at least about 70 consecutive amino acids, at least about 75 consecutive amino acids, at least about 80 consecutive amino acids, at least about 85 consecutive amino acids, at least about 90 consecutive amino acids, at least about 95 consecutive amino acids, at least about 100 consecutive amino acids, at least about 200 consecutive amino acids, at least about 300 consecutive amino acids, at least about 400 consecutive amino acids, at least about 500 consecutive amino acids, at least about 600 consecutive amino acids, at least about 700 consecutive amino acids, or at least about 800 consecutive amino acids of SEQ ID NOS: 79, 88, 150, 158-161, or 539-547. Fragments include all possible amino acid lengths between about 8 and 100 about amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England). The FGFR fusion peptide fragments can be isolated from a natural source, genetically engineered, or chemically prepared. These methods are well known in the art.

A FGFR fusion molecule inhibitor can be a protein, such as an antibody (monoclonal, polyclonal, humanized, chimeric, or fully human), or a binding fragment thereof, directed against a FGFR fusion moleculeof the invention. An antibody fragment can be a form of an antibody other than the full-length form and includes portions or components that exist within full-length antibodies, in addition to antibody fragments that have been engineered. Antibody fragments can include, but are not limited to, single chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like *(see,* Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (*see* United States Patent Nos. 6,914,128, 5,780,597, 5,811,523; Roland E. Kontermann and Stefan Dübel (editors), Antibody Engineering, Vol. I & II, (2010) 2nd ed., Springer; Antony S. Dimitrov (editor), Therapeutic Antibodies: Methods and Protocols (Methods in Molecular Biology), (2009), Humana Press; Benny Lo (editor) Antibody Engineering: Methods and Protocols (Methods in Molecular Biology), (2004) Humana Press, each of which are hereby incorporated by reference in their entireties). For example, antibodies directed to a FGFR fusion molecule can be obtained commercially from Abcam, Santa Cruz Biotechnology, Abgent, R&D Systems, Novus Biologicals, etc. Human antibodies directed to a FGFR fusion molecule (such as monoclonal, humanized, fully human, or chimeric antibodies) can be useful antibody therapeutics for use in humans. In one embodiment, an antibody or binding fragment thereof is directed against SEQ ID NOS: 79, 88, 150, 158-161, or 539-547. Inhibition of RNA encoding a FGFR fusion molecule can effectively modulate the expression of a FGFR fusion molecule. Inhibitors are selected from the group comprising: siRNA; interfering RNA or RNAi; dsRNA; RNA Polymerase III transcribed DNAs; ribozymes; and antisense nucleic acids, which can be RNA, DNA, or an artificial nucleic acid.

Antisense oligonucleotides, including antisense DNA, RNA, and DNA/RNA molecules, act to directly block the translation of mRNA by binding to targeted mRNA and preventing protein translation. For example, antisense oligonucleotides of at least about 15 bases and complementary to unique regions of the DNA sequence encoding a FGFR fusion molecule can be synthesized, e.g., by conventional phosphodiester techniques (Dallas et al., (2006) Med. Sci. Monit.12(4):RA67-74; Kalota et al., (2006) Handb. Exp. Pharmacol. 173:173-96; Lutzelburger et al., (2006) Handb. Exp. Pharmacol. 173:243-59). Antisense nucleotide sequences include, but are not limited to: morpholinos, 2'-O-methyl polynucleotides, DNA, RNA and the like.

siRNA comprises a double stranded structure containing from about 15 to about 50 base pairs, for example from about 21 to about 25 base pairs, and having a nucleotide sequence identical or nearly identical to an expressed target gene or RNA within the cell. The siRNA comprise a sense RNA strand and a complementary antisense RNA strand annealed together by standard Watson-Crick base-pairing interactions. The sense strand comprises a nucleic acid sequence which is substantially identical to a nucleic acid sequence contained within the target miRNA molecule. "Substantially identical" to a target sequence contained within the target mRNA refers to a nucleic acid sequence that differs from the target sequence by about 3% or less. The sense and antisense strands of the siRNA can comprise two complementary, single-stranded RNA molecules, or can comprise a single molecule in which two complementary portions are base-paired and are covalently linked by a single-stranded "hairpin" area. See also, McMnaus and Sharp (2002) Nat Rev Genetics, 3:737-47, and Sen and Blau (2006) FASEB J., 20:1293-99, the entire disclosures of which are herein incorporated by reference.

The siRNA can be altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, or modifications that make the siRNA resistant to nuclease digestion, or the substitution of one or more nucleotides in the siRNA with deoxyribo-nucleotides. One or both strands of the siRNA can also comprise a 3' overhang. As used herein, a 3' overhang refers to at least one unpaired nucleotide extending from the 3'-end of a duplexed RNA strand. For example, the siRNA can comprise at least one 3' overhang of from 1 to about 6 nucleotides (which includes ribonucleotides or deoxyribonucleotides) in length, or from 1 to about 5 nucleotides in length, or from 1 to about 4 nucleotides in length, or from about 2 to about 4 nucleotides in length. For example, each strand of the siRNA can comprise 3' overhangs of dithymidylic acid ("TT") or diuridylic acid ("uu").

siRNA can be produced chemically or biologically, or can be expressed from a recombinant plasmid or viral vector (for example, see U.S. Patent No. 7,294,504 and U.S. Patent No. 7,422,896, the entire disclosures of which are herein incorporated by reference). Exemplary methods for producing and testing dsRNA or siRNA molecules are described in U.S. Patent Application Publication No. 2002/0173478 to Gewirtz, U.S. Patent No. 8,071,559 to Hannon et al., and in U.S. Patent No. 7,148,342 to Tolentino et al., the entire disclosures of which are herein incorporated by reference.

In one embodiment, an siRNA directed to a human nucleic acid sequence comprising a FGFR fusion molecule can be generated against any one of SEQ ID NOS: 80-82, 84, 94-145, 515, 517, 519-527 or 530-538. In another embodiment, an siRNA directed to a human nucleic acid sequence comprising a breakpoint of anFGFR fusion molecule can be generated against any one of SEQ ID NOS: 1-77, 80-82, 84-145, 515, 517, 519-527 or 530-538. In one embodiment, the hairpin sequences targeting the *FGFR3* gene comprise SEQ ID NOS: 182, 183, or 184.

RNA polymerase III transcribed DNAs contain promoters, such as the U6 promoter. These DNAs can be transcribed to produce small hairpin RNAs in the cell that can function as siRNA or linear RNAs, which can function as antisense RNA. The FGFR fusion molecule inhibitor can comprise ribonucleotides, deoxyribonucleotides, synthetic nucleotides, or any suitable combination such that the target RNA and/or gene is inhibited. In addition, these forms of nucleic acid can be single, double, triple, or quadruple stranded. (see for example Bass (2001) Nature, 411:428-429; Elbashir et al., (2001) Nature, 411:494 498; U.S. Patent No. 6,509,154; U.S. Patent Application Publication No. 2003/0027783; and PCT Publication Nos. WO 00/044895, WO 99/032619, WO 00/01846, WO 01/029058, WO 00/044914).

FGFR fusion molecule inhibitor can be a small molecule that binds to a FGFR fusion protein described herein and disrupts its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that inhibit a FGFR fusion protein can be identified via *in silico* screening or high-through-put (HTP) screening of combinatorial libraries according to methods established in the art (e.g., see Potyrailo et al., (2011) 4CS Comb Sci. 13(6):579-633; Mensch et al., (2009) J Pharm Sci. 98(12):4429-68; Schnur (2008) Curr Opin Drug Discov Devel. 11(3):375-80; and Jhoti (2007) Ernst Schering Found Symp Proc. (3):169-85, each of which are hereby incorporated by reference in their entireties.) Most conventional pharmaceuticals, such as aspirin, penicillin, and many chemotherapeutics, are small molecules, can be obtained commercially, can be chemically synthesized, or can be obtained from random or combinatorial libraries as described below (*see,* e.g., Werner et al., (2006) Brief Funct. Genomic Proteomic 5(1):32-6).

Non-limiting examples of FGFR fusion molecule inhibitors include the FGFR inhibitors AZD4547 (see Gavine et al., (2012) Cancer Res, 72(8); 2045-56; *see also* PCT Application Publication No. WO 2008/075068, each of which are hereby incorporated by reference in their entireties); NVP-BGJ398 (see Guagnano et al., (2011) J. Med. Chem., 54:7066-7083; *see also* U.S. Patent Application Publication No. 2008-0312248 A1, each of which are hereby incorporated by reference in their entireties); PD173074 (see Guagnano et al., (2011) J. Med. Chem., 54:7066-7083; see also Mohammadi et al., (1998) EMBO J., 17:5896-5904, each of which are hereby incorporated by reference in their entireties); NF449 (EMD Millipore (Billerica, MA) Cat. No. 480420; *see also* Krejci, (2010) the Journal of Biological Chemistry, 285(27):20644-20653, which is hereby incorporated by reference in its entirety); LY2874455 (Active Biochem; see Zhao et al. (2011) Mol Cancer Ther. (11):2200-10; *see also* PCT Application Publication No. WO 2010129509, each of which are hereby incorporated by reference in their entireties); TKI258 (Dovitinib); BIBF-1120 (Intedanib-Vargatef); BMS-582664 (Brivanib alaninate); AZD-2171 (Cediranib); TSU-68 (Orantinib); AB-1010 (Masitinib); AP-24534 (Ponatinib); and E-7080 (by Eisai). A non-limiting example of an FGFR fusion molecule inhibitor includes the TACC inhibitor KHS101 (Wurdak et al., (2010) PNAS, 107(38): 16542-47, which is hereby incorporated by reference in its entirety).

Structures of FGFR fusion molecule inhibitors useful for the invention include, but are not limited to: the FGFR inhibitor AZD4547, 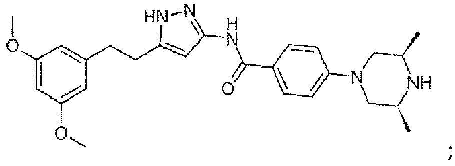 the FGFR inhibitor NVP-BGJ398, 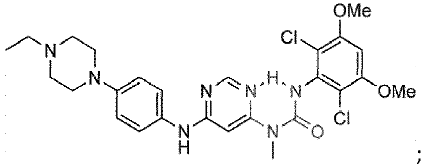 the FGFR inhibitor PD173074, 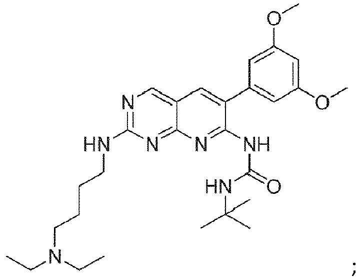 the FGFR inhibitor LY2874455 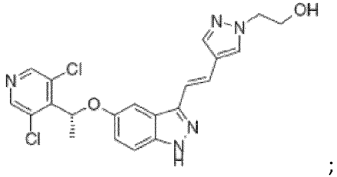 and the FGFR inhibitor NF449 (EMD Millipore (Billerica, MA) Cat. No. 480420),

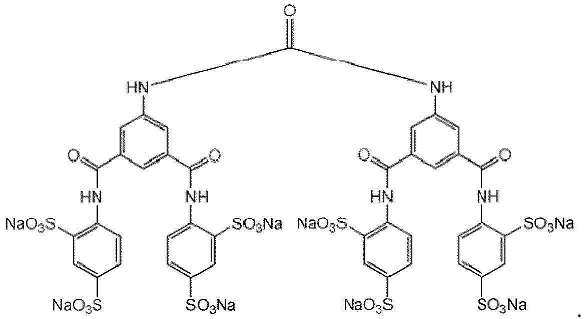

Other FGFR inhibitors include, but are not limited to:

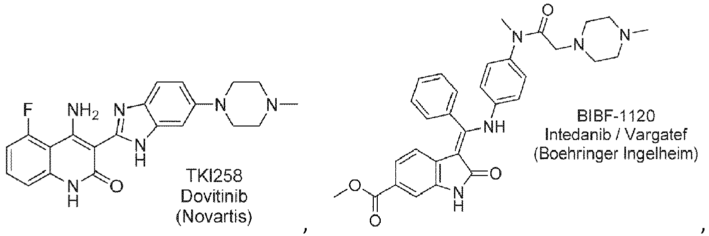

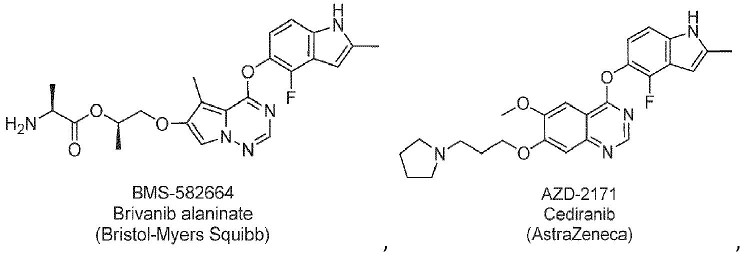

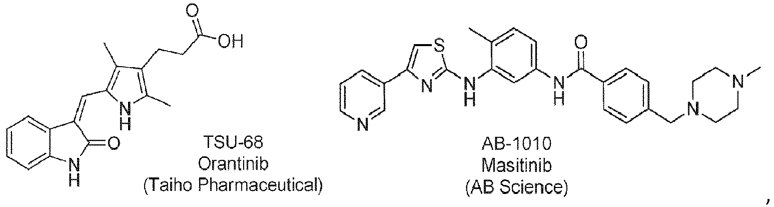

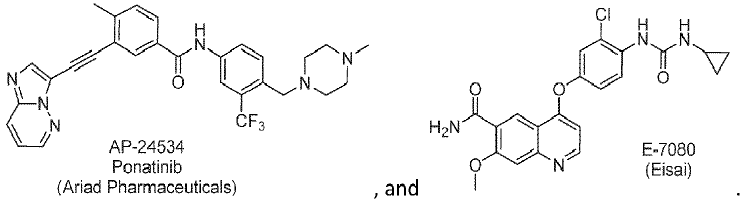

In other embodiments, the FGFR fusion molecule inhibitor comprises an oral pan-FGFR tyrosine kinase inhibitor. In other embodiments, the FGFR fusion molecule inhibitor comprises JNJ-42756493. Structures of FGFR fusion molecule inhibitors useful for the invention include, but are not limited to, the FGFR inhibitor JNJ-42756493.

A structure of an FGFR fusion molecule inhibitor useful for the invention include, but is not limited to the TACC inhibitor KHS101,

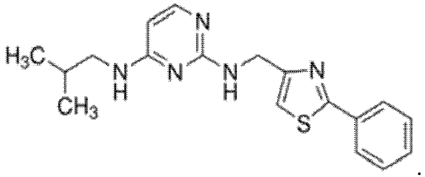

### Assessment and Therapuetic Treatment

The invention provides a method of decreasing the growth of a solid tumor in a subject. The tumor is associated with, but not limited to, glioblastoma multiforme, breast cancer, lung cancer, prostate cancer, or colorectal carcinoma. In another embodiment, the tumor is associated with, but not limited to, bladder carcinoma, squamous lung carcinoma and head and neck carcinoma. In one embodiment, the tumor is associated with, but not limited to, glioma. In one embodiment, the tumor is associated with, but not limited to, grade II or III glioma. In one embodiment, the tumor is associated with, but not limited to, IDH wild-type grade II or III glioma. In one embodiment, the method comprises detecting the presence of a FGFR fusion molecule in a sample obtained from a subject. In some embodiments, the sample is incubated with an agent that binds to an FGFR fusion molecule, such as an antibody, a probe, a nucleic acid primer, and the like. In further embodiments, the method comprises administering to the subject an effective amount of a FGFR fusion molecule inhibitor, wherein the inhibitor decreases the size of the solid tumor. In further embodiments, the method comprises further detecting the presence or absence of IDH1 mutations, IDH2 mutations, EGFR gene amplification, CDK4 gene amplification, or MDM2 gene amplification. In further embodiments, a FGFR fusion molecule inhibitor can be administered in combination with CDK4 inhibitors, MDM2 inhibitors, or a combination thereof.

The invention also provides a method for treating or preventing a gene-fusion associated cancer in a subject. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In some embodiments, the epithelial cancer comprises bladder urothelial carcinoma, breast carcinoma, colorectal cancer, prostate carcinoma, lung squamous cell carcinoma, head and neck squamous cell carcinoma, or a combination of the epithelial cancers decribed.

In one embodiment, the gene-fusion associated cancer comprises glioma. In one embodiment, the gene-fusion associated cancer comprises grade II or III glioma. In one embodiment, the gene-fusion associated cancer comprises IDH wild-type grade II or III glioma. In one embodiment, the method comprises detecting the presence of a FGFR fusion molecule in a sample obtained from a subject, the presence of the fusion being indicative of a gene-fusion associated cancer, and, administering to the subject in need a therapeutic treatment against a gene-fusion associated cancer. In some embodiments, the sample is incubated with an agent that binds to an FGFR fusion molecule, such as an antibody, a probe, a nucleic acid primer, and the like. In further embodiments, the method comprises further detecting the presence or absence of IDH1 mutations, IDH2 mutations, EGFR gene amplification, CDK4 gene amplification, or MDM2 amplification. In further embodiments, an agent that binds to an FGFR fusion molecule can be administered in combination with CDK4 inhibitors, MDM2 inhibitors, or a combination thereof.

The invention also provides a method for decreasing in a subject in need thereof the expression level or activity of a fusion protein comprising the tyrosine kinase domain of an FGFR protein fused to a polypeptide that constitutively activates the tyrosine kinase domain of the FGFR protein. In some embodiments, the method comprises obtaining a biological sample from the subject. In some embodiments, the sample is incubated with an agent that binds to an FGFR fusion molecule, such as an antibody, a probe, a nucleic acid primer, and the like. In some embodiments, the method comprises administering to the subject a therapeutic amount of a composition comprising an admixture of a pharmaceutically acceptable carrier an inhibitor of the fusion protein of the invention. In one embodiment, the inhibitor is JNJ-42756493. In another embodiment, the method further comprises determining the fusion protein expression level or activity. In another embodiment, the method further comprises detecting whether the fusion protein expression level or activity is decreased as compared to the fusion protein expression level or activity prior to administration of the composition, thereby decreasing the expression level or activity of the fusion protein. In some embodiments, the fusion protein is an FGFR-TACC fusion protein. In further embodiments, the method comprises further detecting the presence or absence of IDH1 mutations, IDH2 mutations, EGFR amplification, CDK4 amplification, or MDM2 amplification.

The administering step in each of the claimed methods can comprise a drug administration, such as FGFR fusion molecule inhibitor (for example, a pharmaceutical composition comprising an antibody that specifically binds to a FGFR fusion molecule or a fragment thereof; a small molecule that specifically binds to a FGFR protein; a small molecule that specifically binds to a TACC protein; an antisense RNA or antisense DNA that decreases expression of a FGFR fusion molecule; a siRNA that specifically targets a gene encoding a FGFR fusion molecule; a small molecule such as JNJ-42756493; or a combination thereof). In one embodiment, the therapeutic molecule to be administered comprises a polypeptide of a FGFR fusion molecule, comprising at least about 75%, at least about 80%, at least about 85%, at least about 90%, at least about 93%, at least about 95%, at least about 97%, at least about 98%, at least about 99%, or 100% of the amino acid sequence of SEQ ID NOS: 79, 88, 150, 158-161, or 539-547 and exhibits the function of decreasing expression of such a protein, thus treating a gene fusion-associated cancer. In another embodiment, administration of the therapeutic molecule decreases the size of the solid tumor associated with glioblastoma multiforme, breast cancer, lung cancer, prostate cancer, colorectal carcinoma, bladder carcinoma, squamous lung carcinoma and head and neck carcinoma, glioma, grade II or III glioma, or IDH wild-type grade II or III glioma. In further embodiments, the therapeutic molecule can be administered in combination with CDK4 inhibitors, MDM2 inhibitors, or a combination thereof.

In another embodiment, the therapeutic molecule to be administered comprises an siRNA directed to a human nucleic acid sequence comprising a FGFR fusion molecule. In one embodiment, the siRNA is directed to any one of SEQ ID NOS: 80-82, 84, 94-145, 515, 517, 519-527, or 530-538. In another embodiment, the siRNA is directed to any one of SEQ ID NOS: 1-77, 80-82, 84-145, 515, 517, 519-527, or 530-538. In a further embodiment, the therapeutic molecule to be administered comprises an antibody or binding fragment thereof, which is directed against SEQ ID NOS: 79, 88, 150, 158-161, or 539-547. In some embodiments, the therapeutic molecule to be administered comprises a small molecule that specifically binds to a FGFR protein, such as AZD4547, NVP-BGJ398, PD173074, NF449, TK1258, BIBF-1120, BMS-582664, AZD-2171, TSU68, AB1010, AP24534, E-7080, or LY2874455. In some embodiments, the therapeutic molecule to be administered is JNJ-42756493. In other embodiments, the therapeutic molecule to be administered comprises a small molecule that specifically binds to a TACC protein, such as KHS101.

In one embodiment, the invention provides for the detection of a chromosomal rearrangement at given chromosomal coordinates. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, immunostaining, ELISA, or other antibody detection methods.

In one embodiment, the biological sample comprises neuronal cells, serum, bone marrow, blood, peripheral blood, lymph nodes, cerebro-spinal fluid, urine, a saliva sample, a buccal swab, a serum sample, a sputum sample, a lacrimal secretion sample, a semen sample, a vaginal secretion sample, a fetal tissue sample, or a combination thereof. In some embodiments the sample is a tissue sample. In some embodiments, the sample is a paraffi- embedded tissue section. In some embodiments, the tissue sample is a tumor sample.

A FGFR fusion molecule, for example, a fusion between FGFR1, FGFR2, FGFR3, or any other FGFR, and TACC1, TACC2, TACC3 or any other TACC, can be determined at the level of the DNA, RNA, or polypeptide. Optionally, detection can be determined by performing an oligonucleotide ligation assay, a confirmation based assay, a hybridization assay, a sequencing assay, an allele-specific amplification assay, a microsequencing assay, a melting curve analysis, a denaturing high performance liquid chromatography (DHPLC) assay (for example, see Jones et al, (2000) Hum Genet., 106(6):663-8), or a combination thereof. In one embodiment, the detection is performed by sequencing all or part of a FGFR fusion molecule (e.g., a FGFR1-TACC1, FGFR2-TACC2, FGFR3-TACC3 or other FGFR-TACC nucleic acid, or a FGFR1, TACC1, FGFR2, TACC2, FGFR3, TACC3 or other FGFR or TACC nucleic acid), or by selective hybridization or amplification of all or part of a FGFR fusion molecule (e.g., a FGFR1-TACC1, FGFR2-TACC2, FGFR3-TACC3 or other FGFR-TACC nucleic acid, or a FGFR1, TACC1, FGFR2, TACC2, FGFR3, TACC3 or other FGFR or TACC nucleic acid). A FGFR fusion molecule specific amplification (e.g., a FGFR1-TACC1, FGFR2-TACC2, FGFR3-TACC3 or other FGFR-TACC nucleic acid specific amplification) can be carried out before the fusion identification step.

The invention provides for a method of detecting a chromosomal alteration in a subject afflicted with a gene-fusion associated cancer. In one embodiment, the chromosomal alteration is an in-frame fused transcript described herein, for example an FGFR fusion molecule. In some embodiments, the chromosomal alteration is a chromosomal translocation, for example an FGFR fusion molecule. An alteration in a chromosome region occupied by a FGFR fusion molecule, such as a FGFR1-TACC1, FGFR2-TACC2, FGFR3-TACC3 or other FGFR-TACC nucleic acid, can be any form of mutation(s), deletion(s), rearrangement(s) and/or insertions in the coding and/or non-coding region of the locus, alone or in various combination(s). Mutations can include point mutations. Insertions can encompass the addition of one or several residues in a coding or non-coding portion of the gene locus. Insertions can comprise an addition of between 1 and 50 base pairs in the gene locus. Deletions can encompass any region of one, two or more residues in a coding or non-coding portion of the gene locus, such as from two residues up to the entire gene or locus. Deletions can affect smaller regions, such as domains (introns) or repeated sequences or fragments of less than about 50 consecutive base pairs, although larger deletions can occur as well. Rearrangement includes inversion of sequences. The alteration in a chromosome region occupied by a FGFR fusion molecule, e.g., a FGFR1-TACC1, FGFR2-TACC2, FGFR3-TACC3 or other FGFR-TACC nucleic acid, can result in amino acid substitutions, RNA splicing or processing, product instability, the creation of stop codons, production of oncogenic fusion proteins, frame-shift mutations, and/or truncated polypeptide production. The alteration can result in the production of a FGFR fusion molecule, for example, one encoded by a FGFR1-TACC1, FGFR2-TACC2, FGFR3-TACC3 or other FGFR-TACC nucleic acid, with altered function, stability, targeting or structure. The alteration can also cause a reduction, or even an increase in protein expression. In one embodiment, the alteration in the chromosome region occupied by a FGFR fusion molecule can comprise a chromosomal rearrangement resulting in the production of a FGFR fusion molecule, such as a FGFR1-TACC1, FGFR2-TACC2, FGFR3-TACC3 or other FGFR-TACC fusion. This alteration can be determined at the level of the DNA, RNA, or polypeptide. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, ELISA, immunostaining or other antibody detection methods. In one embodiment, the coordinates comprising FGFR1 translocations comprise chr8:38,268,656-38,325,363. In another embodiment, the coordinates comprising FGFR2 translocations comprise chr10:123,237,844-123,357,972. In a further embodiment, the coordinates comprising FGFR3 translocations comprise chr4:1,795,039-1,810,599. In yet another embodiment, the coordinates comprising FGFR4 translocations comprise chr5:176,513,921-176,525,126. In one embodiment, the coordinates comprising TACC1 translocations comprise chr8:38,644,722-38,710,546. In another embodiment, the coordinates comprising TACC2 translocations comprise chr10:123,748,689-124,014,057. In a further embodiment, the coordinates comprising TACC3 translocations comprise chr4:1,723,217-1,746,905.

The present invention provides a method for treating a gene-fusion associated cancer in a subject in need thereof. In one embodiment, the method comprises obtaining a sample from the subject to determine the level of expression of an FGFR fusion molecule in the subject. In some embodiments, the sample is incubated with an agent that binds to an FGFR fusion molecule, such as an antibody, a probe, a nucleic acid primer, and the like. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, immunostaining, ELISA, or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. In one embodiment, the FGFR fusion molecule inhibitor is JNJ-42756493. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In some embodiments, the epithelial cancer comprises bladder urothelial carcinoma, breast carcinoma, colorectal cancer, prostate carcinoma, lung squamous cell carcinoma, head and neck squamous cell carcinoma, or a combination of the epithelial cancers decribed. In one embodiment, the gene-fusion associated cancer comprises glioma, grade II or III glioma, or IDH wild-type grade II or III glioma. In further embodiments, the method comprises further detecting the presence or absence of IDH1 mutations, IDH2 mutations, EGFR gene amplification, CDK4 gene amplification, or MDM2 gene amplification. In further embodiments, a FGFR fusion molecule inhibitor can be administered in combination with CDK4 inhibitors, MDM2 inhibitors, or a combination thereof.

In one embodiment, the invention provides for a method of detecting the presence of altered RNA expression of an FGFR fusion molecule in a subject, for example one afflicted with a gene-fusion associated cancer. In another embodiment, the invention provides for a method of detecting the presence of an FGFR fusion molecule in a subject. In some embodiments, the method comprises obtaining a sample from the subject to determine whether the subject expresses an FGFR fusion molecule. In some embodiments, the sample is incubated with an agent that binds to an FGFR fusion molecule, such as an antibody, a probe, a nucleic acid primer, and the like. In other embodiments, the detection or determination comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. In one embodiment, the FGFR fusion molecule inhibitor is JNJ-42756493. Altered RNA expression includes the presence of an altered RNA sequence, the presence of an altered RNA splicing or processing, or the presence of an altered quantity of RNA. These can be detected by various techniques known in the art, including sequencing all or part of the RNA or by selective hybridization or selective amplification of all or part of the RNA. In a further embodiment, the method can comprise detecting the presence or expression of a FGFR fusion molecule, such as one encoded by a FGFR1-TACC1, FGFR2-TACC2, FGFR3-TACC3 or other FGFR-TACC nucleic acid. Altered polypeptide expression includes the presence of an altered polypeptide sequence, the presence of an altered quantity of polypeptide, or the presence of an altered tissue distribution. These can be detected by various techniques known in the art, including by sequencing and/or binding to specific ligands (such as antibodies). In one embodiment, the detecting comprises using a northern blot; real time PCR and primers directed to SEQ ID NOS: 80-82, 84, 94-145, 515, 517, 519-527, or 530-538; a ribonuclease protection assay; a hybridization, amplification, or sequencing technique to detect an FGFR fusion molecule, such as one comprising SEQ ID NOS: 80-82, 84, 94-145, 515, 517, 519-527, or 530-538; or a combination thereof. In another embodiment, the PCR primers comprise SEQ ID NOS: 162, 163, 164, 165 166, 167, 168, 169, 495, 496, 497, 498, 507, 508, 509, 510, 511, 512, 513, or 514. In a further embodiment, primers used for the screening of FGFR fusion molecules, such as FGFR-TACC fusions, comprise SEQ ID NOS: 166, 167, 168, 169, 495, 496, 497, 498, 507, 508, 509, 510, 511, 512, 513, or 514. In some embodiments, primers used for genomic detection of an FGFR3-TACC3 fusion comprise SEQ ID NOS: 170 171, 499, 500, 501, 502, 503, 504, 505, or 506.

In some aspects of the invention, the method comprises further detecting the presence or absence of IDH1 mutations, IDH2 mutations, EGFR gene amplification, CDK4 gene amplification, or MDM2 gene amplification. MDM2 encodes a nuclear-localized E3 ubiquitin ligase. Alternative splicing results in a multitude of transcript variants, many of which may be expressed only in tumor cells. EGFR (epidermal growth factor receptor) is a transmembrane glycoprotein that is a member of the protein kinase superfamily. This protein is a receptor for members of the epidermal growth factor family. EGFR is a cell surface protein that binds to epidermal growth factor. Multiple alternatively spliced transcript variants that encode different protein isoforms have been found for this gene. CDK4 (cyclin dependent kinase 4) is a member of the Ser/Thr protein kinase family. It is a catalytic subunit of the protein kinase complex that is important for cell cycle G1 phase progression. The activity of this kinase is restricted to the G1-S phase, which is controlled by the regulatory subunits D-type cyclins and CDK inhibitor p16(INK4a). Multiple polyadenylation sites of this gene have been reported. IDH1 (isocitrate dehydrogenase 1 (NADP+), soluble) catalyzes the oxidative decarboxylation of isocitrate to 2-oxoglutarate. Alternatively spliced transcript variants encoding the same protein have been found for this gene. IDH1 mutations, EGFR amplification, CDK4 amplification, or MDM2 amplification can be detected using various techniques know in the art, including, but not limited to sequencing, qPCR, and microarrays (e.g. CGH arrays, SNP arrays).

Various techniques known in the art can be used to detect or quantify altered gene or RNA expression or nucleic acid sequences, which include, but are not limited to, hybridization, sequencing, amplification, and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, denaturing HLPC, melting curve analysis, heteroduplex analysis, RNase protection, chemical or enzymatic mismatch cleavage, ELISA, radio-immunoassays (RIA) and immuno-enzymatic assays (IEMA).

Some of these approaches (such as SSCA and constant gradient gel electrophoresis (CGGE)) are based on a change in electrophoretic mobility of the nucleic acids, as a result of the presence of an altered sequence. According to these techniques, the altered sequence is visualized by a shift in mobility on gels. The fragments can then be sequenced to confirm the alteration. Some other approaches are based on specific hybridization between nucleic acids from the subject and a probe specific for wild type or altered gene or RNA. The probe can be in suspension or immobilized on a substrate. The probe can be labeled to facilitate detection of hybrids. Some of these approaches are suited for assessing a polypeptide sequence or expression level, such as Northern blot, ELISA and RIA. These latter require the use of a ligand specific for the polypeptide, for example, the use of a specific antibody.

Hybridization. Hybridization detection methods are based on the formation of specific hybrids between complementary nucleic acid sequences that serve to detect nucleic acid sequence alteration(s). A detection technique involves the use of a nucleic acid probe specific for a wild type or altered gene or RNA, followed by the detection of the presence of a hybrid. The probe can be in suspension or immobilized on a substrate or support (for example, as in nucleic acid array or chips technologies). The probe can be labeled to facilitate detection of hybrids. In one embodiment, the probe according to the invention can comprise a nucleic acid directed to SEQ ID NOS: 80-82, 84, 94-145, 515, 517, 519-527, or 530-538. For example, a sample from the subject can be contacted with a nucleic acid probe specific for a gene encoding a FGFR fusion molecule, and the formation of a hybrid can be subsequently assessed. In one embodiment, the method comprises contacting simultaneously the sample with a set of probes that are specific for an FGFR fusion molecule. Also, various samples from various subjects can be investigated in parallel.

According to the invention, a probe can be a polynucleotide sequence which is complementary to and specifically hybridizes with a, or a target portion of a, gene or RNA corresponding to a FGFR fusion molecule. Useful probes are those that are complementary to the gene, RNA, or target portion thereof. Probes can comprise single-stranded nucleic acids of between 8 to 1000 nucleotides in length, for instance between 10 and 800, between 15 and 700, or between 20 and 500. Longer probes can be used as well. A useful probe of the invention is a single stranded nucleic acid molecule of between 8 to 500 nucleotides in length, which can specifically hybridize to a region of a gene or RNA that corresponds to a FGFR fusion molecule.

The sequence of the probes can be derived from the sequences of the FGFR fusion genes provided herein. Nucleotide substitutions can be performed, as well as chemical modifications of the probe. Such chemical modifications can be accomplished to increase the stability of hybrids (e.g., intercalating groups) or to label the probe. Some examples of labels include, without limitation, radioactivity, fluorescence, luminescence, and enzymatic labeling.

A guide to the hybridization of nucleic acids is found in e.g., Sambrook, ed., Molecular Cloning: A Laboratory Manual (3rd Ed.), Vols. 1-3, Cold Spring Harbor Laboratory, 1989; Current Protocols In Molecular Biology, Ausubel, ed. John Wiley & Sons, Inc., New York, 2001; Laboratory Techniques In Biochemistry And Molecular Biology: Hybridization With Nucleic Acid Probes, Part I. Theory and Nucleic Acid Preparation, Tijssen, ed. Elsevier, N.Y., 1993.

Sequencing. Sequencing can be carried out using techniques well known in the art, using automatic sequencers. The sequencing can be performed on the complete FGFR fusion molecule or on specific domains thereof.

Amplification. Amplification is based on the formation of specific hybrids between complementary nucleic acid sequences that serve to initiate nucleic acid reproduction. Amplification can be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Useful techniques in the art encompass real-time PCR, allele-specific PCR, or PCR based single-strand conformational polymorphism (SSCP). Amplification usually requires the use of specific nucleic acid primers, to initiate the reaction. For example, nucleic acid primers useful for amplifying sequences corresponding to a FGFR fusion molecule are able to specifically hybridize with a portion of the gene locus that flanks a target region of the locus. In one embodiment, amplification comprises using forward and reverse PCR primers directed to SEQ ID NOS: 80-82, 84, 94-145, 515, 517, 519-527, or 530-538. Nucleic acid primers useful for amplifying sequences from a FGFR fusion molecule (e.g., a FGFR1-TACC1, FGFR2-TACC2, FGFR3-TACC3 or other FGFR-TACC nucleic acid); the primers specifically hybridize with a portion of an FGFR fusion molecule. In certain subjects, the presence of an FGFR fusion molecule corresponds to a subject with a gene fusion-associated cancer. In one embodiment, amplification can comprise using forward and reverse PCR primers comprising nucleotide sequences of SEQ ID NOS: 80-82, 84, 94-145, 515, 517, 519-527, or 530-538. In one embodiment, amplification can comprise using forward and reverse PCR primers comprising nucleotide sequences of SEQ ID NOS: 162-169, or 495-514.

Non-limiting amplification methods include, e.g., polymerase chain reaction, PCR (PCR Protocols, A Guide To Methods And Applications, ed. Innis, Academic Press, N.Y., 1990 and PCR Strategies, 1995, ed. Innis, Academic Press, Inc., N.Y.); ligase chain reaction (LCR) (Wu (1989) Genomics 4:560; Landegren (1988) Science 241:1077; Barringer (1990) Gene 89:117); transcription amplification (Kwoh (1989) PNAS 86:1173); and, self-sustained sequence replication (Guatelli (1990) PNAS 87:1874); Q Beta replicase amplification (Smith (1997) J. Clin. Microbiol. 35:1477-1491), automated Q-beta replicase amplification assay (Burg (1996) Mol. Cell. Probes 10:257-271) and other RNA polymerase mediated techniques (e.g., NASBA, Cangene, Mississauga, Ontario; *see also* Berger (1987) Methods Enzymol. 152:307-316; U.S. Pat. Nos. 4,683,195 and 4,683,202; and Sooknanan (1995) Biotechnology 13:563-564). All the references stated above are incorporated by reference in their entireties.

The invention provides for a nucleic acid primer, wherein the primer can be complementary to and hybridize specifically to a portion of a FGFR fusion molecule, such as a FGFR1-TACC1, FGFR2-TACC2, FGFR3-TACC3 or other FGFR-TACC nucleic acid (e.g., DNA or RNA) in certain subjects having a gene fusion-associated cancer. In one embodiment, the gene-fusion associated cancer comprises glioblastoma multiforme, breast cancer, lung cancer, prostate cancer, or colorectal carcinoma. Primers of the invention can be specific for fusion sequences in a FGFR1-TACC1, FGFR2-TACC2, FGFR3-TACC3 or other FGFR-TACC nucleic acid (DNA or RNA). By using such primers, the detection of an amplification product indicates the presence of a fusion of a FGFR1 and TACC1, FGFR2 and TACC2, FGFR3 and TACC3 or other FGFR and TACC nucleic acid. Examples of primers of this invention can be single-stranded nucleic acid molecules of about 5 to 60 nucleotides in length, or about 8 to about 25 nucleotides in length. The sequence can be derived directly from the sequence of a FGFR fusion molecule, e.g. FGFR1-TACC1, FGFR2-TACC2, FGFR3-TACC3 or other FGFR-TACC nucleic acid. Perfect complementarity is useful to ensure high specificity; however, certain mismatch can be tolerated. For example, a nucleic acid primer or a pair of nucleic acid primers as described above can be used in a method for detecting the presence of a gene fusion-associated cancer in a subject. In one embodiment, primers can be used to detect an FGFR fusion molecule, such as a primer comprising SEQ ID NOS: 80-82, 84, 94-145, 515, 517, 519-527, or 530-538; or a combination thereof. In another embodiment, the PCR primers comprise SEQ ID NOS: 162, 163, 164, 165, 166, 167, 168, 169, 495, 496, 497, 498, 507, 508, 509, 510, 511, 512, 513, or 514. In a further embodiment, primers used for the screening of FGFR fusion molecules, such as FGFR-TACC fusions, comprise SEQ ID NOS: 166, 167, 168, 169, 495, 496, 497, 498, 507, 508, 509, or 510. In some embodiments, primers used for genomic detection of an FGFR3-TACC3 fusion comprise SEQ ID NOS: 170, 171, 499, 500, 501, 502, 503, 504, 505, or 506.ln one embodiment, the method can comprise contacting a sample from the subject with primers specific for a FGFR fusion molecule, and determining the presence of an PCR product. In another embodiment, the method can comprise contacting a sample from the subject with primer specific for a FGFR molecule, or a TACC molecule, and determining the presence of a PCR product. In another embodiment, the primers can recognize the nucleic acids encoding a FGFR3 C-terminal region, or nucleic acids encoding a TACC3 N-terminal region, or a combination thereof. In another embodiment, the method can comprise contacting a sample from the subject with primers specific for a FGFR molecule, or a TACC molecule, or a FGFR fusion molecule, and determining the amount of PCR product formed compared to the amount of PCR product formed in non-tumor cells or tissue, wherein an increased amount of PCR product indicates the presence of an FGFR fusion. In one embodiment, primers and/or the PCR product are labeled to enable detection of the PCR product. For example, nucleic acid primers useful for amplifying sequences corresponding to a FGFR fusion molecules can be labeled with fluorescent molecules, radioactive molecules, chemiluminescent molecules, or affinity molecules (e.g. biotin) which can then be detected by methods known in the art (e.g. fluorescently labeled streptavidin). PCR products can also be detected by using dyes that can be incorporated into newly formed PCR products, such as, but not limited to, SYBR Green.

Specific Ligand Binding. As discussed herein, a nucleic acid encoding a FGFR fusion molecule or expression of a FGFR fusion molecule, can also be detected by screening for alteration(s) in a sequence or expression level of a polypeptide encoded by the same. Different types of ligands can be used, such as specific antibodies. In one embodiment, the sample is contacted with an antibody specific for a polypeptide encoded by a FGFR fusion molecule and the formation of an immune complex is subsequently determined. Various methods for detecting an immune complex can be used, such as ELISA, immunostaining, radioimmunoassays (RIA) and immuno-enzymatic assays (IEMA).

For example, an antibody can be a polyclonal antibody, a monoclonal antibody, as well as fragments or derivatives thereof having substantially the same antigen specificity. Fragments include Fab, Fab'2, or CDR regions. Derivatives include single-chain antibodies, humanized antibodies, or poly-functional antibodies. An antibody specific for a polypeptide encoded by a FGFR fusion molecule can be an antibody that selectively binds such a polypeptide. In one embodiment, the antibody is raised against a polypeptide encoded by a FGFR fusion molecule (such as FGFR1-TACC1, FGFR2-TACC2, FGFR3-TACC3 or other FGFR-TACC fusion) or an epitope-containing fragment thereof. Although non-specific binding towards other antigens can occur, binding to the target polypeptide occurs with a higher affinity and can be reliably discriminated from non-specific binding. In one embodiment, the method can comprise contacting a sample from the subject with an antibody specific for a FGFR fusion molecule, and determining the presence of an immune complex. Optionally, the sample can be contacted to a support coated with antibody specific for a FGFR fusion molecule. In one embodiment, the sample can be contacted simultaneously, or in parallel, or sequentially, with various antibodies specific for different forms of a FGFR fusion molecule, e.g., FGFR1-TACC1, FGFR2-TACC2, FGFR3-TACC3 or other FGFR-TACC fusion.

In one embodiment, the method can comprise contacting a sample from the subject with an antibody specific for a FGFR fusion molecule, and determining the presence of an immune complex. In another embodiment, the method can comprise contacting a sample from the subject with an antibody specific for a FGFR molecule, or a TACC molecule, and determining the presence of an immune complex. In another embodiment, the antibody can recognize the FGFR3 C-terminal region, or the TACC3 N-terminal region, or a combination thereof. In another embodiment, the antibody can recognize the FGFR3 C-terminal region, or the TACC3 N-terminal region, or a combination thereof. In another embodiment, the method can comprise contacting a sample from the subject with an antibody specific for a FGFR molecule, or a TACC molecule, or a FGFR fusion molecule, and determining the amount of an immune complex formed compared to the amount of immune complex formed in non-tumor cells or tissue, wherein an increased amount of an immune complex indicates the presence of an FGFR fusion.

Detection the formation of a complex between an antibody and a protein can be performed by a variety of method known in the art. For example, an antibody-protein complex can be detected by using antibodies or secondary antibodies labeled with fluorescent molecules, chromogenic molecules, chemiluminescent molecules, radioactive isotopes, or affinity molecules (e.g. biotin) which can then be detected by methods known in the art (e.g. fluorescently labeled streptavidin).

The invention also provides for a diagnostic kit comprising products and reagents for detecting in a sample from a subject the presence of a FGFR fusion molecule. The kit can be useful for determining whether a sample from a subject exhibits increased or reduced expression of a FGFR fusion molecule. For example, the diagnostic kit according to the present invention comprises any primer, any pair of primers, any nucleic acid probe and/or any ligand, or any antibody directed specifically to a FGFR fusion molecule. The diagnostic kit according to the present invention can further comprise reagents and/or protocols for performing a hybridization, amplification, or antigen-antibody immune reaction. In one embodiment, the kit can comprise nucleic acid primers that specifically hybridize to and can prime a polymerase reaction from a FGFR fusion molecule comprising SEQ ID NOS: 80-82, 84, 94-145, 515, 517, 519-527, or 530-538, or a combination thereof. In one embodiment, primers can be used to detect a FGFR fusion molecule, such as a primer directed to SEQ ID NOS: 80-82, 84, 94-145, 515, 517, 519-527, or 530-538; or a combination thereof. In another embodiment, the PCR primer comprises SEQ ID NOS: 162, 163, 164, 165, 166, 167, 168, 169, 495, 496, 497, 498, 507, 508, 509, 510, 511, 512, 513, or 514. In a further embodiment, primers used for the screening of FGFR fusion molecules, such as FGFR-TACC fusions, comprise SEQ ID NOS: 166, 167, 168, 169, 495, 496, 497, 498, 507, 508, 509, or 510. In some embodiments, primers used for genomic detection of an FGFR3-TACC3 fusion comprise SEQ ID NOS: 170, 171, 499, 500, 501, 502, 503, 504, 505, or 506. In some embodiments, the kit comprises an antibody that specifically binds to a FGFR fusion molecule comprising SEQ ID NOS: 79, 85-89, 150, 158-161, or 539-547, wherein the antibody will recognize the protein only when a FGFR fusion molecule is present.The diagnosis methods can be performed *in vitro, ex vivo,* or *in vivo.* These methods utilize a sample from the subject in order to assess the status of a FGFR fusion molecule. The sample can be any biological sample derived from a subject, which contains nucleic acids or polypeptides. Examples of such samples include, but are not limited to, fluids, tissues, cell samples, organs, and tissue biopsies. Non-limiting examples of samples include blood, liver, plasma, serum, saliva, urine, or seminal fluid. In some embodiments the sample is a tissue sample. In some embodiments, the sample is a paraffin embedded tissue section. In some embodiments, the tissue sample is a tumor sample. The sample can be collected according to conventional techniques and used directly for diagnosis or stored. The sample can be treated prior to performing the method, in order to render or improve availability of nucleic acids or polypeptides for testing. Treatments include, for instance, lysis (e.g., mechanical, physical, or chemical), centrifugation. The nucleic acids and/or polypeptides can be pre-purified or enriched by conventional techniques, and/or reduced in complexity. Nucleic acids and polypeptides can also be treated with enzymes or other chemical or physical treatments to produce fragments thereof. In one embodiment, the sample is contacted with reagents, such as probes, primers, or ligands, in order to assess the presence of a FGFR fusion molecule. Contacting can be performed in any suitable device, such as a plate, tube, well, or glass. In some embodiments, the contacting is performed on a substrate coated with the reagent, such as a nucleic acid array or a specific ligand array. The substrate can be a solid or semi-solid substrate such as any support comprising glass, plastic, nylon, paper, metal, or polymers. The substrate can be of various forms and sizes, such as a slide, a membrane, a bead, a column, or a gel. The contacting can be made under any condition suitable for a complex to be formed between the reagent and the nucleic acids or polypeptides of the sample.

### Nucleic Acid Delivery Methods

Delivery of nucleic acids into viable cells can be effected *ex vivo, in situ,* or *in vivo* by use of vectors, such as viral vectors (e.g., lentivirus, adenovirus, adeno-associated virus, or a retrovirus), or ex vivo by use of physical DNA transfer methods (e.g., liposomes or chemical treatments). Non-limiting techniques suitable for the transfer of nucleic acid into mammalian cells *in vitro* include the use of liposomes, electroporation, microinjection, cell fusion, DEAE-dextran, and the calcium phosphate precipitation method (See, for example, Anderson, Nature, 1998) supplement to 392(6679):25(). Introduction of a nucleic acid or a gene encoding a polypeptide of the invention can also be accomplished with extrachromosomal substrates (transient expression) or artificial chromosomes (stable expression). Cells can also be cultured ex vivo in the presence of therapeutic compositions of the present invention in order to proliferate or to produce a desired effect on or activity in such cells. Treated cells can then be introduced in vivo for therapeutic purposes.

Nucleic acids can be inserted into vectors and used as gene therapy vectors. A number of viruses have been used as gene transfer vectors, including papovaviruses, e.g., SV40 (Madzak et al., (1992) J Gen Virol. 73 (Pt 6):1533-6), adenovirus (Berkner (1992) Curr Top Microbiol Immunol.158:39-66; Berkner (1988) Biotechniques, 6(7):616-29; Gorziglia and Kapikian (1992) J Virol. 66(7):4407-12; Quantin et al., (1992) Proc Natl Acad Sci U S A. 89(7):2581-4; Rosenfeld et al., (1992) Cell. 68(1):143-55; Wilkinson et al., (1992) Nucleic Acids Res. 20(9):2233-9; Stratford-Perricaudet et al., (1990) Hum Gene Ther. 1(3):241-56), vaccinia virus (Moss (1992) Curr Opin Biotechnol. 3(5):518-22), adeno-associated virus (Muzyczka, (1992) Curr Top Microbiol Immunol. 158:97-129; Ohi et al., (1990) Gene. 89(2):279-82), herpesviruses including HSV and EBV (Margolskee (1992) Curr Top Microbiol Immunol. 158:67-95; Johnson et al., (1992) Brain Res Mol Brain Res.12(1-3):95-102; Fink et al., (1992) Hum Gene Ther. 3(1):11-9; Breakefield and Geller (1987) Mol Neurobiol. 1(4):339-71; Freese et al., (1990) Biochem Pharmacol. 40(10):2189-99), and retroviruses of avian (Bandyopadhyay and Temin (1984) Mol Cell Biol. 4(4):749-54; Petropoulos et al., (1992) J Virol. 66(6):3391-7), murine (Miller et al. (1992) Mol Cell Biol. 12(7):3262-72; Miller et al., (1985) J Virol. 55(3):521-6; Sorge et al., (1984) Mol Cell Biol. 4(9):1730-7; Mann and Baltimore (1985) J Virol. 54(2):401-7; Miller et al., (1988) J Virol. 62(11):4337-45), and human origin (Shimada et al., (1991) J Clin Invest. 88(3):1043-7; Helseth et al., (1990) J Virol. 64(12):6314-8; Page et al., (1990) J Virol. 64(11):5270-6; Buchschacher and Panganiban (1992) J Virol. 66(5):2731-9).

Non-limiting examples of *in vivo* gene transfer techniques include transfection with viral (e.g., retroviral) vectors (see U.S. Pat. No. 5,252,479, which is incorporated by reference in its entirety) and viral coat protein-liposome mediated transfection (Dzau et al., (1993) Trends in Biotechnology 11:205-210), incorporated entirely by reference). For example, naked DNA vaccines are generally known in the art; *see* Brower, (1998) Nature Biotechnology, 16:1304-1305, which is incorporated by reference in its entirety. Gene therapy vectors can be delivered to a subject by, for example, intravenous injection, local administration (*see*, e.g., U.S. Pat. No. 5,328,470) or by stereotactic injection (*see*, e.g., Chen, et al., (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

For reviews of nucleic acid delivery protocols and methods see Anderson et al. (1992) Science 256:808-813; U.S. Pat. Nos. 5,252,479, 5,747,469, 6,017,524, 6,143,290, 6,410,010 6,511,847; and U.S. Application Publication No. 2002/0077313, which are all hereby incorporated by reference in their entireties. For additional reviews, see Friedmann (1989) Science, 244:1275-1281; Verma, Scientific American: 68-84 (1990); Miller (1992) Nature, 357: 455-460; Kikuchi et al. (2008) J Dermatol Sci. 50(2):87-98; Isaka et al. (2007) Expert Opin Drug Deliv. 4(5):561-71; Jager et al.(2007) Curr Gene Ther. 7(4):272-83; Waehler et al.(2007) Nat Rev Genet. 8(8):573-87; Jensen et al. (2007) Ann Med. 39(2):108-15; Herweijer et al. (2007) Gene Ther. 14(2):99-107; Eliyahu et al. (2005) Molecules 10(1):34-64; and Altaras et al. (2005) Adv Biochem Eng Biotechnol. 99:193-260, all of which are hereby incorporated by reference in their entireties.

A FGFR fusion nucleic acid can also be delivered in a controlled release system. For example, the FGFR fusion molecule can be administered using intravenous infusion, an implantable osmotic pump, a transdermal patch, liposomes, or other modes of administration. In one embodiment, a pump can be used (see Sefton (1987) Biomed. Eng. 14:201; Buchwald et al. (1980) Surgery 88:507; Saudek et al. (1989) N. Engl. J. Med. 321:574). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, (1983) J. Macromol. Sci. Rev. Macromol. Chem. 23:61; *see also* Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target thus requiring only a fraction of the systemic dose (*see*, e.g., Goodson, in Medical Applications of Controlled Release, supra, vol. 2, pp. 115-138 (1984)). Other controlled release systems are discussed in the review by Langer (Science (1990) 249:1527-1533).

### Pharmaceutical Compositions and Administration for Therapy

An inhibitor of the invention can be incorporated into pharmaceutical compositions suitable for administration, for example the inhibitor and a pharmaceutically acceptable carrier A FGFR fusion molecule or inhibitor of the invention (e.g. JNJ-42756493) can be administered to the subject once (e.g., as a single injection or deposition). Alternatively, a FGFR fusion molecule or inhibitor can be administered once or twice daily to a subject in need thereof for a period of from about two to about twenty-eight days, or from about seven to about ten days. A FGFR fusion molecule or inhibitor can also be administered once or twice daily to a subject for a period of 1, 2, 3, 4, 5, 6, 7, 8, 9, 10, 11, 12 times per year, or a combination thereof. A FGFR fusion molecule or inhibitor can also be administered in seven to ten day repeating cycles (i.e. administration of a FGFR fusion molecule or inhibitor for seven to ten days, followed by no administration of a FGFR fusion molecule or inhibitor for seven to ten days). Furthermore, a FGFR fusion molecule or inhibitor of the invention can be co-administrated with another therapeutic. Where a dosage regimen comprises multiple administrations, the effective amount of the FGFR fusion molecule or inhibitor administered to the subject can comprise the total amount of gene product administered over the entire dosage regimen.

A FGFR fusion molecule or inhibitor can be administered to a subject by any means suitable for delivering the FGFR fusion molecule or inhibitor to cells of the subject, such as cancer cells, e.g., glioblastoma multiforme, breast cancer, lung cancer, prostate cancer, colorectal carcinoma, bladder carcinoma, squamous lung carcinoma, head and neck carcinoma, glioma, grade II or III glioma, or IDH wild-type grade II or III glioma. For example, a FGFR fusion molecule or inhibitor can be administered by methods suitable to transfect cells. Transfection methods for eukaryotic cells are well known in the art, and include direct injection of the nucleic acid into the nucleus or pronucleus of a cell; electroporation; liposome transfer or transfer mediated by lipophilic materials; receptor mediated nucleic acid delivery, bioballistic or particle acceleration; calcium phosphate precipitation, and transfection mediated by viral vectors.

The compositions of this invention can be formulated and administered to reduce the symptoms associated with a gene fusion-associated cancer, e.g., glioblastoma multiforme, breast cancer, lung cancer, prostate cancer, colorectal carcinoma, bladder carcinoma, squamous lung carcinoma, head and neck carcinoma, glioma, grade II or III glioma, or IDH wild-type grade II or III glioma, by any means that produces contact of the active ingredient with the agent's site of action in the body of a subject, such as a human or animal (e.g., a dog, cat, or horse). They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

A therapeutically effective dose of FGFR fusion molecule or inhibitor (e.g. JNJ-42756493) can depend upon a number of factors known to those or ordinary skill in the art. The dose(s) of the FGFR fusion molecule inhibitor can vary, for example, depending upon the identity, size, and condition of the subject or sample being treated, further depending upon the route by which the composition is to be administered, if applicable, and the effect which the practitioner desires the a FGFR fusion molecule inhibitor to have upon the nucleic acid or polypeptide of the invention. For example, 12 mg of JNJ-42756493 can be orally administered daily. JNJ-42756493 can be administered in seven to ten day repeating cycles (i.e. administration of JNJ-42756493 for seven to ten days, followed by no administration of JNJ-42756493 for seven to ten days). These amounts can be readily determined by a skilled artisan. Any of the therapeutic applications described herein can be applied to any subject in need of such therapy, including, for example, a mammal such as a dog, a cat, a cow, a horse, a rabbit, a monkey, a pig, a sheep, a goat, or a human. Pharmaceutical compositions for use in accordance with the invention can be formulated in conventional manner using one or more physiologically acceptable carriers or excipients. The therapeutic compositions of the invention can be formulated for a variety of routes of administration, including systemic and topical or localized administration. Techniques and formulations generally can be found in Remmington's Pharmaceutical Sciences, Meade Publishing Co., Easton, Pa (20th Ed., 2000), the entire disclosure of which is herein incorporated by reference. For systemic administration, an injection is useful, including intramuscular, intravenous, intraperitoneal, and subcutaneous. For injection, the therapeutic compositions of the invention can be formulated in liquid solutions, for example in physiologically compatible buffers such as Hank's solution or Ringer's solution. In addition, the therapeutic compositions can be formulated in solid form and redissolved or suspended immediately prior to use. Lyophilized forms are also included. Pharmaceutical compositions of the present invention are characterized as being at least sterile and pyrogen-free. These pharmaceutical formulations include formulations for human and veterinary use.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

A pharmaceutical composition containing FGFR fusion molecule inhibitor can be administered in conjunction with a pharmaceutically acceptable carrier, for any of the therapeutic effects discussed herein. Such pharmaceutical compositions can comprise, for example antibodies directed to a FGFR fusion molecule, or a variant thereof, or antagonists of a FGFR fusion molecule, or JNJ-42756493. The compositions can be administered alone or in combination with at least one other agent, such as a stabilizing compound, which can be administered in any sterile, biocompatible pharmaceutical carrier including, but not limited to, saline, buffered saline, dextrose, and water. The compositions can be administered to a patient alone, or in combination with other agents, drugs or hormones.

Sterile injectable solutions can be prepared by incorporating the FGFR fusion molecule inhibitor (e.g., a polypeptide or antibody) in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

In some embodiments, the FGFR fusion molecule inhibitor can be applied via transdermal delivery systems, which slowly releases the active compound for percutaneous absorption. Permeation enhancers can be used to facilitate transdermal penetration of the active factors in the conditioned media. Transdermal patches are described in for example, U.S. Pat. No. 5,407,713; U.S. Pat. No. 5,352,456; U.S. Pat. No. 5,332,213; U.S. Pat. No. 5,336,168; U.S. Pat. No. 5,290,561; U.S. Pat. No. 5,254,346; U.S. Pat. No. 5,164,189; U.S. Pat. No. 5,163,899; U.S. Pat. No. 5,088,977; U.S. Pat. No. 5,087,240; U.S. Pat. No. 5,008,110; and U.S. Pat. No. 4,921,475.

"Subcutaneous" administration can refer to administration just beneath the skin (i.e., beneath the dermis). Generally, the subcutaneous tissue is a layer of fat and connective tissue that houses larger blood vessels and nerves. The size of this layer varies throughout the body and from person to person. The interface between the subcutaneous and muscle layers can be encompassed by subcutaneous administration. This mode of administration can be feasible where the subcutaneous layer is sufficiently thin so that the factors present in the compositions can migrate or diffuse from the locus of administration. Thus, where intradermal administration is utilized, the bolus of composition administered is localized proximate to the subcutaneous layer.

Administration of the cell aggregates (such as DP or DS aggregates) is not restricted to a single route, but can encompass administration by multiple routes. For instance, exemplary administrations by multiple routes include, among others, a combination of intradermal and intramuscular administration, or intradermal and subcutaneous administration. Multiple administrations can be sequential or concurrent. Other modes of application by multiple routes will be apparent to the skilled artisan.

In other embodiments, this implantation method will be a one-time treatment for some subjects. In further embodiments of the invention, multiple cell therapy implantations will be required. In some embodiments, the cells used for implantation will generally be subject-specific genetically engineered cells. In another embodiment, cells obtained from a different species or another individual of the same species can be used. Thus, using such cells can require administering an immunosuppressant to prevent rejection of the implanted cells. Such methods have also been described in United States Patent No. 7,419,661and PCT application publication WO 2001/32840, and are hereby incorporated by reference.

A pharmaceutical composition of the invention is formulated to be compatible with its intended route of administration. Examples of routes of administration include parenteral, e.g., intravenous, intradermal, subcutaneous, oral (e.g., inhalation or ingestion), transdermal (topical), transmucosal, and rectal administration. For example, JNJ-42756493 can be orally administered. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it can be useful to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the inhibitor (e.g., a polypeptide or antibody or small molecule) of the invention in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

Oral compositions (e.g. of JNJ-42756493) generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier and subsequently swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

In some embodiments, the effective amount of the administered FGFR fusion molecule inhibitor (e.g. JNJ-42756493) is at least about 0.0001 µg/kg body weight, at least about 0.00025 µg/kg body weight, at least about 0.0005 µg/kg body weight, at least about 0.00075 µg/kg body weight, at least about 0.001 µg/kg body weight, at least about 0.0025 µg/kg body weight, at least about 0.005 µg/kg body weight, at least about 0.0075 µg/kg body weight, at least about 0.01 µg/kg body weight, at least about 0.025 µg/kg body weight, at least about 0.05 µg/kg body weight, at least about 0.075 µg/kg body weight, at least about 0.1 µg/kg body weight, at least about 0.25 µg/kg body weight, at least about 0.5 µg/kg body weight, at least about 0.75 µg/kg body weight, at least about 1 µg/kg body weight, at least about 5 µg/kg body weight, at least about 10 µg/kg body weight, at least about 25 µg/kg body weight, at least about 50 µg/kg body weight, at least about 75 µg/kg body weight, at least about 100 µg/kg body weight, at least about 150 µg/kg body weight, at least about 200 µg/kg body weight, at least about 250 µg/kg body weight, at least about 300 µg/kg body weight, at least about 350 µg/kg body weight, at least about 400 µg/kg body weight, at least about 450 µg/kg body weight, at least about 500 µg/kg body weight, at least about 550 µg/kg body weight, at least about 600 µg/kg body weight, at least about 650 µg/kg body weight, at least about 700 µg/kg body weight, at least about 750 µg/kg body weight, at least about 800 µg/kg body weight, at least about 850 µg/kg body weight, at least about 900 µg/kg body weight, at least about 950 µg/kg body weight, at least about 1,000 µg/kg body weight, at least about 2,000 µg/kg body weight, at least about 3,000 µg/kg body weight, at least about 4,000 µg/kg body weight, at least about 5,000 µg/kg body weight, at least about 6,000 µg/kg body weight, at least about 7,000 µg/kg body weight, at least about 8,000 µg/kg body weight, at least about 9,500 µg/kg body weight, or at least about 10,000 µg/kg body weight.

In some embodiments, the effective amount of the administered FGFR fusion molecule inhibitor (e.g. JNJ-42756493) is at least about 1mg, 2mg, 3mg, 4mg, 5mg, 6mg, 7mg, 8mg, 9mg, 10mg, 11mg, 12mg, 14mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention.

All publications and other references mentioned herein are incorporated by reference in their entirety, as if each individual publication or reference were specifically and individually indicated to be incorporated by reference. Publications and references cited herein are not admitted to be prior art.

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

The invention is further illustrated in Singh et al., Science (2012), 337(6099):1231-5 (including the accompanying Supplementary Information). The entire contents of Singh et al., Science (2012), 337(6099):1231-5, including the accompanying "Supplementary Information," is expressly incorporated by reference. The invention is also further illustrated in Di Stefano et al., "Detection, characterization and inhibition of FGFR-TACC fusions in IDH wild type glioma" Clin. Cancer Res. (2015), the entire contents of which are expressly incorporated by reference, including the accompanying Supplementary Information. **Example 1**: *Transforming and recurrent fusions of FGFR and TACC gene in glioblastoma* The history of successful targeted therapy of cancer largely coincides with the inactivation of recurrent, oncogenic and addicting gene fusions in hematological malignancies and recently in some types of epithelial cancer. Glioblastoma multiforme (GBM) is among the most lethal forms of human cancer. Here, an integrated gene fusion discovery pipeline was developed for the detection of in-frame fused transcripts from RNA-seq and genomic fusions from whole exome sequences. The application of the pipeline to human GBM unraveled recurrent chromosomal translocations, which fuse in-frame the tyrosine kinase domain of FGFR genes (FGFR1 or FGFR3) to the TACC domain of TACC1 or TACC3, respectively. The frequency of FGFR-TACC fusions is 3 of 97 GBM (3.1%). The FGFR-TACC fusion protein displays strong oncogenic activity when introduced into astrocytes or transduced by lentivirus-mediated stereotactic delivery to the adult mouse brain. The FGFR-TACC fusion protein mis-localizes over the mitotic spindle pole, has constitutive tyrosine kinase activity and dysregulates the mitotic cycle with delayed mitotic progression. The impaired mitotic fidelity triggers chromatid cohesion defects, defective spindle checkpoint activation, chromosomal mis-segregation, and rampant aneuploidy. Inhibition of FGFR kinase corrects the aneuploidy and oral administration of a specific FGFR tyrosine kinase inhibitor under clinical investigation arrests tumor growth and prolongs survival of mice harboring intracranial FGFR3-TACC3-initiated glioma. FGFR-TACC fusions identify a subset of GBM patients who may benefit from targeted inhibition of the tyrosine kinase activity of FGFR.

Glioblastoma multiforme (GBM) is among the most difficult forms of cancer to treat in humans (Ohgaki and Kleihues, 2005). So far, the targeted therapeutic approaches that have been tested against potentially important oncogenic drivers in GBM have met limited success (Lo, 2010; Reardon et al., 2010; van den Bent et al., 2009). Recurrent chromosomal translocations leading to production of oncogenic fusion proteins are viewed as initiating and addicting events in the pathogenesis of human cancer, thus providing the most desirable molecular targets for cancer therapy (Ablain et al., 2011; Mitelman et al., 2007). Chromosomal rearrangements resulting in recurrent and oncogenic gene fusions are hallmarks of hematological malignancies and recently they have also been uncovered in subsets of solid tumors (breast, prostate, lung and colorectal carcinoma), but they have not been found in GBM (Bass et al., 2011; Prensner and Chinnaiyan, 2009). Important and successful targeted therapeutic interventions for patients whose tumors carry these rearrangements have stemmed from the discovery of functional gene fusions, especially when the translocations involve kinase-coding genes (*BCR-ABL, EML4-ALK*) (Druker, 2009; Gerber and Minna, 2010).

A hallmark of GBM is rampant chromosomal instability (CIN), which leads to aneuploidy (Furnari et al., 2007). CIN and aneuploidy are early events in the pathogenesis of cancer (Cahill et al., 1999). It has been suggested that genetic alterations targeting mitotic fidelity might be responsible for mis-segregation of chromosomes during mitosis, resulting in aneuploidy (Gordon et al., 2012; Solomon et al., 2011). Here, the first cases of recurrent and oncogenic gene fusions in human GBM are described. The resulting fusion protein localizes to mitotic cells, disrupts the normal control of chromosome segregation and induces aneuploidy. A therapeutic strategy with FGFR tyrosine kinase inhibitors is also reported for the targeted therapy of GBM patients harboring these chromosomal rearrangements.

***Identification of recurrent fusions of FGFR and TACC genes.*** To identify genomic rearrangements in GBM that generate functional fusion proteins and are recurrent, gene pairs discovered as in-frame fused transcripts from the analysis of massively parallel, paired-end sequencing of expressed transcripts (RNA-seq) would also emerge as fused gene pairs from the genomic analysis of human GBM. Towards this aim, two complementary gene fusion discovery methods were devised and were applied to two GBM cohorts. The first, TX-Fuse, is an algorithm for the discovery of candidate fusion transcripts from RNA-seq **(****Figure 8****).** The second, Exome-Fuse, detects fusion genes from whole exome DNA sequences **(****Figure 8****).** As first step for the detection of fused transcripts, RNA-seq data was generated from short-term cultures of glioma stem-like cells (GSCs) freshly isolated from nine patients carrying primary GBM. The culture of primary GBM tumors under serum-free conditions selects cells that retain phenotypes and genotypes closely mirroring primary tumor profiles as compared to serum-cultured glioma cell lines that have largely lost their developmental identities (Lee et al., 2006). Therefore, without being bound by theory, if glioma cells carry gene fusions causally responsible for the most aggressive hallmarks of GBM, they should be selected in GSCs. RNA-seq generated an average of 60.3 million paired reads for each GSC culture, of which over 80% were mapped to the reference transcriptome and genome. TX-Fuse detects two main sources of evidence: split reads and split inserts (see Experimental Procedures). The application of TX-Fuse to the RNA-seq dataset from nine GSCs led to the discovery of five candidate rearrangements (all of which were intrachromosomal) that give rise to in-frame fusion transcripts (Table 1).

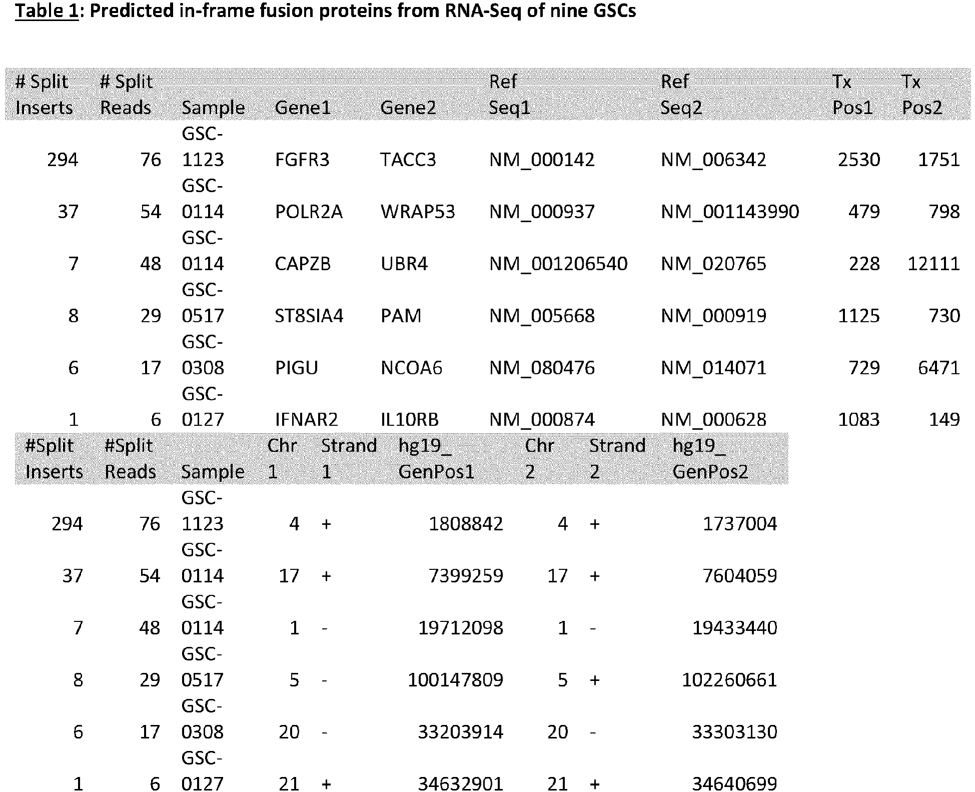

Next, genomic rearrangements leading to gene fusions were identified in GBM by applying Exome-Fuse to a dataset of paired-end exome DNA sequences from 84 GBM samples from TCGA (Table 2).

This analysis detected 147 paired gene fusions, thus producing an average of 1.75 gene fusion events per tumor (Table 3).

The FGFR and TACC families of genes were markedly enriched among those recurrently involved in genomic fusions, with eight tumors harboring FGFR rearrangements and seven tumors harboring fusions that implicate TACC genes (**Figure 1A**)**.** The comparative analysis of the TX-Fuse and Exon-Fuse outputs revealed that FGFR3-TACC3 was the only fusion pair identified as either an in-frame transcript by TX-Fuse and genomic fusions by Exome-Fuse (Tables 1, 2 and 3).

A list of recurrent gene fusions identified by Exome-fuse analysis of 84 GBM from TCGA can be found in Table 2 of WO2014/018673, the contents of which is hereby incorporated by reference in it entirety. As multiple junctions may exist in each fusion candidate, information for all breakpoints is displayed. Column definitions include: sample = TCGA sample ID, virtForSplitReads/virtRevSplitReads/virtTotSplitReads = # forward/reverse/total split reads, splitlnserts = # split inserts, dirA/dirB = forward (1) or reverse (0) direction of split read portion mapping to gene A/B, dirAB_matepair = direction of mate pair of split read, cosmicA+B = # recorded mutations of gene A+B in COSMIC.

Table 3 Recurrent gene fusion pairs from Exome-fuse analysis of 84 GBM from TCGA.

**Table 3 above shows recurrent gene fusion pairs from Exome-fuse analysis of 84 GBM from TCGA. Fusion candidates have been nominated if they have at least two split inserts and at least two split reads. To further filter the list on recurrence, any fusion candidate was kept in which one of the genes is involved in at least two fusions across different samples.**

| **Sample** | **gene A** | **gene B** | **Sample** | **gene A** | **gene B** |
|---|---|---|---|---|---|
| TCGA-12-0820 | ABCA13 | NHSL2 | TCGA-12-0820 | CAMKK1 | FAM184B |
| TCGA-12-1089 | ABCC1 | RNF216 | TCGA-12-1089 | CAMSAP1 | NCF2 |
| TCGA-12-1088 | ABCC1 | AGBL4 | TCGA-12-1088 | CAMTA1 | TMPRSS3 |
| TCGA-12-0827 | ABCC12 | FGFR4 | TCGA-06-1801 | CAMTA1 | GDPD2 |
| TCGA-12-0829 | ABCC6 | SUMF1 | TCGA-06-1801 | CAP2 | DNAJC6 |
| TCGA-06-1801 | ABCC6 | CMTM7 | TCGA-19-0957 | CCDC147 | STK4 |
| TCGA-12-1088 | ABL1 | TNFRSF10B | TCGA-12-0829 | CCDC147 | ISX |
| TCGA-06-1802 | ADAM12 | PTPRD | TCGA-06-0166 | CCDC158 | SNX5 |
| TCGA-12-0829 | ADAM12 | DAPK1 | TCGA-19-0957 | CDH11 | RERE |
| TCGA-12-1088 | ADCY10 | DUSP27 | TCGA-06-1802 | CELF2 | PLA2G2F |
| TCGA-19-0957 | ADCY10 | AKT3 | TCGA-12-0826 | CELF2 | NME4 |
| TCGA-12-0828 | ADCY2 | SDAD1 | TCGA-12-1600 | CILP | PARP16 |
| TCGA-12-0829 | ADCY2 | C14orf174 | TCGA-12-1089 | CLK3 | LRP1 |
| TCGA-12-0829 | ADCY8 | SSX3 | TCGA-12-1088 | CMYA5 | STK24 |
| TCGA-12-0829 | AGBL4 | NUP188 | TCGA-27-1835 | CMYA5 | SRRM1 |
| TCGA-06-1805 | AGBL4 | NOX4 | TCGA-12-1092 | CNTN2 | DNAJC6 |
| TCGA-12-1089 | AHNAK | C21orf29 | TCGA-06-1805 | COL14A1 | NCRNA00157 |
| TCGA-12-0829 | ANXA7 | CACNA1C | TCGA-12-0829 | COL14A1 | MMP12 |
| TCGA-06-1801 | AP4S1 | EYS | TCGA-12-1093 | CORO7 | PLEK2 |
| TCGA-12-0828 | AQP2 | ECE1 | TCGA-12-0829 | CORO7 | DYRK3 |
| TCGA-19-0957 | AQP2 | CDH4 | TCGA-06-1801 | CROCC | CSMD2 |
| TCGA-19-1790 | ARMC6 | FAM184B | TCGA-19-0957 | CSMD2 | MDH2 |
| TCGA-19-1786 | ATP5B | USP48 | TCGA-06-1805 | CUL3 | SLC44A2 |
| TCGA-12-0829 | ATP5B | PRC1 | TCGA-12-0827 | CUL3 | LY75 |
| TCGA-12-1600 | ATP6AP1L | FAM172A | TCGA-12-0829 | DDI2 | KIDINS220 |
| TCGA-12-0829 | ATP6V0D2 | RERE | TCGA-19-5958 | DEPDC5 | SLC5A4 |
| TCGA-12-0829 | ATXN1 | CACNA1G | TCGA-06-1801 | DEPDC5 | ROBO1 |
| TCGA-06-1802 | BAHD1 | OSBPL10 | TCGA-06-1801 | DEPDC7 | EIF2C2 |
| TCGA-12-0820 | BBX | CUL3 | TCGA-12-0829 | DIS3L | GLI3 |
| TCGA-19-2621 | BCAS3 | TTYH1 | TCGA-12-0829 | DMD | N4BP2L2 |
| TCGA-12-1088 | BCAS3 | CACNA1G | TCGA-06-1802 | DNM1L | SYNPO2 |
| TCGA-06-1801 | C15orf23 | DMD | TCGA-12-3644 | EDA | SSX3 |
| TCGA-12-3644 | C15orf33 | PARP16 | TCGA-12-3644 | EFHC1 | LRBA |
| TCGA-12-0829 | C21orf29 | MYT1 | TCGA-12-0829 | EFS | NRXN3 |
| TCGA-06-1805 | C2CD3 | XRRA1 | TCGA-06-1802 | EIF2C2 | TNFRSF10B |
| TCGA-12-0829 | C2CD3 | CAPZB | TCGA-19-0957 | EML1 | NRXN3 |
| TCGA-12-1089 | C6orf170 | NKAIN2 | TCGA-12-0829 | ENTPD2 | FREM2 |
| TCGA-12-0822 | C7orf44 | TACC2 | TCGA-12-0829 | EYS | IL1RN |
| TCGA-12-1089 | CACNA1C | ITGAV | TCGA-14-0781 | FAM160A1 | UNC93B1 |
| TCGA-12-0829 | CACNA1G | CNTNAP4 | TCGA-12-0829 | FAM160A1 | LY75 |

To experimentally validate the computational predictions that emerged from TX-Fuse, the PCR products spanning the fusion breakpoint were sequenced and validated each of the five in-frame fusion predictions **(****Figures 1A-D** and **9A-D****).** FGFR3-TACC3 gene fusions were identified by whole transcriptome sequencing of GSCs. SEQ ID NOs: 2-77 disclose 76 split-reads . SEQ ID NO: 1 discloses the predicted reading frame at the breakpoint (2530bp of NM_000142 fused to 1751bp of NM_006342), with the FGFR3 sequence (nucleotides 1-88) preceding the TACC3 sequence (nucleotides 89-178). SEQ ID NO: 78 discloses the putative amino acid sequence corresponding to SEQ ID NO: 1. Amino acids 1-29 of SEQ ID NO: 78 correspond to the FGFR3 sequence and amino acids 30-59 correspond to the TACC3 sequence. In **Figure 1B**, the cDNA sequence validation for the fusion with the highest read support involving FGFR3 fused in-frame with TACC3 in GSC-1123 is shown. The same FGFR3-TACC3 fusion transcript was also detected in the primary GBM-1123 tumor specimen from which the GSC-1123 culture was established (**Figure 1B**)**.** The amplified cDNA contained an open reading frame for a protein of 1,048 amino acids resulting from the fusion of a FGFR3 amino-terminal portion of residues 1-758 with a TACC3 carboxy-terminal portion of residues 549-838 (**Figure 1C**)**.** SEQ ID NO: 79 discloses the amino acid sequence of the FGFR3-TACC3 protein. Amino acids 1-758 of SEQ ID NO: 79 correspond to FGFR3 and amino acids 759-1048 of SEQ ID NO: 79 correspond to TACC3. FGFR3 is a member of the FGFR receptor tyrosine kinase (TK) family that transduces intracellular signals after binding to FGF ligands (Turner and Grose, 2010). TACC3 belongs to the evolutionarily conserved TACC gene family, which also includes TACC1 and TACC2. The distinctive feature of TACC proteins is the presence of a coiled-coil domain at the C-terminus, known as the TACC domain. Through the TACC domain, TACC proteins localize to the mitotic spindle during metaphase and stabilize the microtubule spindle network (Hood and Royle, 2011; Peset and Vernos, 2008). In the predicted fusion protein the intracellular TK domain of FGFR3 is fused upstream of the TACC domain of TACC3 (**Figure 1C**)**.**

Exon-specific gene expression analysis from the RNA-seq coverage in GSC-1123 demonstrated that the FGFR3 and TACC3 exons implicated in the fusion are highly overexpressed compared with the mRNA sequences not included in the fusion event (**Figure 10A**)**.** Quantitative RT-PCR showed that the expression of the fused FGFR3-TACC3 exons is significantly higher in GSC-1123 than other GSCs and the normal brain (80 to 130-fold, **Figure 10B**)**.** Without being bound by theory, functionally significant genetic rearrangements may result in marked overexpression (outlier) of the genes implicated in the fusion events (Tomlins et al., 2007; Tomlins et al., 2005). The FGFR3-TACC3 fusion protein was also abundantly expressed in GSC-1123 and in the primary tumor GBM-1123, as shown by Western blot and immunohistochemistry **(****Figures 10C** **and** **10D****).** On a Western Blot, the FGFR3-TACC3 fusion protein migrated at a size of ∼150 kD and immunoprecipitation followed by mass spectrometry revealed the presence of FGFR3 and TACC3 peptides consistent with the cDNA translation prediction **(****Figure 10E1-E6****).** The position of the peptides from **FIG. 10E1-E6** in the amino acid sequence of the FGFR3-TACC3 fusion protein (SEQ ID NO: 79) are 77-93 and 298-310 (FGFR3) and 773-778, 985-996, and 999-1011 (TACC3). Using PCR, the genomic breakpoint coordinates were mapped to chromosome 4 (#1,808,966 for FGFR3 and #1,737,080 for TACC3, genome build GRCh37/hg19) falling within FGFR3 exon 17 and TACC3 intron 7, which gives rise to a transcript in which the 5' FGFR3 exon 16 is spliced to the 3' TACC3 exon 8. The DNA junctions of FGFR3 and TACC3 show microhomology within a 10-base region, an observation consistent with results previously reported for other chromosomal rearrangements in human cancer (Bass et al., 2011; Stephens et al., 2009) **(****Figure 1D****).**

The experimental validation of the inferred genomic fusions was focused on FGFR3-TACC3. Exome-Fuse identified FGFR3-TACC3 gene fusions in four GBM samples with breakpoints spanning invariably within intron 16 of FGFR3 (which is downstream to the coding region for the TK domain) and intron 7-10 of TACC3 (which is upstream to the TACC domain) (Tables 4 and 5).

For sample TCGA-27-1835, SEQ ID NOS: 96-107 disclose split-reads of the genomic breakpoints of *FGFR3* within intron 16 and *TACC3* genes within intron 10. SEQ ID NO: 95 discloses the reading frame at the breakpoint (1778595 of FGFR3 fused to 1709397 of TACC3), with the FGFR3 sequence (nucleotides 1-76) preceding the TACC3 sequence (nucleotides 77-152).

For sample TCGA-19-5958, SEQ ID NOS: 109-111 disclose split-reads of the genomic breakpoints of *FGFR3* within intron 16 and *TACC3* genes within intron 7. SEQ ID NO: 108 discloses the reading frame at the breakpoint (1778539 of FGFR3 fused to 1707202 of TACC3), with the FGFR3 sequence (nucleotides 1-76) preceding the TACC3 sequence (nucleotides 77-153).

For sample TCGA-06-6390, SEQ ID NOS: 113-131 disclose split-reads of the genomic breakpoints of *FGFR3* within intron 16 and *TACC3* genes within exon 9. SEQ ID NO: 112 discloses the reading frame at the breakpoint (1778521 of FGFR3 fused to 1708787 of TACC3), with the FGFR3 sequence (nucleotides 1-76) preceding the TACC3 sequence (nucleotides 77-152).

For sample TCGA-12-0826, SEQ ID NOS: 133-145 disclose split-reads of the genomic breakpoints of *FGFR3* within intron 16 and *TACC3* genes within intron 7. SEQ ID NO: 132 discloses the reading frame at the breakpoint (1778502 of FGFR3 fused to 1707185 of TACC3), with the FGFR3 sequence (nucleotides 1-75) preceding the TACC3 sequence (nucleotides 76-151).

Among the four positive TCGA GBM specimens, two were available from TCGA centers for molecular analysis (TCGA-27-1835 and TCGA-06-6390) and, by Sanger sequencing, each of them were confirmed to carry an in-frame fusion transcript that is consistent with the predicted genomic breakpoints **(****Figures 2A** and **2B****).** Thus, the frames of the FGFR3-TACC3 fusion proteins invariably result in juxtaposing the TK domain of FGFR3 upstream of the TACC domain of TACC3. Consistent with the abundant expression of FGFR3-TACC3 in GSC-1123 and GBM-1123, the mRNA expression analysis of the TCGA tumors revealed that the four FGFR3-TACC3-positive GBM display marked co-outlier expression of FGFR3 and TACC3 **(****Figure 2C****).** Recurrent gene fusions can be associated with local copy number variations (CNV) of the breakpoint regions (Wang et al., 2009). Accordingly, the analysis of SNP arrays in the TCGA dataset revealed the presence of microamplification events of the FGFR3 and TACC3 genes in all four FGFR3-TACC3-positive GBM **(****Figure 2D****).**

**SEQ ID NOS 187-224** disclose the split inserts supporting the identification of FGFR3-TACC3 fusion genes in four GBM samples from the ATLAS-TCGA exome collection (TCGA-12-0826; TCGA-06-6390; TCGA-27-1835; TCGA-19-5958).

**SEQ ID NOS 225-318** disclose the split reads supporting the identification of FGFR3-TACC3 fusion genes in four GBM samples from the ATLAS-TCGA exome collection (TCGA-12-0826; TCGA-06-6390; TCGA-27-1835; TCGA-19-5958).

The FGFR3 and TACC3 genes are located 48-Kb apart on human chromosome 4p16. The other members of the FGFR and TACC families retain the close physical association of FGFR3 and TACC3, with FGFR1 and TACC1 paired on chromosome 8p11 and FGFR2 and TACC2 paired on chromosome 10q26. Without being bound by theory, the ancestral FGFR and TACC genes were physically linked and that this tandem gene cluster was duplicated at least twice to generate the FGFR1-TACC1, FGFR2-TACC2 and FGFR3-TACC3 pairs that mark mammalian evolution (Still et al., 1999). The highly conserved TK domains among FGFR genes and TACC domains among TACC genes together with their invariable fusion in the FGFR3-TACC3 rearrangements prompted to ask whether other intra-chromosomal FGFR-TACC fusion combinations exist in human GBM.

cDNA from a panel of 88 primary GBM were screened using pairs of upstream PCR primers that bind the amino-terminal coding region of the TK domains of FGFR1, FGFR2 and FGFR3 and downstream primers that bind to the carboxy-terminal coding region of the TACC domains of TACC1, TACC2 and TACC3 genes, respectively. The screening resulted in the identification of intrachromosomal FGFR-TACC fusions in two additional cases (one harboring FGFR1-TACC1 and one FGFR3-TACC3), corresponding to three of 97 total GBM (3.1%), including the GBM-1123 case. The FGFR1-TACC1 fusion breakpoint in GBM-51 joined in-frame exon 17 of FGFR1 to exon 7 of TACC1, resulting in a novel protein in which the TK domain of FGFR1 is fused upstream of the TACC domain of TACC1 **(****Figure 2E****).** The same structure was conserved again in GBM-22 in which exon 16 of FGFR3 is joined in-frame to exon 10 of TACC3 **(****Figure 2F****).** None of the tumors harboring FGFR-TACC fusions had mutations in IDH1 or IDH2 genes, thus indicating that FGFR-TACC-positive GBM mark an independent subgroup of patients from those carrying IDH mutations (Table 6) (Yan et al., 2009). The constant linkage of the FGFR-TK to the TACC domain created in each of the seven GBM harboring FGFR-TACC rearrangements suggests that FGFR-TACC fusion proteins may generate important functional consequences for oncogenesis in the brain.

**Table 6.**

| **Samples** | **Type** | **Time** | **Status** | **Age at initial pathologic diagnosis** | **IDH1-2 status (Sanger)** | **IDH1-2 status (exome)** |
|---|---|---|---|---|---|---|
| TCGA-12-0826 | FGFR3-TACC3 | 845 | DECEASED | 38 | WT | WT |
| TCGA-27-1835 | FGFR3-TACC3 | 648 | DECEASED | 53 | NA | WT |
| TCGA-19-5958 | FGFR3- TACC3 | 164 | LIVING | 56 | NA | WT |
| TCGA-06-6390 | FGFR3- TACC3 | 163 | DECEASED | 58 | WT | WT |
| GBM-22 | FGFR3-TACC3 | 390 | DECEASED | 60 | WT | NA |
| GBM-1123 | FGFR3-TACC3 | NA | DECEASED | 62 | WT | NA |
| GBM-51 | FGFR1-TACC1 | NA | NA | NA | WT | NA |

| | | | | | | |
|---|---|---|---|---|---|---|
| Time = Survival (days after diagnosis) Sanger = analysis done by Sanger sequencing of genomic DNA Exome = alnalysis done by the SAVI (Statistical Algorithm for Variant Identification), an algorithm developed to detect point mutation in cancer (BRAF Mutations in Hairy-Cell Leukemia, Tiacci E et al. The New England Journal of Medicine 2011 Jun 16;364(24):2305-15) NA= Not Available WT = Wild type sequence for R132 and R172 of IDH1 and IDH2, respectively | | | | | | |

***Transforming activity of FGFR-TACC fusions.*** To test the functional importance of the FGFR-TACC fusions in GBM, the FGFR3-TACC3 cDNA was cloned from GSC-1123 and recombinant lentiviruses were prepared expressing FGFR3-TACC3, FGFR1-TACC1, a kinase-dead FGFR3-TACC3 protein (FGFR3-TACC3-K508M), *wild type* FGFR3 and *wild type* TACC3. Transduction of Rat1A fibroblasts and *Ink4A;Arf-*/*-* astrocytes with the FGFR3-TACC3 lentivirus resulted in the expression of the fusion protein at levels comparable to those present in GSC-1123 (**Figure 11A-C**)**.** Having reconstituted in non-transformed cells the endogenous level of the FGFR-TACC protein that accumulates in GBM cells, it was determined whether it was sufficient to initiate oncogenic transformation *in vitro* and *in vivo.* Rat1A cells expressing FGFR3-TACC3 and FGFR1-TACC1 but not those expressing FGFR3-TACC3-K508M, FGFR3, TACC3 or the empty lentivirus acquired the ability to grow in anchorage-independent conditions in soft agar **(****Figure 3A****).** Transduction of the same lentiviruses in primary *Ink4A;Arf-*/*-* astrocytes followed by subcutaneous injection into immunodeficient mice revealed that only astrocytes expressing FGFR3-TACC3 and FGFR1-TACC1 formed tumors. The tumors emerged in 100% of the mice injected with astrocytes expressing the fusion proteins and were glioma-like lesions with strong positivity for Ki67, phospho-histone H3, nestin, GFAP and Olig2 **(****Figure 3B****).**

Next, it was determined whether the FGFR3-TACC3 fusion protein is oncogenic when transduced to a small number of cells directly into the brain of immunocompetent animals. A recently described mouse glioma model was used in which brain tumors are initiated by lentiviral transduction of oncogenes and inactivation of p53 in the mouse brain (Marumoto et al., 2009). To target adult NSCs, the adult mouse hippocampus was stereotactically transduced with purified lentivirus expressing the FGFR3-TACC3 protein and shRNA against p53 (pTomo-FGFR3-TACC3-shp53). Seven of eight mice (87.5%) transduced with FGFR3-TACC3 succumbed from malignant brain tumors within 240 days **(****Figure 3C****).** None of the mice transduced with a lentivirus expressing the most frequent gain-of-function mutation in GBM (the constitutively active EGFRvIII, pTomo-EGFRvIII-shp53) or the pTomo-shp53 control lentivirus died or developed clinical signs of brain tumors **(****Figure 3C****).** The FGFR3-TACC3 tumors were high-grade glioma with strong propensity to invade the normal brain and stained positive for the glioma stem cell markers nestin and Olig2 and the glial marker GFAP. They were also highly positive for Ki67 and phospho-histone H3, thus displaying rapid tumor growth **(****Figure 3D****).** The expression of FGFR3-TACC3 in the xenograft and intracranial tumor models was comparable to the expression of the endogenous protein in the human GSCs and tumor **(****Figure 11D, 11E and 11F****).**

These data show that FGFR-TACC fusion proteins possess transforming activity in two independent cellular models and this activity is not the result of the overexpression of individual FGFR and TACC genes. They also show that direct transduction of the FGFR3-TACC3 protein to the adult mouse brain leads to efficient development of malignant glioma.

*T**he FGFR-TACC fusions interfere with mitotic progression and induce chromosome missegregation and aneuploidy.*** To elucidate the mechanism by which the FGFR-TACC fusion drives oncogenesis, it was explored whether it activates downstream FGFR signaling. FGFR3-TACC3 failed to hyperactivate the canonical signaling events downstream of FGFR (pERK and pAKT) in the presence or absence of the ligands FGF-1, FGF-2 or FGF-8 (Wesche et al., 2011). However, FGFR3-TACC3 displayed constitutive phosphorylation of its TK domain and the adaptor protein FRS2, both of which were abolished by the specific inhibitor of FGFR-associated TK activity PD173074 (Mohammadi et al., 1998) or the K508M mutation **(****Figure 4A****).** Thus, FGFR3-TACC3 gains constitutive kinase activity that is essential for oncogenic transformation but the downstream signaling of this aberrant activity is distinct from the canonical signaling events downstream to FGFR. By driving the localization of the fusion protein, the TACC domain can create entirely novel TK-dependent functions. The TACC domain is essential for the localization of TACC proteins to the mitotic spindle (Hood and Royle, 2011; Peset and Vernos, 2008). Confocal imaging showed that FGFR3-TACC3 designed an arc-shaped structure bending over and encasing the metaphase spindle poles, frequently displaying asymmetry towards one of the two poles and relocated to the midbody as cells progressed into the late stages of mitosis (telophase and cytokinesis). Conversely, the localization of TACC3 was restricted to spindle microtubules and did not relocalize to the midbody . Wild type FGFR3 lacked discrete localization patterns in mitosis.

The mitotic localization of FGFR3-TACC3 indicates that it may impact the fidelity of mitosis and perturb the accurate delivery of the diploid chromosomal content to daughter cells, thus generating aneuploidy. Mitotic progression of individual cells was examined in vector-transduced and FGFR3-TACC3 expressing cells co-expressing histone H2B-GFP by time-lapse microscopy. The average time from nuclear envelope breakdown to anaphase onset was increased in cells expressing FGFR3-TACC3 in comparison with control cells. The mitotic delay was further exacerbated by difficulties in completing cytokinesis .

Next, it was determined whether the expressions of the FGFR-TACC fusion proteins induce defects of chromosomal segregation. Quantitative analyses of mitoses revealed that cells expressing FGFR3-TACC3 or FGFR1-TACC1 exhibit a three to five fold increase of chromosomal segregation errors than control cells. The most frequent mitotic aberrations triggered by the fusion proteins were misaligned chromosomes during metaphase, lagging chromosomes at anaphase and chromosome bridges that impaired cytokinesis and generated micronuclei in the daughter cells **(****Figure 4B****).** Aberrations at the metaphase-anaphase transition frequently lead to the inability of mitotic cells to maintain a metaphase arrest after treatment with a spindle poison. Over 18% of cells expressing FGFR3-TACC3 displayed prematurely separated sister chromatids in contrast with less than 3% in control, FGFR3 or TACC3-expressing cells. Accordingly, cells expressing the fusion protein were unable to efficiently arrest in metaphase after nocodazole treatment. The above findings indicate that expression of the FGFR3-TACC3 fusion protein may spark aneuploidy. Karyotype analysis revealed that FGFR3-TACC3 increased over 2.5 fold the percent of aneuploidy and led to the accumulation of cells with broad distribution of chromosome counts in comparison with cells transduced with empty vector, FGFR3 or TACC3 **(****Figure 5A****).** Accordingly, GSC-1123 contained aneuploid modal number of chromosomes (49) and manifested a broad distribution of chromosome counts characterized by 60% of metaphase spreads that deviate from the mode.

Next, it was determined whether aneuploidy is a direct consequence of FGFR3-TACC3 expression and is induced in human diploid neural cells. Primary human astrocytes analyzed six days after transduction with the FGFR3-TACC3 lentivirus exhibited a 5-fold increase of the rate of aneuploidy and a significantly wider distribution of chromosome counts **(****Figures 5B, 5C** **and** **5D****).** Consistent that aneuploidy is detrimental to cellular fitness, acute expression of FGFR3-TACC3 compromised the proliferation capacity of human astrocytes. However, continuous culture of FGFR3-TACC3-expressing human astrocytes led to progressive gain of proliferative capacity that overrode that of control cells **(****Figure 12A, 12B****).** Thus, the acute expression of FGFR3-TACC3 in primary normal human cells from the central nervous system causes CIN and aneuploidy with an acute fitness cost manifested by slower proliferation.

It was also determined whether the CIN and aneuploidy caused by FGFR3-TACC3 requires the TK activity of FGFR3 and can be corrected. Treatment with PD173074 rescued the aneuploidy caused by FGFR3-TACC3 by over 80%, restored the narrow distribution of chromosome counts typical of control cells and largely corrected the cohesion defect **(****Figures 6A****,** **6B and 6C****).** Together, these findings indicate that the CIN and aneuploidy caused by rearrangements of FGFR and TACC genes are reversible and suggest that specific FGFR kinase inhibition may be a valuable therapeutic strategy in tumor cells expressing FGFR-TACC fusion proteins.

***FGFR-TACC fusion proteins are new therapeutic targets in GBM.*** Driver genetic alterations trigger a state of oncogene addiction in the cancer cells harboring them that can be exploited therapeutically. To ask whether FGFR-TACC fusions confer addiction to FGFR-TK activity, cell growth was analyzed in the presence of PD173074, AZD4547 or BGJ398, the latter being two highly specific inhibitors of FGFR-TK under clinical investigation (Gavine et al., 2012; Guagnano et al., 2011). Each of the three drugs inhibited growth of cells expressing FGFR3-TACC3 and FGFR1-TACC1 at concentrations <10 nM whereas they were ineffective at concentrations as high as 1 µM in cells transduced with vector, FGFR3, TACC3 and the FGFR3-TACC3-K508M mutant **(****Figures 7A****,** **12C** and **12D****).** These findings underscore the elevated degree of specificity for FGFR kinase inhibition towards cells carrying the fusion protein. The growth of GSC-1123 cells, which naturally harbor the FGFR3-TACC3 translocation, was also abolished by nanomolar concentrations of FGFR-TK inhibitors **(****Figure 7B****).** Targeting of the fusion gene by FGFR3 shRNA inhibited the growth of cells ectopically expressing FGFR3-TACC3 and GSC-1123 proportionally to the silencing efficiency of FGFR3-TACC3 **(****Figures 7C** **and** **12E****).**

Finally, it was determined whether treatment with PD173074 of mice bearing glioma xenografts of FGFR3-TACC3 transformed astrocytes inhibits tumor growth. Twelve days after injection of tumor cells, subcutaneous tumors were present in all animals. The mice were randomized in two cohorts and treated with PD173074 or vehicle. PD173074 elicited a potent growth inhibition of FGFR3-TACC3 glioma **(****Figure 7D****).** To confirm the efficacy of a clinically meaningful FGFR-TK inhibitor using a more anatomically relevant model, the AZD4547 FGFR inhibitor, a compound under clinical investigation (Gavine et al., 2012), was used against intracranial luciferase-expressing FGFR3-TACC3-driven glioma xenografts. After an engraftment period, tumor-bearing animals were treated with either AZD4547 or vehicle. Oral administration of AZD4547 markedly prolonged survival **(****Figure 7E****).** Taken together, the data provide a strong rationale for a clinical trial based on FGFR inhibitors in GBM harboring FGFR-TACC rearrangements.

### DISCUSSION

This work has established that recurrent, oncogenic and addicting gene fusions identify a subset of GBM patients. The functional characterization of FGFR-TACC fusions indicates that the constitutively active FGFR-TK and the TACC domain of the fusion protein are both essential for oncogenesis. The TACC-dependent mis-localization to mitotic cells of the FGFR kinase results in aberrant compartmentalization of a constitutively active TK to the mitotic spindle pole, thus providing a mechanistic explanation for the impaired mitotic fidelity, chromosome mis-segregation and aneuploidy instigated by the fusion protein. Without being bound by theory, mutation of the genes that control chromosome segregation during mitosis can explain the high rate of CIN and aneuploidy, which is typical of most solid tumors including GBM (Gordon et al., 2012). A few examples of mutational inactivation of candidate genes have been reported in human cancer (Solomon et al., 2011; Thompson et al., 2010). However, gain-of-function mutations causally implicated in the control of mitotic fidelity have not been described. This clashes with the classical observation from cell fusion experiments that the underlying mechanisms that cause CIN behave as dominant traits, indicating that the CIN phenotype results from gain-of-function events rather than gene inactivation (Lengauer et al., 1997, 1998). The FGFR-TACC gene fusion is a novel mechanism for the initiation of CIN and provides a clue to the nature of dominant mutations responsible for aneuploidy in human cancer.

The rapid emergence of mitotic defects and aneuploid cell populations triggered by the fusion protein in normal human astrocytes, combined with the correction of aneuploidy after short inhibition of FGFR-TK activity indicate that aneuploidy is a key event in tumor induction by the FGFR-TACC gene fusions. Induction of aneuploidy *per se* is detrimental to cellular fitness (Sheltzer and Amon, 2011). Full-blown tumorigenesis requires cooperation between aneuploidy and genetic lesions that confer growth advantage and protect cells against the detrimental effects of aneuploidy (Coschi and Dick, 2012; Holland and Cleveland, 2009; Weaver and Cleveland, 2009). Therefore, the potent tumor-initiating activity of FGFR-TACC fusions shows that the novel oncoproteins have growth-promoting signaling functions that complement the loss of mitotic fidelity with ensuing karyotypic alterations (Sheltzer and Amon, 2011). Targeted therapies against common genetic alterations in GBM have not changed the dismal clinical outcome of the disease, most likely because they have systematically failed to eradicate the truly addicting oncoprotein activities of GBM. The highly specific anti-tumor effects and the correction of aneuploidy precipitated by FGFR-TK inhibition of FGFR-TACC-driven GBM provide a strong rationale for clinical trials based on FGFR inhibitors in patients harboring FGFR-TACC rearrangements. The computational gene fusion discovery pipeline reported here detected other GBM cases in which FGFR family genes are implicated in additional gene fusions beyond the FGFR-TACC rearrangements. Therefore, the frequency of 3.1% is likely to be an underestimate of the target GBM patient population that may benefit from FGFR-TK inhibition.

### EXPERIMENTAL PROCEDURES

***Cell culture and isolation and maintenance of GSCs.*** Rat1A, mouse astrocytes *Ink4A;Arf-*/*-,* and human astrocytes were cultured in DMEM supplemented with 10% FBS. Isolation and culture of GSCs was performed as described (Carro et al., 2010). For treatment *in vitro* with PD173074, AZD4547 or BJG398, cells infected with vector control, FGFR3, TACC3, FGFR-TACC fusions or FGFR3-TACC3-K508M were seeded in 96-well plates and treated with increasing concentrations of FGFR inhibitors. After 72-120 h, growth rate was measured using the 3-(4,5-dimethythiazol-2-yl)-2,5-diphenyl tetrazolium bromide (MTT) assay. Data were expressed as mean±SD. Proliferation rate in GSC-1123 infected with FGFR3 shRNA lentivirus was determined by plating dissociated gliomaspheres at 2x10⁴ cells/well in twelve-well plates 5 days after infection. The number of viable cells was determined by trypan blue exclusion in triplicate cultures obtained from triplicate independent infections. Cell number was scored every other day.

### DNA, RNA preparation, genomic and real-time quantitative PCR (qRT-PCR).

The validation of fusion transcripts was performed using both genomic and RT-PCR with forward and reverse primer combinations designed within the margins of the paired-end read sequences detected by RNA-seq. DNA, RNA preparation and qRT-PCR were performed as described (Carro et al., 2010; Zhao et al., 2008). To identify novel fusion transcripts within the GBM cohort, PCR primers pairs were designed to bind upstream to the TK domain of the FGFR genes and inside or downstream the Coiled Coil domain of the TACC genes. Expressed fusion transcript variants were subjected to direct sequencing to confirm sequence and frame. Primer sequences are included below.

***Subcutaneous xenografts and drug treatment.*** Rat1A or *Ink4A;Arf-*/*-* astrocytes (5x10⁵) transduced with different lentiviral constructs were suspended in 150 µl of PBS, together with 30 µl of Matrigel (BD Biosciences), and injected subcutaneously in the flank of athymic nude (Nu/Nu) mice (Charles River Laboratories, Wilmington, MA). For experiments with FGFR inhibitors, mice carrying ∼200-300 mm³ subcutaneous tumors derived from *Ink4A;Arf-*/*-* astrocytes were randomized to receive 50 mg/kg PD173074 in 0.05 M lactate buffer (pH 5) or an equal volume of lactate buffer by oral gavage. Treatment was administered for three cycles consisting of four consecutive days followed by two days of rest. Tumor diameters were measured with caliper, and tumor volumes estimated using the formula: 0.5 x length x width². Data are expressed as mean±SE. Mice were sacrificed when tumors in the control group reached the maximal size allowed.

***Orthotopic transplantation and drug treatment.** Ink4A;Arf-*/*-* astrocytes carrying a luciferase expressing vector were transduced with FGFR3-TACC3 lentivitus. 1x10³ cells in 2 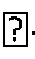 | of saline were injected in the caudate-putamen of 4-6 week old male athymic nude (Nu/Nu) mice using a stereotaxic frame (coordinates relative to bregma: 0.5 mm anterior; 1.1 mm lateral; 3.0 mm ventral) and a 26 gauge Hamilton syringe. Six days after injection, mice underwent bioluminescence imaging using a Xenogen CCD apparatus and were randomized to receive 50 mg/kg AZD4547 in 1% Tween 80 (treatment group) or DMSO in an equal volume of vehicle by oral gavage (control group). AZD4547 was administered daily for two cycles of 10 days with a two day interval. Mice were monitored daily and sacrificed when neurological symptoms appeared. Kaplan-Meier survival curve was generated using the DNA Statview software package (AbacusConcepts, Berkeley, CA). Log-rank analysis was performed on the Kaplan-Meier survival curve to determine statistical significance.

***Intracranial injections of lentiviruses.*** Intracranial injection of FGFR3-TACC3-shp53, EGFRvIII-shp53 or shp53 pTomo lentiviruses was performed in 4 week-old C57/BL/6J mice in accordance with guidelines of IACUC Committee. Briefly, 1.8 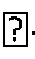 | of purified lentiviral particles in PBS (1X10⁹/ml) were injected into the dentate gyrus using a stereotaxic frame (coordinates relative to bregma: 1.45 mm posterior; 1.65 mm lateral; 2.4 mm ventral) and a 26 gauge Hamilton syringe. Mice were monitored daily and sacrificed when neurological symptoms appeared. Mouse brain was analyzed histopathologically and by immunofluorescence staining.

***Histology and immunostaining.*** Tissue preparation and immunohistochemistry on brain tumors and immunofluorescence staining were performed as previously described (Carro et al., 2010; Zhao et al., 2009; Zhao et al., 2008). Antibodies used in immunostaining and immunoblotting are listed below.

***Cloning and Lentiviral production.*** Lentivirus preparation and infections were performed as described (Carro et al., 2010) and are detailed in Extended Experimental Procedures.

***Karyotype analysis.*** Cultured cells were colcemid (20 ng/ml) treated for 90 minutes before harvesting for karyotopic analysis as detailed in Extended Experimental procedures. At least one hundred cells in metaphase were examined for chromosome count. PMSCS was scored in cells where a majority of the sister chromosomes were no longer associated. Two-tailed unpaired t-tests with Welch's correction were performed for comparison of means analysis.

***Immunofluorescence and live-cell microscopy.*** Immunofluorescence microscopy was performed on cells fixed with 4% PFA in PHEM (60 mM Pipes, 27 mM Hepes, 10 mM EGTA, 4 mM MgSO₄, pH 7.0). Cells were permeabilized using 1% Triton X-100. Mitotic spindles were visualized by anti- 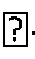 -tubulin antibody (Sigma). Secondary antibodies conjugated to Alexa Fluor-488/-594 (Molecular Probes) were used. All staining with multiple antibodies were performed in a sequential manner. DNA was stained by DAPI (Sigma). Fluorescence microscopy was performed on a Nikon A1R MP microscope.

***Identification of gene fusions from whole transcriptome (RNA-seq) and exome sequencing.*** RNA-Sequencing was performed from total RNA extracted from GSC cultures isolated from nine GBM patients using Illumina HiSeq 2000, producing roughly 60.3 million paired reads per sample. Using the global alignment software Burrows-Wheeler Aligner (BWA) (Li and Durbin, 2009) with modified Mott's trimming, an initial seed length of 32, maximum edit distance of 2 and a maximum gap number of 1, on average 43.1 million reads were mapped properly to the RefSeq transcriptome and, of the remaining, 8.6 million were mapped to the hg19 genome per sample. The remaining 14.3% of paired reads-including those that failed to map to either transcriptome or genome with proper forward-reverse (F-R) orientation, within expected insert size, and with minimal soft clipping (unmapped portions at the ends of a read) - were considered to be appropriate for gene fusion analysis.

A novel computational pipeline was constructed called ***TX-Fuse*** that identifies two sources of evidence for the presence of a gene fusion: 1. *Split inserts,* in which each read of a mate pair maps entirely to one side of a breakpoint, and 2. Individual *split reads* that span a breakpoint. Split inserts are readily detected from BWA mapping. On the other hand, split reads demand precision alignment of smaller nucleotide stretches. To that end, the pipeline employs the local alignment package BLAST with word size of 20, identity cutoff of 95%, expectation cutoff of 10⁻⁴, and soft filtering to map raw paired reads against the RefSeq transcriptome. From this procedure, a list of potential split reads were obtained that were filtered to ensure maintenance of coding frame in the predicted fusion transcript given the proper F-R orientation in the read pair. False positive candidates produced from paralogous gene pairs were also screened out using the Duplicated Genes Database and the EnsemblCompara GeneTrees (Vilella et al., 2009). Pseudogenes in the candidate list were annotated using the list from HUGO Gene Nomenclature Committee (HGNC) database (Seal et al., 2011) and given lower priority. For each remaining gene fusion candidate, a virtual reference was created based on the predicted fusion transcript and re-mapped all unmapped reads using BLAST with word size of 16, identity cutoff of 85%, query coverage greater than 85%, and expectation cutoff of 10⁻⁴ to obtain a final count of split reads and inserts. Moreover, sequencing depth per base of the virtual reference was calculated to corroborate that components of each gene participating in the gene fusion were highly expressed.

To establish the recurrence of the initial panel of gene fusion candidates, the gene fusion discovery pipeline was modified to produce ***EXome-Fuse,*** which probes for fusions within the available dataset of paired-read exome DNA sequencing of 84 matched GBM samples from TCGA. To increase sensitivity for gene fusion identification, reads unmapped by BWA were aligned to the gene pair participating in each fusion candidate using a BLAST word size of 24 for split inserts and 16 for split read and split insert discovery. Given that the breakpoint detected in DNA cannot directly indicate the resulting breakpoint in the transcribed RNA, no restriction was made on split insert orientation. For split reads, it was only required that the component of the split read mapped to the same gene as its mate maintained F-R directionality.

***Co-outlier expression and CNV analysis from TCGA GBM samples.*** Tomlins et al. (Tomlins et al., 2005) reported that outlier gene expression from microarray datasets identifies candidate oncogenic gene fusions. Wang et al. (Wang et al., 2009) suggested a "breakpoint principle" for intragenic copy number aberrations in fusion partners. The two principles (outlier expression and intragenic CNV) were combined to identify candidate gene fusions in GBM samples from Atlas-TCGA. Genomic and expression data sets were downloaded from TCGA public data portal as available on December 1, 2011, where a description of TCGA data types, platforms, and analyses is also available (2008). Specific data sources were (according to Data Levels and Data Types) as follows: Expression data, "Level 2" normalized signals per probe set (Affymetrix HT_HG-U133A) of 84 samples; Copy number data, "Level 1" raw signals per probe (Affymetrix Genome-Wide Human SNP *Array* 6.0) of the 4 *FGFR3-TACC*3 gene fusion positive samples (tumor and matched normal control).

The gene expression analysis was performed first using R³. The median absolute deviation (MAD) was calculated and then a gene was labeled as an outlier according to the following formula: Z_{i,j} = 0.6745(x_{i,j} - mean(xᵢ))/MADᵢ > 3.5 (Iglewicz and Hoaglin, 1993). Samples were identified as ECFS (expression candidate fusion sample) if both genes of interest (e. g. FGFR3 and TACC3) displayed outlier behavior (co-outliers). Next, ECFS were analyzed for CNV using pennCNV (Wang et al., 2007). Tumors samples were paired to their normal controls to obtain the log ratio values and the VEGA algorithm was used to obtain a more accurate segmentation (Morganella et al., 2010).

***Karyotypic Analysis.*** The colcemid treated cells were trypsinized, centrifuged for 7 minutes at 200 x g, and the cell pellet re-suspended in warmed hypotonic solution and incubated at 37°C for 13 minutes. The swollen cells were then centrifuged and the pellet re-suspended in 8 ml of Carnoy's fixative (3:1 methanol:glacial acetic acid). The cell suspension was centrifuged and washed twice in Carnoy's fixative. After the last centrifugation, the cells were resuspended in 0.5 to 1 ml of freshly prepared fixative to produce an opalescent cell suspension. Drops of the final cell suspension were placed on clean slides and air-dried. Slides were stained with DAPI and metaphases were analyzed under a fluorescent microscope. **Cloning and Lentiviral production.** Lentiviral expression vectors, pLOC-GFP (Open Biosystems) and pTomo-shp53, were used to clone FGFR3, TACC3, FGFR3-TACC3, FGFR3-TACC3-K508M, and FGFR1-TACC1. pTomo-shp53 was a gift of Inder Verma and Dinorah Friedman-Morvinski (Salk Institute, San Diego). The FGFR3-TACC3-K508M mutant was generated using the Phusion Site Direct Mutagenesis kit (NEB, USA). MISSION shRNAs clones (pLKO.1 lentiviral expression vectors) against FGFR3 were purchased from Sigma. The hairpin sequences targeting the *FGFR3* gene are SEQ ID NO: 182 (#TRCN0000000372; Sh#2); SEQ ID NO: 183 (#TRCN0000430673; Sh#3); and SEQ ID NO: 184 (#TRCN0000000374; Sh#4).

**Genomic and mRNA RT-PCR.** Total RNA was extracted from cells by using RNeasy Mini Kit (QIAGEN), following the manufacturer instructions. 500 ng of total RNA was retro-transcribed by using the Superscript III kit (Invitrogen), following the manufacturer instructions. The cDNAs obtained after the retro-transcription was used as templates for qPCR. The reaction was performed with a Roche480 thermal cycler, by using the Absolute Blue QPCR SYBR Green Mix from Thermo Scientific. The relative amount of specific mRNA was normalized to 18S. Results are presented as the mean ± SD of triplicate amplifications. Primers used are: hFGFR3-RT-FW1 (SEQ ID NO: 162); hFGFR3-RT-REV1 (SEQ ID NO: 163); hTACC3-RT-FW2 (SEQ ID NO: 164); hTACC3-RT-REV2 (SEQ ID NO: 165); hWRAP53-RT-FW1 (SEQ ID NO: 180); hWRAP53-RT-REV1 (SEQ ID NO: 181).

Primers used for the screening of FGFR-TACC fusions are: FGFR3-FW1 (SEQ ID NO: 166); TACC3-REV1 (SEQ ID NO: 167); FGFR1-FW1 (SEQ ID NO: 168); TACC1-REV1 (SEQ ID NO: 169).

Primers used for genomic detection of FGFR3-TACC3 fusion in GBM-1123 and GSC-1123 are: Genomic FGFR3-FW1 (SEQ ID NO: 170); genomicTACC3-REV1 (SEQ ID NO: 171).

Primers used to validate fusions detected by RNA-seq are: POLR2A-FW1 (SEQ ID NO: 172); WRAP53-REV1 (SEQ ID NO: 173); PIGU-FW1 (SEQ ID NO: 174); NCOA6-REV1 (SEQ ID NO: 175); ST8SIA4-FW1 (SEQ ID NO: 176); PAM-REV1 (SEQ ID NO: 177); CAPZB-FW (SEQ ID NO: 178);UBR4-REV1 (SEQ ID NO: 179).

**Confocal microscopy imaging.** For immunofluorescence of fixed cells, images were recorded with a Z-optical spacing of 0.25 µm using a Nikon A1R MP and a 60X1.3 oil objective and analyzed using ImageJ software (National Institute of Health). For live-cell analyses, Rat1A cells infected with pLNCX-H2B retrovirus and transduced with lentiviral vector or FGFR3-TACC3 fusion were seeded in glass bottom dishes in phenol red free DMEM and followed by time-lapse microscopy using the Nikon A1R MP biostation at 37°C and 5% CO₂/95% air. Images with a Z-optical spacing of 1 µm were recorded every 4 min for 8 h. Images of unchallenged mitosis from early prophase until cytokinesis were processed using ImageJ software (National Institute of Health). The time-point of nuclear envelope breakdown (NEB) was defined as the first frame showing loss of smooth appearance of chromatin and anaphase was the first frame when chromosome movement towards the poles became apparent. Nuclear envelope reconstitution (NER) was defined as the first frame showing nuclei decondensation.

Box and whisker plots were calculated from image sequences from at least 50 recorded cells. Two-tailed unpaired t-tests with Welch's correction were performed for comparison of means analysis using StatView software (AbacusConcepts, Berkeley, CA).

**Immunofluorescence.** Antibodies and concentrations used in immunofluorescence staining are:

| | | | |
|---|---|---|---|
| Anti-Ki67 | Rabbit | 1:1000 | Vector Labs |
| Anti-pHH3 | Rabbit | 1:500 | Millipore |
| Anti-FGFR3 | Mouse | 1:1000 | Santa Cruz |
| Anti-Tacc3 | Goat | 1:1000 | USBiological |
| Anti-a-tubulin | Mouse | 1:1000 | Sigma |
| Anti-Nestin | Mouse | 1:1000 | BD Pharmingen |
| Anti-Olig2 | Rabbit | 1:200 | IBL |
| Anti-GFAP | Rabbit | 1:200 | Dako |
| Anti-ERK | Rabbit | 1:1000 | Cell Signaling |
| Anti-pERK | Rabbit | 1:1000 | Cell Signaling |
| AntiFRS | Rabbit | 1:250 | Santa Cruz |
| Anti-pFRS | Rabbit | 1:1000 | Cell Signaling |
| Anti-AKT | Rabbit | 1:1000 | Cell Signaling |
| Anti-pAKT473 | Rabbit | 1:1000 | Cell Signaling |

### Example 2- Fusions in GBM

**Table 8: Soft agar colony assay**

| **Cell line** | **Vector** | **FGFR3** | **TACC3** | **F1-T1 Fusion** | **F3-T3 Fusion** | **F3-T3-K508M Fusion** |
|---|---|---|---|---|---|---|
| Rat1 | 0 | 0 | 0 | 225.3±10.0 | 198.7±8.0 | 0 |
| Balb 3T3 | 0 | 0 | 0 | n.d. | 45.5±8.9 | n.d. |

| | | | | | | |
|---|---|---|---|---|---|---|
| n.d.: not done | | | | | | |

**Table 9: Subcutaneous tumor xenografts**

| **Cell line** | **Vector** | **FGFR3** | **TACC3** | **F1-T1 Fusion** | **F3-T3 Fusion** | **F3-T3-K508M Fusion** |
|---|---|---|---|---|---|---|
| Rat1 | 0/5 | 0/5 | 0/5 | n.d. | 5/5 | n.d. |
| *Ink4A*/*Arf-*/*-* Astrocytes | 0/9 | 0/5 | 0/5 | 8/8 | 12/12 | 0/8 |

| | | | | | | |
|---|---|---|---|---|---|---|
| n.d.: not done | | | | | | |

**Table 10: Analysis of chromosomal number in Rat1 cells**

| **Cell line** | **Number of cells counted** | **Percent aneuploidy** | **Range** | **Mean number** | **Average variation from mean number** | ***p*-value** |
|---|---|---|---|---|---|---|
| Rat1A Vector | 100 | 27 | 35-43 | 41.2 | 1.2 | |
| Rat1A FGFR3 | 100 | 33 | 35-44 | 42.1 | 1.3 | n.s. |
| Rat1ATACC3 | 100 | 41 | 34-46 | 40.7 | 1.1 | n.s. |
| Rat1A FGFR3-TACC3 | 100 | 69 | 35-73 | 43.8 | 3.1 | <0.0001 |

**Table 11: Analysis of chromosomal number in human astrocytes**

| **Cell line** | **Number of cells counted** | **Percent aneuploidy** | **Range** | **Mean number** | **Average variation from mean number** | ***p*- value** |
|---|---|---|---|---|---|---|
| Human Astrocytes Vector | 100 | 8 | 42-46 | 45.85 | 0.28 | p = <0.001 |
| Human Astrocytes FGFR3-TACC3 | 100 | 42 | 28-48 | 42.24 | 3.33 | |

### Example 3 - Fusions in Other Cancers

The inventors reported in Example 1 that 3.1% of human glioblastoma harbor FGFR3-TACC3 and FGFR1-TACC1 gene fusions. Tumors harboring FGFR3-TACC3 gene fusions are identified by the presence of highly specific focal micro-amplification events of the rearranged portions of the FGFR3 and TACC3 genes (See **FIG. 2D**). Therefore, these micro-amplification events can be used as distinctive marks for the presence of FGFR3-TACC3 gene fusions. It was asked whether other types of human tumors also harbor FGFR3-TACC3 gene fusions from the analysis of Copy Number Variations (CNVs) of SNP arrays generated from the Atlas-TCGA project. This analysis was performed using segmented CNVs data visualized using the Integrated Genomic Viewers software. The analysis revealed that the following tumors display focal micro-amplification events of FGFR3 and TACC3 that indicate the presence of FGFR3-TACC3 gene fusions: Bladder Urothelial Carcinoma; Breast Carcinoma; Colorectal Carcinoma; Lung Squamous Cell Carcinoma; Head and Neck Squamous Cell Carcinoma.

Taken together, these data indicate that the same FGFR3-TACC3 gene fusions reported for the first time in Glioblastoma also occur in several other types of human tumors. Therefore, as for Glioblastoma and other epithelial cancers (such as the human tumors discussed herein), the identification of FGFR-TACC gene fusions also provides a new diagnostic and therapeutic target for treatment with drugs that inhibit FGFR-TACC gene fusions.

### Example 4 - fusion transcripts identified by RNA-seq of nine GSCs

POLR2A-WRAP53, CAPZB-UBR4, ST8SIA4-PAM, PIGU-NCOA6, IFNAR2-IL10RB fusions were identified by RNA-seq of nine GSCs **(****FIGS. 9A-D****).**

The POLR2A-WRAP53 gene fusion was identified by whole transcriptome sequencing of GSCs. SEQ ID NOs: 329-382 disclose 54 split-reads . SEQ ID NO: 327 discloses the predicted reading frame at the breakpoint (479bp of NM_000937 fused to 798bp of NM_001143990), with the POLR2A sequence (nucleotides 1-88) preceding the WRAP53 sequence (nucleotides 89-176). SEQ ID NO: 328 discloses the putative amino acid sequence corresponding to SEQ ID NO: 327. Amino acids 1-29 of SEQ ID NO: 328 correspond to the POLR2A sequence and amino acids 30-59 correspond to the WRAP53 sequence.

The CAPZB-UBR4 gene fusion was identified by whole transcriptome sequencing of GSCs. SEQ ID NOs: 385-432 disclose 48 split-reads . SEQ ID NO: 383 discloses the predicted reading frame at the breakpoint (227bp of NM_00120650 fused to 12110bp of NM_020/65), with the CAPZB sequence (nucleotides 1-82) preceding the UBR4 sequence (nucleotides 83-170). SEQ ID NO: 384 discloses the putative amino acid sequence corresponding to SEQ ID NO: 383. Amino acids 1-27 of SEQ ID NO: 384 correspond to the CAPZB sequence and amino acids 27-56 correspond to the UBR4 sequence.

The ST8SIA4-PAM gene fusion was identified by whole transcriptome sequencing of GSCs. SEQ ID NOs: 435-463 disclose 29 split-reads . SEQ ID NO: 433 discloses the predicted reading frame at the breakpoint (1125bp of NM_005668 fused to 730bp of NM_000919), with the ST8SIA4 sequence (nucleotides 1-87) preceding the PAM sequence (nucleotides 88-176). SEQ ID NO: 434 discloses the putative amino acid sequence corresponding to SEQ ID NO: 433. Amino acids 1-29 of SEQ ID NO: 434 correspond to the ST8SIA4 sequence and amino acids 30-59 correspond to the PAM sequence.

The PIGU-NCOA6 gene fusion was identified by whole transcriptome sequencing of GSCs. SEQ ID NOs: 466-482 disclose 17 split-reads . SEQ ID NO: 464 discloses the predicted reading frame at the breakpoint (729bp of NM_080476 fused to 6471bp of NM_014071), with the PIGU sequence (nucleotides 1-84) preceding the NCOA6 sequence (nucleotides 85-167). SEQ ID NO: 465 discloses the putative amino acid sequence corresponding to SEQ ID NO: 464. Amino acids 1-28 of SEQ ID NO: 465 correspond to the PIGU sequence and amino acids 39-56 correspond to the NCOA6 sequence.

The IFNAR2-IL10RB gene fusion was identified by whole transcriptome sequencing of GSCs. SEQ ID NOs: 495-490 disclose 6 split-reads . SEQ ID NO: 483 discloses the predicted reading frame at the breakpoint (1083bp of NM_000874 fused to 149bp of NM_000628), with the IFNAR2 sequence (nucleotides 1-78) preceding the IL10RB sequence (nucleotides 79-166). SEQ ID NO: 484 discloses the putative amino acid sequence corresponding to SEQ ID NO: 483. Amino acids 1-26 of SEQ ID NO: 484 correspond to the IFNAR2 sequence and amino acids 27-55 correspond to the IL10RB sequence.

### Example 4 - Detection, characterization and inhibition of FGFR-TACC fusions in IDH wild type glioma TRANSLATIONAL RELEVANCE

Described herein is an unbiased screening assay for FGFR-TACC fusions in glioma that overcomes the great variability of variants that are generated by FGFR-TACC chromosomal translocation in human cancer. FGFR-TACC fusions occur in grade II and III glioma harboring wildtype IDH1 with frequency similar to glioblastoma (GBM), therefore providing a clue to the aggressive clinical behavior of this glioma subtype. The molecular characterization of fusion-positive glioma revealed that FGFR-TACC is mutually exclusive with EGFR amplification but co-occurs with CDK4 amplification. FGFR-TACC-positive glioma displays strikingly uniform and strong expression of the fusion protein at the single cell level. Preclinical experiments with FGFR3-TACC3-positive glioma cells treated with the FGFR inhibitor JNJ-42756493 showed strong antitumor effects and treatment of two patients with recurrent GBM harboring FGFR3-TACC3 resulted in clinical improvement and radiological tumor reduction. These findings validate the treatment with FGFR inhibitors of glioma patients harboring FGFR-TACC chromosomal translocations.

**Purpose.** Oncogenic fusions consisting of FGFR and TACC are present in a subgroup of glioblastoma (GBM) and other human cancers and have been proposed as new therapeutic targets. Frequency, molecular features of FGFR-TACC fusions, and the therapeutic efficacy of inhibiting FGFR kinase in GBM and grade-II-III glioma were analyzed.

**Experimental Design.** Overall, 795 gliomas (584 GBM, 85 grade-II-III with wild-type and 126 with IDH1/2 mutation) were screened for FGFR-TACC breakpoints and associated molecular profile. Expression of the FGFR3 and TACC3 components of the fusions were also analyzed. The effects of the specific FGFR inhibitor JNJ-42756493 for FGFR3-TACC3-positive glioma were determined in preclinical experiments. Two patients with advanced FGFR3-TACC3-positive GBM received JNJ-42756493 and were assessed for therapeutic response.

**Results.** Three of 85 IDH1/2 wild type (3.5%) but none of 126 IDH1/2 mutant grade-II-III glioma harbored FGFR3-TACC3 fusions. FGFR-TACC rearrangements were present in 17 of 584 GBM (2.9%). FGFR3-TACC3 fusions were associated with strong and homogeneous FGFR3 immunostaining. They are mutually exclusive with IDH1/2 mutations and EGFR amplification whereas co-occur with CDK4 amplification. JNJ-42756493 inhibited growth of glioma cells harboring FGFR3-TACC3 in vitro and in vivo. The two patients with FGFR3-TACC3 rearrangements who received JNJ-42756493 manifested clinical improvement with stable disease and minor response, respectively.

**Conclusions.** RT-PCR-sequencing is a sensitive and specific method to identify FGFR-TACC-positive patients. FGFR3-TACC3 fusions are associated with uniform intra-tumor expression of the fusion protein. The clinical response observed in the FGFR3-TACC3-positive patients treated with a FGFR inhibitor supports clinical studies of FGFR inhibition in FGFR-TACC-positive patients.

### INTRODUCTION

The history of successful targeted therapy of cancer largely coincides with the inactivation of recurrent, oncogenic and addicting gene fusions in hematological malignancies and recently in some types of epithelial cancer (1, 2). Glioblastoma multiforme (GBM) is among the most lethal forms of human cancer and targeted therapies against common genetic alterations in GBM have not changed the dismal outcome of the disease (3, 4). Underlying biological features including infiltrative growth behavior, intratumoral heterogeneity, and adaptive resistance mechanisms coupled with the unique challenges of intracranial location present significant problems in its effective management. Despite surgery and chemo-radiotherapy, most patients rapidly recur and no effective treatment options are available at that stage. Beside GBM, which features the highest grade of malignancy among glioma (grade IV), lower grade glioma which include grade II and grade III are a heterogeneous group of tumors in which specific molecular features are associated with divergent clinical outcome. The majority of grade II-III glioma (but only a small subgroup of GBM) harbor mutations in IDH genes (IDH1 or IDH2), which confer a more favorable clinical outcome. Conversely, the absence of IDH mutations is associated with the worst prognosis (5).

Described herein is the identification of FGFR-TACC gene fusions (mostly FGFR3-TACC3, and rarely FGFR1-TACC1) as the first example of highly oncogenic and recurrent gene fusions in GBM. The FGFR-TACC fusions that have been identified so far include the Tyrosine Kinase (TK) domain of FGFR and the coiled-coil domain of TACC proteins, both necessary for the oncogenic function of FGFR-TACC fusions. Tumor dependency on FGFR-TACC fusions was also tested in preclinical mouse models of FGFR-TACC glioma and observed marked anti-tumor effects by FGFR inhibition (6). FGFR3-TACC3 fusions have been identified in pediatric and adult glioma, bladder carcinoma, squamous lung carcinoma and head and neck carcinoma, thus establishing FGFR-TACC fusions as one of the chromosomal translocation most frequently found across multiple types of human cancers (6-15).

From a mechanistic standpoint, the unexpected capacity of FGFR-TACC fusions to trigger aberrant chromosome segregation during mitosis, thus initiating chromosome instability (CIN) and aneuploidy, two hallmarks of cancer, is described herein. However, the full repertoire of the structural variants of FGFR-TACC fusions occurring in GBM and lower grade glioma is not completely understood. Furthermore, it remains unknown whether FGFR-TACC fusions mark distinct grades of glioma and GBM subtypes.

To date eight variants of the FGFR3-TACC3 fusion have been reported that mostly differ for the breakpoint in the TACC3 gene (6-15). Because of the close proximity of FGFR3 and TACC3 (the two genes map at a distance of 70 Kb on chromosome 4p16.3), detection of FGFR3-TACC3 rearrangements by FISH is not a feasible option with the currently available methods. Here a screening method for FGFR-TACC fusions is reported that includes a RT-PCR assay designed to identify the known and novel FGFR3-TACC3 fusion transcripts, followed by confirmation of the inframe breakpoint by Sanger sequencing. Using this assay, a dataset of 584 GBM and 211 grade II and grade III gliomas has been analyzed.

A crucial question with fundamental clinical relevance for any novel candidate target mutation is the frequency of the alteration in the cancer cell population, thus discriminating between a clonal or sub-clonal origin of the mutation. In fact, GBM is characterized by a formidable degree of subclonal heterogeneity, whereby neighboring cells display amplification and expression of different Receptor Tyrosine Kinase (RTK)-coding genes (16-19). This notion poses major therapeutic challenges for targeting any individual RTK will result, at best, in the eradication of a limited tumor sub-clone. Described herein, it was determined that brain tumors harboring FGFR-TACC fusions manifest strong and homogeneous intra-tumor expression of the FGFR3 and TACC3 component invariably included in the fusion protein, when analyzed by immunostaining. A significant clinical benefit following treatment with a specific inhibitor of FGFR-TK is reported in two GBM patients who harbored FGFR3-TACC3 rearrangement.

### MATERIALS AND METHODS

**Patients and tissue samples.** This example includes a cohort of 746 untreated patients with histologic diagnosis of glioma from 5 institutions. Forty-nine recurrent gliomas from Pitié-Salpêtrière Hospital and one recurrent glioma from the University of Calgary were also included. A summary of the patient cohort is provided in Table 12.

**Table 12 - Frequency of FGFR3-TACC3 Fusions in GBM and Grade II-III glioma. Distribution of the FGFR3-TACC3 fusions in GBM (upper panel) and lower grade glioma (lower panel) samples stratified according to the Institution of origin. The table reports number of cases analyzed, number of tumors harboring FGFR3-TACC3 fusion transcripts, and results of FGFR3 immunostaining. Lower grade glioma samples are further classified according to IDH status (IDH1 and IDH2). The respective frequency of FGFR3-TACC3 in GBM, Glioma grade II-III IDH wild type (wt), and IDH mutant (Mut) glioma is reported in parentheses. R^{§}, Recurrent GBM. ^{£}, Recurrent GBM from the University of Calgary Dataset is not included in the total count of GBM. *, 25 cases out of 85 are unknown for IDH2 status.**

| **Tumor sample source** | | **No of case (GBM)** | **No of detected fusions** | **Immunostaining FGFR3 positive /Sample analyzed** |
|---|---|---|---|---|
| **Pitié-Salpêtrière Hospital** | | 380 | 9 | 9/9 |
| **Besta Neurological Institute** | | 85 | 5 | 2/2 |
| **University of Calgary** | | 60 + 1R^{§} | 2 + 1R^{§} | **1/1 +1/1R^{§}** |
| **Montreal Neurological Institute** | | 51 | 1 | - |
| **University of British Columbia** | | 8 | 0 | |
| **Total** | | 584 (100%)^{£} | 17 (2.9%) | |

| **Tumor sample source** | **IDH Status** | **No of cases (Grade II-III)** | **No of detected fusions** | **Immunostaining FGFR3 positive/Sample analyzed** |
|---|---|---|---|---|
| **Pitié-Salpêtrière Hospital** | IDH wt | 85* (100%) | 3 (3.5%) | 3/3 |
| | IDH1/IDH2 Mut | 126 (100%) | 0 (0%) | **0** |

Tumor specimens, blood samples and clinico-pathological information were collected with informed consent and relevant ethical board approval in accordance with the tenets of the Declaration of Helsinki. For the samples from the Pitié-Salpêtrière Hospital, clinical data and follow-up are available in the neurooncology database (Onconeurotek, GH Pitié-Salpêtrière, Paris).

Two recurrent GBM patients harboring FGFR3-TACC3 were enrolled in the dose escalation part of JNJ-42756493 trial at the Gustave Roussy Institute.

**Identification of fusion transcripts and analysis of genomic breakpoints.** Total RNA was extracted from frozen tissues using Trizol (Invitrogen) according to manufacturer instructions. Two to three hundred nanograms of total RNA were retro-transcribed with the Maxima First Strand cDNA Synthesis Kit (Thermo Scientific) or SuperScript II (Invitrogen). RT-PCR was performed using AccuPrime Taq DNA Polymerase (Invitrogen). Primer pairs used for the FGFR3-TACC3 fusions screening were: FGFR3ex12-FW: 5'-CGTGAAGATGCTGAAAGACGATG-3 (SEQ ID NO: 495) and TACC3ex14-RV: 5'-AAACGCTTGAAGAGGTCGGAG-3 (SEQ ID NO: 496); amplification conditions were 94°C-3min, (94°C-30sec/61°C-30sec/68°C-1min40sec) for 35 cycles, 68°C-7min. FGFR1-TACC1 fusions were amplified with FGFR1ex16-FW: 5'-TGCCTGTGGAGGAACTTTTCA-3' (SEQ ID NO: 497) and TACC1ex13-RV: 5'-CCCAAACTCAGCAGCCTAAG-3' (SEQ ID NO: 498) primers (94°C-30sec/60°C-30sec/68°C-1min40sec for 35 cycles). PCR products were subjected to Sanger sequencing.

FGFR3-TACC3 genomic breakpoints were analyzed in 6 FGFR3-TACC3 positive samples, 5 of which from the Pitié-Salpêtrière Hospital and 1 from Montreal Neurological Institute. Three additional samples (MB-22, TCGA 27-1835 and TCGA 06-6390) available from the previous study (6) were also included in the analysis. Fifty nanograms of genomic DNA were used in the PCR reaction, performed with Accuprime Taq Polymerase (Invitrogen) and PCR products were Sanger sequenced. Primers used in genomic PCR were designed according to the breakpoint sequence in the mRNA; the list of primers used are: FGFR3ex17-FW 5'-TGGACCGTGTCCTTACCGT-3' (SEQ ID NO: 499) (PCR Samples 3048, 4373, 4867, 4451, MB-22, OPK-14, 06-6390, 27-1835 and Sequencing samples 3048, 4373, 4867, 4451, MB-22, OPK14, 06-6390, 27-1835); FGFR3ex16-FW 5'- GGTCCTTTGGGGTCCTGCT-3' (SEQ ID NO: 500) (PCR and Sequencing Sample 3808); TACC3ex6-RV 5'- CCTCTTTCAGCTCCAAGGCA-3' (SEQ ID NO: 501) (PCR and Sequencing Samples PCR 4451 and OPK-14); TACC3ex8-RV 5'-TCTACCAGGACTGTCCCTCAG-3' (SEQ ID NO: 502) (Sequencing Samples 3048 and 4373); TACC3ex9-RV 5'-GGGAGTCTCATTTGCACCGT-3' (SEQ ID NO: 503) (PCR Samples 3048,4373,4867 and Sequencing Sample 4867); TACC3ex10-RV 5'- CTGCATCCAGGTCCTTCTGG-3' (SEQ ID NO: 504) (PCR and Sequencing Samples MB-22 and 06-6390); TACC3ex11-RV 5'- CCAGTTCCAGGTTCTTCCCG-3' (SEQ ID NO: 505) (Sequencing Samples 27-1837 and 3808); TACC3ex12-RV 5'-CAACCTCTTCGAACCTGTCCA-3' (SEQ ID NO: 506) (PCR and Sequencing Samples 27-1837 and 3808). PCR conditions were 94°C-30sec/60°C-30sec/68°C-2min30sec for 40 cycles. For amplifications performed with the primer TACC3ex9-RV, the program was 94°C-30sec/56°C-30sec/68°C-2min30sec) for 40 cycles.

**Quantitation of FGFR3 and TACC3 transcripts in GBM.** The relative expression of FGFR3 and TACC3 regions included in or excluded from the fusion transcript was assessed by qRT-PCR. Primer pairs with comparable efficiency of amplification were identified and efficiency was assessed using serial dilutions of cDNA (20) prepared from OAW28 ovarian carcinoma cells that contain wild type FGFR3 and TACC3 (21). Primers used are: N-terminal region of FGFR3, FGFR3-N: Forward 5'-AAGACGATGCCACTGACAAG-3' (SEQ ID NO: 507), Reverse 5'-CCCAGCAGGTTGATGATGTTTTTG-3' (SEQ ID NO: 508); C-terminal region of TACC3, TACC3-C: Forward 5'-TCCTTCTCCGACCTCTTCAAGC-3' (SEQ ID NO: 509), Reverse 5'-TAATCCTCCACGCACTTCTTCAG-3' (SEQ ID NO: 510). To amplify transcripts in regions excluded from FGFR3-TACC3 fusion, primers were designed in the C-terminal region of FGFR3, FGFR3-C: Forward 5'-TACCTGGACCTGTCGGCG-3' (SEQ ID NO: 511), Reverse 5'-TGGGCAAACACGGAGTCG-3' (SEQ ID NO: 512) and N-terminal domain of TACC3, TACC3-N: Forward 5'-CCACAGACGCACAGGATTCTAAGTC-3' (SEQ ID NO: 513), Reverse 5'-TGAGTTTTCCAGTCCAAGGGTG-3' (SEQ ID NO: 514). All reactions were performed in triplicate and the data are reported as Fold Change ± Standard Deviation.

**Immunofluorescence and immunohistochemistry.** For immunofluorescence (IF) staining of FGFR3, 5µm FFPE sections subjected to antigen retrieval with citrate buffer for 8 min. Primary antibodies were: FGFR3-N (1:400, sc-13121, Santa Cruz Biotechnology), FGFR3-C (1:2000, sc-123, Santa Cruz Biotechnology), TACC3-N (1:600, ab134153, Abcam), and TACC3-C (1:300, NBP1-01032, Novus Biological). Secondary biotinylated antibodies were used at 1:50,000 followed by streptavidin and TSA Cy3-conjugated. Nuclei were counterstained with DAPI. For immunohistochemical analysis (IHC) of FGFR3 expression, antigen retrieval was performed for 12 min and FGFR-3 antibody (sc-13121, Santa Cruz Biotechnology) was diluted 1:500. Biotinylated anti-mouse antibody (1:30,000) and streptavidin were added before incubation with the chromogen. Nuclei were counterstaining with hematoxylin.

**Molecular characterization of tumor samples.** Mutational status of IDH1, IDH2, TERT promoter, as well as the methylation status of the MGMT promoter was analyzed in the Pitié-Salpêtrière cohort. Expression of IDH1-R132H mutant was analyzed by IHC in 500 cases as previously described (22). IDH1 and IDH2 gene mutations were identified by Sanger sequencing in 464 and 388 gliomas, respectively (5). IDH wild-type tumors are defined according to the absence of IDH1-R132H immunopositivity and/or mutations in IDH1 and IDH2 genes. TERT promoter status was determined by the same technique in 277 samples (23). Hypermethylation of the MGMT promoter was tested in 242 samples by bisulfite pyro-sequencing (24). The presence of EGFRvIII was evaluated by RT-PCR in 118 samples using EGFR-FW 5'-CTTCGGGGAGCAGCGATGCGAC-3' (SEQ ID NO: 548) and EGFR-RV 5'CTGTCCATCCAGAGG AGGAGTA-3' (SEQ ID NO: 549) primers (25).

Copy number variations analyses have been performed on 192 tissue samples using CGH arrays using BAC arrays (N=187), Agilent 4x180K (N=2), Nimblegen 3x720K (N=2), Agilent 8x60K (N=1). Results were normalized using control DNA from matched blood samples as previously described (26). Additional analyses of 193 tumor specimens were performed by SNP array, using Illumina Omni (N=110), Illumina HumCore (N=32), Illumina 370K (N=27), or Illumina 610K (N=24), as previously described (27). Array processing was outsourced to Integragen. Raw copy numbers were estimated at each of the SNP and copy-number markers. Biodiscovery property SNP-FASST2 algorithm was then used to segment copy number data. Segments were mapped to hg18 genome assembly (28). Copy number alterations (CAN) magnitudes called log-R ratio (LRR) were classified using simple thresholds: deletion (x ≤ -1), loss (-1< x ≤ -0.2), gain (0.2 ≤ x < 1) or amplification (x ≥ 1) according to default Nexus 7.5 software. For additional 56 gliomas, 10q loss was assessed on tumor and blood DNA by microsatellite analysis, while amplification of EGFR, MDM2 and CDK4, and deletion of CDKN2A gene, were determined by qPCR, as previously reported (29, 30).

The molecular profiles obtained in Pitié-Salpêtrière dataset were combined with those available in the TCGA dataportal. TCGA GBM segmented copy number variation profile was downloaded from The UCSC Cancer Genomics Browser (31). Copy Number Variations (CNVs) were measured experimentally using the Affymetrix Genome-Wide Human SNP Array 6.0 platform at the Broad TCGA genome characterization center (32). Raw copy numbers were estimated at each of the SNP and copy-number markers. Circular binary segmentation was then used to segment the copy number data (28). Segments are mapped to hg18 genome assembly at Broad.

For CNV analysis of the regions across FGFR3 and TACC3 genes, samples for which RNAseq and CNV data were available or samples for which only CNV data were available and RT-PCR-sequencing of FGFR3-TACC3 fusion had been performed were considered. Overall, 158 GBM (all with a wild type IDH1 gene) satisfied these criteria. Among them, 5 harbored an FGFR3-TACC3 fusion whereas 153 were FGFR-TACC-negative. The CNV magnitudes, called log-R ratio (LRR), were classified using the following thresholds: deletion (x < - 1), loss (-1 ≤ x ≤ -0.2), gain (0.2 ≤ x ≤ 1) or amplification (x > 1), according to the Atlas-TCGA (32). The analysis of the genomic regions encompassing EGFR, MDM2, CDK4, CDKN2A, 7p, 10q, according to hg18 genome assembly, was performed to evaluate their CNV. EGFRvIII mutation status was inferred according to Brennan et al. (32). The frequencies of the aberrations of these genes in FGFR3-TACC3 positive and negative samples were calculated and the obtained data were then combined with the Pitié-Salpêtrière Hospital dataset.

**Statistical Analysis.** Differences in the distribution on categorical variables were analyzed using Fisher Exact test. The p-values were adjusted for multiple testing according to Benjamini and Hochberg false discovery rate (FDR). A q-value ≤ 0.05 (two-sided) was considered to be statistically significant.

Overall survival (OS) was defined as the time between the diagnosis and death or last follow-up. Patients who were still alive at the last follow-up were considered as censored events in the analysis. Progression-free survival (PFS) was defined as the time between the diagnosis and recurrence or last follow-up. Patients who were recurrence-free at the last follow-up were considered as censored events in the analysis. Survival curves were calculated by the Kaplan-Meier method and differences between curves assessed using the Log-Rank test. A Log-Rank test p-value ≤ 0.05 (two-sided) was considered to be statistically significant.

**Cell culture and cell growth assay.** GIC-1123 gliomaspheres were cultured in neurobasal medium (Invitrogen) supplemented with B27, N2 (Invitrogen), EGF and FGF2 (20 ng/ml, PeproTech). Mouse astrocytes Ink4A-Arf-/- were cultured in DMEM supplemented with 10% Fetal Bovine Serum. Cells were seeded at 1,000 cells/well in a 96-well plate and treated with JNJ-42756493. After 72 hours cell viability was assessed using the 3-(4,5- dimethythiazol-2-yl)-2,5-diphenyl tetrazolium bromide (MTT) assay. Data are mean ± SEM of six replicates. Experiments were performed three times.

**Subcutaneous xenografts and drug treatment.** GIC-1123 cells (5x10⁵) were injected subcutaneously in the flank of athymic nude (Nu/Nu) mice (Charles River Laboratories). Mice carrying ∼200 mm³ subcutaneous tumors were randomized to receive 12 mg/kg JNJ-42756493 or DMSO in 1% Tween 80 by oral gavage. Tumor diameters were measured with caliper and tumor volumes estimated using the formula: 0.5 x length x width². Data are mean ± SD of nine mice in each group. Mice were sacrificed when tumors in the control group reached the maximal size allowed by the IACUC Committee at Columbia University.

**MRI imaging and evaluation of clinical response to JNJ-42756493.** Baseline and follow-up imaging assessments were performed on 1.5 Tesla MR imaging systems, including at least axial T1 weighted images before gadolinium injection, Axial or 3D FLAIR (Fluid-Attenuated Inversion-Recovery), dynamic susceptibility contrast MR perfusion (0.1 mmol/kg of gadobutrol), axial and 3D T1 weighted images after gadolinium injection. Tumor response was assessed according to the RANO criteria (33). Contrast-enhancing lesion volume was assessed with the help of a semi-automated volumetry tool (SegmentiX), based on shape-detection and thresholding, with control and manual correction of edges when necessary. Since exclusion of cystic or necrotic portions of the lesion may be affected by operator subjectivity, both were included for volumetric and axial measurements.

DSC (dynamic susceptibility contrast) perfusion datasets were processed with vendor's software suite (Neuroperfusion, Philips), including coregistration and rCBV (relative cerebral blood volume) parametric maps generation with 3 different algorithms (Gamma-variate fitting, Arterial Input Function based deconvolution and Model Free).

### RESULTS

**Detection of FGFR1-TACC1 and FGFR3-TACC3 fusions in GBM and grade II-III glioma.** To determine the frequency and molecular features of FGFR-TACC fusions in human glioma patients, a cohort of 584 GBM and 211 grade II-III glioma treated at five Neurooncology centers **(Table 12)** were screened. 108 were grade III (49 IDH wild type, 52 IDH1 mutant and 7 IDH2 mutant) and 103 were grade II (36 IDH wild type, 63 IDH1 mutant and 4 IDH2 mutant). The IDH mutational status of 333 GBM was also established and it was determined that 303 harbored wild type IDH1/2 and 30 were mutated at codon 132 of IDH1. A RT-PCR assay was designed for the detection of all known and possibly new variants of FGFR1-TACC1 and FGFR3-TACC3 fusions that retain the mRNA sequences coding for the key FGFR-TK and TACC domains required for the oncogenic activity of the fusion protein (Fig. 19 and Figs. 20A-D). Overall, 20 tumors with an FGFR3-TACC3 fusion were found, of which 17 were GBM (2.9% positives) and 3 lower grade glioma harboring wild type IDH1/2 genes (3.5% positives). The size of the FGFR3-TACC3 RT-PCR amplicons ranged from 928 bp (for FGFR3ex18-TACC3ex13) to 1706 bp (for FGFR3ex18-TACC3ex4). The FGFR1-TACC1 fusion was detected in one grade II IDH wild type glioma (Fig. 19). Conversely, an IDH1/2 mutant glioma harboring FGFR-TACC fusions (p<0.02) was not found. Sanger sequencing of the fusion amplicons revealed that each FGFR-TACC cDNA joined in-frame the sequence coding for the entire TK domain upstream of TACC-coding sequences that invariably include the coiled-coil TACC domain (Fig. 19). However, a notable variability among FGFR3-TACC3 fusion isoforms was detected, whereby 5 of the identified variants occurred only in individual cases (Fig. 19). Furthermore, 6 fusion transcripts emerged as new variants that have not been reported before in human cancer (marked in red in Fig. 19).

Next, suitable PCR primers were designed to map the genomic breakpoint coordinates for 9 FGFR3-TACC3-positive samples for which genomic DNA was available (Figs. 23 and 24). The genomic breakpoints were successfully reconstructed by Sanger sequencing and found that they differ for each of the 9 positive cases. Interestingly, even cases harboring the same FGFR3-TACC3 transcript splice variants (#4451 and #OPK-14 joining exon 17 of FGFR3 to exon 6 of TACC3; #3048 and #4373 joining exon 17 of FGFR3 to exon 8 of TACC3; #3808 and #27-1835 joining exon 17 of FGFR3 to exon 11 of TACC3) had different genomic breakpoints (Fig. 24). Taken together, the above findings indicate that the noticeable variability among FGFR3-TACC3 fusion transcripts and genomic breakpoints is efficiently resolved by the RT-PCR screening assay.

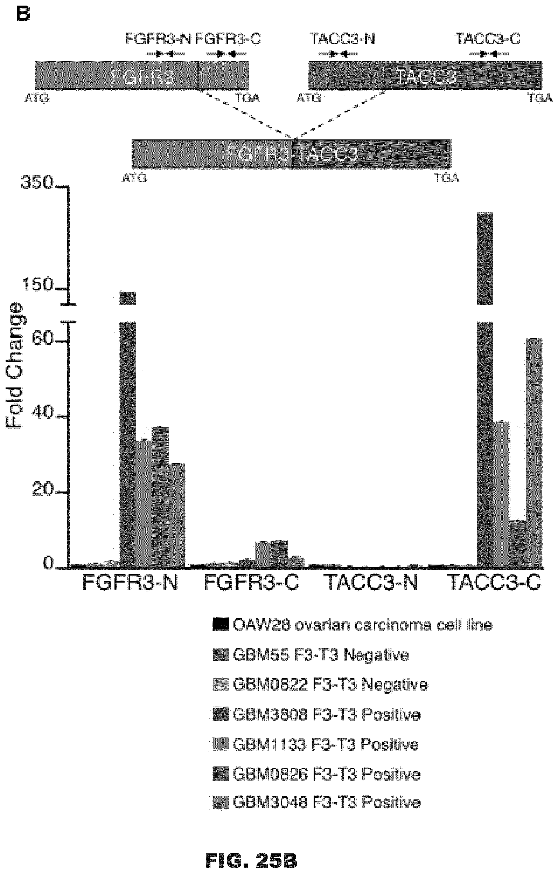

**Immunostaining analysis of FGFR3-TACC3-positive tumors.** The expression of the FGFR3 fusion protein was analyzed by IHC or IF using an antibody that recognizes the N-terminal region of FGFR3 (FGFR3-N) in 12 GBM and 3 lower grade glioma harboring FGFR3-TACC3 fusions for which sufficient tissue was available. Remarkably, each of the 15 positive tumors but none of those that had scored negative in the RT-PCR assay, displayed strong positivity for FGFR3 in the vast majority of tumor cells but not endothelial cells throughout the analyzed tumor section (Figs. 20A-H). Notably, IF using an antibody that recognizes an epitope at the C-terminus of TACC3, which is invariably retained within FGFR3-TACC3 variants (TACC3-C), reproduced the staining pattern of the FGFR3-N antibody in FGFR3-TACC3 positive tumors. Conversely, negative or very weak staining was obtained in FGFR3-TACC3-positive tumors with antibodies recognizing the regions of FGFR3 (FGFR3 C-terminal region, FGFR3-C) and TACC3 (TACC3 N-terminal region, TACC3-N) constantly excluded from FGFR3-TACC3 fusion proteins (Fig. 25A). Consistently, quantitative RT-PCR of GBM harboring FGFR3-TACC3 fusions showed that the expression of the N-terminal coding region of FGFR3 and the C-terminal coding region of TACC3 (which are included in the fusion genes) is markedly higher than the expression of the C-terminal coding region of FGFR3 and the N-terminal coding region of TACC3, which are excluded from the fusion transcripts (Fig. 25B). One recurrent GBM from a patient whose tumor had been found positive for FGFR3-TACC3 at the initial diagnosis and who had recurred after concurrent radiotherapy and temozolomide treatment was analyzed. The recurrent tumor retained the same FGFR3-TACC3 fusion gene and protein that was present in the untreated GBM as determined by RT-PCR-sequencing and FGFR3 IF, respectively (Fig. 26A-C). Although this requires additional evaluation, the retained uniform positivity for FGFR3 in this recurrent GBM suggests that targeting the FGFR3-TACC3 fusion protein at relapse is a valid therapeutic strategy.

Clinical and molecular characteristics of glioma patients with FGFR3-TACC3 fusions. Clinical and molecular profiling data were available for 591 patients including 380 GBM (9 with FGFR3-TACC3 fusions) and all 211 lower grade glioma (3 with FGFR3-TACC3 fusions). Of these 12 patients 5 are males and 7 females, aged 48y to 82y (median= 61y). The molecular profile of FGFR3-TACC3-positive glioma was determined. To do so, the analysis of CNVs and somatic mutations of key GBM genes in the dataset was combined with the SNP6.0 high-density genomic array analysis of 158 TCGA-derived GBM samples fully annotated for FGFR3-TACC3 fusion genes (the RNA-seq and/or RT-PCR analysis of these samples had revealed that 5 of them harbor FGFR3-TACC3 fusions) (6). Patients with FGFR3-TACC3 fusions displayed unique characteristics (Table 13). FGFR3-TACC3 fusions were mutually exclusive with EGFR amplification (0/16 vs. 166/411; p=0.0004, FDR q-value corrected for multiple comparisons=0.0012) and showed a clear trend against the presence of the EGFRvIII transcript variant (0/16 vs. 37/219; p=0.083). Conversely, CDK4 amplification was significantly more frequent in FGFR3-TACC3-positive tumors (7/16 vs 41/408, p=0.0008; FDR q-value= 0.0024). A less significant association of FGFR3-TACC3 fusions was also seen with amplification of MDM2, which as CDK4, maps to chromosome 12q (4/16 vs 24/408, p=0.016; FDR q-value= 0.048). No statistical association between FGFR3-TACC3 fusions and other genetic and epigenetic alterations that commonly occur in gliomas harboring wild type IDH genes was found (CDKN2A deletion, TERT promoter mutations, gain of chromosome 7p, loss of chromosome 10q and methylation of the MGMTpromoter, Table 13). When compared with the IDH wild type patient population of grade II and grade III glioma and GBM, there was no significant difference in progression free survival (PFS) or overall survival (OS) between patients positive or negative for FGFR3-TACC3 (Figs. 27A-B). Finally, it was established whether the CNV analysis of the FGFR3 and TACC3 genomic loci could be used to predict positivity for FGFR3-TACC3 fusions. The analysis of high-density SNP6.0 arrays of the 158 GBM samples from the Atlas-TCGA revealed that 10 samples displayed different degrees of copy number gains encompassing the entire FGFR3 and TACC3 loci (Fig. 28). However, none of them harbored FGFR3-TACC3 fusions. Conversely, the 5 FGFR3-TACC3-positive samples in the dataset harbor micro-amplification events involving only the exons of the FGFR3 gene that are included in the fusion breakpoint. This finding suggests that any CNV survey that is less accurate than high-density SNP arrays, could fail to identify the genomic marks associated with true FGFR3-TACC3-positive cases.

**Table 13 - Molecular alterations in IDH wild type glioma harboring FGFR3-TACC3 fusions. The table reports the absolute number and frequency (percentage) of individual glioma-specific molecular alterations in tumors scoring positive or negative for FGFR3-TACC3 fusions. The analysis is done on the Union dataset (TCGA and "Pitié-Salpêtrière Hospital" datasets, see methods for details). Statistically significant associations are indicated in bold (Fisher Exact test, q-values adjusted with FDR).**

| | **N of FGFR3-TACC3 Positive** | **% of FGFR3-TACC3 Positive** | **N of FGFR3-TACC3 Negative** | **% of FGFR3-TACC Negative** | ***P*-value (Fisher test)** | ***q*-value (FDR)** |
|---|---|---|---|---|---|---|
| **EGFR amplification** | **0/16** | **0.0 %** | **166/411** | **40.4%** | **4.E-04** | **0.0012** |
| **CDK4 amplification** | **7/16** | **43.7 %** | **41/408** | **10.0%** | **8.E-04** | **0.0024** |
| **MDM2 amplification** | **4/16** | **25.0 %** | **24/408** | **5.9 %** | **0.016** | **0.048** |
| **EGFRvIII** | **0/16** | **0.0 %** | **37/219** | **16.9 %** | **0.083** | **0.25** |
| **CDKN2A deletion** | **4/16** | **25.0 %** | **188/411** | **45.7 %** | **0.13** | **0.39** |
| **Chr. 7p gain** | **12/15** | **80.0 %** | **242/374** | **64.7 %** | **0.28** | **0.84** |
| **Chr. 10q deletion** | **12/16** | **75.0 %** | **253/420** | **60.2 %** | **0.3** | **0.9** |
| **TERT promoter mutation** | **9/11** | **81.8 %** | **128/163** | **78.5 %** | **0.8** | **1** |
| **MGMT promoter hypermethylation** | **6/12** | **50.0%** | **73/160** | **45.6 %** | **0.7** | **1** |

**Preclinical and clinical relevance of targeting FGFR3-TACC3 fusions.** JNJ-42756493 is a potent, oral pan-FGFR tyrosine kinase inhibitor with IC50 values in the low nanomolar range for all members of the FGFR family. It has demonstrated potent antitumor activities in nonclinical models with FGFR aberrations including squamous non-small cell lung cancer, gastric, breast, hepatocellular cancer (HCC), endometrial, and bladder (34, 35). To ask whether JNJ-42756493 is effective in targeting specifically FGFR-TACC-positive cells, mouse astrocytes expressing FGFR3-TACC3, FGFR3-TACC3 containing a mutation that inactivates the kinase activity of FGFR3 (FGFR3-TACC3-KD), or the empty vector were treated with JNJ-42756493. The effect of JNJ-42756493 on human glioma stem cells GIC-1123 that harbor the FGFR3-TACC3 gene fusion (6) was also studied. These experiments revealed that both mouse astrocytes and GIC-1123 that express FGFR3-TACC3 but not cells expressing the KD mutant fusion or the empty vector are highly sensitive to FGFR inhibition by JNJ-42756493 with an IC50 of 3.03 nM and 1.55 nM, respectively (Figs. 21A-B). Next, the effect of oral treatment with JNJ-42756493 of mice bearing xenografts of human GIC-1123 affects tumor growth was tested. Mice were randomized to receive vehicle or JNJ-42756493 (12 mg/kg). Mirroring the in vitro results, JNJ-42756493 elicited a potent growth inhibition of GIC-1123 tumor xenografts (Figs. 21C-D) with a statistically significant tumor regression after two weeks (p-value of the slope calculated from the treatment starting point = 0.04). The above findings provide a strong foundation for the treatment of GBM patients harboring FGFR-TACC rearrangements with JNJ-42756493.

Two patients with recurrent GBM harboring FGFR3-TACC3 fusions were treated with JNJ-42756493 in a first-in-man phase I trial. Patient 1, male aged 52, underwent partial surgical resection of a right parietal GBM, followed by fractionated radiotherapy and concomitant temozolomide (TMZ) as first line treatment (36). The RT-PCR-sequencing analysis of the GBM specimen revealed positivity for the FGFR3-TACC3 fusion (FGFR3-exon17-TACC3-exon 6, sample 4451, Figs 40 and 41) and the immunostaining using FGFR3 antibody on paraffin embedded sections showed strong positivity in a large fraction of tumor cells. After 5 cycles of TMZ, the patient presented with dizziness and headache and brain MRI revealed tumor progression (Figs. 22A). At this time the patient was enrolled in the JNJ-42756493 trial and received JNJ-42756493 (12 mg/day administered in cycles of 7 days followed by 7 days off treatment). After 3 weeks the patient reported a marked clinical improvement (complete regression of dizziness and headache). On MRI, the sum of product diameters (RANO criteria, Fig. 22B) and volumetry (Fig. 22C) measured without excluding cystic and necrotic components showed disease stabilization. However, the tumor mass underwent significant decrease of the enhancing parenchyma (-44%) with formation of a cystic portion in the central core (33). The objective response was further corroborated by the marked reduction of the extent of tumor vascularity estimated by quantitative analysis of rCBV (relative cerebral blood volume) from dynamic susceptibility MR perfusion maps (37) (Fig. 22D). Stabilization lasted for 115 days. During JNJ-42756493 treatment mild and manageable toxicity was observed (grade I hyperphosphatemia, asthenia, dysgueusia, dry mouth, keratitis, and grade II nail changes). After 4 months, tumor progressed on MRI locally both on T1 contrast-enhanced area and T2/FLAIR hypersignal. The patient was re-operated and subsequently treated with CCNU. He is still alive, but in progression after 21 months from diagnosis and 287 days from the start of the anti-FGFR therapy.

Patient 2 is a 64 years old woman, affected by left parietal GBM, diagnosed by stereotactic biopsy. The tumor was positive for FGFR3-TACC3 gene fusion by RT-PCR-sequencing and showed diffuse FGFR3 expression in most tumor cells (Figs. 20A, 20C, 20E, sample 4620). The patient received as first line treatment fractionated radiotherapy and TMZ according to the Stupp protocol (36), but after 2 cycles of monthly TMZ she presented with clinical deterioration including progressive headaches, right homonymous hemianopsia and memory impairment. Brain MRI performed 3 and 4 months after the completion of concomitant chemo-radiotherapy revealed tumor progression with increase of the left parietal mass and the appearance of a small contralateral lesion (Fig. 22E). The patient was thus enrolled in the JNJ-42756493 trial (12 mg/day administered in cycles of 7 days followed by 7 days off treatment) and showed clinical improvement after 4 weeks (regression of headaches, visual field defect and memory impairment). Best response was observed after 104 days of treatment with a 22% reduction of tumor size according to the RANO criteria (Fig. 22F) and 28% according to volumetry (Fig. 22G). Grade I hyperphosphatemia, nail changes, and mucositis were observed. Clinical status remained stable until disease progression occurring 134 days after the start of the anti-FGFR. The patient is still alive and is receiving a third-line chemotherapy with nitrosoureas and bevacizumab.

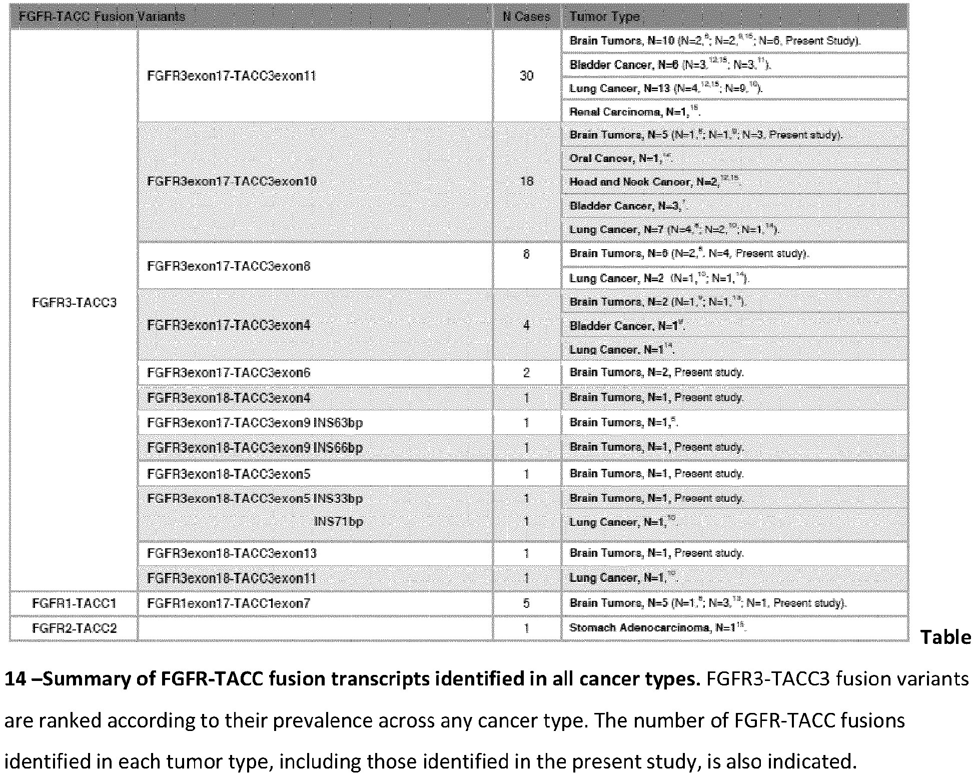

### DISCUSSION

FGFR-TACC fusions are potent oncogenic events that when present in brain tumor cells confer sensitivity to FGFR inhibitors (6). Since the original identification of recurrent FGFR-TACC fusions in GBM, small subgroups of patients harboring FGFR-TACC translocations have been identified in several other tumor types (7-15). Here, an unbiased RT-PCR-sequencing analysis for the identification of all possible functional FGFR-TACC fusion transcripts is reported. The screening of a large glioma dataset from multiple Institutions not only confirmed that FGFR-TACC rearrangements occur in ∼3% of human GBM but also revealed that FGFR-TACC fusions are present in the subgroup of IDH wild type lower grade glioma (grade II-III) with prevalence similar to that of GBM. IDH wild type grade II and III glioma have a significantly worse clinical outcome than the IDH mutant glioma and manifests molecular and clinical features that resemble GBM (5). The finding that FGFR-TACC fusions occur in IDH wild type but not IDH mutant glioma provides an important clue for the molecular characterization of this glioma subtype. Furthermore, the clustering of such potent oncogenic events in IDH wild type glioma underscores the particularly aggressive nature of this group of glioma. While it was shown that FGFR-TACC fusions cluster within the poor clinical outcome subgroup of IDH wild type glioma, these translocations do not seem to carry prognostic value within the IDH wild type subgroup of glioma patients. Without being bound by theory, the sample size of patients harboring FGFR-TACC fusions is too small to draw definitive conclusions with respect to the impact on survival and larger studies may be necessary to clarify the prognostic role of FGFR-TACC fusions in IDH wild type glioma.

Beside mutual exclusivity between IDH1 mutations and FGFR-TACC fusions, the results showed that patients with FGFR3-TACC3 rearrangements lack EGFR amplification and EGFRvIII but are significantly enriched for amplification of CDK4 (and MDM2 to a lesser extent). Knowledge of these molecular characteristics will help select those patients who most likely harbor FGFR-TACC rearrangements and design combinatorial targeted therapies that might be more effective in the FGFR-TACC-positive glioma subgroup.

The molecular screen uncovered 6 new FGFR3-TACC3 fusion events. Together with the previously identified variants, 12 distinct isoforms of FGFR3-TACC3 have been reported, thus revealing a remarkable variability of FGFR3-TACC3 transcripts in human cancer (see Table 14 summarizing the structure of all the FGFR-TACC variants identified to date). The structural heterogeneity of FGFR3-TACC3 fusions is yet more pronounced at the genomic level, whereby each fusion event harbors distinct genomic breakpoints, even for identical fusion transcripts. This finding underscores the notion that targeted genomic analyses are unlikely to be suitable approaches for the molecular diagnosis of FGFR3-TACC3 positivity. Conversely, the unbiased identification of FGFR3-TACC3-positive tumors with the RT-PCR-sequencing assay reported here overcomes the limitations of screening only for previously identified FGFR3-TACC3 fusions and provides a simple molecular diagnostic assay.

Rather than displaying uniform amplifications of the FGFR3 and TACC3 genomic loci, FGFR3-TACC3-positive samples harbor small, intragenic micro-amplification events typically encompassing only the exons of the FGFR3 and TACC3 genes included in the breakpoint (6). This finding is consistent with the notion that a "fusion breakpoint principle" sustains the CNVs of driver gene fusions such as FGFR3-TACC3 in which local CNVs target exclusively the breakpoint region (38). It is noted that such small and irregular CNVs may easily go undetected from CNV analyses performed using platforms less sensitive than the high-density SNP6.0 genomic arrays. Furthermore, the notion that FGFR3-TACC3-negative GBM may harbor uniform amplifications across the FGFR3 and TACC3 loci argues against the standard analysis of FGFR3 and/or TACC3 CNVs as a method for the selection of FGFR3-TACC3-positive tumors.

There is a growing body of evidence supporting the notion that GBM is a markedly heterogeneous tumor. The formidable degree of intra-tumor heterogeneity of GBM is a potential cause of failure of targeted therapies in these tumors. In particular, the intra-tumor heterogeneity of GBM has previously been recognized in light of the mosaic expression of the RTK genes EGFR, PDGFRA and MET by neighboring cells (16-19). Thus, in the majority of GBM, amplification or overexpression of individual RTK genes are present in a sub-clonal fraction of tumor cells and co-exist with amplification/expression of other RTK-coding genes within the tumor mass. Therefore, it was essential to determine whether such heterogeneity was also present in gliomas harboring FGFR-TACC translocations. The immunostaining of FGFR3-TACC3-positive tumors revealed that positive specimens manifest strong and uniform expression of the fusion protein, which is also retained after recurrence. This behavior is reminiscent of other driver chromosome translocations (BCR-ABL, EML4-ALK) and is compatible with the glioma-initiating functions of FGFR-TACC fusions (6). It is also the scenario expected for a driver oncogene whose activity remains essential for tumor maintenance regardless of secondary genetic alterations that occur during tumor progression. The strong antitumor effects obtained with JNJ-42756493 in glioma cells harboring FGFR3-TACC3 fusions have built a compelling rationale for the treatment of glioma patients positive for FGFR-TACC rearrangements. JNJ-42756493 is an oral ATP-competitive pan-FGFR selective inhibitor that inhibits tyrosine phosphorylation of activated FGFR at nanomolar concentrations (34, 35). The enrollment of two patients with recurrent FGFR3-TACC3-positive GBM in a phase I trial with JNJ-42756493 showed that this treatment has tolerable toxicity and clear anti-tumor activity, thus validating FGFR-TACC as a therapeutic target. Therefore, targeted inhibition of FGFR-TK in preselected IDH wild type FGFR-TACC-positive glioma may provide clinical benefits for patients with recurrent glioma who currently lack valuable therapeutic options. In conclusion, described herein is the importance and feasibility of prospective genotyping for FGFR-TACC fusions in glioma patients and provided a preliminary evidence of clinical response that warrants the investigation of the sensitivity of gliomas harboring FGFR-TACC rearrangements to FGFR kinase inhibition in clinical trials.

## Claims

1. JNJ-42756493 substance for use in treating a subject having a FGFR1-TACC1 or FGFR3-TACC3 fusion associated cancer, wherein:
a. the FGFR3-TACC3 fusion comprises a tyrosine kinase domain of FGFR3 fused to the TACC domain of transforming acidic coiled-coil-containing (TACC)-3, or
b. the FGFR1-TACC1 fusion comprises a tyrosine kinase domain of FGFR1 fused to the TACC domain of TACC1.

2. Substance according to claim 1, wherein the subject has
a. FGFR3-TACC3 fusion associated epithelial cancer,
b. FGFR3-TACC3 fusion associated glioblastoma multiforme, or
c. FGFR3-TACC3 fusion associated glioma.

3. Substance according to claim 1, wherein the subject has FGFR3-TACC3 fusion associated epithelial cancer, glioma, glioblastoma multiforme, breast cancer, lung cancer, prostate cancer, bladder carcinoma, squamous lung carcinoma, head and neck carcinoma or colorectal carcinoma.

4. Substance according to claim 3, wherein the glioma is a grade II or III glioma.

5. Substance according to claim 3 or 4,
a. wherein the subject does not have mutations in IDH1 or IDH2 genes,
b. wherein the subject does not have a gene amplification in EGFR,
c. wherein the subject does not express EGFRγIII transcript variant, or
d. wherein the subject has a gene amplification of CDK4, MDM2, or both.

6. Substance according to claim 1, wherein the subject has FGFR1-TACC1 fusion associated epithelial cancer, glioma, glioblastoma multiforme, breast cancer, lung cancer, prostate cancer, bladder carcinoma, squamous lung carcinoma, head and neck carcinoma or colorectal carcinoma.

7. Substance according to claim 1, wherein the subject has
a. FGFR1-TACC1 fusion associated epithelial cancer,
b. FGFR1-TACC1 fusion associated glioblastoma multiforme, or
c. has FGFR1-TACC1 fusion associated glioma.

8. Substance according to claim 7, wherein the glioma is a grade II or III glioma.

9. Substance according to claim 7, wherein if the subject has FGFR1-TACC1 fusion associated glioblastoma multiforme, or has FGFR1-TACC1 fusion associated glioma,
a. the subject does not have mutations in IDH1 or IDH2 genes,
b. the subject does not have a gene amplification in EGFR,
c. the subject does not express EGFRγIII transcript variant, or
d. the subject has a gene amplification of CDK4, MDM2, or both.

10. Substance according to claim 1, wherein the FGFR3-TACC3 fusion comprises a sequence selected from the group of SEQ ID NO: 85, 86, 87, 89, 158, 159, 160, 161, 542, 543, 544, 545, 546, and 547.

11. Substance according to claim 1, wherein the FGFR3-TACC3 fusion comprises a tyrosine kinase domain of FGFR3 transcript variant 1 fused to the TACC domain of TACC3.

12. Substance according to claim 1, wherein a nucleotide sequence encoding the FGFR3-TACC3 fusion comprises a sequence selected from the group of SEQ ID NO: 1 to 77, 80 to 82, 84, 519 to 527, 530 to 538, and 533 to 538

13. Substance according to claim 1, wherein the FGFR1-TACC1 fusion comprises a sequence selected from the group of SEQ ID NO: 88 and 150.

14. JNJ-42756493 substance for use in treating a subject having a FGFR1-TACC1 or FGFR3-TACC3 fusion associated cancer, wherein:
a. if the FGFR1-TACC1 or FGFR3-TACC3 fusion is detected in the subject,
i. wherein the FGFR3-TACC3 fusion comprises a tyrosine kinase domain of FGFR3 fused to the TACC domain of transforming acidic coiled-coil-containing (TACC)-3,
ii. or wherein the FGFR1-TACC1 fusion comprises a tyrosine kinase domain of FGFR1 fused to the TACC domain of TACC1,
b. an effective amount of JNJ-42756493 is given to the subject.
